# EUROPEAN PATENT SPECIFICATION

(11) **EP 3 735 412 B1**
(45) Date of publication and mention of the grant of the patent: **21.02.2024**
(21) Application number: 19705235.0
(22) Date of filing: 03.01.2019
(51) Int. Cl.: C07D 471/04, C07D 487/04, C07D 519/00, C12N 5/0789, A61K 35/12

(54) **COMPOSITIONS AND METHODS FOR THE EXPANSION OF HEMATOPOIETIC STEM AND PROGENITOR CELLS AND TREATMENT OF INHERITED METABOLIC DISORDERS**
ZUSAMMENSETZUNGEN UND VERFAHREN ZUR VERMEHRUNG VON HEMOPOIETISCHEN STAMZELLEN UND VORLÄUFERZELLEN UND DIE BEHANDLUNG VON METABOLISCHEN STÖRUNGEN.
COMPOSITIONS ET PROCÉDÉS DE MULTIPLICATION DE CELLULES SOUCHES ET PROGÉNETRICES HEMAPOIÉTIQUES ET LA TRAITEMENT DES DÉSORDRES MÉTABOLIQUES.

(30) Priority: 03.01.2018 US 201862613382 P; 03.01.2018 US 201862613383 P; 02.02.2018 US 201862625896 P; 02.02.2018 US 201862625917 P; 20.02.2018 US 201862633056 P; 23.02.2018 US 201862634638 P; 17.10.2018 US 201862747068 P; 31.10.2018 US 201862753835 P; 30.11.2018 US 201862773950 P
(43) Date of publication of application: 11.11.2020
(73) Proprietor: EdiGene Biotechnology, Inc., Beijing (CN)
(72) Inventor: BOITANO, Anthony, Newton, Massachusetts 02468 (US); GONCALVES, Kevin A., Boston, Massachusetts 02116 (US); COOKE, Michael, Brookline, Massachusetts 02445 (US)
(74) Representative: Russell, Tim
(86) International application number: PCT/US2019/012195
(87) International publication number: WO 2019/136159

(56) References cited:
- WO-A1-2013/086436
- WO-A1-2017/161001
- WO-A2-2010/059401
- US-B2- 9 580 426
- A. E. BOITANO ET AL: "Aryl Hydrocarbon Receptor Antagonists Promote the Expansion of Human Hematopoietic Stem Cells", SCIENCE, vol. 329, no. 5997, 10 September 2010 (2010-09-10), pages 1345-1348, XP055056853, ISSN: 0036-8075, DOI: 10.1126/science.1191536
- E. ZONARI ET. AL.: "Efficient Ex Vivo Engineering and Expansion of Highly Purified Human Hematopoietic Stem and Progenitor Cell Populations for Gene Therapy", STEM CELL REPORTS, vol. 8, 11 April 2017 (2017-04-11), pages 977-990, XP002791722,
- M. D. HOBAN ET. AL.: "Aryl Hydrocarbon Receptor Antagonists Expand Adult Hematopoietic Stem Cells from Mobilized Peripheral Blood and Bone Marrow and Increase the Dose of CRISPR/Cas9 Gene Edited NSG-Repopulating Cells", BLOOD, vol. 130, 7 December 2017 (2017-12-07), pages 3341-3343, XP055544684,

## Description

### Field

The present disclosure relates to compositions and methods useful for expansion, for instance, by treatment ex vivo with an aryl hydrocarbon receptor antagonist, of hematopoietic stem and progenitor cells, such as those that result in microglia engraftment in the brain, as well as methods of treating various related pathologies, such as inherited metabolic disorders.

### Background

Despite advances in the medicinal arts, there remains a demand for treating pathologies of the hematopoietic system, such as diseases of a particular blood cell, metabolic disorders, cancers, and autoimmune conditions, among others. While hematopoietic stem cells have significant therapeutic potential, a limitation that has hindered their use in the clinic has been the difficulty associated with expanding populations of hematopoietic stem cells to achieve quantities sufficient for transplantation while preserving hematopoietic stem cell functional potential. There is currently a need for compositions and methods for effectuating the expansion of hematopoietic stem and progenitor cells.

WO 2017/161001 A1 discloses hematopoietic stem cell compositions that have been expanded using agents, such as SR1, and their use in treating diseases, including Hurler syndrome and adrenoleukodystrophy (ALD), by administration to a subject.

### Summary

Provided herein is a composition for use in treating an inherited metabolic disorder in a subject in need thereof, said composition comprising an expanded population of hematopoietic stem cells.The expanded hematopoietic stem cells result in microglia engraftment in the brain of the subject.

In one embodiment, the expanded population of hematopoietic stem cells are produced *ex vivo.*

In one embodiment, producing the expanded population of hematopoietic stem cells comprises contacting a population of hematopoietic stem cells with an aryl hydrocarbon receptor antagonist in an amount sufficient to produce the expanded population of hematopoietic stem cells.

In one embodiment, the population of hematopoietic stem cells comprises CD34+ cells.

In one embodiment, the population of hematopoietic stem cells comprises a population enriched for CD90+ cells.

In one embodiment, the enrichment for CD90+ cells comprises flow cytometry.

In one embodiment, the expanded population of hematopoietic stem cells that contribute to microglia engraftment in the brain of the subject comprise CD90+ cells.

In one embodiment, the expanded population of hematopoietic stem cells that contribute to microglia engraftment in the brain comprises a population enriched for CD90+ cells.

In one embodiment, the enrichment for CD90+ cells comprises flow cytometry.

In one embodiment, the hematopoietic stem cells are human cells.

In one embodiment, the human cells are derived from umbilical cord blood cells.

In one embodiment, the number of expanded hematopoietic stem cells administered is 10³ cells/kg, 10⁴ cells/kg, 10⁵ cells/kg, 10⁶ cells/kg, 10⁷ cells/kg, 10⁸ cells/kg, or any number in between, inclusive of the end points.

In one embodiment, the number of expanded hematopoietic stem cells administered is equal to or greater than the amount of hematopoietic stem cells needed to achieve a therapeutic benefit.

In one embodiment, the therapeutic benefit achieved is proportional to the number of expanded hematopoietic stem cells that are administered.

In one embodimentthe expanded hematopoietic stem cells are administered intravenously.

In one embodiment, the intravenous administration comprises an injection or an infusion.

In one embodiment, the expanded population of hematopoietic stem cells are administered every day, every other day, every three days, every week, every 10 days, every two weeks, every month, every two months, every three months, every four months, every six months or every year.

In one embodiment, the inherited metabolic disorder has a neurological component.

In one embodiment, the inherited metabolic disorder is Hurler syndrome (Hurler's Disease), a mucopolysaccharide disorder (Maroteaux Lamy syndrome), a lysosomal storage disorder, a peroxisomal disorder (X-linked adrenoleukodystrophy), a glycogen storage disease, a mucopolysaccharidose disorder, Gaucher's Disease, a sphingolipidose disorder, Mucolipidosis II, or metachromatic leukodystrophy.

In one embodiment, the treatment of the subject may comprise engraftment of expanded hematopoietic stem cells in the patient, wherein the implanted cells secrete an enzyme in which the patient is deficient, and wherein said deficient enzyme is then taken up by cells in the patient which are deficient in that enzyme.

In one embodiment, the treatment of the subject further comprises busulfan conditioning, wherein the busulfan conditioning occurs prior to the administration of the expanded population of hematopoietic stem cells.

In some embodiments, the busulfan conditioning comprises administering busulfan at an amount of less than about 40 mg/kg, less than about 35 mg/kg, less than about 30 mg/kg, less than about 25 mg/kg, less than about 20 mg/kg, less than about 15 mg/kg, less than about 10 mg/kg, less than about 5 mg/kg, less than about 4 mg/kg, less than about 3 mg/kg, less than about 2 mg/kg, less than about 1 mg/kg, less than about 0.5 mg/kg, or less than about 0.1 mg/kg prior to administration of the expanded population of hematopoietic stem cells.

A method of producing an expanded population of hematopoietic stem or progenitor cells ex vivo may comprise contacting the population of hematopoietic stem or progenitor cells with an expanding amount of an aryl hydrocarbon receptor antagonist (i.e., an amount of an aryl hydrocarbon receptor antagonist sufficient to increase the quantity of hematopoietic stem or progenitor cells in the population by, for example, 1.1-fold to about 1,000-fold, about 1 .1-fold to about 5,000-fold or more (e.g., about 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.1-fold, 3.2-fold, 3.3-fold, 3.4-fold, 3.5-fold, 3.6-fold, 3.7-fold, 3.8-fold, 3.9-fold, 4-fold, 4.1-fold, 4.2-fold, 4.3-fold, 4.4-fold, 4.5-fold, 4.6-fold, 4.7-fold, 4.8-fold, 4.9-fold, 5-fold, 5.1-fold, 5.2-fold, 5.3-fold, 5.4-fold, 5.5-fold, 5.6-fold, 5.7-fold, 5.8-fold, 5.9-fold, 6-fold, 6.1-fold, 6.2-fold, 6.3-fold, 6.4-fold, 6.5-fold, 6.6-fold, 6.7-fold, 6.8-fold, 6.9-fold, 7-fold, 7.1-fold, 7.2-fold, 7.3-fold, 7.4-fold, 7.5-fold, 7.6-fold, 7.7-fold, 7.8-fold, 7.9-fold, 8-fold, 8.1-fold, 8.2-fold, 8.3-fold, 8.4-fold, 8.5-fold, 8.6-fold, 8.7-fold, 8.8-fold, 8.9-fold, 9-fold, 9.1-fold, 9.2-fold, 9.3-fold, 9.4-fold, 9.5-fold, 9.6-fold, 9.7-fold, 9.8-fold, 9.9-fold, 10-fold, 50-fold, 100-fold, 200-fold, 300-fold, 400-fold, 500-fold, 600-fold, 700-fold, 800-fold, 900-fold, 1,000-fold, or more), while maintaining hematopoietic stem cell functional potential).

Prior to expansion, the hematopoietic stem or progenitor cells may be mobilized and isolated from a donor, such as a human. The mobilization may be conducted, e.g., by treating the donor with a mobilizing amount of a CXCR4 antagonist, such as plerixafor, and/or a CXCR2 agonist, such as Gro-β, Gro-β T, or a variant thereof. In some embodiments, the Gro-β, Gro-β T, or variant thereof has a purity that is at least 95% (e.g., from about 95% to about 99.99%, about 96%, to about 99.99%, about 97% to about 99.99%, about 98% to about 99.99%, about 99% to about 99.99%, about 95% to about 99.9%, about 97% to about 99.9%, about 99% to about 99.9%, such as 95%, 96%, 97%, 98%, 99%, 99.9%, 99.99%, or more) relative to deamidated versions of these peptides.

In some embodiments, the expanded hematopoietic stem cells result in microglia engraftment in the brain of the subject in less than about 10 weeks after administering, less than about 8 weeks after administering, less than about 6 weeks after administering, less than about 4 weeks after administering, less than about 3 weeks after administering, less than about 2 weeks after administering, or less than about 1 week after administering.

In some embodiments, the expanded hematopoietic stem cells result in microglia engraftment in the brain of the subject that is maintained for greater than 1 week after administering, greater than 2 weeks after administering, greater than 4 weeks after administering, greater than 8 weeks after administering, greater than 16 weeks after administering, greater than 32 weeks after administering, or greater than 40 weeks after administering.

In some embodiments, the expanded hematopoietic stem cell results in microglia engraftment in the brain of the subject in a period of time that is decreased as the number of expanded hematopoietic stem cells that are administered is increased.

In some embodiments, the expanded hematopoietic stem cells result in microglia engraftment in the brain of the subject in a period of time that is shorter than a period of time for a substantially similar population of hematopoietic stem cells that is not expanded in the presence of an aryl hydrocarbon receptor antagonist.

In some embodiments, the expanded hematopoietic stem cell results in microglia engraftment in the brain of the subject that is maintained for a period of time that is increased as the number of expanded hematopoietic stem cells that are administered is increased.

In some embodiments, the expanded hematopoietic stem cells result in microglia engraftment in the brain of the subject that is maintained for a period of time that is longer than a period of time for a substantially similar population of hematopoietic stem cells that is not expanded in the presence of an aryl hydrocarbon receptor antagonist.

A method of treating a stem cell disorder in a patient (e.g., a human patient) may be by producing an expanded population of hematopoietic stem or progenitor cells in accordance with the any of the above and infusing the resulting cells into the patient.

A method of treating a stem cell disorder in a patient (e.g., a human patient) may be by infusing into the patient an expanded population of hematopoietic stem or progenitor cells produced according to any of the above.

A method of treating a stem cell disorder in a patient (e.g., a human patient) may be by contacting a population of hematopoietic stem or progenitor cells with an expanding amount of an aryl hydrocarbon receptor antagonist and infusing the resulting cells into the patient.

A method of treating a stem cell disorder in a patient (e.g., a human patient) may be by infusing into the patient an expanded population of hematopoietic stem or progenitor cells produced by contacting a population of hematopoietic stem or progenitor cells with an expanding amount of an aryl hydrocarbon receptor antagonist.

A method of treating a disorder in a patient (e.g., a human patient) in need thereof, may comprise administering an expanded population of hematopoietic stem cells to the patient, wherein the expanded population of hematopoietic stem cells is prepared by contacting a first population of hematopoietic stem cells with an aryl hydrocarbon receptor antagonist for a time sufficient to produce the expanded population of hematopoietic stem cells.

The stem cell disorder may be a hemoglobinopathy disorder. The hemoglobinopathy disorder may be, for example, sickle cell anemia, thalassemia, Fanconi anemia, aplastic anemia, or Wiskott-Aldrich syndrome.

The stem cell disorder may be a myelodysplastic disorder. The stem cell disorder may be an immunodeficiency disorder, such as a congenital immunodeficiency or an acquired immunodeficiency, such as human immunodeficiency virus or acquired immune deficiency syndrome.

The stem cell disorder may be a metabolic disorder, such as glycogen storage diseases, mucopolysaccharidoses, Gaucher's Disease, Hurler syndrome or Hurler's Disease, sphingolipidoses, Mucolipidosis II, or metachromatic leukodystrophy.

The stem cell disorder may be cancer, such as leukemia, lymphoma, multiple myeloma, or neuroblastoma. The cancer may be, for instance, a hematological cancer. The cancer may be myeloid leukemia, acute lymphoid leukemia, chronic myeloid leukemia, chronic lymphoid leukemia, multiple myeloma, diffuse large B-cell lymphoma, or non-Hodgkin's lymphoma.

The stem cell disorder may be adenosine deaminase deficiency and severe combined immunodeficiency, hyper immunoglobulin M syndrome, Chediak-Higashi disease, hereditary lymphohistiocytosis, osteopetrosis, osteogenesis imperfecta, storage diseases, thalassemia major, systemic sclerosis, systemic lupus erythematosus, multiple sclerosis, or juvenile rheumatoid arthritis.

The stem cell disorder may be an autoimmune disorder, such as multiple sclerosis, human systemic lupus, rheumatoid arthritis, inflammatory bowel disease, treating psoriasis, Type 1 diabetes mellitus, acute disseminated encephalomyelitis, Addison's disease, alopecia universalis, ankylosing spondylitisis, antiphospholipid antibody syndrome, aplastic anemia, autoimmune hemolytic anemia, autoimmune hepatitis, autoimmune inner ear disease, autoimmune lymphoproliferative syndrome, autoimmune oophoritis, Balo disease, Behcet's disease, bullous pemphigoid, cardiomyopathy, Chagas' disease, chronic fatigue immune dysfunction syndrome, chronic inflammatory demyelinating polyneuropathy, Crohn's disease, cicatrical pemphigoid, coeliac sprue-dermatitis herpetiformis, cold agglutinin disease, CREST syndrome, Degos disease, discoid lupus, dysautonomia, endometriosis, essential mixed cryoglobulinemia, fibromyalgia-fibromyositis, Goodpasture's syndrome, Grave's disease, Guillain-Barre syndrome, Hashimoto' s thyroiditis, Hidradenitis suppurativa, idiopathic and/or acute thrombocytopenic purpura, idiopathic pulmonary fibrosis, IgA neuropathy, interstitial cystitis, juvenile arthritis, Kawasaki's disease, lichen planus, Lyme disease, Meniere disease, mixed connective tissue disease, myasthenia gravis, neuromyotonia, opsoclonus myoclonus syndrome, optic neuritis, Ord's thyroiditis, pemphigus vulgaris, pernicious anemia, polychondritis, polymyositis and dermatomyositis, primary biliary cirrhosis, polyarteritis nodosa, polyglandular syndromes, polymyalgia rheumatica, primary agammaglobulinemia, Raynaud phenomenon, Reiter's syndrome, rheumatic fever, sarcoidosis, scleroderma, Sjögren's syndrome, stiff person syndrome, Takayasu's arteritis, temporal arteritis, ulcerative colitis, uveitis, vasculitis, vitiligo, vulvodynia, and Wegener's granulomatosis.

The stem cell disorder may be a neurological disorder, such as Parkinson's disease, Alzheimer's disease, multiple sclerosis, Amyotrophic lateral sclerosis, Huntington's disease, mild cognitive impairment, amyloidosis, AIDS-related dementia, encephalitis, stroke, head trauma, epilepsy, mood disorders, or dementia.

In some embodiments, the hematopoietic stem cells are autologous with respect to the patient. For instance, autologous hematopoietic stem cells can be removed from a donor and the cells can subsequently be administered to (e.g., infused into) the patient so as to repopulate one or more cell types of the hematopoietic lineage.

In some embodiments, the hematopoietic stem cells are allogeneic with respect to the patient. For instance, allogeneic hematopoietic stem cells can be removed from a donor, such as donor that is HLA-matched with respect to the patient, for instance, a closely related family member of the patient. In some embodiments, the allogenic hematopoietic stem cells are HLA-mismatched with respect to the patient. Following withdrawal of the allogeneic hematopoietic stem cells from a donor, the cells can subsequently be administered to (e.g., infused into) the patient so as to repopulate one or more cell types of the hematopoietic lineage.

The hematopoietic stem or progenitor cells, or progeny thereof, may maintain hematopoietic stem cell functional potential after two or more days following infusion of the hematopoietic stem or progenitor cells into the patient. The hematopoietic stem or progenitor cells, or progeny thereof, may localize to hematopoietic tissue and/or reestablish hematopoiesis following infusion of the hematopoietic stem or progenitor cells into the patient. For instance, upon infusion into the patient, the hematopoietic stem or progenitor cells may give rise to recovery of a population of cells selected from the group consisting of megakaryocytes, thrombocytes, platelets, erythrocytes, mast cells, myeoblasts, basophils, neutrophils, eosinophils, microglia, granulocytes, monocytes, osteoclasts, antigen-presenting cells, macrophages, dendritic cells, natural killer cells, T-lymphocytes, and B-lymphocytes.

A method of producing microglia in the central nervous system of a human patient in need thereof, may include administering an expanded population of hematopoietic stem cells to the patient, wherein the expanded population of hematopoietic stem cells is prepared by contacting a first population of hematopoietic stem cells with an aryl hydrocarbon receptor antagonist for a time sufficient to produce the expanded population of hematopoietic stem cells, and wherein administration of the expanded population of hematopoietic stem cells results in formation of microglia in the central nervous system of the patient.

A kit may contain a plurality of hematopoietic stem or progenitor cells and a package insert that instructs a user according to any of the above.

In some embodiments, the aryl hydrocarbon receptor antagonist is a compound represented by formula **(IV)** 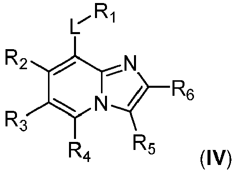
wherein L is selected from the group consisting of -NR₇ₐ(CR₈ₐR_{8b})ₙ-, -O(CR₈ₐR_{8b})ₙ-,-C(O)(CR₈ₐR_{8b})ₙ-, -C(S)(CR₈ₐR_{8b})ₙ-, -S(O)₀₋₂₍CR₈ₐR_{8b})ₙ-, -(CR₈aR_{8b})ₙ-, -NR₇ₐC(O)(CR₈ₐR_{8b})ₙ-,-NR₇ₐC(S)(CR₈ₐR_{8b})ₙ-, -OC(O)(CR₈ₐR_{8b})ₙ-, -OC(S)(CR₈ₐR_{8b})n-, -C(O)NR₇ₐ(CR₈ₐR_{8b})ₙ-,-C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-,-NR₇ₐS(O)₂(CR₈ₐR_{8b})ₙ-, -NR₇ₐC(O)NR_{7b}(CR₈ₐR_{8b})ₙ-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, -NR₉ₐC(S)R_{9b},-NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b},-C(S)NR₉ₐR_{9b}, -NR₉ₐS(O)₂R_{9b}, -NR₉ₐC(O)OR_{9b}, -OC(O)CR₉ₐR_{9b}R_{9c}, -OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
R₂ is selected from the group consisting of hydrogen and optionally substituted C1-4 alkyl;
R₃ is selected from the group consisting of optionally substituted aryl, optionally substituted heteroaryl, optionally substituted cycloalkyl, and optionally substituted heterocycloalkyl;
R₄ is selected from the group consisting of hydrogen and optionally substituted C1-4 alkyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, wherein the aryl hydrocarbon receptor antagonist is a compound represented by formula (V) 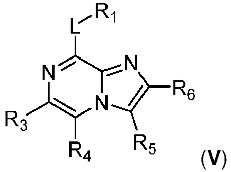
wherein L is selected from the group consisting of -NR₇ₐ(CR₈ₐR_{8b})ₙ-, -O(CR₈ₐR_{8b})ₙ-,-C(O)(CR₈ₐR_{8b})ₙ-, -C(S)(CR₈ₐR_{8b})ₙ-, -S(O)₀₋₂₍CR₈ₐR_{8b})ₙ-, -(CR₈ₐR_{8b})ₙ-, -NR₇ₐC(O)(CR₈ₐR_{8b})ₙ-,-NR₇ₐC(S)(CR₈ₐR_{8b})ₙ-, -OC(O)(CR₈ₐR_{8b})ₙ-, -OC(S)(CR₈ₐR_{8b})n-, -C(O)NR₇ₐ(CR₈ₐR_{8b})ₙ-,-C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-,-NR₇ₐS(O)₂(CR₈ₐR_{8b})ₙ-, -NR₇ₐC(O)NR_{7b}(CR₈ₐR_{8b})ₙ-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, -NR₉ₐC(S)R_{9b},-NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b},-C(S)NR₉ₐR_{9b}, -NR₉ₐS(O)₂R_{9b}, -NR₉ₐC(O)OR_{9b}, -OC(O)CR₉ₐR_{9b}R_{9c}, -OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
R₃ is selected from the group consisting of optionally substituted aryl, optionally substituted heteroaryl, optionally substituted cycloalkyl, and optionally substituted heterocycloalkyl;
R₄ is selected from the group consisting of hydrogen and optionally substituted C1-4 alkyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

The disclosure features a composition for use in treating a disorder in a patient, said composition comprising hematopoietic stem or progenitor cells, or progeny thereof, prepared according to any of the above.

The disclosure features use of a composition comprising hematopoietic stem or progenitor cells, or progeny thereof, prepared according to any one of the above in preparing a medicament for treating a disorder in a patient.

In the embodiments of the invention, the disorder is an inherited metabolic disorder.

In the embodiments of the invention, the hematopoietic stem or progenitor cells, or progeny thereof, comprise an expanded population of hematopoietic stem cells.

In the embodiments of the invention, the expanded hematopoietic stem cells result in microglia engraftment in the brain of the patient.

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar or equivalent to those described herein can be used in the practice or testing of the present disclosure, suitable methods and materials are described below. The references cited herein are not admitted to be prior art to the claimed invention. In the case of conflict, the present specification, including definitions, will control. In addition, the materials, methods and examples are illustrative only and are not intended to be limiting. In the case of conflict between the chemical structures and names of the compounds disclosed herein, the chemical structures will control.

Other features and advantages of the disclosure will be apparent from the following detailed description and claims.

### Brief Description of the Figures

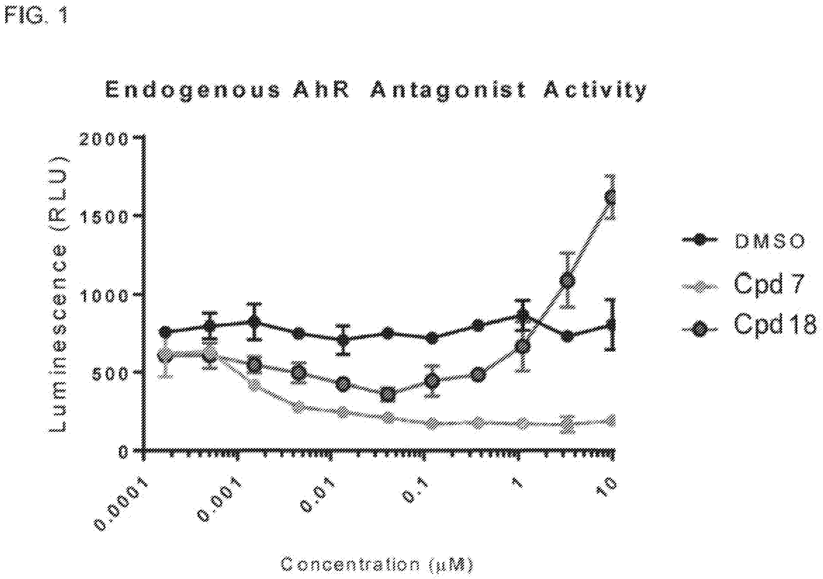

**FIG. 1** is a graph demonstrating the effect of compound **(7)** and compound **(18)** on the aryl hydrocarbon receptor-driven expression of luciferase in the absence of the aryl hydrocarbon receptor agonist VAF347 in transiently transfected HepG2 cells in vitro. Experimental details for this experiment are reported in Example 3, below.

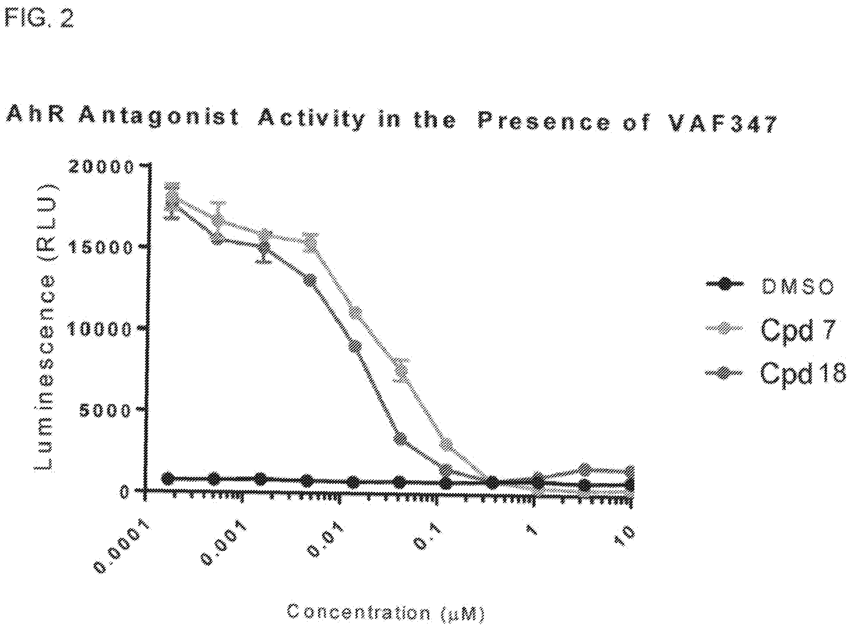

**FIG. 2** is a graph demonstrating the effect of compound **(7)** and compound **(18)** on the aryl hydrocarbon receptor-driven expression of luciferase in the presence of the aryl hydrocarbon receptor agonist VAF347 in transiently transfected HepG2 cells in vitro. Experimental details for this experiment are reported in Example 3, below.

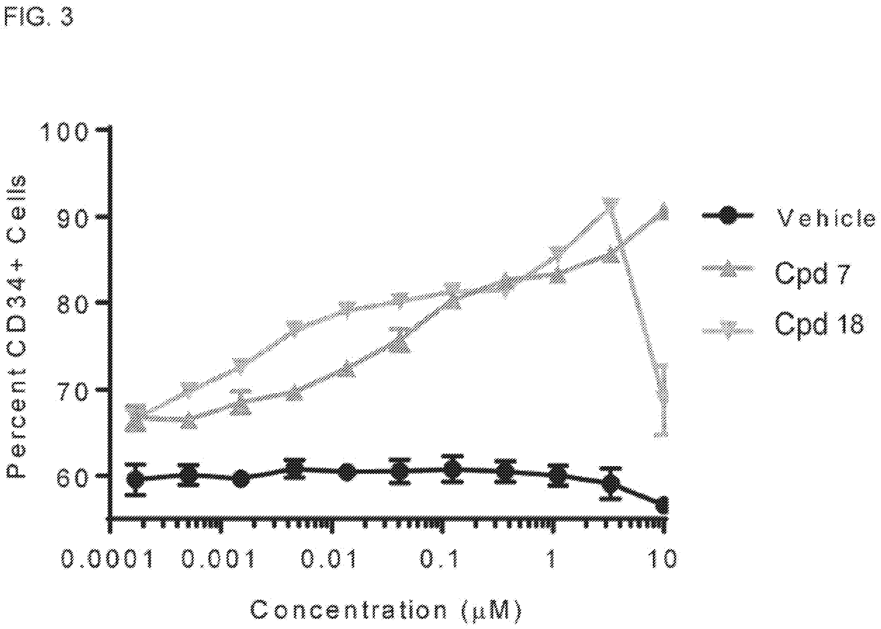

**FIG. 3** is a graph demonstrating the effect of compound **(7)** and compound **(18)** on the quantity of CD34+ cells in a hematopoietic stem cell population over the course of a seven-day experiment. Experimental details for this experiment are reported in Example 3, below.

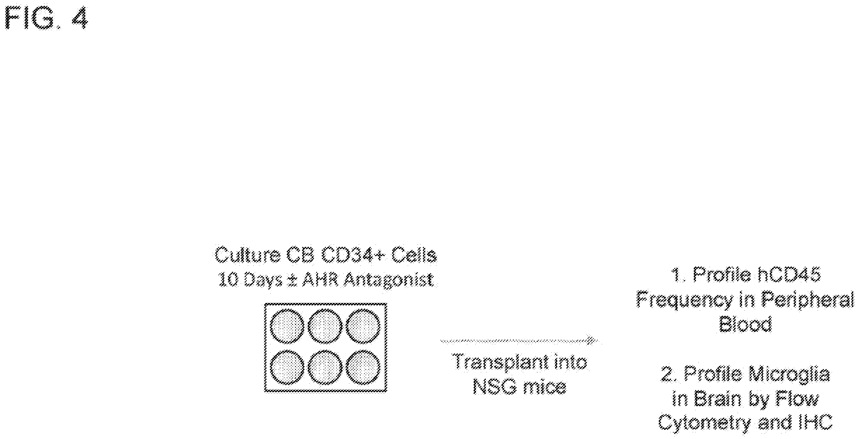

**FIG. 4** is a scheme showing the design of experiments, described in Example 5, below, aimed at examining the ability of hematopoietic stem cells to migrate to central nervous system tissue and promote the engraftment of microglial cells in the brain.

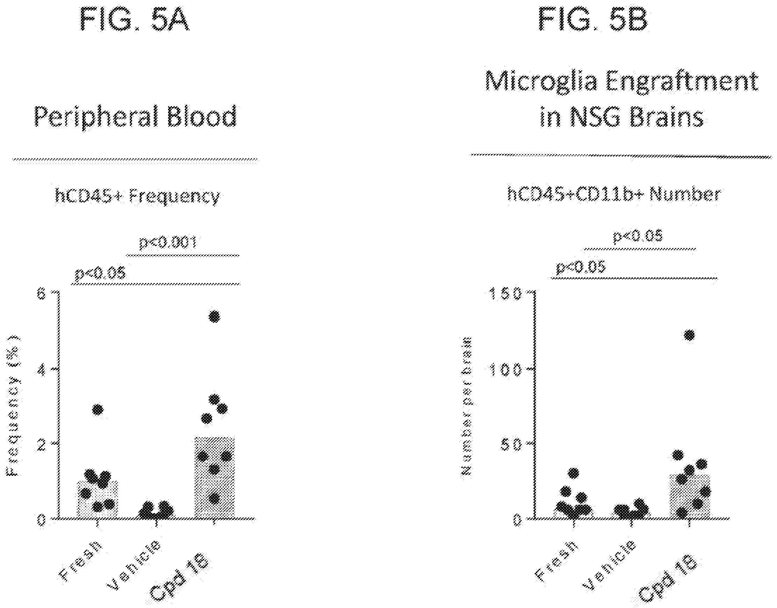

**FIGS. 5A and 5B** are graphs showing the ability of hematopoietic stem cells expanded, ex vivo, in the presence of compound **(18)** to increase the frequency of CD45+ cells in peripheral blood of NSG mice, and to promote the engraftment of microglial cells in the brains of NSG mice. Each bar graph shows the median value obtained upon examination of n=8 NSG mice.

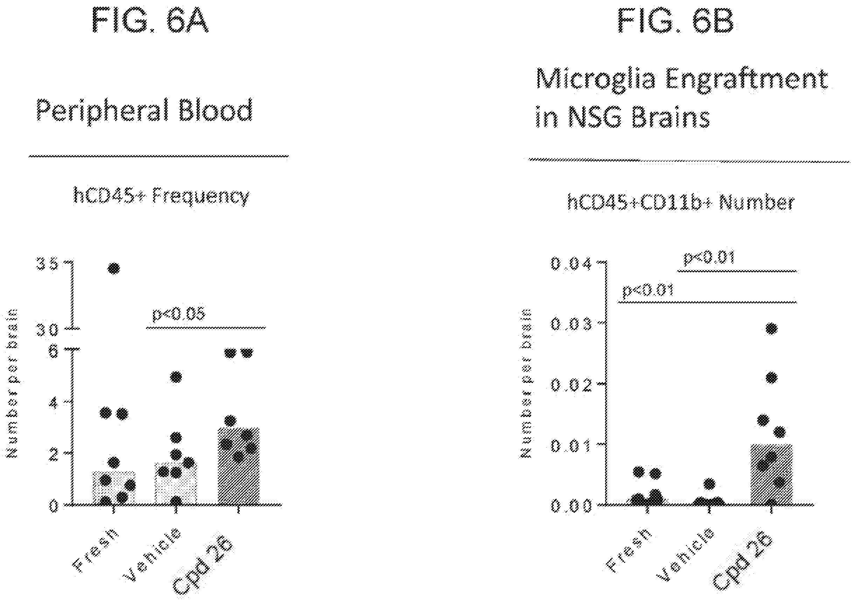

**FIGS. 6A and 6B** are graphs showing the ability of hematopoietic stem cells expanded, ex vivo, in the presence of compound **(26)** to increase the frequency of CD45+ cells in peripheral blood of NSG mice, and to promote the engraftment of microglial cells in the brains of NSG mice. Each bar graph shows the median value obtained upon examination of n=6-8 NSG mice.

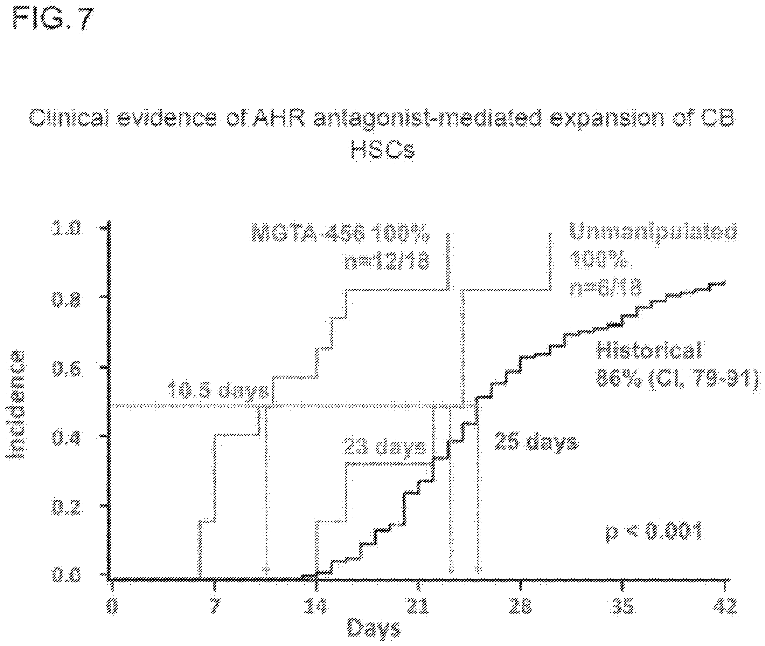

**FIG.** 7 is a graph illustrating the clinical evidence of aryl hydrocarbon receptor (AHR) antagonist-mediated expansion of cord blood-derived hematopoietic stem cells. Particularly, the graph shows the incidence of neutrophil recovery for patients transplanted with expanded hematopoietic stem cells (n=17) compared to that in the historical cohort (n=111). Neutrophil recovery was defined as the absolute neutrophil count greater than or equal to 0.5 × 10⁹/L for three consecutive days.

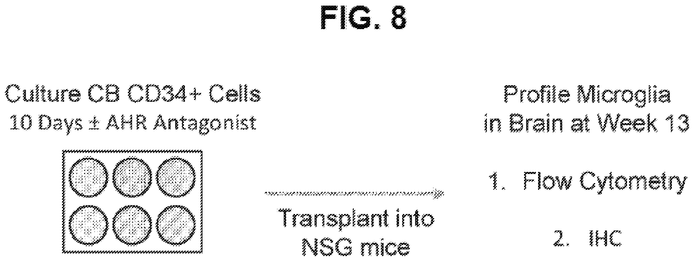

**FIG. 8** is a scheme showing the design of experiments aimed at investigating the ability of hematopoietic stem cells to migrate to central nervous system tissue and engraft as microglial cells in the brains of NSG mice, as described in Example 6, below.

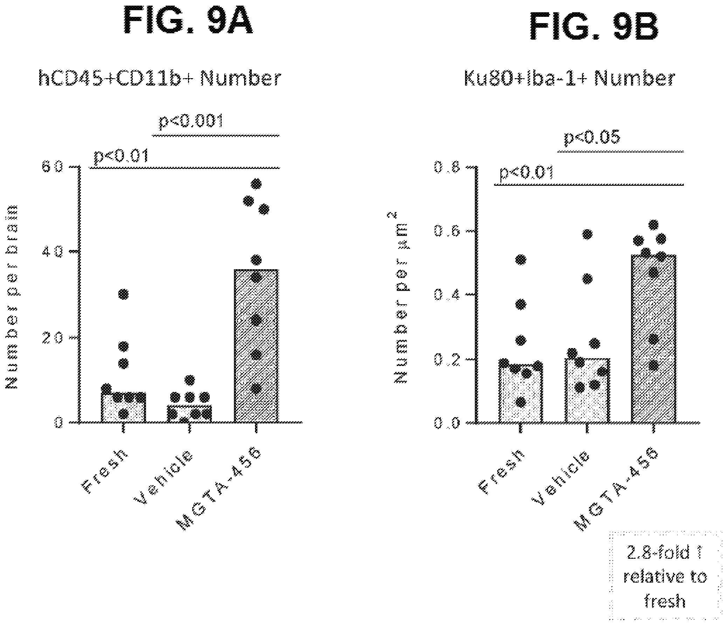

**FIGS. 9A and 9B** are graphs showing the quantity of hCD45+ CD11b+ cells and Ku80+ Iba-1+ cells, respectively, in the brains of NSG mice, upon treatment of the mice with freshly isolated hematopoietic stem cells, vehicle, or MGTA-456, a hematopoietic stem cell composition obtained upon expansion of cord blood *ex vivo* using an aryl hydrocarbon receptor (AHR) antagonist. The graphs show the median values obtained upon observation of n=8 individual mice per group.

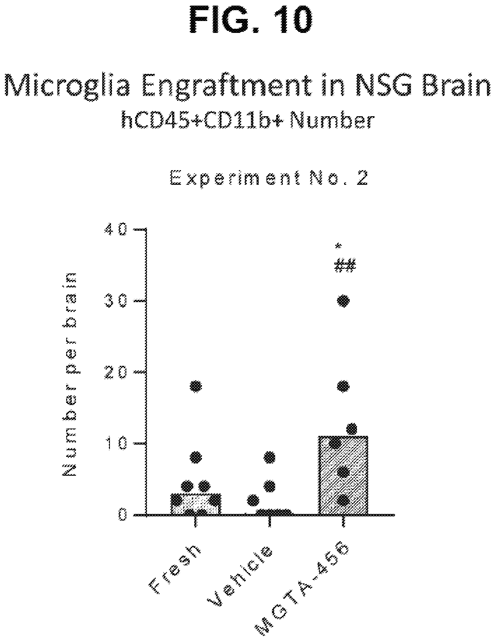

**FIG. 10** is a graph showing the results of a second, independent experiment in which a second flow cytometry quantitation of microglial engraftment in NSG mice was conducted following transplantation of the mice with MGTA-456. Asterisk designates a p value of p<0.05 relative to freshly isolated hematopoietic stem cells. "##" notation designates a p value of p<0.01 relative to vehicle-expanded hematopoietic stem cells. Statistics were calculated using a one-tailed, two-sample equal variance Student's t-test.

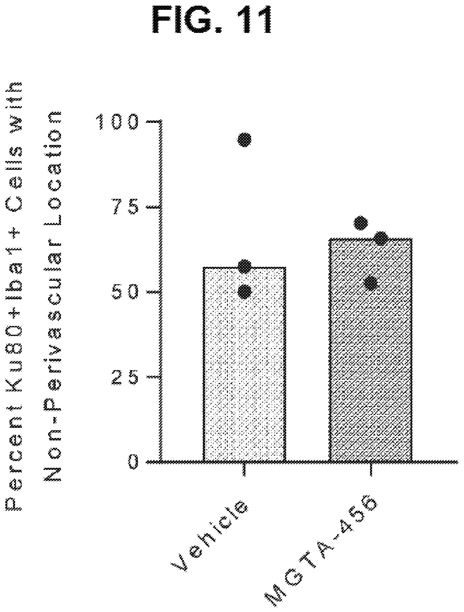

**FIG. 11** is a graph showing the proportion of Ku80+lba1 + microglia in the brains of NSG mice transplanted with vehicle-expanded hematopoietic stem cells or MGTA-456. The frequency of Ku80+lba1 + microglia in the brains of mice transplanted with vehicle-expanded or MGTA-456 were quantitated by IHC from selected sections. The majority of Ku80+lba1+ microglia are non-perivascular. The bar graph shows the median values obtained upon observation of n=3 mice per group.

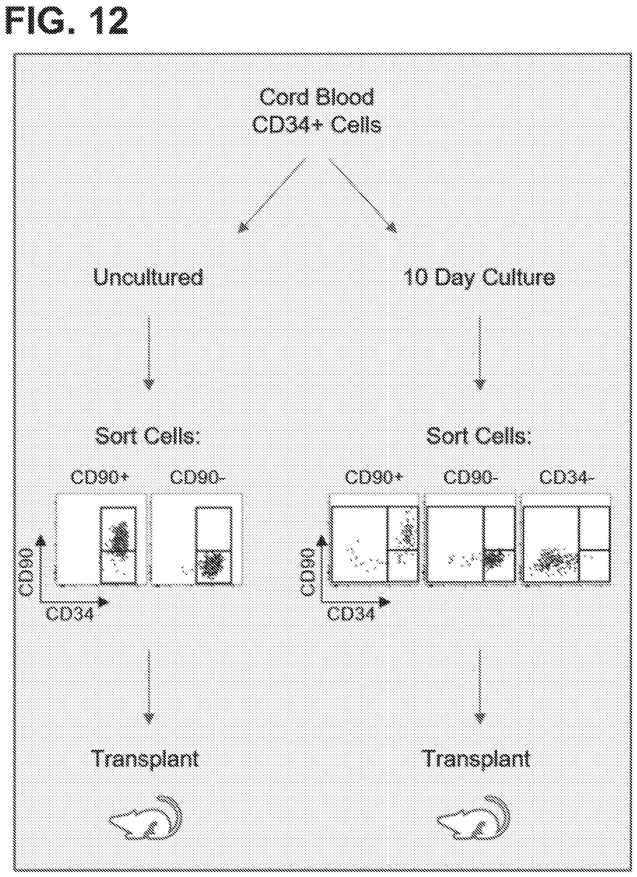

**FIG. 12** is a diagram showing an experimental protocol for transplanting CD34+ cells in NSG immunodeficient mice. CD34+ cord blood cells were either cultured for 10 days or uncultured, then sorted by fluorescent activated flow cytometry using CD34 and CD90 markers. Sorted cell populations were then transplanted into NSG mice.

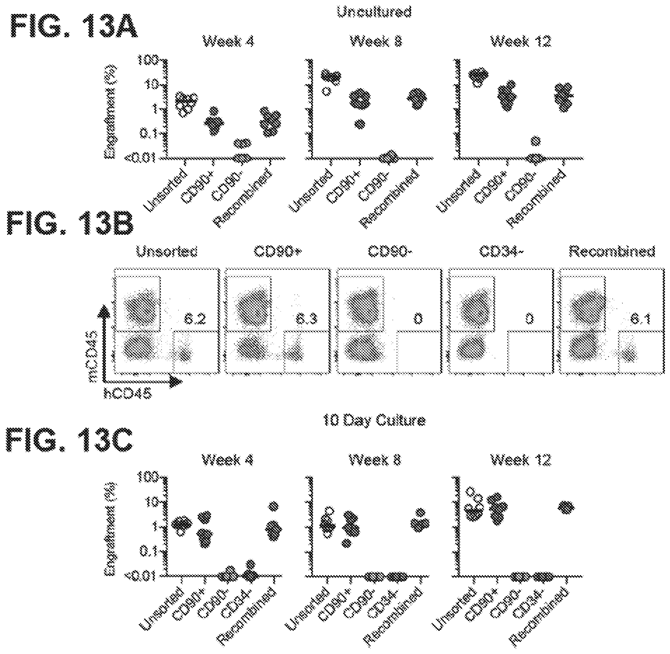

**FIG. 13A** is a series of three graphs showing the percent engraftment of uncultured cells at weeks 4, 8 and 12 following transplantation. Percent engraftment, from <0.01 to 100% is shown on the Y-axis, while unsorted cells (white), CD90+ cells (red), CD90- cells (green) and recombined cells (purple) are shown on the X-axis. The line indicates the median, each symbol represents an individual mouse, cells were injected intravenously.
**FIG. 13B** is a series of 4 fluorescent activated flow cytometry (FACS) plots showing mCD45 (Y axis) versus hCD45 (X axis) expression in, from left to right, unsorted, CD90+, CD90- CD34- and recombined transplanted cell populations.
**FIG. 13C** is a series of three graphs showing the percent engraftment of cells that were cultured 10 days, at weeks 4, 8 and 12 following transplantation.. Percent engraftment, from <0.01 to 100% is shown on the Y-axis, while unsorted cells (white), CD90+ cells (red), CD90- cells (green), CD34- cells (turquoise) and recombined cells (blue) are shown on the X-axis. The line indicates the median, each symbol represents an individual mouse, cells were injected intravenously.

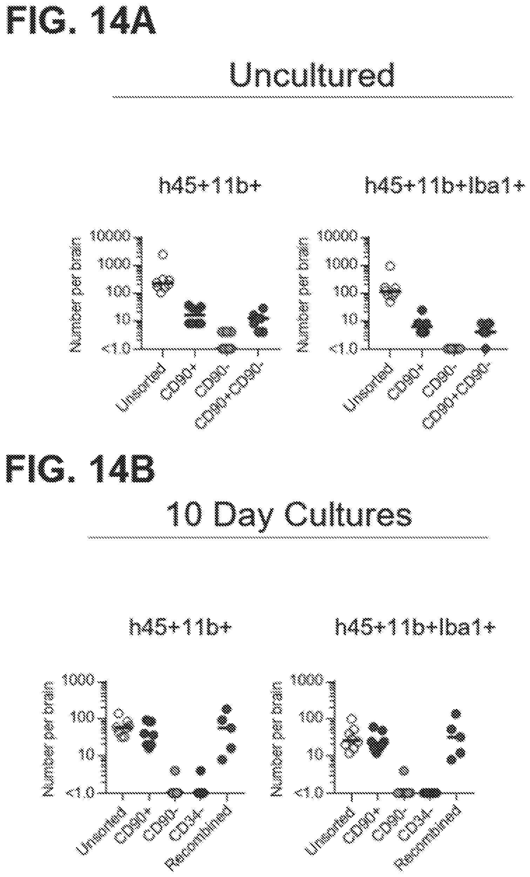

**FIG. 14A** is a pair of graphs showing which cell type contributes to microglia engraftment in the brains of NSG mice transplanted with uncultured cells. The left hand graphs shows the quantity of hCD45+ CD11b+ cells in the brains of mice transplanted with unsorted cells, CD90+ cells, CD90-cells or CD90+ and CD90- cells (X axis, from left to right). The right hand graph shows the quantity of hCD45+ CD11b+ Iba1+ cells in in the brains of mice transplanted with uncultured unsorted cells, CD90+ cells, CD90- cells or CD90+ and CD90- cells (X axis, from left to right). The line indicates the median, each symbol represents an individual mouse, cells were injected intravenously.
**FIG. 14B** is a pair of graphs showing which cell type contributes to microglia engraftment in the brains of NSG mice transplanted with 10 day cultured cells. The left hand graphs shows the quantity of hCD45+ CD11b+ cells in the brains of mice transplanted with unsorted cells, CD90+ cells, CD90- cells CD34 - cells or recombined cells (X axis, from left to right). The right hand graph shows the quantity of hCD45+ CD11b+ Iba1 + cells in in the brains of mice transplanted with unsorted cells, CD90+ cells, CD90- cells, CD34- cells or recombined cells (X axis, from left to right). The line indicates the median, each symbol represents an individual mouse, cells were injected intravenously.

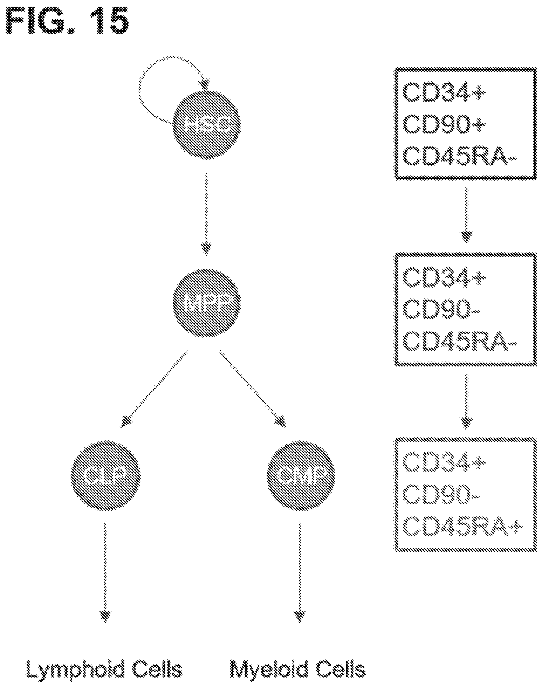

**FIG. 15** is a diagram showing the developmental pathway leading to lymphoid cells and myeloid cells, and the markers expressed in different cell types. HSC = hematopoietic stem cell; MPP = multipotent progenitor; CLP = common lymphoid progenitor; CMP = common myeloid progenitor.

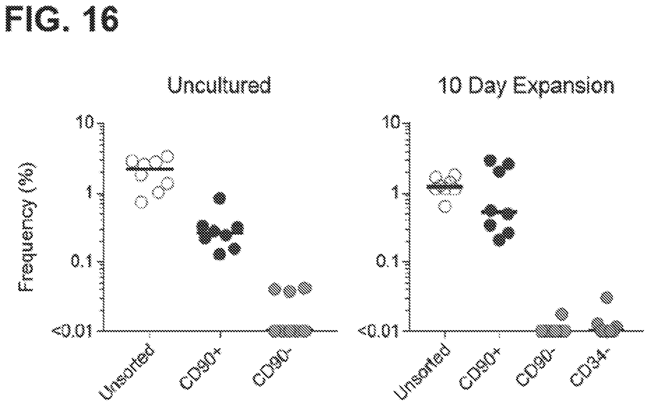

**FIG. 16** is a pair of graphs showing the relevancy of CD90 as a marker for expanded hematopoietic stem cells (HSCs). CD34+ cord blood cells were uncultured or expanded for 10 days, sorted for CD34 and CD90 using FACs and then transplanted into mice. Uncultured cells are shown in the left graph, while 10 day cultured cells are shown in the right. Percent frequency is plotted on the Y axis, while the X-axis, in each plot shows, from left to right, unsorted cells, CD90+ cells and CD90-cells. The 10 day expanded cells also show CD34- cells. The line indicates the median, each symbol represents an individual mouse (n = 8), cells were injected intravenously.

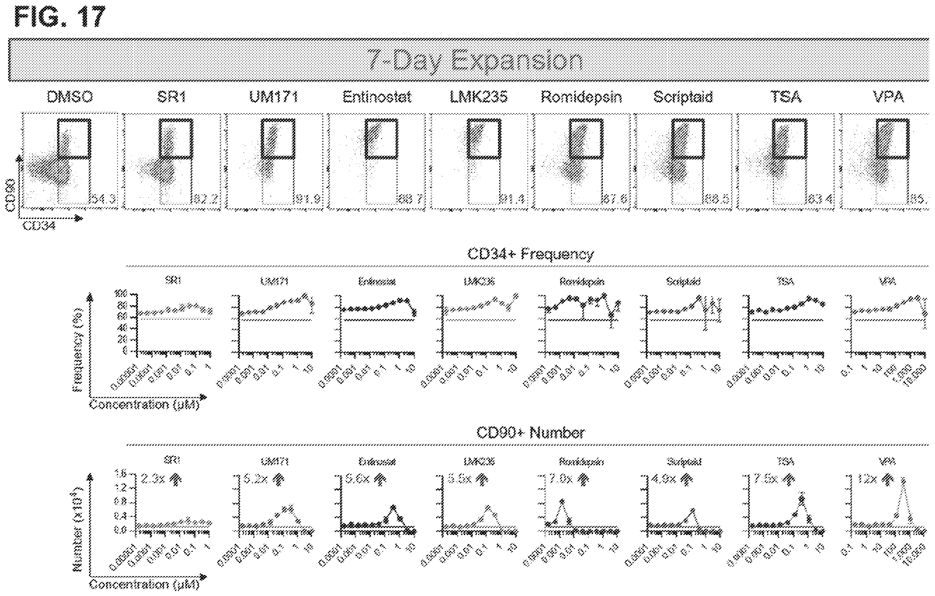

**FIG.** 17 is a series of 9 FACS plots (top row), 8 graphs showing CD34+ frequency versus concentration (frequency is expressed as % on the Y axis, concentration as µM on the X axis, middle row) and 8 graphs showing the number of CD90+ cells versus concentration (Number as ×10⁴ on the Y axis, concentration in µM on the X, bottom row). Cells were expanded 7 days using DMSO (vehicle), SR1, UM171, Entinostat, LMK235, Romidepsin, Scriptaid, TSA and VPA. On day 7, about half of the cells cultured in DMSO vehicle differentiated into CD34- cells. In contrast, SR1 prevented the differentiation of CD34+ cells. UM171 and HDAC inhibitors also had a similar effect. In CD90+ cells, it was observed that while SR1 increased the number of CD90+ cells, and UM171 had a similar effect, the histone deacetylase (HDAC) HDAC inhibitors showed an even greater increase in the number of CD90+ cells after 7 days of expansion. Line Graph: Mean ± SD, n=2.

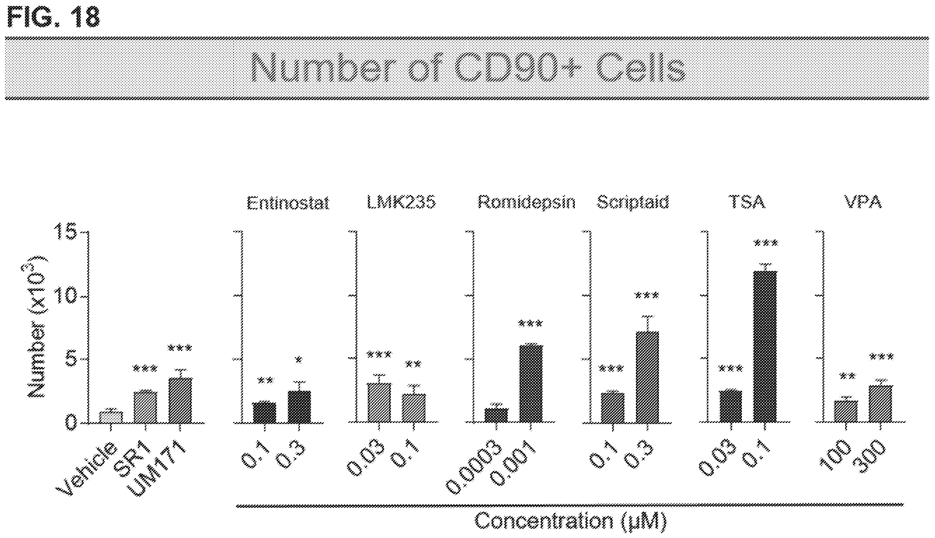

**FIG. 18** is a graph showing the phenotypic expansion of cord blood CD34+ cells with SR1, UM171 and HDAC inhibitors. The graph shows the number of CD90+ cells on the Y axis, in ×10³, while the X axis shows, from left to right, cells cultured with vehicle, SR1, UM171, and different concentrations of Entinostat, LMK235, Romidepsin, Scriptaid, TSA and VPA. Concentrations are as indicated in, µM. Bar Graph: Mean ± SD, n=4, *p<0.05, **p<0.01, ***p<0.001, relative to vehicle-treated cells. All compounds led to a significant increase in CD90+ number after expansion.

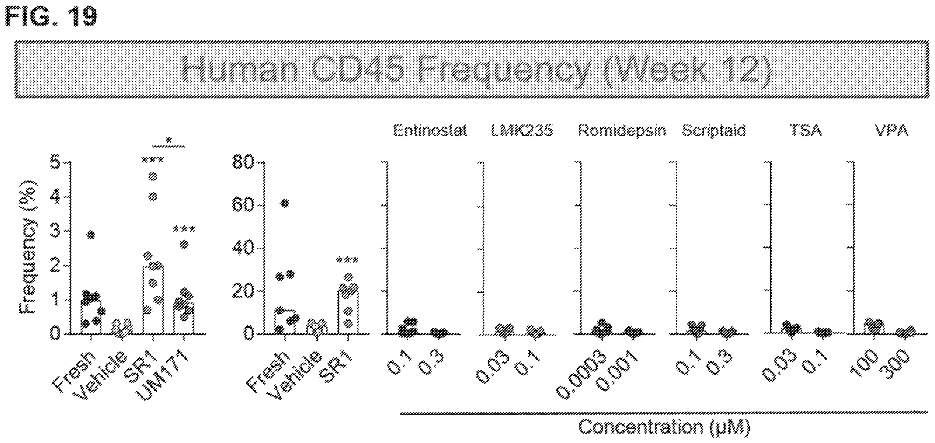

**FIG. 19** is a graph showing that SR1 show the largest increase in engraftable HSCs. CD34+ cord blood cells were transplanted either fresh, or following 10 days of culture with or without vehicle, SR1, UM171, Entinostat, LMK235, Romidepsin, Scriptaid, TSA or VPA at the concentrations shown, and transplanted into mice. The frequency of human CD45 cells was assayed at 12 weeks post transplantation. The Bar graph indicates the median. Each symbol represents individual mouse (n=6-8) *p<0.05, *p<0.01, ***p<0.001 relative to vehicle-cultured cells.

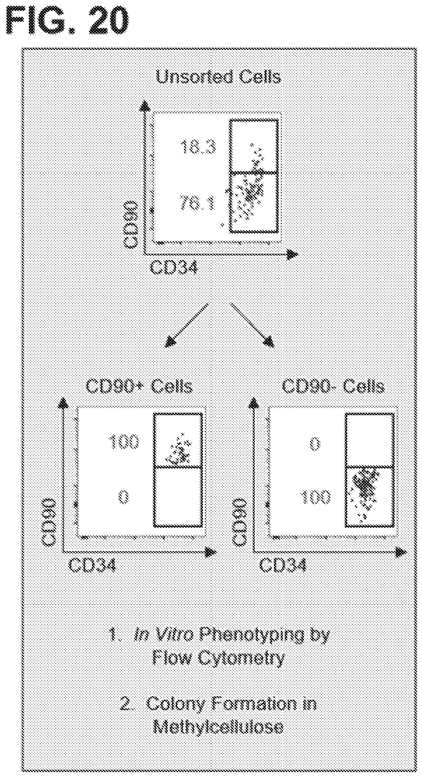

**FIG. 20** is a diagram for an experimental protocol to determine the disconnect between phenotype and function of CD90+ cells. Unsorted cells will be sorted using CD90 and CD34 as markers, and cultured with compounds. Cultured CD34+ CD90+ and CD34+ CD90 - cells will be phenotyped in vitro via FACS and/or assayed for colony formation on methylcellulose.

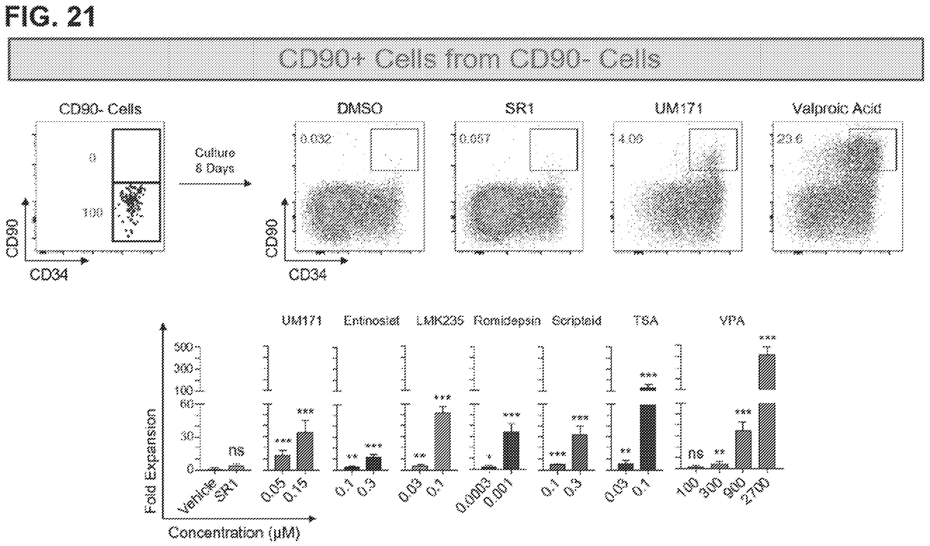

**FIG. 21** is a series of 5 FACS plots (top row) and 8 graphs showing that HDAC inhibitors and UM171 upregulated CD90 on progenitor cells. Top row: CD90+ cells were sorted from CD90- cells, and CD90-cultured 8 days with DMSO, SRT, UM171 or valproic acid. Cultured cells were assayed for the expression of CD90 and CD34 via FACS. The bottom row shows the fold expansion (Y axis) of cells cultured with vehicle, SR1, UM171, Entinostat, LMK235, Romidepsin, Scriptaid, TSA and VAP (concentrations in µM, as shown).

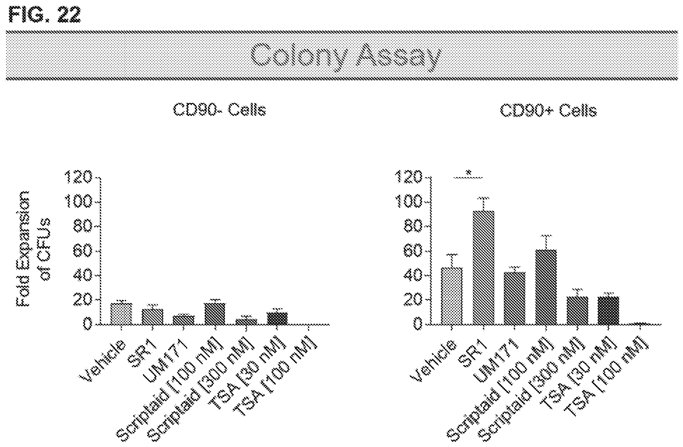

**FIG. 22** is a pair of graphs showing that only SR1 increases colony formation units (CFUs) after expansion. The Y axis shows fold expansion of CFUs. The X axis, from left to right, shows cells cultured with vehicle, SR1, UM171, Scriptaid (100 nM), Scriptaid (300 nM), TSA (30 nM) and TSA (100 nM). CD90- cells are shown in the left hand plot, CD90+ in the right hand plot. CFU formation ability correlates with in vivo transplant ability.

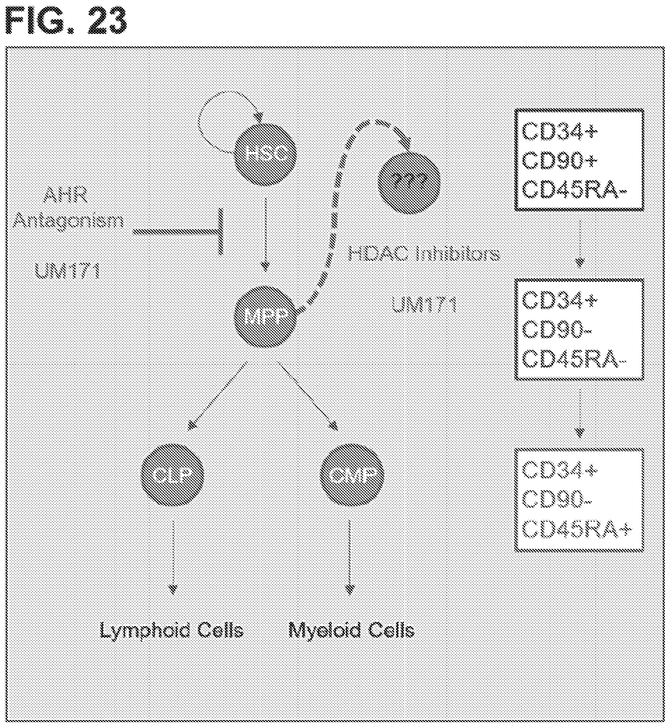

**FIG. 23** is a diagram showing the proposed effect of compounds on HSC differentiation and gene expression. HDAC inhibitors and UM171 upregulate HSC markers on progenitor cells. AHR antagonism is an optimal mechanism to expand HSCs. Expansion of functional HSCs has potential for significant impact on patient outcomes.

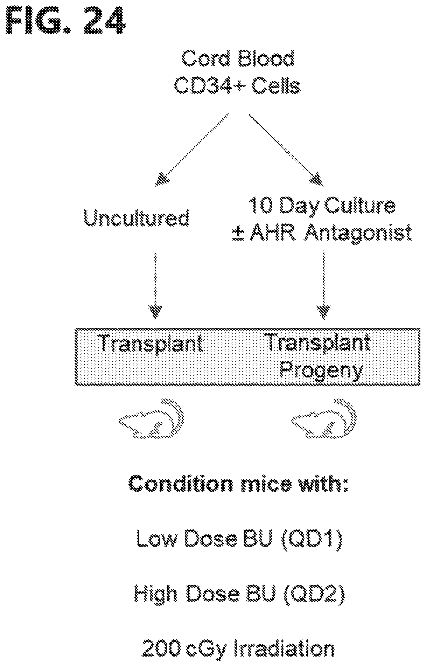

**FIG. 24** is a diagram showing an experimental protocol for transplanting CD34+ cells in NSG immunodeficient mice. CD34+ cord blood cells were either cultured for 10 days with an AHR antagonist (MGTA 456) or uncultured, then transplanted into NSG mice following conditioning with low dose busulfan (QD1, 20 mg/kg), high dose busulfan (QD2, 40 mg/kg), or irradiation (200 cGy).

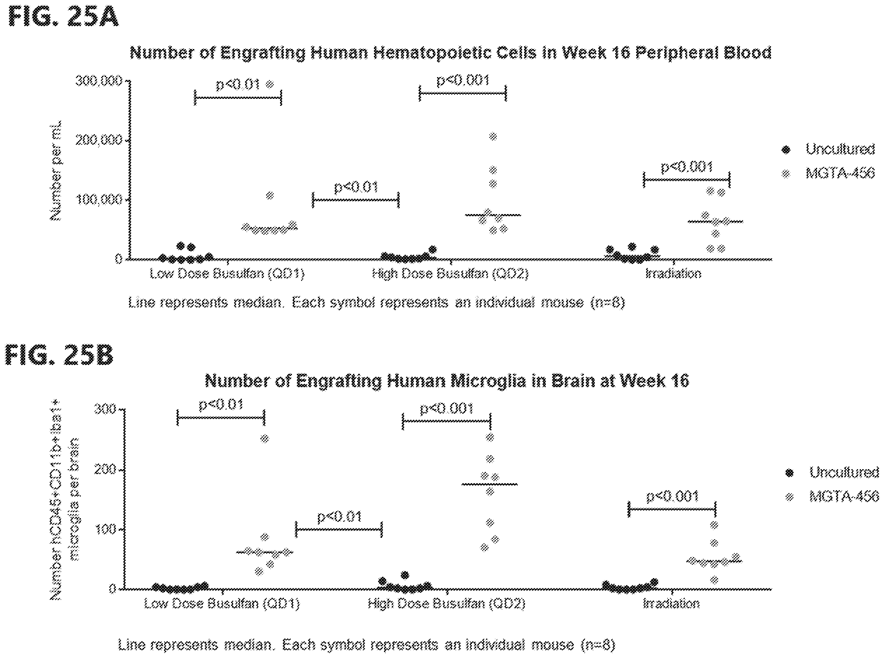

**FIG. 25A** and **FIG. 25B** are graphs showing the number of engrafting human hematopoietic cell in peripheral blood at week 16 following transplantation **(****FIG. 25A****)** and the number of engrafting human microglia in the brain at week 16 following transplantation **(****FIG. 25B****)** for CD34+ cord blood cells that were either uncultured or cultured for 10 days with an AHR antagonist (MGTA 456) and transplanted into NSG mice following conditioning with low dose busulfan (QD1, 20 mg/kg), high dose busulfan (QD2, 40 mg/kg), or irradiation (200 cGy). The line indicates the median, each symbol represents an individual mouse (n = 8), cells were injected intravenously.

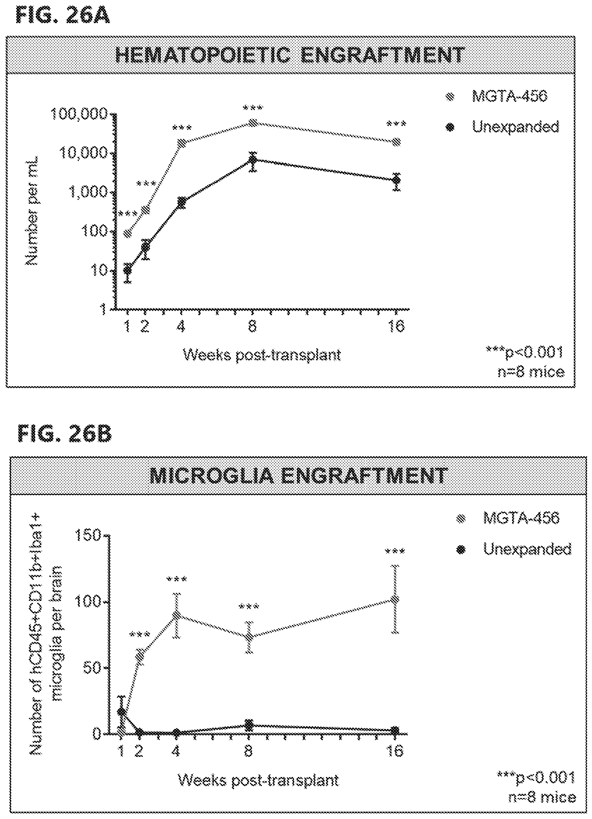

**FIG. 26A** and **FIG. 26B** are graphs showing the number of engrafting human hematopoietic cells in peripheral blood **(****FIG. 26A****)** and the number of engrafting human microglia in the brain as a function of weeks post-transplantation for CD34+ cord blood cells that were either uncultured or cultured for 10 days with an AHR antagonist (MGTA 456) and transplanted into NSG mice.

### Detailed Description

Disclosed herein are compositions and methods for the expansion of hematopoietic stem and progenitor cells. It has presently been discovered that the use of an aryl hydrocarbon receptor antagonist to expand a population of hematopoietic stem or progenitor cells produces a population of cells that can maintain long-term engraftment potential.

Compositions and methods for expanding hematopoietic stem cells from various sources like bone marrow (BM) mobilized peripheral blood (mPB), or cord blood (CB) can: have significant impact on patient outcomes by leading to faster engraftment, which allows for patients to leave the hospital sooner and allows for the expansion of usable CB inventory, which allows for more patients to receive a better matched graft.

The sections that follow describe, in further detail, the compositions and methods that can be used to effectuate the expansion of hematopoietic stem and progenitor cells.

### Methods of Treating Inherited Metabolic Disorders - Administration of Expanded CD90+ Stem Cells for Microglial Engraftment in the Brain

A significant number of inherited metabolic disorders (IMDs) are characterized by defective enzyme function in patients. Whereas a wild-type (WT) cell is able to degrade substrates, in an enzyme-deficient cell, lack of enzyme function may lead to an accumulation of toxic substrates, which can result in cell and tissue death, and in some IMDs, neurological defects. As these diseases can be uniformly fatal if left untreated, a therapeutic goal for these IMDs is to restore functional enzyme levels. Many of these diseases, such as Fabry or some mucopolysaccharidosis (MPS) subtypes, can be treated with enzyme replacement therapy. For many disorders, however, enzymes cannot translocate to the brain and therefore, enzyme replacement therapy is not a suitable treatment option where patients present with neurological symptoms. For these diseases, hematopoietic stem cell (HSC) transplant may be the only disease-modifying treatment option. Cross correction of neurological defects remains a possibility with HSC transplantation as hematopoietic cells are able to cross the blood brain barrier.

Following transplant, donor-derived myeloid cells, including microglial, are able to enter the brain. Without wishing to be bound by any theory, microglia secrete wild-type enzyme, which is taken up by the cell and degrades substrate in brain cells. Two possible ways in which cross-correction by transplant may occur are: i) allogeneic HSC transplant and ii) autologous gene therapy. Allogenic transplantation has shown promise as the standard of care for treating IMD patients due to the documented functional curative capabilities.

As described herein, hematopoietic stem cell transplant therapy can be administered to a subject in need of treatment so as to populate or repopulate one or more blood cell types, such as a blood cell lineage that is deficient or defective in a patient suffering from a stem cell disorder. Hematopoietic stem and progenitor cells exhibit multi-potency, and can thus differentiate into multiple different blood lineages including, microglia.

In one embodiment, hematopoietic stem cell transplant therapy or hematopoietic stem cell transplantation of inherited metabolic disorders may be accomplished using cross-correction. (Wynn, R. "Stem Cell Transplantation in Inherited Metabolic Disorders" Hematology 2011, pp. 285-291.) Cross correction involves engraftment of expanded HSCs in the patient or host tissue, where the implanted cells secrete the deficient enzyme and said deficient enzyme is then taken up by cells in the patient which are deficient in that enzyme.

In one embodiment, the inherited metabolic disorder to be treated is selected from Hurler syndrome (Hurler's Disease), mucopolysaccharide disorders (e.g., Maroteaux Lamy syndrome), lysosomal storage disorders, and peroxisomal disorders (e.g., X-linked adrenoleukodystrophy), glycogen storage diseases, mucopolysaccharidoses, Mucolipidosis II, Gaucher's Disease, sphingolipidoses, and metachromatic leukodystrophy.

In certain embodiments, HSCs in the patient or in a healthy donor are mobilized using a CXCR2 agonist and/or CXCR4 antogonist of the disclosure. The CXCR4 antagonist may be plerixafor or a variant thereof, and a CXCR2 agonist may be Gro-β or a variant thereof, such as a truncation of Gro-β, for instance, Gro-β T. Mobilized HSCs are then isolated from a peripheral blood sample of the subject. Methods of isolating HSCs will be readily apparent to one of ordinary skill in the art. Alternatively, HSCs may be mobilized using a CXCR2 agonist and/or CXCR4 antogonist of the disclosure in a healthy individual who (1) does not suffer from an inherited metabolic disorder and (2) is a compatible donor for the subject who does suffer from the inherited metabolic disorder. HSCs can be isolated from a blood sample taken from this healthy individual collected following mobilization, the HSCs can then be expanded using the expansion methods of the disclosure, and the expanded cells transplanted into the subject with the inherited metabolic disorder.

It has been found that HSCs prepared with the methods of the disclosure lead to more microglia engraftment than fresh cells or cells cultured in the presence of cytokines. This is due to the presence of more CD90+ cells in expanded cell populations.

In some embodiments, the expanded hematopoietic stem cells result in microglia engraftment in the brain of the subject in less than about 10 weeks after administering, less than about 8 weeks after administering, less than about 6 weeks after administering, less than about 4 weeks after administering, less than about 3 weeks after administering, less than about 2 weeks after administering, or less than about 1 week after administering.

In some embodiments, the expanded hematopoietic stem cells result in microglia engraftment in the brain of the subject that is maintained for greater than 1 week after administering, greater than 2 weeks after administering, greater than 4 weeks after administering, greater than 8 weeks after administering, greater than 16 weeks after administering, greater than 32 weeks after administering, or greater than 40 weeks after administering.

In some embodiments, the expanded hematopoietic stem cell results in microglia engraftment in the brain of the subject in a period of time that is decreased as the number of expanded hematopoietic stem cells that are administered is increased.

In some embodiments, the expanded hematopoietic stem cells result in microglia engraftment in the brain of the subject in a period of time that is shorter than a period of time for a substantially similar population of hematopoietic stem cells that is not expanded in the presence of an aryl hydrocarbon receptor antagonist.

In some embodiments, the expanded hematopoietic stem cell results in microglia engraftment in the brain of the subject that is maintained for a period of time that is increased as the number of expanded hematopoietic stem cells that are administered is increased.

In some embodiments, the expanded hematopoietic stem cells result in microglia engraftment in the brain of the subject that is maintained for a period of time that is longer than a period of time for a substantially similar population of hematopoietic stem cells that is not expanded in the presence of an aryl hydrocarbon receptor antagonist.

The methods disclosed herein for treating inherited metabolic disorders in a subject in need thereof comprise the administration of an expanded population of hematopoietic stem cells to a subject in need thereof. The number of expanded hematopoietic stem cells administered to the subject may be equal to or greater than the amount of hematopoietic stem cells needed to achieve a therapeutic benefit. The number of expanded hematopoietic stem cells administered to the subject may be greater than the amount of hematopoietic stem cells needed to achieve a therapeutic benefit. The therapeutic benefit achieved may be proportional to the number of expanded hematopoietic stem cells that are administered.

A dose of the expanded hematopoietic stem cell composition of the disclosure is deemed to have achieved a therapeutic benefit if it alleviates a sign or a symptom of the disease. The sign or symptom of the disease may comprise one or more biomarkers associated with the disease, or one or more clinical symptoms of the disease.

For example, administration of the expanded hematopoietic stem cell composition may result in the reduction of a biomarker that is elevated in individuals suffering from the disease, or elevate the level of a biomarker that is reduced in individuals suffering from the disease.

For example, administering the expanded hematopoietic stem cell composition of the disclosure may elevate the level of an enzyme that is reduced in an individual suffering from a metabolic disorder. This change in biomarker level may be partial, or the level of the biomarker may return to levels normally seen in healthy individuals.

In one embodiment, when the disease is, for example, an inherited metabolic disorder with a neurological component, the expanded hematopoietic stem cell composition may partly or fully reduce one or more clinical symptoms of the inherited metabolic disorder. Exemplary but non-limiting symptoms that may be affected by administration of the expanded hematopoietic stem cell composition of the disclosure comprise ataxias, dystonia, movement, disorders, epilepsies, and peripheral neuropathy.

In some cases, the sign or symptom of the inherited metabolic disorder with a neurological component comprises psychological signs or symptoms. For example, the sign or symptom of the disorder may comprise acute psychotic disorder, hallucinations, depressive syndrome, other symptoms or combinations of symptoms. Methods of evaluating psychological signs or symptoms associated with metabolic disorders with a neurological component will be known to one of ordinary skill in the art.

The onset of the inherited metabolic disorder may be adult or pediatric.

The inherited metabolic disorder may lead to degeneration of the nervous system.

Alleviating a sign or a symptom of the disorder may comprise slowing the rate of neurodegeneration or the rate of the progression of the disease.

Alleviating a sign or a symptom of the disorder may comprise reversing neurodegeneration or reversing the progression of the disease. Exemplary symptoms of neurodegeneration comprise memory loss, apathy, anxiety, agitation, loss of inhibition and mood changes. Methods of evaluating neurodegeneration, and the progression thereof, will be known to one of ordinary skill in the art.

For example, in a patient suffering from Hurler syndrome, heparan and dermatan sulfate accumulation follows from α-L-iduronidase deficiency. Treatments that better clear these accumulated substrates will better correct the underlying disorder.

### Definitions

Listed below are definitions of various terms used in this application. These definitions apply to terms as they are used throughout this specification and claims, unless otherwise limited in specific instances, either individually or as part of a larger group.

As used herein, the term "about" refers to a value that is within 10% above or below the value being described. For example, the term "about 5 nM" indicates a range of from 4.5 nM to 5.5 nM.

As used herein, "CRU (competitive repopulating unit)" refers to a unit of measure of long-term engrafting stem cells, which can be detected after in-vivo transplantation.

As used herein in the context of Gro-β, Gro-β T, or a variant thereof, the term "deamidated version" of one or more of these peptides refers to a form of the peptide in which the C-terminal asparagine residue that is located at position 69 in the amino acid sequence of Gro-β, at position 65 in the amino acid sequence of Gro-β T, and at equivalent positions in variant peptides, has been converted to an aspartic acid residue. Deamidated versions of Gro-β and Gro-β T (Gro-β N69D and Gro-β T N65D, respectively) are described in Table 2, herein.

As used herein with respect a hematopoietic stem cell, the term "progenitor" refers to a parent cell or an ancestor thereof that gave rise to the hematopoietic stem cell by way of cell division. For instance, a progenitor of a hematopoietic stem cell may be a parent cell that gave rise to the hematopoietic stem cell by mitotic reproduction, or an ancestor of the parent cell.

As used herein, the term "donor" refers to a subject, such as a mammalian subject (e.g., a human subject) from which one or more cells are isolated prior to administration of the cells, or progeny thereof, into a recipient. The one or more cells may be, for example, a population of hematopoietic stem or progenitor cells.

As used herein, the term "endogenous" describes a substance, such as a molecule, cell, tissue, or organ (e.g., a hematopoietic stem cell or a cell of hematopoietic lineage, such as a megakaryocyte, thrombocyte, platelet, erythrocyte, mast cell, myeoblast, basophil, neutrophil, eosinophil, microglial cell, granulocyte, monocyte, osteoclast, antigen-presenting cell, macrophage, dendritic cell, natural killer cell, T-lymphocyte, or B-lymphocyte) that is found naturally in a particular organism, such as a human patient.

As used herein, the term "engraftment potential" is used to refer to the ability of hematopoietic stem and progenitor cells to repopulate a tissue, whether such cells are naturally circulating or are provided by transplantation. The term encompasses all events surrounding or leading up to engraftment, such as tissue homing of cells and colonization of cells within the tissue of interest. The engraftment efficiency or rate of engraftment can be evaluated or quantified using any clinically acceptable parameter as known to those of skill in the art and can include, for example, assessment of competitive repopulating units (CRU); incorporation or expression of a marker in tissue(s) into which stem cells have homed, colonized, or become engrafted; or by evaluation of the progress of a subject through disease progression, survival of hematopoietic stem and progenitor cells, or survival of a recipient. Engraftment can also be determined by measuring white blood cell counts in peripheral blood during a post-transplant period. Engraftment can also be assessed by measuring recovery of marrow cells by donor cells in a bone marrow aspirate sample.

As used herein, the term "exogenous" describes a substance, such as a molecule, cell, tissue, or organ (e.g., a hematopoietic stem cell or a cell of hematopoietic lineage, such as a megakaryocyte, thrombocyte, platelet, erythrocyte, mast cell, myeoblast, basophil, neutrophil, eosinophil, microglial cell, granulocyte, monocyte, osteoclast, antigen-presenting cell, macrophage, dendritic cell, natural killer cell, T-lymphocyte, or B-lymphocyte) that is not found naturally in a particular organism, such as a human patient. Exogenous substances include those that are provided from an external source to an organism or to cultured matter extracted therefrom.

As used herein, the term "expanding amount" refers to a quantity or concentration of an agent, such as an aryl hydrocarbon receptor antagonist described herein, sufficient to induce the proliferation of a population of CD34+ cells (e.g., a CD34+ CD90+ cells), for example, by from about 1.1 -fold to about 1,000-fold, about 1.1 -fold to about 5,000-fold or more (e.g., about 1.1 -fold, 1.2-fold, 1.3-fold, 1.4-fold, 1.5-fold, 1.6-fold, 1.7-fold, 1.8-fold, 1.9-fold, 2-fold, 2.1 -fold, 2.2-fold, 2.3-fold, 2.4-fold, 2.5-fold, 2.6-fold, 2.7-fold, 2.8-fold, 2.9-fold, 3-fold, 3.1-fold, 3.2-fold, 3.3-fold, 3.4-fold, 3.5-fold, 3.6-fold, 3.7-fold, 3.8-fold, 3.9-fold, 4-fold, 4.1-fold, 4.2-fold, 4.3-fold, 4.4-fold, 4.5-fold, 4.6-fold, 4.7-fold, 4.8-fold, 4.9-fold, 5-fold, 5.1-fold, 5.2-fold, 5.3-fold, 5.4-fold, 5.5-fold, 5.6-fold, 5.7-fold, 5.8-fold, 5.9-fold, 6-fold, 6.1-fold, 6.2-fold, 6.3-fold, 6.4-fold, 6.5-fold, 6.6-fold, 6.7-fold, 6.8-fold, 6.9-fold, 7-fold, 7.1-fold, 7.2-fold, 7.3-fold, 7.4-fold, 7.5-fold, 7.6-fold, 7.7-fold, 7.8-fold, 7.9-fold, 8-fold, 8.1-fold, 8.2-fold, 8.3-fold, 8.4-fold, 8.5-fold, 8.6-fold, 8.7-fold, 8.8-fold, 8.9-fold, 9-fold, 9.1-fold, 9.2-fold, 9.3-fold, 9.4-fold, 9.5-fold, 9.6-fold, 9.7-fold, 9.8-fold, 9.9-fold, 1 0-fold, 50-fold, 100-fold, 200-fold, 300-fold, 400-fold, 500-fold, 600-fold, 700-fold, 800-fold, 900-fold, 1,000-fold, or more).

In some embodiments, the expanding amount, refers to a quantity or concentration of an agent, such as an aryl hydrocarbon receptor antagonist described herein, sufficient to induce the proliferation of a population of CD34+ cells (e.g., a CD34+ CD90+ cells), for example, by from about 60-fold to about 900-fold, from about 80-fold to about 800-fold, from about 100-fold to about 700-fold, from about 150-fold to about 600-fold, from about 200-fold to about 500-fold, from about 250-fold to about 400-fold, from about 275-fold to about 350-fold, or about 325-fold.

As used herein, the term "hematopoietic progenitor cells" includes pluripotent cells capable of differentiating into several cell types of the hematopoietic system, including, without limitation, granulocytes, monocytes, erythrocytes, megakaryocytes, B-cells and T- cells, among others. Hematopoietic progenitor cells are committed to the hematopoietic cell lineage and generally do not self-renew. Hematopoietic progenitor cells can be identified, for example, by expression patterns of cell surface antigens, and include cells having the following immunophenotype: Lin- KLS+ Flk2-CD34+. Hematopoietic progenitor cells include short-term hematopoietic stem cells, multi-potent progenitor cells, common myeloid progenitor cells, granulocyte-monocyte progenitor cells, and megakaryocyte-erythrocyte progenitor cells. The presence of hematopoietic progenitor cells can be determined functionally, for instance, by detecting colony-forming unit cells, e.g., in complete methylcellulose assays, or phenotypically through the detection of cell surface markers using flow cytometry and cell sorting assays described herein and known in the art.

As used herein, the term "hematopoietic stem cells" ("HSCs") refers to immature blood cells having the capacity to self-renew and to differentiate into mature blood cells containing diverse lineages including but not limited to granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells). Such cells may include CD34+ cells. CD34+ cells are immature cells that express the CD34 cell surface marker. In humans, CD34+ cells are believed to include a subpopulation of cells with the stem cell properties defined above, whereas in mice, HSCs are CD34-. In addition, HSCs also refer to long term repopulating HSCs (LT-HSC) and short term repopulating HSCs (ST-HSC). LT-HSCs and ST-HSCs are differentiated, based on functional potential and on cell surface marker expression. For example, human HSCs are CD34+, CD38-, CD45RA-, CD90+, CD49F+, and lin- (negative for mature lineage markers including CD2, CD3, CD4, CD7, CD8, CD10, CD11B, CD19, CD20, CD56, CD235A). In mice, bone marrow LT-HSCs are CD34-, SCA-1+, C-kit+, CD135-, Slamfl/CD150+, CD48-, and lin-(negative for mature lineage markers including Ter119, CD11b, Gr1, CD3, CD4, CD8, B220, IL7ra), whereas ST-HSCs are CD34+, SCA-1+, C-kit+, CD135-, Slamfl/CD150+, and lin- (negative for mature lineage markers including Ter119, CD11b, Gr1, CD3, CD4, CD8, B220, IL7ra). In addition, ST-HSCs are less quiescent and more proliferative than LT-HSCs under homeostatic conditions. However, LT-HSC have greater self renewal potential (i.e., they survive throughout adulthood, and can be serially transplanted through successive recipients), whereas ST-HSCs have limited self renewal (i.e., they survive for only a limited period of time, and do not possess serial transplantation potential). Any of these HSCs can be used in the methods described herein. ST-HSCs are particularly useful because they are highly proliferative and thus, can more quickly give rise to differentiated progeny.

As used herein, the term "hematopoietic stem cell functional potential" refers to the functional properties of hematopoietic stem cells which include 1) multi-potency (which refers to the ability to differentiate into multiple different blood lineages including, but not limited to, granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells), 2) self-renewal (which refers to the ability of hematopoietic stem cells to give rise to daughter cells that have equivalent potential as the mother cell, and further that this ability can repeatedly occur throughout the lifetime of an individual without exhaustion), and 3) the ability of hematopoietic stem cells or progeny thereof to be reintroduced into a transplant recipient whereupon they home to the hematopoietic stem cell niche and re-establish productive and sustained hematopoiesis.

As used herein, the terms "Major histocompatibility complex antigens" ("MHC", also referred to as "human leukocyte antigens" ("HLA") in the context of humans) refer to proteins expressed on the cell surface that confer a unique antigenic identity to a cell. MHC/HLA antigens are target molecules that are recognized by T cells and NK cells as being derived from the same source of hematopoietic stem cells as the immune effector cells ("self") or as being derived from another source of hematopoietic reconstituting cells ("non-self"). Two main classes of HLA antigens are recognized: HLA class I and HLA class II. HLA class I antigens (A, B, and C in humans) render each cell recognizable as "self," whereas HLA class II antigens (DR, DP, and DQ in humans) are involved in reactions between lymphocytes and antigen presenting cells. Both have been implicated in the rejection of transplanted organs. An important aspect of the HLA gene system is its polymorphism. Each gene, MHC class I (A, B and C) and MHC class II (DP, DQ and DR) exists in different alleles. For example, two unrelated individuals may carry class I HLA-B, genes B5, and Bw41, respectively. Allelic gene products differ in one or more amino acids in the α and/or β domain(s). Large panels of specific antibodies or nucleic acid reagents are used to type HLA haplotypes of individuals, using leukocytes that express class I and class II molecules. The genes commonly used for HLA typing are the six MHC Class I and Class II proteins, two alleles for each of HLA- A; HLA-B and HLA-DR. The HLA genes are clustered in a "super-locus" present on chromosome position 6p21, which encodes the six classical transplantation HLA genes and at least 132 protein coding genes that have important roles in the regulation of the immune system as well as some other fundamental molecular and cellular processes. The complete locus measures roughly 3.6 Mb, with at least 224 gene loci. One effect of this clustering is that "haplotypes", i.e. the set of alleles present on a single chromosome, which is inherited from one parent, tend to be inherited as a group. The set of alleles inherited from each parent forms a haplotype, in which some alleles tend to be associated together. Identifying a patient's haplotypes can help predict the probability of finding matching donors and assist in developing a search strategy, because some alleles and haplotypes are more common than others and they are distributed at different frequencies in different racial and ethnic groups.

As used herein, the term "HLA-matched" refers to a donor-recipient pair in which none of the HLA antigens are mismatched between the donor and recipient, such as a donor providing a hematopoietic stem cell graft to a recipient in need of hematopoietic stem cell transplant therapy. HLA-matched (i.e., where all of the 6 alleles are matched) donor-recipient pairs have a decreased risk of graft rejection, as endogenous T cells and NK cells are less likely to recognize the incoming graft as foreign, and are thus less likely to mount an immune response against the transplant.

As used herein, the term "HLA-mismatched" refers to a donor-recipient pair in which at least one HLA antigen, in particular with respect to HLA-A, HLA-B, HLA-C, and HLA-DR, is mismatched between the donor and recipient, such as a donor providing a hematopoietic stem cell graft to a recipient in need of hematopoietic stem cell transplant therapy. In some embodiments, one haplotype is matched and the other is mismatched. HLA-mismatched donor-recipient pairs may have an increased risk of graft rejection relative to HLA-matched donor-recipient pairs, as endogenous T cells and NK cells are more likely to recognize the incoming graft as foreign in the case of an HLA-mismatched donor-recipient pair, and such T cells and NK cells are thus more likely to mount an immune response against the transplant.

As used herein, the term "aryl hydrocarbon receptor (AHR) modulator" refers to an agent that causes or facilitates a qualitative or quantitative change, alteration, or modification in one or more processes, mechanisms, effects, responses, functions, activities or pathways mediated by the AHR receptor. Such changes mediated by an AHR modulator, such as an inhibitor or a non-constitutive agonist of the AHR described herein, can refer to a decrease or an increase in the activity or function of the AHR, such as a decrease in, inhibition of, or diversion of, constitutive activity of the AHR.

An "AHR antagonist" refers to an AHR inhibitor that does not provoke a biological response itself upon specifically binding to the AHR polypeptide or polynucleotide encoding the AHR, but blocks or dampens agonist-mediated or ligand-mediated responses, i.e., an AHR antagonist can bind but does not activate the AHR polypeptide or polynucleotide encoding the AHR, and the binding disrupts the interaction, displaces an AHR agonist, and/or inhibits the function of an AHR agonist. Thus, as used herein, an AHR antagonist does not function as an inducer of AHR activity when bound to the AHR, i.e., they function as pure AHR inhibitors.

As used herein, patients that are "in need of" a hematopoietic stem cell transplant include patients that exhibit a defect or deficiency in one or more blood cell types, as well as patients having a stem cell disorder, autoimmune disease, cancer, or other pathology described herein. Hematopoietic stem cells generally exhibit 1) multi-potency, and can thus differentiate into multiple different blood lineages including, but not limited to, granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells), 2) self-renewal, and can thus give rise to daughter cells that have equivalent potential as the mother cell, and 3) the ability to be reintroduced into a transplant recipient whereupon they home to the hematopoietic stem cell niche and re-establish productive and sustained hematopoiesis. Hematopoietic stem cells can thus be administered to a patient defective or deficient in one or more cell types of the hematopoietic lineage in order to re-constitute the defective or deficient population of cells in vivo. For example, the patient may be suffering from cancer, and the deficiency may be caused by administration of a chemotherapeutic agent or other medicament that depletes, either selectively or non-specifically, the cancerous cell population. Additionally or alternatively, the patient may be suffering from a hemoglobinopathy (e.g., a non-malignant hemoglobinopathy), such as sickle cell anemia, thalassemia, Fanconi anemia, aplastic anemia, and Wiskott-Aldrich syndrome. The subject may be one that is suffering from adenosine deaminase severe combined immunodeficiency (ADA SCID), HIV/AIDS, metachromatic leukodystrophy, Diamond-Blackfan anemia, and Schwachman-Diamond syndrome. The subject may have or be affected by an inherited blood disorder (e.g., sickle cell anemia) or an autoimmune disorder. Additionally or alternatively, the subject may have or be affected by a malignancy, such as neuroblastoma or a hematologic cancer. For instance, the subject may have a leukemia, lymphoma, or myeloma. The subject may have acute myeloid leukemia, acute lymphoid leukemia, chronic myeloid leukemia, chronic lymphoid leukemia, multiple myeloma, diffuse large B-cell lymphoma, or non-Hodgkin's lymphoma. The subject may have myelodysplastic syndrome. The subject may have an autoimmune disease, such as scleroderma, multiple sclerosis, ulcerative colitis, Crohn's disease, Type 1 diabetes, or another autoimmune pathology described herein. The subject may be in need of chimeric antigen receptor T-cell (CART) therapy. In some embodiments, the subject has or is otherwise affected by a metabolic storage disorder. The subject may suffer or otherwise be affected by a metabolic disorder selected from the group consisting of glycogen storage diseases, mucopolysaccharidoses, Gaucher's Disease, Hurler syndrome or Hurler's Disease, sphingolipidoses, Mucolipidosis II, metachromatic leukodystrophy, or any other diseases or disorders which may benefit from the treatments and therapies disclosed herein and including, without limitation, severe combined immunodeficiency, Wiscott-Aldrich syndrome, hyper immunoglobulin M (IgM) syndrome, Chediak-Higashi disease, hereditary lymphohistiocytosis, osteopetrosis, osteogenesis imperfecta, storage diseases, thalassemia major, sickle cell disease, systemic sclerosis, systemic lupus erythematosus, multiple sclerosis, juvenile rheumatoid arthritis and those diseases, or disorders described in "Bone Marrow Transplantation for Non-Malignant Disease," ASH Education Book, 1 :319-338 (2000), which pertains to pathologies that may be treated by administration of hematopoietic stem cell transplant therapy. Additionally or alternatively, a patient "in need of" a hematopoietic stem cell transplant may one that is or is not suffering from one of the foregoing pathologies, but nonetheless exhibits a reduced level (e.g., as compared to that of an otherwise healthy subject) of one or more endogenous cell types within the hematopoietic lineage, such as megakaryocytes, thrombocytes, platelets, erythrocytes, mast cells, myeoblasts, basophils, neutrophils, eosinophils, microglia, granulocytes, monocytes, osteoclasts, antigen-presenting cells, macrophages, dendritic cells, natural killer cells, T-lymphocytes, and B-lymphocytes. One of skill in the art can readily determine whether one's level of one or more of the foregoing cell types, or other blood cell type, is reduced with respect to an otherwise healthy subject, for instance, by way of flow cytometry and fluorescence activated cell sorting (FACS) methods, among other procedures, known in the art.

As used herein, the terms "mobilize" and "mobilization" refer to processes by which a population of hematopoietic stem or progenitor cells is released from a stem cell niche, such as the bone marrow of a subject, into circulation in the peripheral blood. Mobilization of hematopoietic stem and progenitor cells can be monitored, for instance, by assessing the quantity or concentration of hematopoietic stem or progenitor cells in a peripheral blood sample isolated from a subject. For example, the peripheral blood sample may be withdrawn from the subject, and the quantity or concentration of hematopoietic stem or progenitor cells in the peripheral blood sample may subsequently be assessed, following the administration of a hematopoietic stem or progenitor cell mobilization regimen to the subject. The mobilization regimen may include, for instance, a CXCR4 antagonist, such as a CXCR4 antagonist described herein (e.g., plerixafor or a variant thereof), and a CXCR2 agonist, such as a CXCR2 agonist described herein (e.g., Gro-β or a variant thereof, such as a truncation of Gro-β, for instance, Gro-β T). The quantity or concentration of hematopoietic stem or progenitor cells in the peripheral blood sample isolated from the subject following administration of the mobilization regimen may be compared to the quantity or concentration of hematopoietic stem or progenitor cells in a peripheral blood sample isolated from the subject prior to administration of the mobilization regimen. An observation that the quantity or concentration of hematopoietic stem or progenitor cells has increased in the peripheral blood of the subject following administration of the mobilization regimen is an indication that the subject is responding to the mobilization regimen, and that hematopoietic stem and progenitor cells have been released from one or more stem cell niches, such as the bone marrow, into peripheral blood circulation.

As used herein, the term "sample" refers to a specimen (e.g., blood, blood component (e.g., serum or plasma), urine, saliva, amniotic fluid, cerebrospinal fluid, tissue (e.g., placental or dermal), pancreatic fluid, chorionic villus sample, and cells) taken from a subject.

As used herein, the phrase "stem cell disorder" broadly refers to any disease, disorder, or condition that may be treated or cured by engrafting or transplanting a population of hematopoietic stem or progenitor cells in a target tissue within a patient. For example, Type I diabetes has been shown to be cured by hematopoietic stem cell transplant, along with various other disorders. Diseases that can be treated by infusion of hematopoietic stem or progenitor cells into a patient include, sickle cell anemia, thalassemias, Fanconi anemia, aplastic anemia, Wiskott-Aldrich syndrome, ADA SCID, HIV/AIDS, metachromatic leukodystrophy, Diamond-Blackfan anemia, and Schwachman-Diamond syndrome. Additional diseases that may be treated by transplantation of hematopoietic stem and progenitor cells as described herein include blood disorders (e.g., sickle cell anemia) and autoimmune disorders, such as scleroderma, multiple sclerosis, ulcerative colitis, and Chrohn's disease. Additional diseases that may be treated using hematopoietic stem and progenitor cell transplant therapy include cancer, such as a cancer described herein. Stem cell disorders include a malignancy, such as a neuroblastoma or a hematologic cancers, such as leukemia, lymphoma, and myeloma. For instance, the cancer may be acute myeloid leukemia, acute lymphoid leukemia, chronic myeloid leukemia, chronic lymphoid leukemia, multiple myeloma, diffuse large B-cell lymphoma, or non-Hodgkin's lymphoma. Disorders that may be treated by transplanting a population of hematopoietic stem cells to a patient include neurological disorders, such as Parkinson's disease, Alzheimer's disease, multiple sclerosis, Amyotrophic lateral sclerosis, Huntington's disease, mild cognitive impairment, amyloidosis, AIDS-related dementia, encephalitis, stroke, head trauma, epilepsy, mood disorders, and dementia. As described herein, without being limited by mechanism, the ability of hematopoietic stem cell transplantation to treat such disorders may be due, in part, to the capacity of hematopoietic stem cells to migrate to the central nervous system and differentiate into microglial cells, thereby repopulating a hematopoietic cell line that may be damaged or deficient in patients having a neurological disorder. Additional diseases treatable using hematopoietic stem or progenitor cell transplant therapy include myelodysplastic syndrome. In some embodiments, the patient has or is otherwise affected by a metabolic storage disorder. For example, the patient may suffer or otherwise be affected by a metabolic disorder selected from the group consisting of glycogen storage diseases, mucopolysaccharidoses, Gaucher's Disease, Hurler syndrome or Hurler's Disease, sphingolipidoses, Mucolipidosis II, metachromatic leukodystrophy, or any other diseases or disorders which may benefit from the treatments and therapies disclosed herein and including, without limitation, severe combined immunodeficiency, Wiscott-Aldrich syndrome, hyper immunoglobulin M (IgM) syndrome, Chediak-Higashi disease, hereditary lymphohistiocytosis, osteopetrosis, osteogenesis imperfecta, storage diseases, thalassemia major, sickle cell disease, systemic sclerosis, systemic lupus erythematosus, multiple sclerosis, juvenile rheumatoid arthritis and those diseases, or disorders described in "Bone Marrow Transplantation for Non-Malignant Disease," ASH Education Book, 1 :319-338 (2000), which pertains to pathologies that may be treated by administration of hematopoietic stem or progenitor cell transplant therapy.

As used herein, the terms "subject" and "patient" refer to an organism, such as a human, that receives treatment for a particular disease or condition as described herein. For instance, a patient, such as a human patient, that is in need of hematopoietic stem cell transplantation may receive treatment that includes a population of hematopoietic stem cells so as to treat a stem cell disorder, such as a cancer, autoimmune disease, or metabolic disorder described herein. A patient, such as a human patient suffering from a stem cell disorder, may, for instance, receive treatment in the form of a population of hematopoietic stem cells, such as a population of from about 1 × 10⁶ to about 1 × 10⁹ hematopoietic stem cells.

As used herein, the term "recipient" refers to a patient that receives a transplant, such as a transplant containing a population of hematopoietic stem cells. The transplanted cells administered to a recipient may be, e.g., autologous, syngeneic, or allogeneic cells.

As used herein, the terms "treat", "treating" or "treatment" refer to a method of alleviating or abating a disease and/or its attendant symptoms. As used herein, the terms "preventing" or "prevent" describes reducing or eliminating the onset of the symptoms or complications of the disease, condition, or disorder. As used herein, the terms "disease(s)", "disorder(s)", and "condition(s)" are used interchangeably, unless the context clearly dictates otherwise.

"Treating" may refer to therapeutic treatment, in which the object is to prevent or slow down (lessen) an undesired physiological change or disorder or to promote a beneficial phenotype in the patient being treated. Beneficial or desired clinical results include, but are not limited to, promoting the engraftment of exogenous hematopoietic cells in a patient following hematopoietic stem or progenitor cell transplant therapy. Additional beneficial results include an increase in the cell count or relative concentration of hematopoietic stem cells in a patient in need of a hematopoietic stem or progenitor cell transplant following administration of an exogenous hematopoietic stem or progenitor cell graft to the patient. Beneficial results of therapy described herein may also include an increase in the cell count or relative concentration of one or more cells of hematopoietic lineage, such as a megakaryocyte, thrombocyte, platelet, erythrocyte, mast cell, myeoblast, basophil, neutrophil, eosinophil, microglial cell, granulocyte, monocyte, osteoclast, antigen-presenting cell, macrophage, dendritic cell, natural killer cell, T-lymphocyte, or B-lymphocyte, following and subsequent hematopoietic stem cell transplant therapy. Additional beneficial results may include the reduction in quantity of a disease-causing cell population, such as a population of cancer cells or autoimmune cells.

As used herein, the terms "variant" and "derivative" are used interchangeably and refer to naturally-occurring, synthetic, and semi-synthetic analogues of a compound, peptide, protein, or other substance described herein. A variant or derivative of a compound, peptide, protein, or other substance described herein may retain or improve upon the biological activity of the original material.

As used herein, the term "alkyl" refers to a straight- or branched-chain alkyl group having, for example, from 1 to 20 carbon atoms in the chain, or, in one embodiment, from 1 to 6 carbon atoms in the chain. Examples of alkyl groups include, but are not limited to, methyl, ethyl, n-propyl, isopropyl, butyl, isobutyl, sec-butyl, tert-butyl, pentyl, isopentyl, tert-pentyl, neopentyl, isopentyl, tert-pentyl, hexyl, isohexyl, and the like.

As used herein, the term "alkylene" refers to a straight- or branched-chain divalent alkyl group. The divalent positions may be on the same or different atoms within the alkyl chain. Examples of alkylene include methylene, ethylene, propylene, isopropylene, and the like.

As used herein, the term "heteroalkyl" refers to a straight or branched-chain alkyl group having, for example, from 1 to 20 carbon atoms in the chain, and further containing one or more heteroatoms (e.g., oxygen, nitrogen, or sulfur, among others) in the chain.

As used herein, the term "heteroalkylene" refers to a straight- or branched-chain divalent heteroalkyl group. The divalent positions may be on the same or different atoms within the heteroalkyl chain. The divalent positions may be one or more heteroatoms.

As used herein, the term "alkenyl" refers to a straight- or branched-chain alkenyl group having, for example, from 2 to 20 carbon atoms in the chain. It denotes a monovalent group derived from a hydrocarbon moiety containing, for example, from two to six carbon atoms having at least one carbon-carbon double bond. The double bond may or may not be the point of attachment to another group. hexenyl, Examples of alkenyl groups include, but are not limited to, vinyl, propenyl, isopropenyl, butenyl, tert-butylenyl, 1-methyl-2-buten-1-yl, hexenyl, and the like.

As used herein, the term "alkenylene" refers to a straight- or branched-chain divalent alkenyl group. The divalent positions may be on the same or different atoms within the alkenyl chain. Examples of alkenylene include ethenylene, propenylene, isopropenylene, butenylene, and the like.

As used herein, the term "heteroalkenyl" refers to a straight- or branched-chain alkenyl group having, for example, from 2 to 20 carbon atoms in the chain, and further containing one or more heteroatoms (e.g., oxygen, nitrogen, or sulfur, among others) in the chain.

As used herein, the term "heteroalkenylene" refers to a straight- or branched-chain divalent heteroalkenyl group. The divalent positions may be on the same or different atoms within the heteroalkenyl chain. The divalent positions may be one or more heteroatoms.

As used herein, the term "alkynyl" refers to a straight- or branched-chain alkynyl group having, for example, from 2 to 20 carbon atoms in the chain and at least one carbon-carbon triple bond. Examples of alkynyl groups include, but are not limited to, propargyl, butynyl, pentynyl, hexynyl, and the like.

As used herein, the term "alkynylene" refers to a straight- or branched-chain divalent alkynyl group. The divalent positions may be on the same or different atoms within the alkynyl chain.

As used herein, the term "heteroalkynyl" refers to a straight- or branched-chain alkynyl group having, for example, from 2 to 20 carbon atoms in the chain, and further containing one or more heteroatoms (e.g., oxygen, nitrogen, or sulfur, among others) in the chain.

As used herein, the term "heteroalkynylene" refers to a straight- or branched-chain divalent heteroalkynyl group. The divalent positions may be on the same or different atoms within the heteroalkynyl chain. The divalent positions may be one or more heteroatoms.

As used herein, the term "cycloalkyl" refers to a monocyclic, or fused, bridged, or spiro polycyclic ring structure that is saturated and has, for example, from 3 to 12 carbon ring atoms. Examples of cycloalkyl groups include cyclopropyl, cyclobutyl, cyclopentyl, cyclohexyl, cycloheptyl, cyclooctyl, bicyclo[3.1.0]hexane, and the like. Also contemplated is a monovalent group derived from a monocyclic or polycyclic carbocyclic ring compound having at least one carbon-carbon double bond by the removal of at least one or two hydrogen atoms. Examples of such groups include, but are not limited to, cyclopropenyl, cyclobutenyl, cyclopentenyl, cyclohexenyl, cycloheptenyl, cyclooctenyl, and the like. Also contemplated is a monovalent group derived from a monocyclic or polycyclic saturated or partially unsaturated carbocyclic ring compound.

As used herein, the term "cycloalkylene" refers to a divalent cycloalkyl group. The divalent positions may be on the same or different atoms within the ring structure. Examples of cycloalkylene include cyclopropylene, cyclobutylene, cyclopentylene, cyclohexylene, and the like.

As used herein, the term "heterocyloalkyl" or "heterocyclyl" refers to a monocyclic, or fused, bridged, or spiro polycyclic ring structure that is saturated and has, for example, from 3 to 12 ring atoms per ring structure selected from carbon atoms and heteroatoms selected from, e.g., nitrogen, oxygen, and sulfur, among others. The ring structure may contain, for example, one or more oxo groups on carbon, nitrogen, or sulfur ring members. Exemplary heterocycloalkyl groups include, but are not limited to, [1,3] dioxolane, pyrrolidinyl, pyrazolinyl, pyrazolidinyl, imidazolinyl, imidazolidinyl, piperazinyl, piperidinyl, oxazolidinyl, isooxazolidinyl, morpholinyl, thiazolidinyl, isothiazolidinyl, and tetrahydrofuryl.

As used herein, the term "heterocycloalkylene" refers to a divalent heterocyclolalkyl group. The divalent positions may be on the same or different atoms within the ring structure.

As used herein, the term "aryl" refers to a monocyclic or multicyclic aromatic ring system containing, for example, from 6 to 19 carbon atoms. Aryl groups include, but are not limited to, phenyl, fluorenyl, naphthyl, tetrahydronaphthyl, indanyl, indenyl, and the like. The divalent positions may be one or more heteroatoms.

As used herein, the term "arylene" refers to a divalent aryl group. The divalent positions may be on the same or different atoms.

As used herein, the term "heteroaryl" refers to a monocyclic heteroaromatic, or a bicyclic or a tricyclic fused-ring heteroaromatic group. In one embodiment, the heteroaryl group contains five to ten ring atoms of which one ring atom is selected from S, O, and N; zero, one, or two ring atoms are additional heteroatoms independently selected from S, O, and N; and the remaining ring atoms are carbon. Heteroaryl groups include, but are not limited to, pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl, benzoquinolyl, and the like.

As used herein, the term "heteroarylene" refers to a divalent heteroaryl group. The divalent positions may be on the same or different atoms. The divalent positions may be one or more heteroatoms.

Unless otherwise constrained by the definition of the individual substituent, the foregoing chemical moieties, such as "alkyl", "alkylene", "heteroalkyl", "heteroalkylene", "alkenyl", "alkenylene", "heteroalkenyl", "heteroalkenylene", "alkynyl", "alkynylene", "heteroalkynyl", "heteroalkynylene", "cycloalkyl", "cycloalkylene", "heterocyclolalkyl", heterocycloalkylene", "aryl," "arylene", "heteroaryl", and "heteroarylene" groups can optionally be substituted. As used herein, the term "optionally substituted" refers to a compound or moiety containing one or more (for example, 1, 2, 3, 4, 5, 6, 7, 8, 9, 10, or more) substituents, as permitted by the valence of the compound or moiety or a site thereof, such as a substituent selected from the group consisting of alkyl, alkenyl, alkynyl, cycloalkyl, heterocycloalkyl, alkyl aryl, alkyl heteroaryl, alkyl cycloalkyl, alkyl heterocycloalkyl, amino, ammonium, acyl, acyloxy, acylamino, aminocarbonyl, alkoxycarbonyl, ureido, carbamate, aryl, heteroaryl, sulfinyl, sulfonyl, alkoxy, sulfanyl, halogen, carboxy, trihalomethyl, cyano, hydroxy, mercapto, nitro, and the like. The substitution may include situations in which neighboring substituents have undergone ring closure, such as ring closure of vicinal functional substituents, to form, for instance, lactams, lactones, cyclic anhydrides, acetals, hemiacetals, thioacetals, aminals, and hemiaminals, formed by ring closure, for example, to furnish a protecting group.

As used herein, the term "optionally substituted" refers to a chemical moiety that may have one or more chemical substituents, as valency permits, such as C1-4 alkyl, C2-4 alkenyl, C2-4 alkynyl, C3-10 cycloalkyl, C3-10 heterocycloalkyl, aryl, alkylaryl, heteroaryl, alkylheteroaryl, amino, ammonium, acyl, acyloxy, acylamino, aminocarbonyl, alkoxycarbonyl, ureido, carbamate, sulfinyl, sulfonyl, alkoxy, sulfanyl, halogen, carboxy, trihalomethyl, cyano, hydroxy, mercapto, nitro, and the like. An optionally substituted chemical moiety may contain, e.g., neighboring substituents that have undergone ring closure, such as ring closure of vicinal functional substituents, thus forming, e.g., lactams, lactones, cyclic anhydrides, acetals, thioacetals, or aminals formed by ring closure, for instance, in order to generate protecting group.

In accordance with the application, any of the aryls, substituted aryls, heteroaryls and substituted heteroaryls described herein, can be any aromatic group.

The terms "hal," "halo," and "halogen," as used herein, refer to an atom selected from fluorine, chlorine, bromine and iodine.

As described herein, compounds of the application and moieties present in the compounds may optionally be substituted with one or more substituents, such as are illustrated generally above, or as exemplified by particular classes, subclasses, and species of the application. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. The terms "optionally substituted", "optionally substituted alkyl," "optionally substituted "optionally substituted alkenyl," "optionally substituted alkynyl", "optionally substituted cycloalkyl," "optionally substituted cycloalkenyl," "optionally substituted aryl", "optionally substituted heteroaryl," "optionally substituted aralkyl", "optionally substituted heteroaralkyl," "optionally substituted heterocycloalkyl," and any other optionally substituted group as used herein, refer to groups that are substituted or unsubstituted by independent replacement of one, two, or three or more of the hydrogen atoms thereon with substituents including, but not limited to:
-F, -Cl, -Br, -I, -OH, protected hydroxy, -NO₂, -CN, -NH₂, protected amino, -NH-C₁-C₁₂-alkyl,-NH-C₂-C₁₂-alkenyl, -NH-C₂-C₁₂-alkenyl, -NH -C₃-C₁₂-cycloalkyl, -NH-aryl, -NH -heteroaryl, -NH -heterocycloalkyl, -dialkylamino, -diarylamino, -diheteroarylamino, -O-C₁-C₂-alkyl, -O-C₂-C₂-alkenyl, -O-C₂-C₂-alkenyl, -O-C3-C₁₂-cycloalkyl, -O-aryl, -O-heteroaryl, -O-heterocycloalkyl, -C(O)-C₁-C₁₂-alkyl, -C(O)- C₂-C₁₂-alkenyl, -C(O)-C₂-C₁₂-alkenyl, -C(O)-C₃-C₂-cycloalkyl, -C(O)-aryl, -C(O)-heteroaryl, -C(O)-heterocycloalkyl, -CONH₂, -CONH-C₁-C₁₂-alkyl, -CONH-C₂-C₁₂-alkenyl, -CONH-C₂-C₁₂-alkenyl, -CONH-C₃-C₁₂-cycloalkyl, -CONH-aryl, -CONH-heteroaryl, -CONH-heterocycloalkyl,-OCO₂-C₁-C₁₂-alkyl, -OCO₂-C₂-C₁₂-alkenyl, -OCO₂-C₂-C₁₂-alkenyl, -OCO₂-C₃-C₁₂-cycloalkyl, -OCO₂-aryl, -OCO₂-heteroaryl, -OCO₂-heterocycloalkyl, -OCONH₂, -OCONH-C₁-C₁₂-alkyl, -OCONH- C₂-C₁₂-alkenyl, -OCONH- C₂-C₁₂-alkenyl, -OCONH-C₃-C₁₂-cycloalkyl, -OCONH-aryl, -OCONH-heteroaryl, -OCONH-heterocycloalkyl, -NHC(O)-C₁-C₁₂-alkyl, -NHC(O)-C₂-C₁₂-alkenyl, -NHC(O)-C₂-C₁₂-alkenyl, -NHC(O)-C₃-C₁₂-cycloalkyl, -NHC(O)-aryl, -NHC(O)-heteroaryl, -NHC(O)-heterocycloalkyl, -NHCO₂-C₁-C₁₂-alkyl, -NHCO₂-C₂-C₁₂-alkenyl, -NHCO₂-C₂-C₁₂-alkenyl, -NHCO₂-C₃-C₁₂-cycloalkyl, -NHCO₂-aryl, -NHCO₂-heteroaryl, -NHCO₂- heterocycloalkyl, NHC(O)NH₂, -NHC(O)NH-C₁-C₁₂-alkyl, -NHC(O)NH-C₂-C₁₂-alkenyl, -NHC(O)NH-C₂-C₁₂-alkenyl, -NHC(O)NH-C₃-C₁₂-cycloalkyl, -NHC(O)NH-aryl, -NHC(O)NH-heteroaryl, NHC(O)NH-heterocycloalkyl, -NHC(S)NH₂, -NHC(S)NH-C₁-C₁₂-alkyl, -NHC(S)NH-C₂-C₁₂-alkenyl, -NHC(S)NH-C₂-C₁₂-alkenyl, -NHC(S)NH-C₃-C₁₂-cycloalkyl, -NHC(S)NH-aryl, -NHC(S)NH-heteroaryl, -NHC(S)NH-heterocycloalkyl, -NHC(NH)NH₂, -NHC(NH)NH- C₁-C₁₂-alkyl, -NHC(NH)NH-C₂-C₁₂-alkenyl, -NHC(NH)NH-C₂-C₁₂-alkenyl, -NHC(NH)NH-C₃-C₁₂-cycloalkyl, -NHC(NH)NH-aryl, -NHC(NH)NH-heteroaryl, -NHC(NH)NHheterocycloalkyl, -NHC(NH)-C₁-C₁₂-alkyl, -NHC(NH)-C₂-C₁₂-alkenyl, -NHC(NH)-C₂-C₁₂-alkenyl, -NHC(NH)-C₃-C₁₂-cycloalkyl, -NHC(NH)-aryl, -NHC(NH)-heteroaryl, -NHC(NH)-heterocycloalkyl, -C(NH)NH-C₁-C₁₂-alkyl, -C(NH)NH-C₂-C₁₂-alkenyl, -C(NH)NH-C₂-C₁₂-alkenyl, C(NH)NH-C₃-C₁₂-cycloalkyl, -C(NH)NH-aryl, -C(NH)NH-heteroaryl, -C(NH)NHheterocycloalkyl, -S(O)-C₁-C₁₂-alkyl,- S(O)-C₂-C₁₂-alkenyl,- S(O)-C₂-C₁₂-alkenyl, -S(O)-C₃-C₁₂-cycloalkyl,- S(O)-aryl, -S(O)-heteroaryl, -S(O)-heterocycloalkyl -SO₂NH₂, -SO₂NH-C₁-C₁₂-alkyl, -SO₂NH-C₂-C₁₂-alkenyl, -SO₂NH-C₂-C₁₂-alkenyl, -SO₂NH-C₃-C₁₂-cycloalkyl, -SO₂NH-aryl, -SO₂NH-heteroaryl, -SO₂NH-heterocycloalkyl, -NHSO₂-C₁-C₁₂-alkyl, -NHSO₂-C₂-C₁₂-alkenyl,- NHSO₂-C₂-C₁₂-alkenyl, -NHSO₂-C₃-C₁₂-cycloalkyl, -NHSO₂-aryl, -NHSO₂-heteroaryl, -NHSO₂-heterocycloalkyl, -CH₂NH₂, -CH₂SO₂CH₃, -aryl, -arylalkyl, -heteroaryl, -heteroarylalkyl, -heterocycloalkyl, -C₃-C₁₂-cycloalkyl, polyalkoxyalkyl, polyalkoxy, -methoxymethoxy, -methoxyethoxy, -SH, -S-C₁-C₁₂-alkyl, -S-C₂-C₁₂-alkenyl, -S-C₂-C₁₂-alkenyl, -S-C₃-C₁₂-cycloalkyl, -S-aryl, -S-heteroaryl, -S-heterocycloalkyl, or methylthiomethyl.

Where the number of any given substituent is not specified, there may be one or more substituents present. For example, "halo-substituted C1-4 alkyl" may include one or more of the same or different halogens.

When the compounds described herein contain olefinic double bonds or other centers of geometric asymmetry, and unless specified otherwise, it is intended that the compounds include both *E* and *Z* geometric isomers. Likewise, all tautomeric forms of carbonyl-containing compounds are also intended to be included.

It is to be understood that the compounds provided herein may contain chiral centers. Such chiral centers may be of either the (*R*) or (*S*) configuration, or may be a mixture thereof. Thus, the compounds provided herein may be enantiomerically pure, or may be stereoisomeric or diastereomeric mixtures. As such, one of skill in the art will recognize that administration of a compound in its (*R*) form is equivalent, for compounds that undergo epimerization *in vivo,* to administration of the compound in its (*S*) form.

Compounds described herein include, but are not limited to, those set forth above, as well as any of their isomers, such as diastereomers and enantiomers, as well as salts, esters, amides, thioesters, solvates, and polymorphs thereof, as well as racemic mixtures and pure isomers of the compounds set forth above.

### Aryl Hydrocarbon Receptor Antagonists

Prior to infusion into a patient, hematopoietic and progenitor cells may be expanded ex vivo, for example, by contacting the cells with an aryl hydrocarbon receptor antagonist. Aryl hydrocarbon receptor antagonists useful in conjunction with the compositions and methods described herein include those described in US Patent No. 9,580,426.

In some embodiments, aryl hydrocarbon receptor antagonists include those represented by formula **(III)** 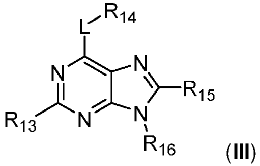 in which:
L is selected from -NR₁₇ₐ(CH₂)₂₋₃, -NR₁₇ₐ(CH₂)₂NR_{17b}-, -NR₁₇ₐ(CH₂)₂S-,-NR₁₇ₐCH₂CH(OH)- and -NR₁₇ₐCH(CH₃)CH₂-; wherein R₁₇ₐ and R_{17b} are independently selected from hydrogen and C₁₋₄alkyl;
R₁₃ is selected from thiophenyl, 1H-benzoimidazolyl, isoquinolinyl, 1H-imidazopyridinyl, benzothiophenyl, pyrimidinyl, pyridinyl, pyrazinyl, pyridazinyl, and thiazolyl; In some embodiments, wherein the thiophenyl, 1H-benzoimidazolyl, isoquinolinyl, 1H-imidazopyridinyl, benzothiophenyl, pyrimidinyl, pyridinyl, pyrazinyl, pyridazinyl, or thiazolyl of R₁₃ can be optionally substituted by 1 to 3 radicals independently selected from cyano, hydroxy, C₁₋₄ alkyl, C₁₋₄ alkoxy, halo, halo-substituted-C₁₋₄ alkyl, halo-substituted-C₁₋₄alkoxy, amino, -C(O)R₂₀ₐ, -S(O)₀₋₂R₂₀ₐ, -C(O)OR₂₀ₐ and-C(O)NR₂₀ₐR_{20b}; wherein R₂₀ₐ and R_{20b} are independently selected from hydrogen and C₁₋₄alkyl;
R₁₄ is selected from -S(O)₂NR₁₈ₐR_{18b}, -NR₁₈ₐC(O)R_{18b}-, -NR₁₈ₐC(O)NR_{18b}R_{18c}, phenyl, 1H-pyrrolopyridin-3-yl, 1H-pyrrolopyridin-5-yl, 1H-indolyl thiophenyl, pyridinyl, 1H-1,2,4-triazolyl, 2-oxoimidazolidinyl, 1H-pyrazolyl, 2-oxo-2,3-dihydro-1H-benzoimidazolyl and 1H-indazolyl; wherein R₁₈ₐ, R_{18b} and R_{18c} are independently selected from hydrogen and C₁₋₄alkyl; and the phenyl, 1H-pyrrolopyridin-3-yl, 1H-pyrrolo[2,3-b]pyridin-5-yl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl or 1H-indazolyl of R₁₄ is optionally substituted with 1 to 3 radicals independently selected from hydroxy, halo, methyl, methoxy, amino, -O(CH₂)₂NR₁₉ₐR_{19b}, -S(O)₂NR₁₉ₐR_{19b}, -OS(O)₂NR₁₉ₐR_{19b}and -NR₁₉ₐS(O)₂R_{19b}; wherein R₁₉ₐ and R_{19b} are independently selected from hydrogen and C₁₋₄ alkyl;
R₁₅ is selected from hydrogen, C₁₋₄alkyl and biphenyl; and
R₁₆ is selected from C₁₋₁₀ alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, and benzyl, (4-pentylphenyl)(phenyl)methyl and 1-(1-(2-oxo-6,9,12-trioxa-3-azatetradecan-14-yl)-1H-1,2,3-triazol-4-yl)ethyl wherein said alkyl, cyclopropyl, cyclohexyl, 2-(2-oxopyrrolidin-1-yl)ethyl, oxetan-3-yl, oxetan-2-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl or 1-(1-(2-oxo-6,9,12-trioxa-3-azatetradecan-14-yl)-1H-1,2,3-triazol-4-yl)ethyl can be optionally substituted with 1 to 3 radicals independently selected from hydroxy, C₁₋₄alkyl and halo-substituted-C₁₋₄alkyl; or a salt thereof.

In some embodiments, aryl hydrocarbon receptor antagonists useful in conjunction with the compositions and methods described herein include SR-1, represented by formula (1), below.

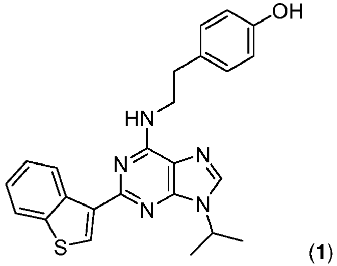

In some embodiments, aryl hydrocarbon receptor antagonists useful in conjunction with the compositions and methods described herein include Compound 2, represented by formula (2), below.

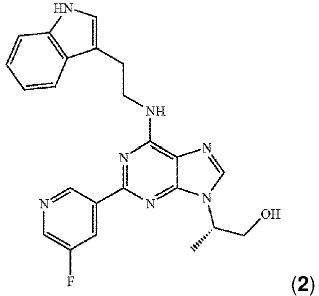

In some embodiments, aryl hydrocarbon receptor antagonists useful in conjunction with the compositions and methods described herein include Compound 2-ent, represented by formula **(2-ent),** below.

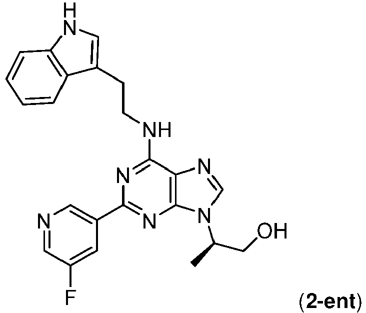

In some embodiments, aryl hydrocarbon receptor antagonists useful in conjunction with the compositions and methods described herein include Compound 2-rac, represented by formula **(2-rac),** below.

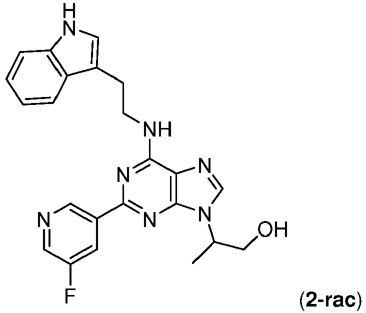

In some embodiments, aryl hydrocarbon receptor antagonists include those represented by formula **(IV)** 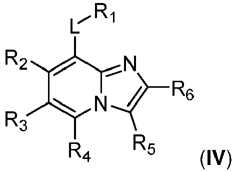
wherein L is a linker selected from the group consisting of -NR₇ₐ(CR₈ₐR_{8b})ₙ-, -O(CR₈ₐR_{8b})ₙ-,-C(O)(CR₈ₐR_{8b})ₙ-, -C(S)(CR₈ₐR_{8b})ₙ-, -S(O)₀₋₂₍CR₈ₐR_{8b})ₙ-, -(CR₈ₐR_{8b})ₙ-, -NR₇ₐC(O)(CR₈ₐR_{8b})ₙ-,-NR₇ₐC(S)(CR₈ₐR_{8b})ₙ-, -OC(O)(CR₈ₐR_{8b})ₙ-, -OC(S)(CR₈ₐR_{8b})n-, -C(O)NR₇ₐ(CR₈ₐR_{8b})ₙ-,-C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-,-NR₇ₐS(O)₂(CR₈ₐR_{8b})ₙ-, -NR₇ₐC(O)NR_{7b}(CR₈ₐR_{8b})ₙ-, -NR₇ₐ(CR₈ₐR_{8b})ₙNR₇ₐ-, -NR₇ₐ(CR₈ₐR_{8b})ₙO-,-NR₇ₐ(CR₈ₐR_{8b})ₙS-, -O(CR₈ₐR_{8b})ₙNR₇ₐ-, -O(CR₈ₐR_{8b})ₙO-, -O(CR₈ₐR_{8b})ₙS-, -S(CR₈ₐR_{8b})ₙNR₇ₐ-,-S(CR₈ₐR_{8b})ₙO-, -S(CR₈ₐR_{8b})ₙS-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, -NR₉ₐC(S)R_{9b},-NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b},-C(S)NR₉ₐR_{9b}, -NR₉ₐS(O)₂R_{9b}, -NR₉ₐC(O)OR_{9b}, -OC(O)CR₉ₐR_{9b}R_{9c}, -OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
R₂ is selected from the group consisting of hydrogen and optionally substituted C1-4 alkyl;
R₃ is selected from the group consisting of optionally substituted aryl, optionally substituted heteroaryl, optionally substituted cycloalkyl, and optionally substituted heterocycloalkyl;
R₄ is selected from the group consisting of hydrogen and optionally substituted C1-4 alkyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

As used herein to describe linkers (represented by "L" in formulas **(IV), (V),** and the like), the notation "- (Linker) -" (wherein "linker" is represented using chemical symbols such as NR₇ₐ(CR₈ₐR_{8b})ₙ, O(CR₈ₐR_{8b})ₙ, C(O)(CR₈ₐR_{8b})ₙ, C(S)(CR₈ₐR_{8b})ₙ, S(O)₀₋₂(CR₈ₐR_{8b})ₙ, (CR₈ₐR_{8b})ₙ, -NR₇ₐC(O)(CR₈ₐR_{8b})ₙ, NR₇ₐC(S)(CR₈ₐR_{8b})ₙ, OC(O)(CR₈ₐR_{8b})ₙ, OC(S)(CR₈ₐR_{8b})ₙ, C(O)NR₇ₐ(CR₈ₐR_{8b})ₙ, C(S)NR₇ₐ(CR₈ₐR_{8b})ₙ, C(O)O(CR₈ₐR_{8b})ₙ, C(S)O(CR₈ₐR_{8b})n, S(O)₂NR₇ₐ(CR₈ₐR_{8b})ₙ, NR₇ₐS(O)₂(CR₈aR_{8b})ₙ, and NR₇ₐC(O)NR_{7b}(CR₈ₐR_{8b})ₙ) designates that the left hyphen represents a covalent bond to the indicated position on the imidazopyridine or imidazopyrazine ring system, while the right hyphen represents a covalent bond to R₁.

In some embodiments, R₁ is selected from the group consisting of -S(O)₂NR₉ₐR_{9b},-NR₉ₐC(O)R_{9b}, -NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ,-C(S)OR₉ₐ, -C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c},-OC(S)CR₉ₐR_{9b}R_{9c}, phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1 H-benzoimidazolyl, or 1H-indazolyl is optionally substituted, for example, with from 1 to 3 substituents independently selected from the group consisting of cyano, hydroxy, C₁₋₄ alkyl, C₁₋₄ alkoxy, halo, halo-substituted-C1-4 alkyl, halo-substituted-C₁₋₄ alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl.

In some embodiments, R₁ is selected from the group consisting of -S(O)₂NR₉ₐR_{9b},-NR₉ₐC(O)R_{9b}, -NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ,-C(S)OR₉ₐ, -C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, and-OC(S)CR₉ₐR_{9b}R_{9c}.

In some embodiments, R₁ is selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted, for example, with from 1 to 3 substituents independently selected from the group consisting of cyano, hydroxy, C1-4 alkyl, C₁₋₄ alkoxy, halo, halo-substituted-C1-4 alkyl, halo-substituted-C₁₋₄ alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}.

In some embodiments, R₁ is selected from the group consisting of phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl, wherein the phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, or 2-oxo-2,3-dihydro-1H-benzo[d]imidazol-5-yl is optionally substituted, for example, with from 1 to 3 substituents independently selected from the group consisting of cyano, hydroxy, C₁₋₄ alkyl, C₁₋₄ alkoxy, halo, halo-substituted-C1-4 alkyl, halo-substituted-C₁₋₄ alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}.

In some embodiments, R₁ is selected from the group consisting of phenyl, phenol-4-yl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl.

In some embodiments, R₁ is selected from the group consisting of:

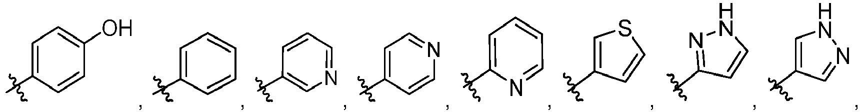

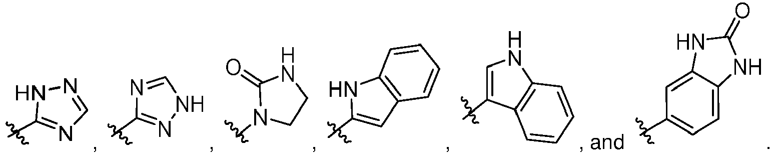

In some embodiments, R₁ is selected from the group consisting of:

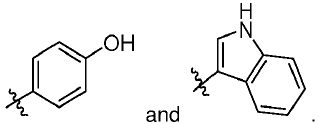

In some embodiments, R₁ is selected from the group consisting of phenol-4-yl and 1H-indol-3-yl.

In some embodiments, L is selected from the group consisting of -NR₇ₐ(CR₈ₐR_{8b})ₙ- and-O(CR₈ₐR_{8b})ₙ-.

In some embodiments, L is selected from the group consisting of -NH(CH₂)₂- and -O(CH₂)₂-. In some embodiments, R₂ is hydrogen.

In some embodiments, R₃ is selected from the group consisting of optionally substituted aryl and optionally substituted heteroaryl.

In some embodiments, R₃ is selected from the group consisting of phenyl, thiophenyl, furanyl, 1H-benzoimidazolyl, quinolinyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl, wherein the phenyl, thiophenyl, furanyl, 1H-benzoimidazolyl, quinolinyl, isoquinolinyl, imidazopyridinyl, benzothiophenyl, pyrimidinyl, pyridinyl, 1H-imidazolyl, pyrazinyl, pyridazinyl, 1H-pyrrolyl, or thiazolyl is optionally substituted, for example, with from 1 to 3 substituents independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and-C(O)NR₁₁ₐR_{11b}, and wherein R₁₁ₐ and R_{11b} are each independently selected from the group consisting of hydrogen and C₁₋₄ alkyl.

In some embodiments, R₃ is selected from the group consisting of thiophen-2-yl, thiophen-3-yl, furan-3-yl, 1H-benzo[d]imidazol-1-yl, isoquinolin-4-yl, 1H-imidazo[4,5-b]pyridin-1-yl, imidazo[1,2-a]pyridin-3-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl and thiazol-5-yl, wherein the thiophen-2-yl, thiophen-3-yl, furan-3-yl, 1H-benzo[d]imidazol-1-yl, isoquinolin-4-yl, 1H-imidazo[4,5-b]pyridin-1-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl, or thiazol-5-yl is optionally substituted, for example, with from 1 to 3 substituents independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}.

In some embodiments, R₃ is selected from the group consisting of thiophen-3-yl, benzo[b]thiophen-3-yl, pyridin-3-yl, pyrimidin-5-yl, 1H-imidazol-1-yl, 1H-benzo[d]imidazol-1-yl, isoquinolin-4-yl, 1H-imidazo[4,5-b]pyridin-1-yl, and imidazo[1,2-a]pyridin-3-yl, wherein the thiophen-3-yl, benzo[b]thiophen-3-yl, pyridin-3-yl, pyrimidin-5-yl, 1H-imidazol-1-yl, 1H-benzo[d]imidazol-1-yl, isoquinolin-4-yl, 1H-imidazo[4,5-b]pyridin-1-yl, or imidazo[1,2-a]pyridin-3-yl is optionally substituted, for example, with from 1 to 3 substituents independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and-C(O)NR₁₁ₐR_{11b}.

In some embodiments, R₃ is selected from the group consisting of optionally substituted:

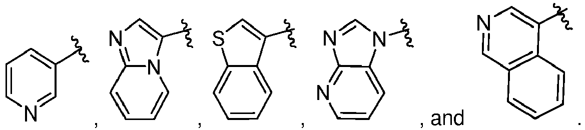

In some embodiments, R₃ is pyridin-3-yl, wherein the pyridin-3-yl is optionally substituted at C5, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, the pyridin-3-yl is substituted at C5 with a substituent selected from the group consisting of ethoxycarbonyl, methoxy, cyano, methyl, methylsulfonyl, fluoro, chloro, trifluoromethyl, ethynyl, and cyclopropyl.

In some embodiments, R₃ is selected from the group consisting of: 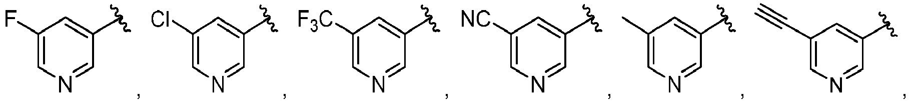 and

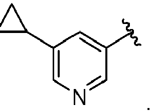

In some embodiments, R₃ is imidazo[1,2-a]pyridin-3-yl, wherein the imidazo[1,2-a]pyridin-3-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₃ is benzo[b]thiophen-3-yl, wherein the benzo[b]thiophen-3-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₃ is 1H-imidazo[4,5-b]pyridin-1-yl, wherein the 1H-imidazo[4,5-b]pyridin-1-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₃ is isoquinolin-4-yl, wherein the isoquinolin-4-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₄ is hydrogen.

In some embodiments, Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1 ,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted, for example, with from 1 to 3 substituents independently selected from the group consisting of hydroxy, C1-4 alkyl, and halo-substituted-C1-4alkyl.

In some embodiments, Rs is selected from the group consisting of isopropyl, methyl, ethyl, prop-1-en-2-yl, isobutyl, cyclohexyl, sec-butyl, (S)-sec-butyl, (R)-sec-butyl, 1-hydroxypropan-2-yl, (S)-1-hydroxypropan-2-yl, (R)-1-hydroxypropan-2-yl, and nonan-2-yl.

In some embodiments, Rs is (S)-1-hydroxypropan-2-yl.

In some embodiments, Rs is (R)-1-hydroxypropan-2-yl

In some embodiments, Rs is (S)-sec-butyl.

In some embodiments, Rs is (R)-sec-butyl.

In some embodiments, Rs is selected from the group consisting of **(i), (ii), (iii), (iv),** and **(v)** 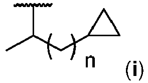 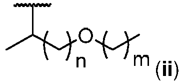 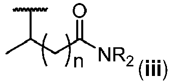 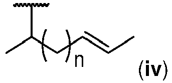 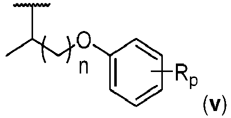 wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl.

In some embodiments, Rs is selected from the group consisting of: 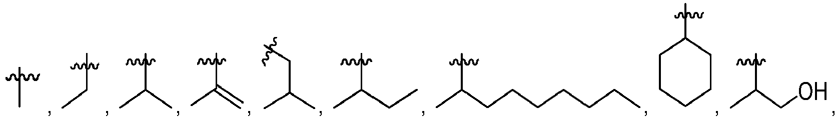 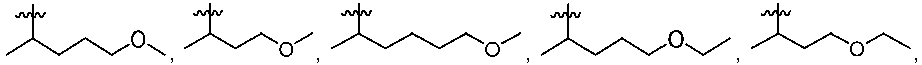 and

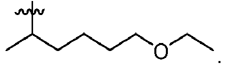

In some embodiments, Rs is **(ii).**

In some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl.

In some embodiments, Rs is (S)-4-methoxybutan-2-yl.

In some embodiments, Rs is (R)-4-methoxybutan-2-yl.

In some embodiments, Rs is (S)-5-methoxypentan-2-yl.

In some embodiments, Rs is (R)-5-methoxypentan-2-yl.

In some embodiments, Rs is (S)-4-ethoxybutan-2-yl.

In some embodiments, Rs is (R)-4-ethoxybutan-2-yl.

In some embodiments, R₆ is hydrogen.

In some embodiments, the disclosure features a compound represented by formula **(IV-a)** 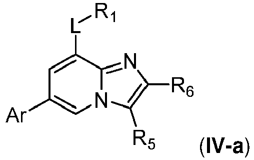
wherein L is a linker selected from the group consisting of -NR₇ₐ(CR₈ₐR_{8b})ₙ-, -O(CR₈ₐR_{8b})ₙ-,-C(O)(CR₈ₐR_{8b})ₙ-, -C(S)(CR₈ₐR_{8b})ₙ-, -S(O)₀₋₂₍CR₈ₐR_{8b})ₙ-, -(CR₈ₐR_{8b})ₙ-, -NR₇ₐC(O)(CR₈ₐR_{8b})ₙ-,-NR₇ₐC(S)(CR₈ₐR_{8b})ₙ-, -OC(O)(CR₈ₐR_{8b})ₙ-, -OC(S)(CR₈ₐR_{8b})n-, -C(O)NR₇ₐ(CR₈ₐR_{8b})ₙ-,-C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-,-NR₇ₐS(O)₂(CR₈ₐR_{8b})ₙ-, -NR₇ₐC(O)NR_{7b}(CR₈ₐR_{8b})ₙ-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, -NR₉ₐC(S)R_{9b},-NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b},-C(S)NR₉ₐR_{9b}, -NR₉ₐS(O)₂R_{9b}, -NR₉ₐC(O)OR_{9b}, -OC(O)CR₉ₐR_{9b}R_{9c}, -OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl (for example, R₁ may be selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1 H-benzoimidazolyl, or 1H-indazolyl is optionally substituted, for example, with from 1 to 3 substituents independently selected from the group consisting of cyano, hydroxy, C₁₋₄ alkyl, C₁₋₄ alkoxy, halo, halo-substituted-C1-4 alkyl, halo-substituted-C₁₋₄ alkoxy, amino, -O(CH₂)₂NR₁₀ₐR_{10b},-S(O)₂NR₁₀ₐR_{10b}, -OS(O)₂NR₁₀aR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl);
Ar is selected from the group consisting of optionally substituted monocyclic aryl and heteroaryl, such as optionally substituted thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, Ar is pyridin-3-yl, wherein the pyridin-3-yl is optionally substituted at C5, for example, with a substituent selected from the group consisting of ethoxycarbonyl, methoxy, cyano, methyl, methylsulfonyl, fluoro, chloro, trifluoromethyl, ethynyl, and cyclopropyl.

In some embodiments, the disclosure features a compound represented by formula **(IV-b)** 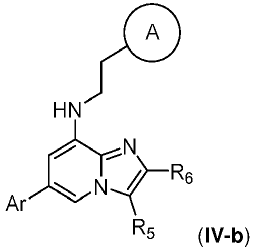
wherein A is an optionally substituted ring system selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted with from 1 to 3 substituents independently selected from the group consisting of cyano, hydroxy, C₁₋₄ alkyl, C₁₋₄ alkoxy, halo, halo-substituted-C1-4 alkyl, halo-substituted-C1-4 alkoxy, amino, -O(CH₂)₂NR₁₀ₐR_{10b},-S(O)₂NR₁₀ₐR_{10b}, -OS(O)₂NR₁₀aR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
Ar is selected from the group consisting of optionally substituted monocyclic aryl and heteroaryl, such as optionally substituted thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, A is selected from the group consisting of phenyl, phenol-4-yl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]im idazol-5-yl.

In some embodiments, A is selected from the group consisting of phenol-4-yl and 1H-indol-3-yl.

In some embodiments, the disclosure features a compound represented by formula **(IV-c)** 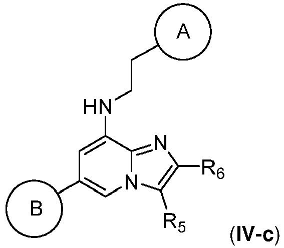
wherein A is an optionally substituted ring system selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted with from 1 to 3 substituents independently selected from the group consisting of cyano, hydroxy, C₁₋₄ alkyl, C₁₋₄ alkoxy, halo, halo-substituted-C1-4 alkyl, halo-substituted-C1-4 alkoxy, amino, -O(CH₂)₂NR₁₀ₐR_{10b},-S(O)₂NR₁₀ₐR_{10b}, -OS(O)₂NR₁₀aR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
B is an optionally substituted ring system selected from the group consisting of thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl, wherein the thiophenyl, furanyl, 1H-benzoimidazolyl, isoquinolinyl, 1H-imidazopyridinyl, benzothiophenyl, pyrimidinyl, pyridinyl, 1H-imidazolyl, pyrazinyl, pyridazinyl, 1H-pyrrolyl, or thiazolyl is optionally substituted with from 1 to 3 substituents independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}, wherein R₁₁ₐand R_{11b} are each independently selected from the group consisting of hydrogen and C₁₋₄ alkyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, B is pyridin-3-yl, wherein the pyridin-3-yl is optionally substituted at C5, for example, with a substituent selected from the group consisting of ethoxycarbonyl, methoxy, cyano, methyl, methylsulfonyl, fluoro, chloro, trifluoromethyl, ethynyl, and cyclopropyl.

In some embodiments, the disclosure features a compound represented by formula **(IV-d)** 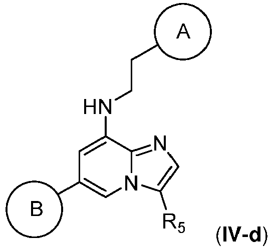
wherein A is an optionally substituted ring system selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted with from 1 to 3 substituents independently selected from the group consisting of cyano, hydroxy, C₁₋₄ alkyl, C₁₋₄ alkoxy, halo, halo-substituted-C1-4 alkyl, halo-substituted-C1-4 alkoxy, amino, -O(CH₂)₂NR₁₀ₐR_{10b},-S(O)₂NR₁₀ₐR_{10b}, -OS(O)₂NR₁₀aR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
B is an optionally substituted ring system selected from the group consisting of thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl, wherein the thiophenyl, furanyl, 1H-benzoimidazolyl, isoquinolinyl, 1H-imidazopyridinyl, benzothiophenyl, pyrimidinyl, pyridinyl, 1H-imidazolyl, pyrazinyl, pyridazinyl, 1H-pyrrolyl, or thiazolyl is optionally substituted with from 1 to 3 substituents independently selected from the group consisting of cyano, hydroxy, C1-4 alkyl, C2-4 alkenyl, C2-4 alkynyl, C3-6 cycloalkyl, C₁₋₄alkoxy, halo, halo-substituted-C1-4 alkyl, halo-substituted-C1-4 alkoxy, amino, -C(O)Rna, -S(O)₀₋₂R₁₁ₐ, -C(O)ORna, and -C(O)NR₁₁ₐR_{11b}, wherein R₁₁ₐ and R_{11b} are each independently selected from the group consisting of hydrogen and C₁₋₄ alkyl; and
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, the disclosure features a compound represented by formula **(IV-e)** 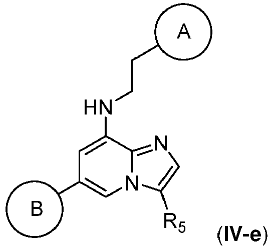
wherein A is an optionally substituted ring system selected from the group consisting of phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl, wherein the phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, or 2-oxo-2,3-dihydro-1H-benzo[d]imidazol-5-yl is optionally substituted with from 1 to 3 substituents independently selected from the group consisting of cyano, hydroxy, C₁₋₄ alkyl, C₁₋₄ alkoxy, halo, halo-substituted-C1-4 alkyl, halo-substituted-C₁₋₄ alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
B is an optionally substituted ring system selected from the group consisting of thiophen-2-yl, thiophen-3-yl, furan-3-yl, 1H-benzo[d]imidazol-1-yl, isoquinolin-4-yl, 1H-imidazo[4,5-b]pyridin-1-yl, imidazo[1,2-a]pyridin-3-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl and thiazol-5-yl, wherein the thiophen-2-yl, thiophen-3-yl, furan-3-yl, 1H-benzo[d]imidazol-1-yl, isoquinolin-4-yl, 1H-imidazo[4,5-b]pyridin-1-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl, or thiazol-5-yl is optionally substituted with from 1 to 3 substituents independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (i), (ii), (iii), (iv), and (v) 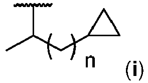 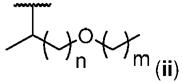 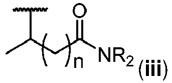 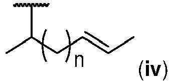 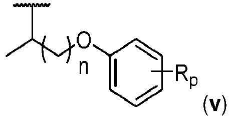
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 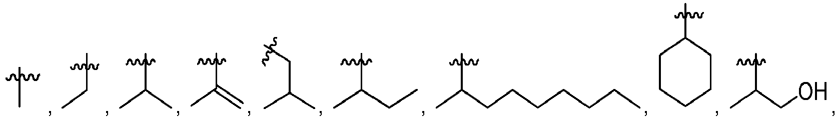 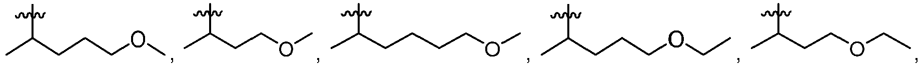 and 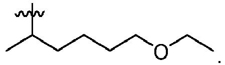
in some embodiments, Rs is **(ii);**
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;or a salt thereof.
In some embodiments, the disclosure features a compound represented by formula **(IV-f)** 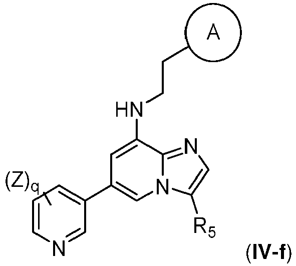
   wherein A is an optionally substituted ring system selected from the group consisting of phenol-4-yl and 1H-indol-3-yl;
   q is an integer from 0 to 4;
   each Z is independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
   Rs is selected from the group consisting of isopropyl, methyl, ethyl, prop-1-en-2-yl, isobutyl, cyclohexyl, sec-butyl, (S)-sec-butyl, (R)-sec-butyl, 1-hydroxypropan-2-yl, (S)-1-hydroxypropan-2-yl, (R)-1-hydroxypropan-2-yl, and nonan-2-yl, or Rs is selected from the group consisting of **(i), (ii), (iii), (iv),** and **(v)** 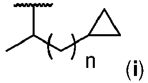 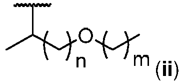 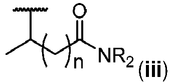 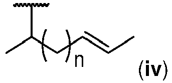 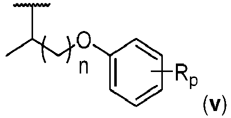
   wherein n is an integer from 1 to 6, m is an integer from 0 to 6, p is an integer from 0 to 5, and each R is independently selected from the group consisting of cyano, hydroxy, C₁₋₄ alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 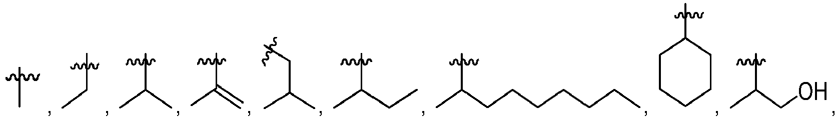 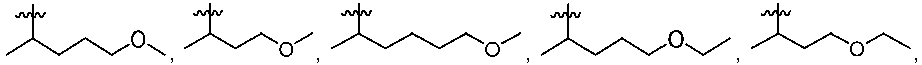 and 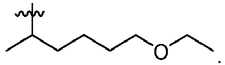
in some embodiments, Rs is (ii);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, each Z is independently a substituent selected from the group consisting of ethoxycarbonyl, methoxy, cyano, methyl, methylsulfonyl, fluoro, chloro, trifluoromethyl, ethynyl, and cyclopropyl.

In some embodiments, the disclosure features a compound represented by formula **(IV-g)** 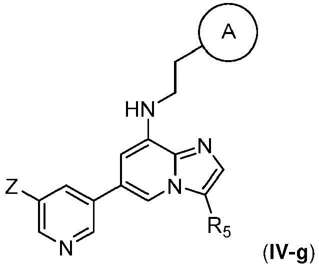
wherein A is an optionally substituted ring system selected from the group consisting of phenol-4-yl and 1H-indol-3-yl;
Z is a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ,-C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of isopropyl, methyl, ethyl, prop-1-en-2-yl, isobutyl, cyclohexyl, sec-butyl, (S)-sec-butyl, (R)-sec-butyl, 1-hydroxypropan-2-yl, (S)-1-hydroxypropan-2-yl, (R)-1 -hydroxypropan-2-yl, and nonan-2-yl, or Rs is selected from the group consisting of **(i), (ii), (iii), (iv),** and **(v)** 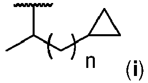 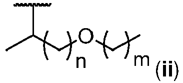 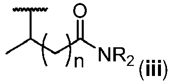 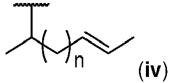 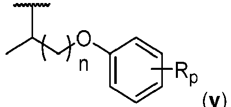
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 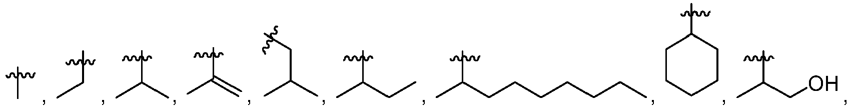 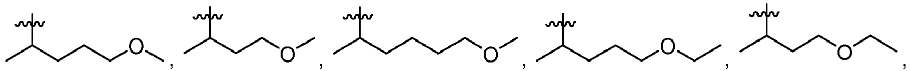 and 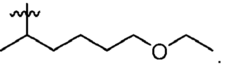
in some embodiments, R₅ is **(ii);**
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula **(IV-h)** 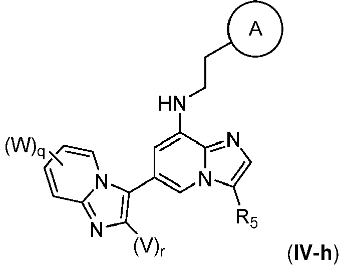
wherein A is an optionally substituted ring system selected from the group consisting of phenol-4-yl and 1H-indol-3-yl;
q is an integer from 0 to 4;
r is 0 or 1;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1 ,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of **(i), (ii), (iii), (iv),** and **(v)** 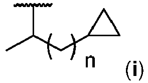 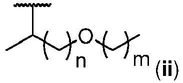 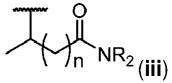 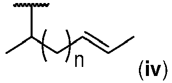 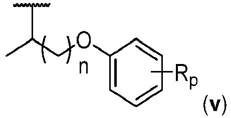
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 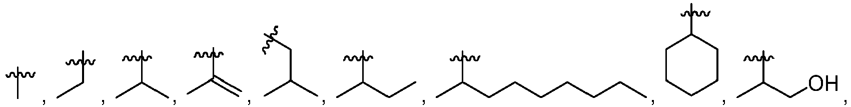 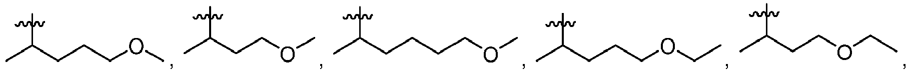 and 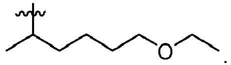
in some embodiments, Rs is **(ii);**
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula **(IV-i)** 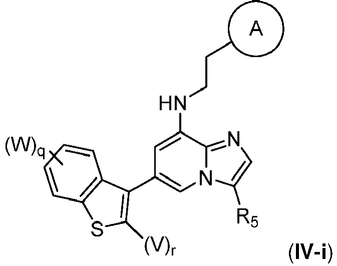
wherein A is an optionally substituted ring system selected from the group consisting of phenol-4-yl and 1H-indol-3-yl;
q is an integer from 0 to 4;
r is 0 or 1 ;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of **(i), (ii), (iii), (iv),** and **(v)** 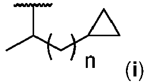 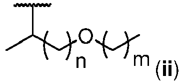 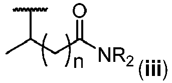 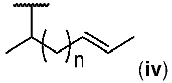 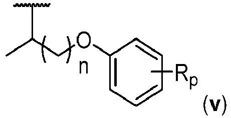
wherein n is an integer from 1 to 6, m is an integer from 0 to 6, p is an integer from 0 to 5, and each R is independently selected from the group consisting of cyano, hydroxy, C₁₋₄ alkyl, C2-4 alkenyl, C2-4 alkynyl, C3-6 cycloalkyl, C₁₋₄ alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 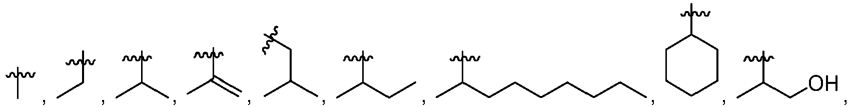 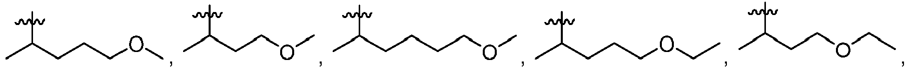 and 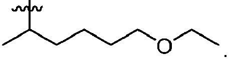
in some embodiments, R₅ is **(ii);**
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula **(IV-j)** 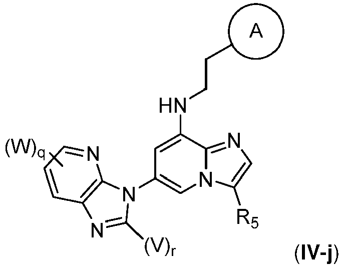
wherein A is an optionally substituted ring system selected from the group consisting of phenol-4-yl and 1H-indol-3-yl;
q is an integer from 0 to 4;
r is 0 or 1 ;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1 -en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1 ,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of **(i), (ii), (iii), (iv),** and **(v)** 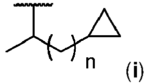 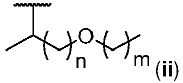 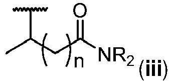 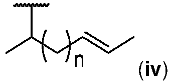 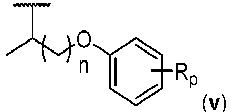
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 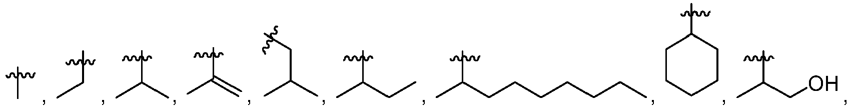 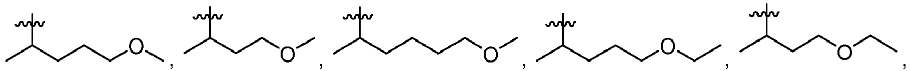 and 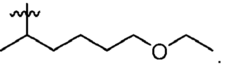
in some embodiments, R₅ is **(ii);**
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula **(IV-k)** 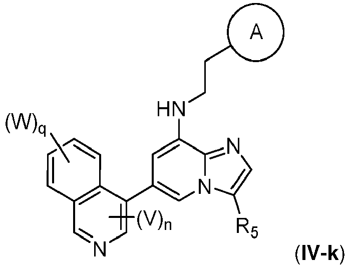
wherein A is an optionally substituted ring system selected from the group consisting of phenol-4-yl and 1H-indol-3-yl;
q is an integer from 0 to 4;
r is 0 or 1;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (**i**), (**ii**), (**iii**), (**iv**), and (**v**) 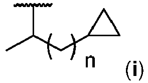 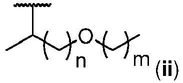 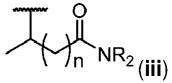 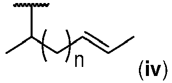 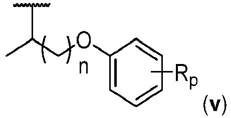
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 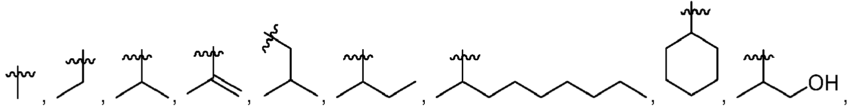 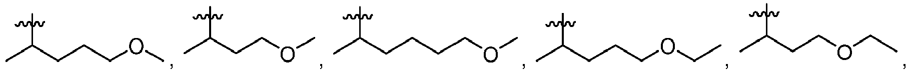 and 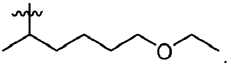
in some embodiments, Rs is (**ii**);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the aryl hydrocarbon receptor antagonist is compound (**3**), compound (**4**), compound (**5**), compound (**6**), compound (**7**), compound (**8**), compound (**9**), compound (**10**), compound (**11**), compound (**12**), compound (**13**), compound (**25**), compound (**27**), or compound (**28**) 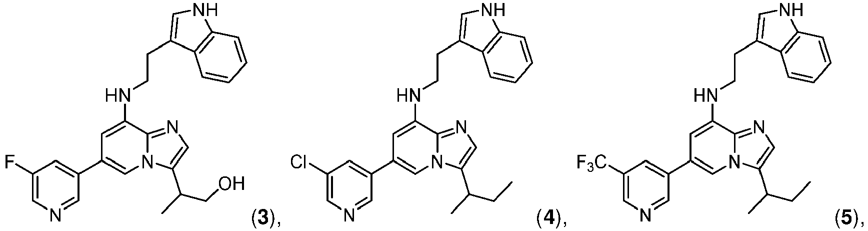 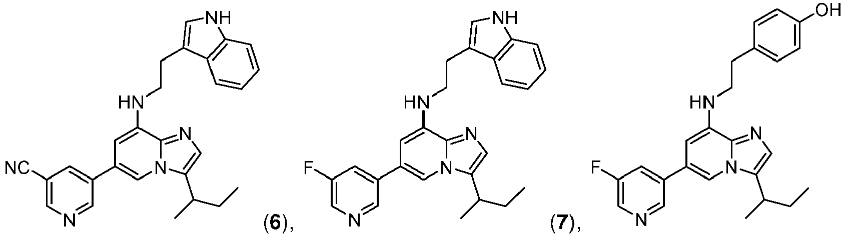 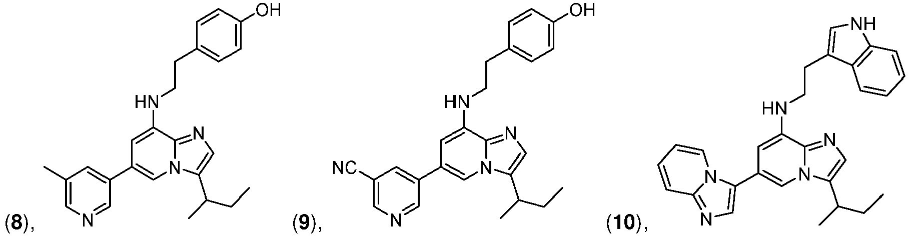 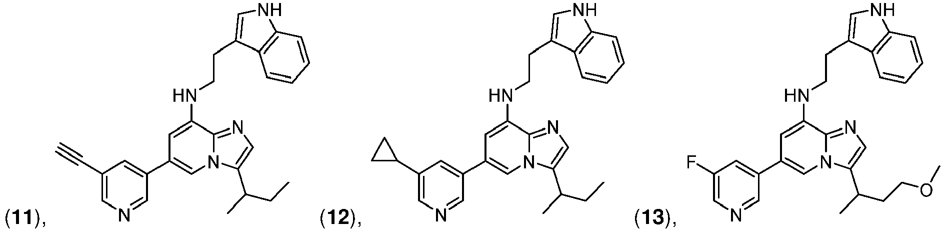 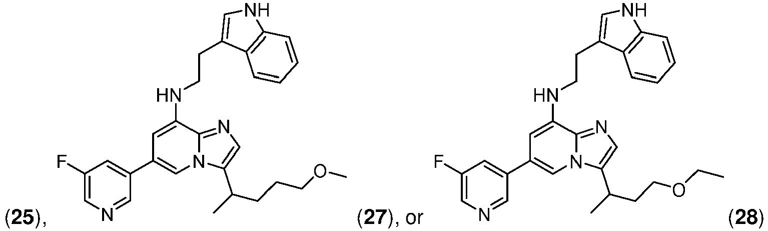 or salts thereof.

In some embodiments, aryl hydrocarbon receptor antagonists include those represented by formula (**V**) 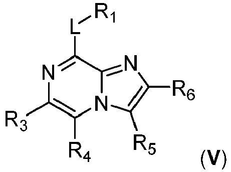
wherein L is a linker selected from the group consisting of -NR₇ₐ(CR₈ₐR_{8b})ₙ-, -O(CR₈ₐR_{8b})ₙ-, - C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, -NR₇ₐC(O)(CR₈ₐR_{8b})ₙ-, - NR₇ₐC(S)(CR₈ₐR_{8b})ₙ-, -OC(O)(CR₈ₐR_{8b})ₙ-, -OC(S)(CR₈ₐR_{8b})ₙ-, -C(O)NR₇ₐ(CR₈ₐR_{8b})ₙ-, - C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-, - NR₇ₐS(O)2(CR₈ₐR_{8b})ₙ-, -NR₇ₐC(O)NR_{7b}(CR₈ₐR_{8b})ₙ-, -NR₇ₐ(CR₈ₐR_{8b})ₙNR₇ₐ-, -NR₇ₐ(CR₈ₐR_{8b})ₙO-, - NR₇ₐ(CR₈ₐR_{8b})ₙS-, -O(CR₈ₐR_{8b})ₙNR₇ₐ-, -O(CR₈ₐR_{8b})ₙO-, -O(CR₈ₐR_{8b})ₙS-, -S(CR₈ₐR_{8b})ₙNR₇ₐ-, - S(CR₈ₐR_{8b})ₙO-, -S(CR₈ₐR_{8b})ₙS-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, -NR₉ₐC(S)R_{9b}, - NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b}, - C(S)NR₉ₐR_{9b}, -NR₉ₐS(O)₂R_{9b}, -NR₉ₐC(O)OR_{9b}, -OC(O)CR₉ₐR_{9b}R_{9c}, -OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
R₃ is selected from the group consisting of optionally substituted aryl, optionally substituted heteroaryl, optionally substituted cycloalkyl, and optionally substituted heterocycloalkyl;
R₄ is selected from the group consisting of hydrogen and optionally substituted C1-4 alkyl;
R₅ is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, R₁ is selected from the group consisting of -S(O)₂NR₉ₐR_{9b}, - NR₉ₐC(O)R_{9b}, -NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ, - C(S)OR₉ₐ, -C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, - OC(S)CR₉ₐR_{9b}R_{9c}, phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted, for example, with from 1 to 3 substituents independently selected from the group consisting of cyano, hydroxy, C1-4 alkyl, C1-4 alkoxy, halo, halo-substituted-C1-4 alkyl, halo-substituted-C1-4 alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}; wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl.

In some embodiments, R₁ is selected from the group consisting of -S(O)₂NR₉ₐR_{9b}, - NR₉ₐC(O)R_{9b}, -NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ, - C(S)OR₉ₐ, -C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, and - OC(S)CR₉ₐR_{9b}R_{9c}.

In some embodiments, R₁ is selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1 H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted, for example, with from 1 to 3 substituents independently selected from the group consisting of cyano, hydroxy, C1-4 alkyl, C1-4 alkoxy, halo, halo-substituted-C1-4 alkyl, halo-substituted-C1-4 alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}.

In some embodiments, R₁ is selected from the group consisting of phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl, wherein the phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, or 2-oxo-2,3-dihydro-1H-benzo[d]imidazol-5-yl is optionally substituted, for example, with from 1 to 3 substituents independently selected from the group consisting of cyano, hydroxy, C1-4 alkyl, C1-4 alkoxy, halo, halo-substituted-C1-4 alkyl, halo-substituted-C1-4 alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}.

In some embodiments, R₁ is selected from the group consisting of phenyl, phenol-4-yl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl.

In some embodiments, R₁ is selected from the group consisting of:

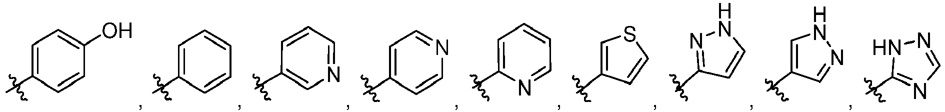

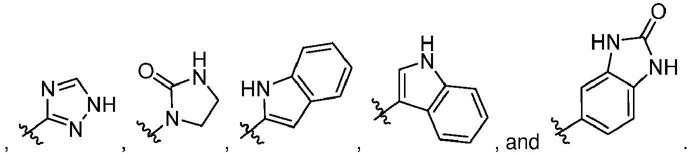

In some embodiments, R₁ is selected from the group consisting of:

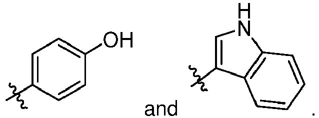

In some embodiments, R₁ is selected from the group consisting of phenol-4-yl and 1H-indol-3-yl.

In some embodiments, L is selected from the group consisting of -NR₇ₐ(CR₈ₐR_{8b})ₙ- and - O(CR₈ₐR_{8b})ₙ-.

In some embodiments, L is selected from the group consisting of -NH(CH₂)₂- and -O(CH₂)₂-.

In some embodiments, Rs is selected from the group consisting of optionally substituted aryl and optionally substituted heteroaryl.

In some embodiments, Rs is selected from the group consisting of phenyl, thiophenyl, furanyl, 1H-benzoimidazolyl, quinolinyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl, wherein the phenyl, thiophenyl, furanyl, 1H-benzoimidazolyl, quinolinyl, isoquinolinyl, imidazopyridinyl, benzothiophenyl, pyrimidinyl, pyridinyl, 1H-imidazolyl, pyrazinyl, pyridazinyl, 1H-pyrrolyl, or thiazolyl is optionally substituted, for example, with from 1 to 3 substituents independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and - C(O)NR₁₁ₐR_{11b}, and wherein R₁₁ₐ and R_{11b} are each independently selected from the group consisting of hydrogen and C₁₋₄ alkyl.

In some embodiments, Rs is selected from the group consisting of thiophen-2-yl, thiophen-3-yl, furan-3-yl, 1H-benzo[d]imidazol-1-yl, isoquinolin-4-yl, 1H-imidazo[4,5-b]pyridin-1-yl, imidazo[1,2-a]pyridin-3-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl and thiazol-5-yl, wherein the thiophen-2-yl, thiophen-3-yl, furan-3-yl, 1H-benzo[d]imidazol-1-yl, isoquinolin-4-yl, 1H-imidazo[4,5-b]pyridin-1-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl, or thiazol-5-yl is optionally substituted, for example, with from 1 to 3 substituents independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}.

In some embodiments, Rs is selected from the group consisting of thiophen-3-yl, benzo[b]thiophen-3-yl, pyridin-3-yl, pyrimidin-5-yl, 1H-imidazol-1-yl, 1H-benzo[d]imidazol-1-yl, isoquinolin-4-yl, 1H-imidazo[4,5-b]pyridin-1-yl, and imidazo[1 ,2-a]pyridin-3-yl, wherein the thiophen-3-yl, benzo[b]thiophen-3-yl, pyridin-3-yl, pyrimidin-5-yl, 1H-imidazol-1-yl, 1H-benzo[d]imidazol-1-yl, isoquinolin-4-yl, 1H-imidazo[4,5-b]pyridin-1-yl, or imidazo[1,2-a]pyridin-3-yl is optionally substituted, for example, with from 1 to 3 substituents independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and - C(O)NR₁₁ₐR_{11b}.

In some embodiments, Rs is selected from the group consisting of optionally substituted:

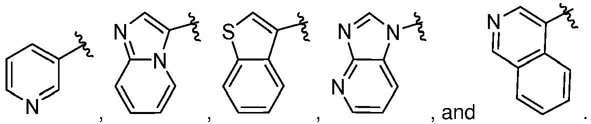

In some embodiments, Rs is pyridin-3-yl, wherein the pyridin-3-yl is optionally substituted at C5, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, the pyridin-3-yl is substituted at C5 with a substituent selected from the group consisting of ethoxycarbonyl, methoxy, cyano, methyl, methylsulfonyl, fluoro, chloro, trifluoromethyl, ethynyl, and cyclopropyl.

In some embodiments, Rs is selected from the group consisting of:

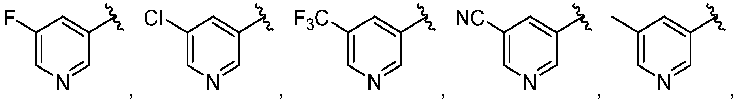

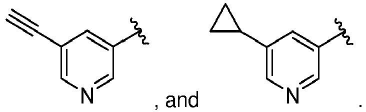

In some embodiments, R₃ is imidazo[1 ,2-a]pyridin-3-yl, wherein the imidazo[1 ,2-a]pyridin-3-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₃ is benzo[b]thiophen-3-yl, wherein the benzo[b]thiophen-3-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₃ is 1H-imidazo[4,5-b]pyridin-1-yl, wherein the 1H-imidazo[4,5-b]pyridin-1-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₃ is isoquinolin-4-yl, wherein the isoquinolin-4-yl is optionally substituted, for example, with a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}.

In some embodiments, R₄ is hydrogen.

In some embodiments, Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1 ,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted, for example, with from 1 to 3 substituents independently selected from the group consisting of hydroxy, C1-4 alkyl, and halo-substituted-C1-4alkyl.

In some embodiments, Rs is selected from the group consisting of isopropyl, methyl, ethyl, prop-1-en-2-yl, isobutyl, cyclohexyl, sec-butyl, (S)-sec-butyl, (R)-sec-butyl, 1-hydroxypropan-2-yl, (S)-1-hydroxypropan-2-yl, (R)-1-hydroxypropan-2-yl, and nonan-2-yl.

In some embodiments, Rs is (S)-1-hydroxypropan-2-yl.

In some embodiments, Rs is (R)-1-hydroxypropan-2-yl.

In some embodiments, Rs is (S)-sec-butyl.

In some embodiments, Rs is (R)-sec-butyl.

In some embodiments, Rs is selected from the group consisting of (**i**), (**ii**), (**iii**), (**iv**), and (**v**) 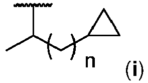 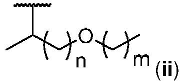 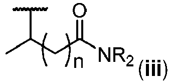 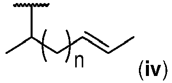 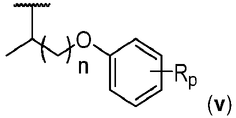 wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl.

In some embodiments, Rs is selected from the group consisting of: 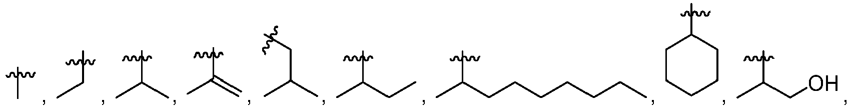 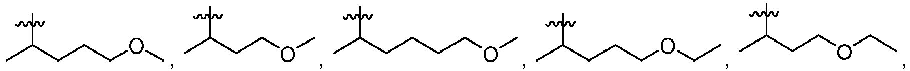 and

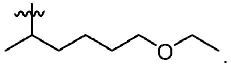

In some embodiments, Rs is (ii).

In some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl.

In some embodiments, Rs is (S)-4-methoxybutan-2-yl.

In some embodiments, Rs is (R)-4-methoxybutan-2-yl.

In some embodiments, Rs is (S)-5-methoxypentan-2-yl.

In some embodiments, Rs is (R)-5-methoxypentan-2-yl.

In some embodiments, Rs is (S)-4-ethoxybutan-2-yl.

In some embodiments, Rs is (R)-4-ethoxybutan-2-yl.

In some embodiments, R₆ is hydrogen.

In some embodiments, the disclosure features a compound represented by formula (**V-a**) 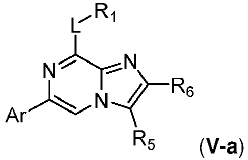
wherein L is a linker selected from the group consisting of -NR₇ₐ(CR₈ₐR_{8b})ₙ-, -O(CR₈ₐR_{8b})ₙ-, - C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, -NR₇ₐC(O)(CR₈ₐR_{8b})ₙ-, - NR₇ₐC(S)(CR₈ₐR_{8b})ₙ-, -OC(O)(CR₈ₐR_{8b})ₙ-, -OC(S)(CR₈ₐR_{8b})ₙ-, -C(O)NR₇ₐ(CR₈ₐR_{8b})ₙ-, - C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-, - NR₇ₐS(O)₂(CR₈ₐR_{8b})ₙ-, -NR₇ₐC(O)NR_{7b}(CR₈ₐR_{8b})ₙ-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, -NR₉ₐC(S)R_{9b}, - NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b}, - C(S)NR₉ₐR_{9b}, -NR₉ₐS(O)₂R_{9b}, -NR₉ₐC(O)OR_{9b}, -OC(O)CR₉ₐR_{9b}R_{9c}, -OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl (for example, R₁ may be selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted, for example, with from 1 to 3 substituents independently selected from the group consisting of cyano, hydroxy, C1-4 alkyl, C₁₋₄ alkoxy, halo, halo-substituted-C1-4 alkyl, halo-substituted-C1-4 alkoxy, amino, -O(CH₂)₂NR₁₀ₐR_{10b}, - S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl);
Ar is selected from the group consisting of optionally substituted monocyclic aryl and heteroaryl, such as optionally substituted thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, Ar is pyridin-3-yl, wherein the pyridin-3-yl is optionally substituted at C5, for example, with a substituent selected from the group consisting of ethoxycarbonyl, methoxy, cyano, methyl, methylsulfonyl, fluoro, chloro, trifluoromethyl, ethynyl, and cyclopropyl.

In some embodiments, the disclosure features a compound represented by formula (**V-b**) 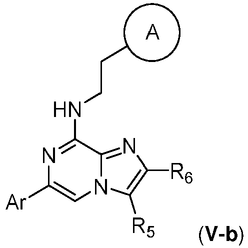
wherein A is an optionally substituted ring system selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted with from 1 to 3 substituents independently selected from the group consisting of cyano, hydroxy, C1-4 alkyl, C₁₋₄ alkoxy, halo, halo-substituted-C1-4 alkyl, halo-substituted-C1-4 alkoxy, amino, -O(CH₂)₂NR₁₀ₐR_{10b},-S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
Ar is selected from the group consisting of optionally substituted monocyclic aryl and heteroaryl, such as optionally substituted thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, A is selected from the group consisting of phenyl, phenol-4-yl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl.

In some embodiments, A is selected from the group consisting of phenol-4-yl and 1H-indol-3-yl.

In some embodiments, the disclosure features a compound represented by formula (V-c) 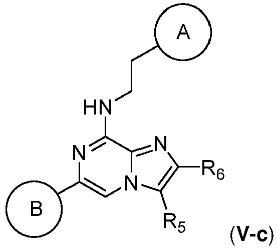
wherein A is an optionally substituted ring system selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted with from 1 to 3 substituents independently selected from the group consisting of cyano, hydroxy, C1-4 alkyl, C₁₋₄ alkoxy, halo, halo-substituted-C1-4 alkyl, halo-substituted-C1-4 alkoxy, amino, -O(CH₂)₂NR₁₀ₐR_{10b},-S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
B is an optionally substituted ring system selected from the group consisting of thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl, wherein the thiophenyl, furanyl, 1H-benzoimidazolyl, isoquinolinyl, 1H-imidazopyridinyl, benzothiophenyl, pyrimidinyl, pyridinyl, 1H-imidazolyl, pyrazinyl, pyridazinyl, 1H-pyrrolyl, or thiazolyl is optionally substituted with from 1 to 3 substituents independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl;
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, B is pyridin-3-yl, wherein the pyridin-3-yl is optionally substituted at C5, for example, with a substituent selected from the group consisting of ethoxycarbonyl, methoxy, cyano, methyl, methylsulfonyl, fluoro, chloro, trifluoromethyl, ethynyl, and cyclopropyl.

In some embodiments, the disclosure features a compound represented by formula (**V-d**) 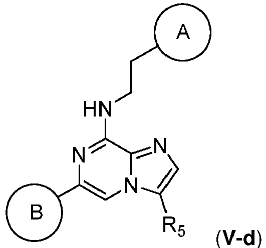
wherein A is an optionally substituted ring system selected from the group consisting of phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, and 1H-indazolyl, wherein the phenyl, 1H-pyrrolopyridinyl, 1H-indolyl, thiophenyl, pyridinyl, 1H-1,2,4-triazolyl, 2-oxoimidazolidinyl, 1H-pyrazolyl, 2-oxo-2,3-dihydro-1H-benzoimidazolyl, or 1H-indazolyl is optionally substituted with from 1 to 3 substituents independently selected from the group consisting of cyano, hydroxy, C1-4 alkyl, C₁₋₄ alkoxy, halo, halo-substituted-C1-4 alkyl, halo-substituted-C1-4 alkoxy, amino, -O(CH₂)₂NR₁₀ₐR_{10b}, - S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
B is an optionally substituted ring system selected from the group consisting of thiophenyl, furanyl, 1H-benzoimidazolyl, isoquinolinyl, imidazopyridinyl, benzothiophenyl, pyrimidinyl, pyridinyl, 1H-imidazolyl, pyrazinyl, pyridazinyl, 1H-pyrrolyl, and thiazolyl, wherein the thiophenyl, furanyl, 1H-benzoimidazolyl, isoquinolinyl, 1H-imidazopyridinyl, benzothiophenyl, pyrimidinyl, pyridinyl, 1H-imidazolyl, pyrazinyl, pyridazinyl, 1H-pyrrolyl, or thiazolyl is optionally substituted with from 1 to 3 substituents independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of optionally substituted aryl, optionally substituted heteroaryl, optionally substituted alkyl, optionally substituted heteroalkyl, optionally substituted cycloalkyl, and optionally substituted heterocycloalkyl;
or a salt thereof.

In some embodiments, the disclosure features a compound represented by formula (**V-e**) 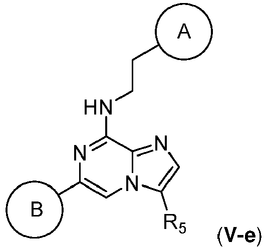
wherein A is an optionally substituted ring system selected from the group consisting of phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, and 2-oxo-2,3-dihydro-1H-benzo[d]imidazol-5-yl, wherein the phenyl, 1H-indol-2-yl, 1H-indol-3-yl, thiophen-3-yl, pyridin-2-yl, pyridin-3-yl, pyridin-4-yl, 1H-1,2,4-triazol-3-yl, 1H-1,2,4-triazol-5-yl, 2-oxoimidazolidin-1-yl, 1H-pyrazol-3-yl, 1H-pyrazol-4-yl, or 2-oxo-2,3-dihydro-1H-benzo[d]imidazol-5-yl is optionally substituted with from 1 to 3 substituents independently selected from the group consisting of cyano, hydroxy, C1-4 alkyl, C1-4 alkoxy, halo, halo-substituted-C1-4 alkyl, halo-substituted-C1-4 alkoxy, amino, -O(CH₂)₂NR₁₀ₐR_{10b}, -S(O)₂NR₁₀ₐR_{10b}, -OS(O)₂NR₁₀ₐR_{10b}, and -NR₁₀ₐS(O)₂R_{10b}, wherein R₁₀ₐ and R_{10b} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
B is an optionally substituted ring system selected from the group consisting of thiophen-2-yl, thiophen-3-yl, furan-3-yl, 1H-benzo[d]imidazol-1-yl, isoquinolin-4-yl, 1H-imidazo[4,5-b]pyridin-1-yl, imidazo[1,2-a]pyridin-3-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl and thiazol-5-yl, wherein the thiophen-2-yl, thiophen-3-yl, furan-3-yl, 1H-benzo[d]imidazol-1-yl, isoquinolin-4-yl, 1H-imidazo[4,5-b]pyridin-1-yl, benzo[b]thiophen-3-yl, pyrimidin-5-yl, pyridin-2-yl, pyridin-3-yl, pyridin-4-yl, 1H-imidazol-1-yl, pyrazin-2-yl, pyridazin-4-yl, 1H-pyrrol-2-yl, or thiazol-5-yl is optionally substituted with from 1 to 3 substituents independently selected from the group consisting of cyano, hydroxy, C1-4 alkyl, C2-4 alkenyl, C2-4 alkynyl, C3-6 cycloalkyl, C1-4alkoxy, halo, halo-substituted-C1-4 alkyl, halo-substituted-C1-4 alkoxy, amino, -C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1 ,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (**i**), (**ii**), (**iii**), (**iv**), and (**v**) 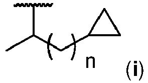 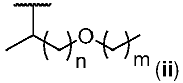 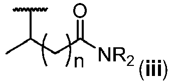 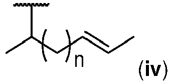 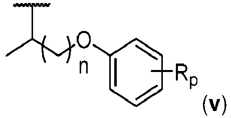
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 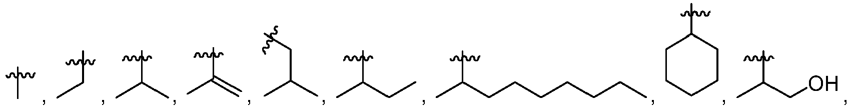 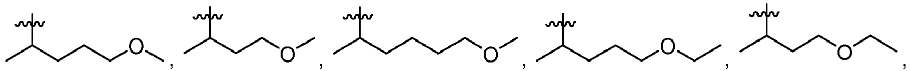 and 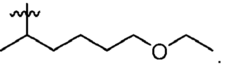
in some embodiments, R₅ is (**ii**);
in some embodiments, R₅ is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula (**V-f**) 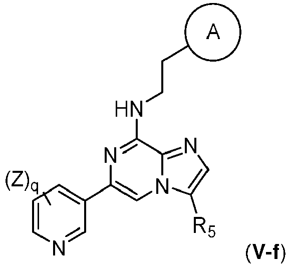
wherein A is an optionally substituted ring system selected from the group consisting of phenol-4-yl and 1H-indol-3-yl;
q is an integer from 0 to 4;
each Z is independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of isopropyl, methyl, ethyl, prop-1-en-2-yl, isobutyl, cyclohexyl, sec-butyl, (S)-sec-butyl, (R)-sec-butyl, 1-hydroxypropan-2-yl, (S)-1-hydroxypropan-2-yl, (R)-1-hydroxypropan-2-yl, and nonan-2-yl, or Rs is selected from the group consisting of (**i**), (**ii**), (**iii**), (**iv**), and (**v**) 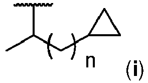 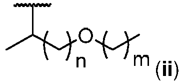 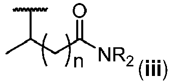 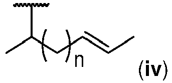 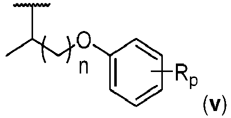
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 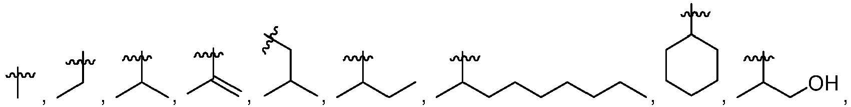 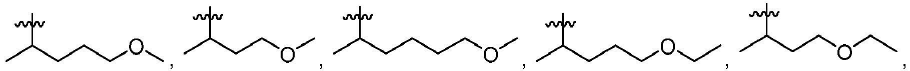 and 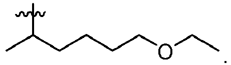
in some embodiments, Rs is (**ii**);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, each Z is independently a substituent selected from the group consisting of ethoxycarbonyl, methoxy, cyano, methyl, methylsulfonyl, fluoro, chloro, trifluoromethyl, ethynyl, and cyclopropyl.

In some embodiments, the disclosure features a compound represented by formula (**V-g**) 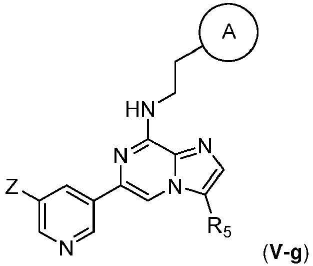
wherein A is an optionally substituted ring system selected from the group consisting of phenol-4-yl and 1H-indol-3-yl;
Z is a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, - C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of isopropyl, methyl, ethyl, prop-1-en-2-yl, isobutyl, cyclohexyl, sec-butyl, (S)-sec-butyl, (R)-sec-butyl, 1-hydroxypropan-2-yl, (S)-1-hydroxypropan-2-yl, (R)-1-hydroxypropan-2-yl, and nonan-2-yl, or Rs is selected from the group consisting of (**i**), (**ii**), (**iii**), (**iv**), and (**v**) 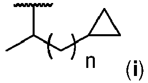 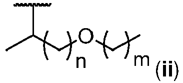 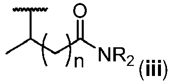 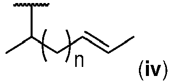 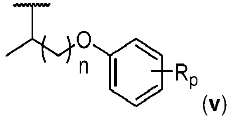
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 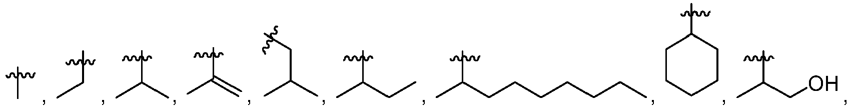 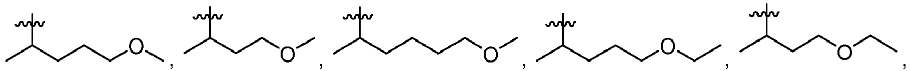 and 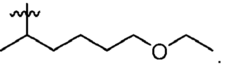
in some embodiments, Rs is (**ii**);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula (**V-h**) 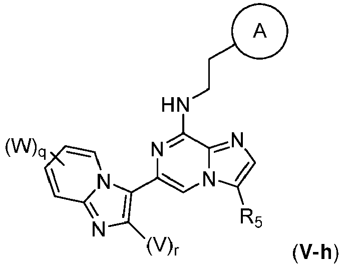
wherein A is an optionally substituted ring system selected from the group consisting of phenol-4-yl and 1H-indol-3-yl;
q is an integer from 0 to 4;
r is 0 or 1;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (i), (ii), (iii), (iv), and (v) 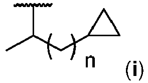 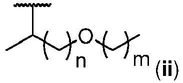 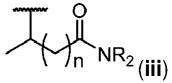 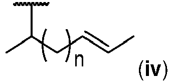 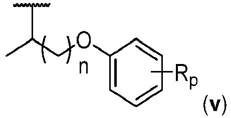
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 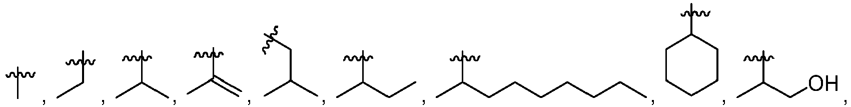 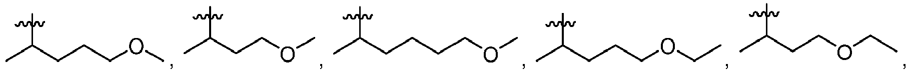 and 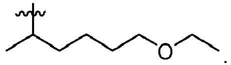
in some embodiments, Rs is (**ii**);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula (**V-i**) 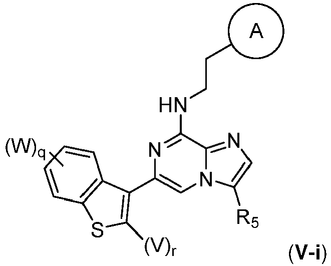
wherein A is an optionally substituted ring system selected from the group consisting of phenol-4-yl and 1H-indol-3-yl;
q is an integer from 0 to 4;
r is 0 or 1;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (**i**), (**ii**), (**iii**), (**iv**), and (**v**) 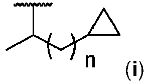 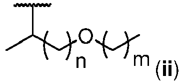 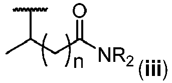 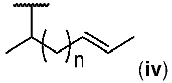 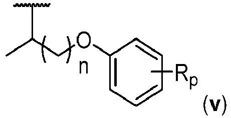
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 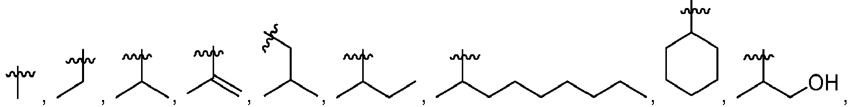 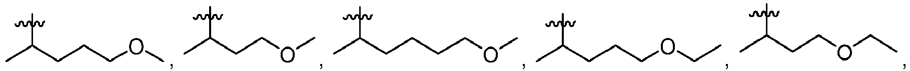 and 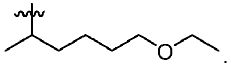
in some embodiments, Rs is (**ii**);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula (**V-j**) 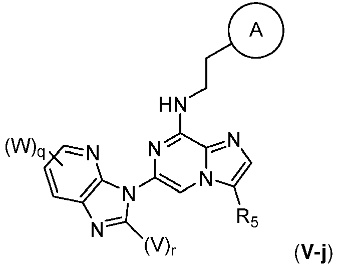
wherein A is an optionally substituted ring system selected from the group consisting of phenol-4-yl and 1H-indol-3-yl;
q is an integer from 0 to 4;
r is 0 or 1;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (**i**), (**ii**), (**iii**), (**iv**), and (**v**) 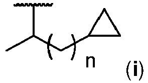 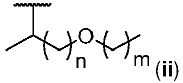 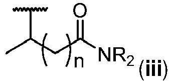 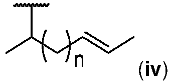 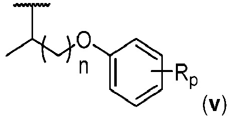
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 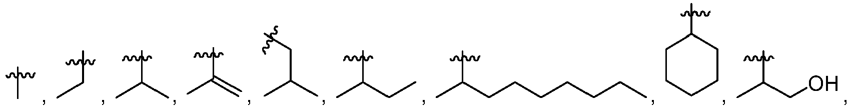 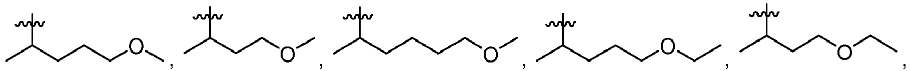 and 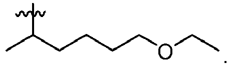
in some embodiments, Rs is (**ii**);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the disclosure features a compound represented by formula (**II-k**) 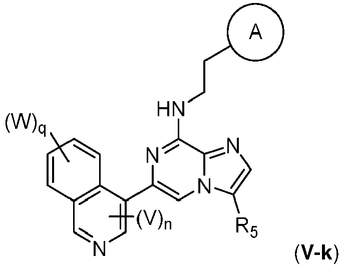
wherein A is an optionally substituted ring system selected from the group consisting of phenol-4-yl and 1H-indol-3-yl;
q is an integer from 0 to 4;
r is 0 or 1;
W and V are each independently a substituent selected from the group consisting of C1-4 alkyl, halo, halo-substituted-C1-4 alkyl, C2-4 alkenyl, C2-4 alkynyl, C3-6 cycloalkyl, C1-4 alkoxy, cyano, amino, C(O)R₁₁ₐ, -S(O)₀₋₂R₁₁ₐ, -C(O)OR₁₁ₐ, and -C(O)NR₁₁ₐR_{11b}, wherein R₁₁ₐ and R_{11b} are each independently selected from the group consisting of hydrogen and C₁₋₄ alkyl; and
Rs is selected from the group consisting of C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, and 1-(1-(2-oxo-6,9,12-trioxa-3-azatetradecan-14-yl)-1H-1,2,3-triazol-4-yl)ethyl, wherein the C1-10 alkyl, prop-1-en-2-yl, cyclohexyl, cyclopropyl, 2-(2-oxopyrrolidin-1-yl)ethyl, oxetan-2-yl, oxetan-3-yl, benzhydryl, tetrahydro-2H-pyran-2-yl, tetrahydro-2H-pyran-3-yl, phenyl, tetrahydrofuran-3-yl, benzyl, (4-pentylphenyl)(phenyl)methyl, or 1-(1-(2-oxo-6,9,12-trioxa-3-azatetradecan-14-yl)-1H-1,2,3-triazol-4-yl)ethyl is optionally substituted with from 1 to 3 substituents independently selected from the group consisting of hydroxy, C1-4 alkyl, and halo-substituted-C1-4alkyl, or Rs is selected from the group consisting of (i), (ii), (iii), (iv), and (v) 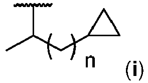 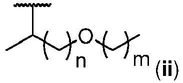 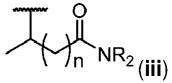 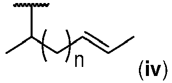 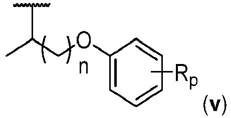
wherein n is an integer from 1 to 6, m is an integer from 0 to 6, p is an integer from 0 to 5, and each R is independently selected from the group consisting of cyano, hydroxy, C1-4 alkyl, C2-4 alkenyl, C2-4 alkynyl, C3-6 cycloalkyl, C1-4 alkoxy, halo, halo-substituted-C1-4 alkyl, halo-substituted-C1-4 alkoxy, amino, -C(O)R₁₂ₐ, -S(O)₀₋₂R₁₂ₐ, -C(O)OR₁₂ₐ, and -C(O)NR₁₂ₐR_{12b}, and wherein R₁₂ₐ and R_{12b} are each independently selected from the group consisting of hydrogen and C₁₋₄ alkyl;

In some embodiments, Rs is selected from the group consisting of: 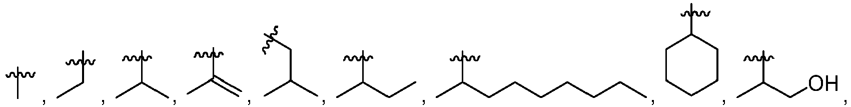 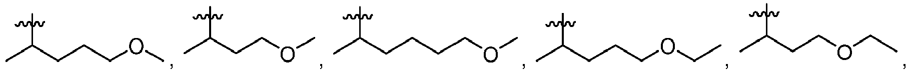 and 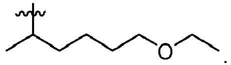
in some embodiments, Rs is (ii);
in some embodiments, Rs is selected from the group consisting of 4-methoxybutan-2-yl, (S)-4-methoxybutan-2-yl, (R)-4-methoxybutan-2-yl, 4-ethoxybutan-2-yl, (S)-4-ethoxybutan-2-yl, (R)-4-ethoxybutan-2-yl, 5-methoxypentan-2-yl, (S)-5-methoxypentan-2-yl, (R)-5-methoxypentan-2-yl, 5-ethoxypentan-2-yl, (S)-5-ethoxypentan-2-yl, (R)-5-ethoxypentan-2-yl, 6-methoxyhexan-2-yl, (S)-6-methoxyhexan-2-yl, (R)-6-methoxyhexan-2-yl, 6-ethoxyhexan-2-yl, (S)-6-ethoxyhexan-2-yl, and (R)-6-ethoxyhexan-2-yl;
or a salt thereof.

In some embodiments, the aryl hydrocarbon receptor antagonist is compound (**14**), compound (**15**), compound (**16**), compound (**17**), compound (**18**), compound (**19**), compound (**20**), compound (**21**), compound (**22**), compound (**23**), compound (**24**), compound (**26**), compound (**29**), or compound (**30**) 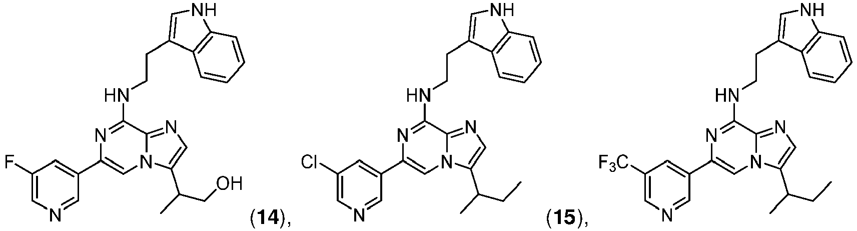 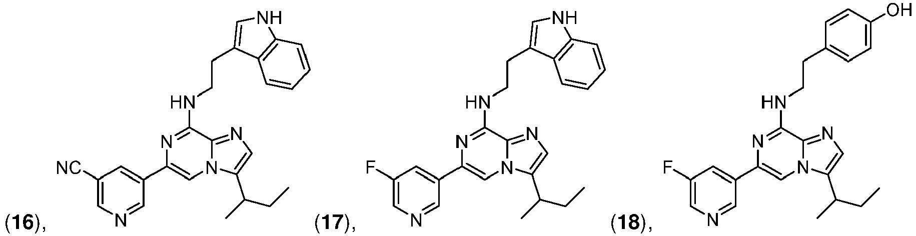 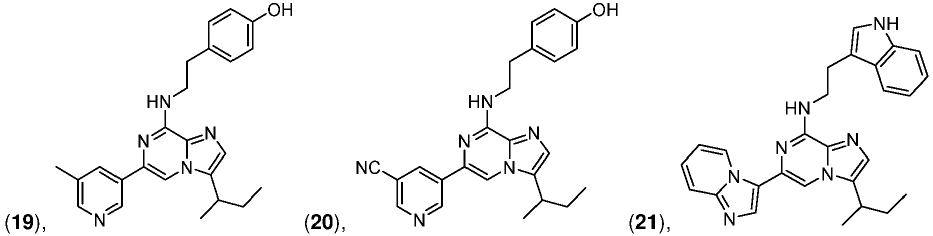 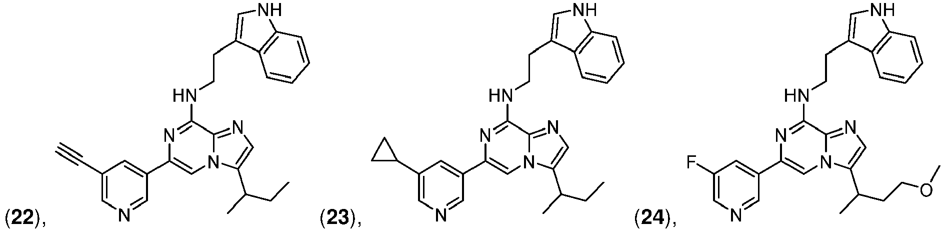 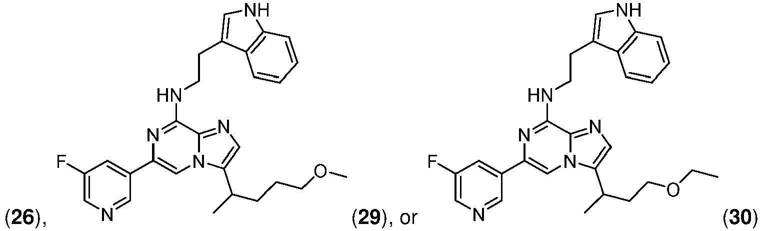 or salts thereof.

### CXCR4 Antagonists

Exemplary CXCR4 antagonists for use in conjunction with the compositions and methods described herein are compounds represented by formula (**I**)

Z - linker - Z' (**I**)

or a pharmaceutically acceptable salt thereof, wherein Z is:
(i) a cyclic polyamine containing from 9 to 32 ring members, wherein from 2 to 8 of the ring members are nitrogen atoms separated from one another by 2 or more carbon atoms; or
(ii) an amine represented by formula (**IA**) 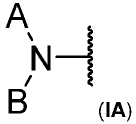
   wherein A includes a monocyclic or bicyclic fused ring system including at least one nitrogen atom and B is H or a substituent of from 1 to 20 atoms;
   and wherein Z' is:
      (i) a cyclic polyamine containing from 9 to 32 ring members, wherein from 2 to 8 of the ring members are nitrogen atoms separated from one another by 2 or more carbon atoms;
      (ii) an amine represented by formula (**IB**) 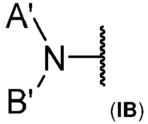 wherein A' includes a monocyclic or bicyclic fused ring system including at least one nitrogen atom and B' is H or a substituent of from 1 to 20 atoms; or
      (iii) a substituent represented by formula (**IC**)

         - N(R) - (CR₂)ₙ - X (**IC**)

         wherein each R is independently H or C₁-C₆ alkyl, n is 1 or 2, and X is an aryl or heteroaryl group or a mercaptan;
wherein the linker is a bond, optionally substituted alkylene (e.g., optionally substituted C₁-C₆ alkylene), optionally substituted heteroalkylene (e.g., optionally substituted C₁-C₆ heteroalkylene), optionally substituted alkenylene (e.g., optionally substituted C₂-C₆ alkenylene), optionally substituted heteroalkenylene (e.g., optionally substituted C₂-C₆ heteroalkenylene), optionally substituted alkynylene (e.g., optionally substituted C₂-C₆ alkynylene), optionally substituted heteroalkynylene (e.g., optionally substituted C₂-C₆ heteroalkynylene), optionally substituted cycloalkylene, optionally substituted heterocycloalkylene, optionally substituted arylene, or optionally substituted heteroarylene.

In some embodiments, Z and Z' may each independently a cyclic polyamine containing from 9 to 32 ring members, of which from 2 to 8 are nitrogen atoms separated from one another by 2 or more carbon atoms. In some embodiments, Z and Z' are identical substituents. As an example, Z may be a cyclic polyamine including from 10 to 24 ring members. In some embodiments, Z may be a cyclic polyamine that contains 14 ring members. In some embodiments, Z includes 4 nitrogen atoms. In some embodiments, Z is 1,4,8,11-tetraazocyclotetradecane.

In some embodiments, the linker is represented by formula (**ID**) 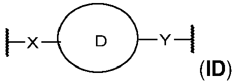
wherein ring D is an optionally substituted aryl group, an optionally substituted heteroaryl group, an optionally substituted cycloalkyl group, or an optionally substituted heterocycloalkyl group; and
X and Y are each independently optionally substituted alkylene (e.g., optionally substituted C₁-C₆ alkylene), optionally substituted heteroalkylene (e.g., optionally substituted C₁-C₆ heteroalkylene), optionally substituted alkenylene (e.g., optionally substituted C₂-C₆ alkenylene), optionally substituted heteroalkenylene (e.g., optionally substituted C₂-C₆ heteroalkenylene), optionally substituted alkynylene (e.g., optionally substituted C₂-C₆ alkynylene), or optionally substituted heteroalkynylene (e.g., optionally substituted C₂-C₆ heteroalkynylene).

As an example, the linker may be represented by formula (**IE**) 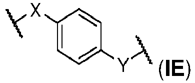
wherein ring D is an optionally substituted aryl group, an optionally substituted heteroaryl group, an optionally substituted cycloalkyl group, or an optionally substituted heterocycloalkyl group; and
X and Y are each independently optionally substituted alkylene (e.g., optionally substituted C₁-C₆ alkylene), optionally substituted heteroalkylene (e.g., optionally substituted C₁-C₆ heteroalkylene), optionally substituted C₂-C₆ alkenylene (e.g., optionally substituted C₂-C₆ alkenylene), optionally substituted heteroalkenylene (e.g., optionally substituted C₂-C₆ heteroalkenylene), optionally substituted alkynylene (e.g., optionally substituted C₂-C₆ alkynylene), or optionally substituted heteroalkynylene (e.g., optionally substituted C₂-C₆ heteroalkynylene). In some embodiments, X and Y are each independently optionally substituted C₁-C₆ alkylene. In some embodiments, X and Y are identical substituents. In some embodiments, X and Y may be each be methylene, ethylene, n-propylene, n-butylene, n-pentylene, or n-hexylene groups. In some embodiments, X and Y are each methylene groups.

The linker may be, for example, 1,3-phenylene, 2,6-pyridine, 3,5-pyridine, 2,5-thiophene, 4,4'-(2,2'-bipyrimidine), 2,9-(1,10-phenanthroline), or the like. In some embodiments, the linker is 1,4-phenylene-bis-(methylene).

CXCR4 antagonists useful in conjunction with the compositions and methods described herein include plerixafor (also referred to herein as "AMD3100" and "Mozibil"), or a pharmaceutically acceptable salt thereof, represented by formula (**II**), 1,1'-[1,4-phenylenebis(methylene)]-bis-1,4,8,11-tetra-azacyclotetradecane.

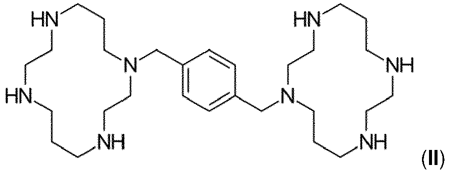

Additional CXCR4 antagonists that may be used in conjunction with the compositions and methods described herein include variants of plerixafor, such as a compound described in US Patent No. 5,583,131, which pertains to CXCR4 antagonists. In some embodiments, the CXCR4 antagonist may be a compound selected from the group consisting of: 1,1'-[1,3-phenylenebis(methylene)]-bis-1,4,8,11-tetra-azacyclotetradecane; 1,1'-[1,4-phenylene-bis-(methylene)]-bis-1,4,8,11-tetraazacyclotetradecane; bis-zinc or bis-copper complex of 1,1'-[1,4-phenylene-bis-(methylene)]-bis-1,4,8,11-tetraazacyclotetradecane; 1,1'-[3,3'-biphenylene-bis-(methylene)]-bis-1,4,8,11-tetraazacyclotetradecane; 11,11'-[1,4-phenylene-bis-(methylene)]-bis-1,4,7,11-tetraazacyclotetradecane; 1,11'-[1,4-phenylene-bis-(methylene)]-1,4,8,11-tetraazacyclotetradecane-1, 4,7,11-tetraazacyclotetradecane; 1,1'-[2,6-pyridine-bis-(methylene)]-bis-1,4,8,11-tetraazacyclotetradecane; 1,1-[3,5-pyridine-bis-(methylene)]-bis-1,4,8,11-tetraazacyclotetradecane; 1,1'-[2,5-thiophene-bis-(methylene)]-bis-1,4,8,11-tetraazacyclotetradecane; 1,1'-[4,4'-(2,2'-bipyridine)-bis-(methylene)]-bis-1,4,8,11-tetraazacyclotetradecane; 1,1'-[2,9-(1,10-phenanthroline)-bis-(methylene)]-bis-1,4,8,11-tetraazacyclotetradecane; 1,1'-[1,3-phenylene-bis-(methylene)]-bis-1,4,7,10-tetraazacyclotetradecane; 1,1'-[1 ,4-phenylene-bis-(methylene)]-bis-1 ,4,7,10-tetraazacyclotetradecane; 1'-[5-nitro-1 ,3-phenylenebis(methylene)]bis-1,4,8,11-tetraazacyclotetradecane; 1',1'-[2,4,5,6-tetrachloro-1,3-phenyleneis(methylene)]bis-1,4,8,11-tetraazacyclotetradecane; 1,1'-[2,3,5,6-tetra-fluoro-1,4-phenylenebis(methylene)]bis-1,4,8,11-tetraazacyclotetradecane; 1,1'-[1,4-naphthylene-bis-(methylene)]bis-1,4,8,11-tetraazacyclotetradecane; 1,1'-[1,3-phenylenebis-(methylene)]bis-1,5,9-triazacyclododecane;
1,1'-[1,4-phenylene-bis-(methylene)]-1,5,9-triazacyclododecane; 1,1'-[2,5-dimethyl-1,4-phenylenebis-(methylene)]-bis-1,4,8,11-tetraazacyclotetradecane; 1,1 '-[2,5-dichloro-1 ,4-phenylenebis-(methylene)]-bis-1,4,8,11-tetraazacyclotetradecane; 1,1'-[2-bromo-1,4-phenylenebis-(methylene)]-bis-1,4,8,11-tetraazacyclotetradecane; and 1,1'-[6-phenyl-2,4-pyridinebis-(methylene)]-bis-1,4,8,11-tetraazacyclotetradecane.

In some embodiments, the CXCR4 antagonist is a compound described in US 2006/0035829, which pertains to CXCR4 antagonists. In some embodiments, the CXCR4 antagonist may be a compound selected from the group consisting of: 3,7,11,17-tetraazabicyclo(13.3.1)heptadeca-1(17),13,15-triene; 4,7,10,17-tetraazabicyclo(13.3.1)heptadeca-1(17),13,15-triene; 1,4,7,10-tetraazacyclotetradecane; 1,4,7-triazacyclotetradecane; and 4,7,10-triazabicyclo(13.3.1)heptadeca-1(17),13,15-triene.

The CXCR4 antagonist may be a compound described in WO 2001/044229, which pertains to CXCR4 antagonists. In some embodiments, the CXCR4 antagonist may be a compound selected from the group consisting of: N-[4-(11-fluoro-1,4,7-triazacyclotetradecanyl)-1,4-phenylenebis(methylene)]-2-(aminomethyl)pyridine; N-[4-(11,11-difluoro-1,4,7-triazacyclotetradecanyl)-1,4-phenylenebis(methylene)]-2-(aminomethyl)pyridine; N-[4-(1,4,7-triazacyclotetradecan-2-onyl)-1,4-phenylenebis(methylene)]-2-(aminomethyl)pyridine; N-[12-(5-oxa-1,9-diazacyclotetradecanyl)-1,4-phenylenebis(methylene)]-2-(aminomethyl)pyridine; N-[4-(11-oxa-1,4,7-triazacyclotetradecanyl)-1,4-phenylenebis(methylene)]-2-(aminomethyl)pyridine; N-[4-(11-thia-1,4,7-triazacyclotetradecanyl)-1,4-phenylenebis(methylene)]-2-(aminomethyl)pyridine; N-[4-(11-sulfoxo-1,4,7-triazacyclotetradecanyl)-1,4-phenylenebis(methylene)]-2-(aminomethyl)pyridine; N-[4-(11-sulfono-1,4,7-triazacyclotetradecanyl)-1,4-phenylenebis(methylene)]-2-(aminomethyl)pyridine; and N-[4-(3-carboxo-1,4,7-triazacyclotetradecanyl)-1,4-phenylenebis(methylene)]-2-(aminomethyl)pyridine.

Additional CXCR4 antagonists useful in conjunction with the compositions and methods described herein include compounds described in WO 2000/002870 which pertains to CXCR4 antagonists. In some embodiments, the CXCR4 antagonist may be a compound selected from the group consisting of: N-[1,4,8,11-tetraazacyclotetra-decanyl-1,4-phenylenebis-(methylene)]-2-(aminomethyl)pyridine; N-[1,4,8,11-tetraazacyclotetra-decanyl-1 ,4-phenylenebis(methylene)]-N-methyl-2-(aminomethyl)pyridine; N-[1,4,8,11-tetraazacyclotetra-decanyl-1,4-phenylenebis(methylene)]-4-(aminomethyl)pyridine; N-[1,4,8,11-tetraazacyclotetra-decanyl-1,4-phenylenebis(methylene)]-3-(aminomethyl)pyridine; N-[1,4,8,11-tetraazacyclotetra-decanyl-1,4-phenylenebis(methylene)]-(2-aminomethyl-5-methyl)pyrazine; N-[1,4,8,11-tetraazacyclotetra-decanyl-1,4-phenylenebis(methylene)]-2-(aminoethyl) pyridine; N-[1,4,8,11-tetraazacyclotetra-decanyl-1,4-phenylenebis(methylene)]-2-(aminomethyl)thiophene; N-[1,4,8,11-tetraazacyclotetra-decanyl-1,4-phenylenebis(methylene)]-2-(aminomethyl)mercaptan; N-[1,4,8,11-tetraazacyclotetra-decanyl-1,4-phenylenebis(methylene)]-2-amino benzylamine; N-[1,4,8,11-tetraazacyclotetra-decanyl-1,4-phenylenebis(methylene)]-4-amino benzylamine; N-[1,4,8,11-tetraazacyclotetra-decanyl-1,4-phenylenebis(methylene)]-4-(aminoethyl)imidazole; N-[1,4,8,11-tetraazacyclotetra-decanyl-1,4-phenylenebis(methylene)]-benzylamine; N-[4-(1,4,7-triazacyclotetra-decanyl)-1,4-phenylenebis(methylene)]-2-(aminomethyl)pyridine; N-[7-(4,7,10,17-tetraazabicyclo[13.3.1]heptadeca-1(17),13,15-trienyl)-1 ,4-phenylenebis(methylene)]-2-(aminomethyl)pyridine; N-[7-(4,7,10-triazabicyclo[13.3.1]heptadeca-1 (17),13,15-trienyl)-1 ,4-phenylenebis(methylene)]-2-(aminomethyl)pyridine; N-[1-(1,4,7-triazacyclotetra-decanyl)-1,4-phenylenebis(methylene)]-2-(aminomethyl)pyridine; N-[4-[4,7,10,17-tetraazabicyclo[13.3.1]heptadeca-1(17),13,15-trienyl]-1,4-phenylenebis(methylene)]-2-(aminomethyl)pyridine; N-[4-[4,7,10-triazabicyclo[13.3.1]heptadeca-1(17),13,15-trienyl]-1 ,4-phenylenebis(methylene)]-2-(aminomethyl)pyridine; N-[1 ,4,8,11-tetraazacyclotetradecanyl-1,4-phenylenebis(methylene)]-purine; 1-[1,4,8,11-tetraazacyclotetradecanyl-1,4-phenylenebix(methylene)]-4-phenylpiperazine; N-[4-(1,7-diazacyclotetradecanyl)-1,4-phenylenebis(methylene)]-2-(aminomethyl)pyridine; and N-[7-(4,10-diazabicyclo[13.3.1]heptadeca-1 (17),13,15-trienyl)-1 ,4-phenylenebis(methylene)]-2-(aminomethyl)pyridine.

In some embodiments, the CXCR4 antagonist is a compound selected from the group consisting of: 1-[2,6-dimethoxypyrid-4-yl(methylene)]-1,4,8,11-tetraazacyclotetradecane; 1-[2-chloropyrid-4-yl(methylene)]-1,4,8,11-tetraazacyclotetradecane; 1-[2,6-dimethylpyrid-4-yl(methylene)]-1,4,8,11-tetraazacyclotetradecane; 1-[2-methylpyrid-4-yl(methylene)]-1,4,8,11-tetraazacyclotetradecane; 1-[2,6-dichloropyrid-4-yl(methylene)]-1,4,8,11-tetraazacyclotetradecane; 1-[2-chloropyrid-5-yl(methylene)]-1,4,8,11-tetraazacyclotetradecane; and 7-[4-methylphenyl (methylene)]-4,7,10,17-tetraazabicyclo[13.3.1]heptadeca-1 (17),13,15-triene.

In some embodiments, the CXCR4 antagonist is a compound described in US Patent No. 5,698,546, which pertains to CXCR4 antagonists. In some embodiments, the CXCR4 antagonist may be a compound selected from the group consisting of: 7,7'-[1,4-phenylene-bis(methylene)]bis-3,7,11,17-tetraazabicyclo[13.3.1]heptadeca-1(17),13,15-triene; 7,7'-[1,4-phenylene-bis(methylene)]bis[15-chloro-3,7,11,17-tetraazabicyclo [13.3.1]heptadeca-1 (17),13,15-triene]; 7,7'-[1,4-phenylene-bis(methylene)]bis[15-methoxy-3,7,11,17-tetraazabicyclo[13.3.1]heptadeca-1(17),13,15-triene]; 7,7'-[1,4-phenylene-bis(methylene)]bis-3,7,11,17-tetraazabicyclo[13.3.1]-heptadeca-13,16-triene-15-one; 7,7'-[1,4-phenylene-bis(methylene)]bis-4,7,10,17-tetraazabicyclo[13.3.1]-heptadeca-1 (17),13,15-triene; 8,8'-[1,4-phenylene-bis(methylene)]bis-4,8,12,19-tetraazabicyclo[15.3.1]nonadeca-1(19),15,17-triene; 6,6'-[1,4-phenylene-bis(methylene)]bis-3,6,9,15-tetraazabicyclo[11.3.1]pentadeca-1 (15),11,13-triene; 6,6'-[1,3-phenylene-bis(methylene)]bis-3,6,9,15-tetraazabicyclo[11.3.1]pentadeca-1 (15),11,13-triene; and 17,17'-[1,4-phenylene-bis(methylene)]bis-3,6, 14,17,23,24-hexaazatricyclo[17.3.1.1^{8,12}]tetracosa-1(23),8,10,12(24),19,21 -hexaene.

In some embodiments, the CXCR4 antagonist is a compound described in US Patent No. 5,021,409, which pertains to CXCR4 antagonists. In some embodiments, the CXCR4 antagonist may be a compound selected from the group consisting of: 2,2'-bicyclam, 6,6'-bicyclam; 3,3'-(bis-1,5,9,13-tetraaza cyclohexadecane); 3,3'-(bis-1,5,8,11,14-pentaazacyclohexadecane); methylene (or polymethylene) di-1-N-1,4,8,11-tetraaza cyclotetradecane; 3,3'-bis-1,5,9,13-tetraazacyclohexadecane; 3,3'-bis-1,5,8,11,14-pentaazacyclohexadecane; 5,5'-bis-1,4,8,11-tetraazacyclotetradecane; 2,5'-bis-1,4,8,11-tetraazacyclotetradecane; 2,6'-bis-1,4,8,11-tetraazacyclotetradecane; 11,11'-(1,2-ethanediyl)bis-1,4,8,11-tetraazacyclotetradecane; 11,11'-(1,2-propanediyl)bis-1,4,8,11-tetraazacyclotetradecane; 11,11'-(1,2-butanediyl)bis-1,4,8,11-tetraazacyclotetradecane; 11,11'-(1,2-pentanediyl)bis-1,4,8,11-tetraazacyclotetradecane; and 11,11'-(1,2-hexanediyl)bis-1,4,8,11-tetraazacyclotetradecane.

In some embodiments, the CXCR4 antagonist is a compound described in WO 2000/056729, which pertains to CXCR4 antagonists. In some embodiments, the CXCR4 antagonist may be a compound selected from the group consisting of: N-(2-pyridinylmethyl)-N'-(6,7,8,9-tetrahydro-5H-cyclohepta[b]pyridin-9-yl)-1,4-benzenedimethanamine; N-(2-pyridinylmethyl)-N'-(5,6,7,8-tetrahydro-8-quinolinyl)-1,4-benzenedimethanamine; N-(2-pyridinylmethyl)-N'-(6,7-dihydro-5H-cyclopenta[b]pyridin-7-yl)-1,4-benzenedimethanamine; N-(2-pyridinylmethyl)-N'-(1,2,3,4-tetrahydro-1-naphthalenyl)-1,4-benzenedimethanamine; N-(2-pyridinylmethyl)-N'-(1-naphthalenyl)-1,4-benzenedimethanamine; N-(2-pyridinylmethyl)-N'-(8-quinolinyl)-1,4-benzenedimethanamine; N-(2-pyridinylmethyl)-N'-[2-[(2-pyridinylmethyl)amino]ethyl]-N'-(1-methyl-1,2,3,4-tetrahydro-8-quinolinyl)-1,4-benzenedimethanamine; N-(2-pyridinylmethyl)-N'-[2-[(1H-imidazol-2-ylmethyl)amino]ethyl]-N'-(1-methyl-1,2,3,4-tetrahydro-8-quinolinyl)-1,4-benzenedimethanamine; N-(2-pyridinylmethyl)-N'-(1,2,3,4-tetrahydro-8-quinolinyl)-1,4-benzenedimethanamine; N-(2-pyridinylmethyl)-N'-[2-[(1H-imidazol-2-ylmethyl)amino]ethyl]-N'-(1,2,3,4-tetrahydro-1-naphthalenyl)-1,4-benzenedimethanamine; N-(2-pyridinylmethyl)-N'-(2-phenyl-5,6,7,8-tetrahydro-8-quinolinyl)-1,4-benzenedimethanamine; N,N'-bis(2-pyridinylmethyl)-N'-(2-phenyl-5,6,7,8-tetrahydro-8-quinolinyl)-1,4-benzenedimethanamine; N-(2-pyridinylmethyl)-N'-(5,6,7,8-tetrahydro-5-quinolinyl)-1,4-benzenedimethanamine; N-(2-pyridinylmethyl)-N'-(1H-imidazol-2-ylmethyl)-N'-(5,6,7,8-tetrahydro-5-quinolinyl)-1,4-benzenedimethanamine; N-(2-pyridinylmethyl)-N'-(1H-imidazol-2-ylmethyl)-N'-(5,6,7,8-tetrahydro-8-quinolinyl)-1,4-benzenedimethanamine; N-(2-pyridinylmethyl)-N'-[(2-amino-3-phenyl)propyl]-N'-(5,6,7,8-tetrahydro-8-quinolinyl)-1,4-benzenedimethanamine; N-(2-pyridinylmethyl)-N'-(1H-imidazol-4-ylmethyl)-N'-(5,6,7,8-tetrahydro-8-quinolinyl)-1,4-benzenedimethanamine; N-(2-pyridinylmethyl)-N'-(2-quinolinylmethyl)-N'-(5,6,7,8-tetrahydro-8-quinolinyl)-1,4-benzenedimethanamine; N-(2-pyridinylmethyl)-N'-(2-(2-naphthoyl)aminoethyl)-N'-(5,6,7,8-tetrahydro-8-quinolinyl)-1,4-benzenedimethanamine; N-(2-pyridinylmethyl)-N'-[(S)-(2-acetylamino-3-phenyl)propyl]-N'-(5,6,7,8-tetrahydro-8-quinolinyl)-1,4-benzenedimethanamine; N-(2-pyridinylmethyl)-N'-[(S)-(2-acetylamino-3-phenyl)propyl]-N'-(5,6,7,8-tetrahydro-8-quinolinyl)-1,4-benzenedimethanamine; N-(2-pyridinylmethyl)-N'-[3-((2-naphthalenylmethyl)amino)propyl]-N'-(5,6,7,8-tetrahydro-8-quinolinyl)-1,4-benzenedimethanamine; N-(2-pyridinylmethyl)-N'-[2-(S)-pyrollidinylmethyl]-N'-(5,6,7,8-tetrahydro-8-quinolinyl)-1,4-benzenedimethanamine; N-(2-pyridinylmethyl)-N'-[2-(R)-pyrollidinylmethyl]-N'-(5,6,7,8-tetrahydro-8-quinolinyl)-1,4-benzenedimethanamine; N-(2-pyridinylmethyl)-N'-[3-pyrazolylmethyl]-N'-(5,6,7,8-tetrahydro-8-quinolinyl)-1,4-benzenedimethanamine; N-(2-pyridinylmethyl)-N'-[2-pyrrolylmethyl]-N'-(5,6,7,8-tetrahydro-8-quinolinyl)-1,4-benzenedimethanamine; N-(2-pyridinylmethyl)-N'-[2-thiopheneylmethyl]-N'-(5,6,7,8-tetrahydro-8-quinolinyl)-1,4-benzenedimethanamine; N-(2-pyridinylmethyl)-N'-[2-thiazolylmethyl]-N'-(5,6,7,8-tetrahydro-8-quinolinyl)-1,4-benzenedimethanamine; N-(2-pyridinylmethyl)-N'-[2-furanylmethyl]-N'-(5,6,7,8-tetrahydro-8-quinolinyl)-1,4-benzenedimethanamine; N-(2-pyridinylmethyl)-N'-[2-[(phenylmethyl)amino]ethyl]-N'-(5,6,7,8-tetrahydro-8-quinolinyl)-1,4-benzenedimethanamine; N-(2-pyridinylmethyl)-N'-(2-aminoethyl)-N'-(5,6,7,8-tetrahydro-8-quinolinyl)-1,4-benzenedimethanamine; N-(2-pyridinylmethyl)-N'-3-pyrrolidinyl-N'-(5,6,7,8-tetrahydro-8-quinolinyl)-1,4-benzenedimethanamine N-(2-pyridinylmethyl)-N'-4-piperidinyl-N'-(5,6,7,8-tetrahydro-8-quinolinyl)-1,4-benzenedimethanamine; N-(2-pyridinylmethyl)-N'-[2-[(phenyl)amino]ethyl]-N'-(5,6,7,8-tetrahydro-8-quinolinyl)-1,4-benzenedimethanamine; N-(2-pyridinylmethyl)-N'-(7-methoxy-1,2,3,4-tetrahydro-2-naphthalenyl)-1,4-benzenedimethanamine; N-(2-pyridinylmethyl)-N'-(6-methoxy-1,2,3,4-tetrahydro-2-naphthalenyl)-1,4-benzenedimethanamine; N-(2-pyridinylmethyl)-N'-(1-methyl-1,2,3,4-tetrahydro-2-naphthalenyl)-1,4-benzenedimethanamine; N-(2-pyridinylmethyl)-N'-(7-methoxy-3,4-dihydronaphthalenyl)-1-(aminomethyl)-4-benzamide; N-(2-pyridinylmethyl)-N'-(6-methoxy-3,4-dihydronaphthalenyl)-1-(aminomethyl)-4-benzamide; N-(2-pyridinylmethyl)-N'-(1H-imidazol-2-ylmethyl)-N'-(7-methoxy-1,2,3,4-tetrahydro-2-naphthalenyl)-1,4-benzenedimethanamine; N-(2-pyridinylmethyl)-N'-(8-hydroxy-1,2,3,4-tetrahydro-2-naphthalenyl)-1,4-benzenedimethanamine; N-(2-pyridinylmethyl)-N'-(1H-imidazol-2-ylmethyl)-N'-(8-hydroxy-1,2,3,4-tetrahydro-2-naphthalenyl)-1,4-benzenedimethanamine; N-(2-pyridinylmethyl)-N'-(8-Fluoro-1,2,3,4-tetrahydro-2-naphthalenyl)-1 ,4-benzenedimethanamine; N-(2-pyridinylmethyl)-N'-(1H-imidazol-2-ylmethyl)-N'-(8-Fluoro-1,2,3,4-tetrahydro-2-naphthalenyl)-1,4-benzenedimethanamine; N-(2-pyridinylmethyl)-N'-(5,6,7,8-tetrahydro-7-quinolinyl)-1,4-benzenedimethanamine; N-(2-pyridinylmethyl)-N'-(1H-imidazol-2-ylmethyl)-N'-(5,6,7,8-tetrahydro-7-quinolinyl)-1,4-benzenedimethanamine; N-(2-pyridinylmethyl)-N'-[2-[(2-naphthalenylmethyl)amino]ethyl]-N'-(5,6,7,8-tetrahydro-8-quinolinyl)-1,4-benzenedimethanamine; N-(2-pyridinylmethyl)-N'-[2-(isobutylamino)ethyl]-N'-(5,6,7,8-tetrahydro-8-quinolinyl)-1,4-benzenedimethanamine; N-(2-pyridinylmethyl)-N'-[2-[(2-pyridinylmethyl)amino]ethyl]-N'-(5,6,7,8-tetrahydro-8-quinolinyl)-1,4-benzenedimethanamine; N-(2-pyridinylmethyl)-N'-[2-[(2-furanylmethyl)amino]ethyl]-N'-(5,6,7,8-tetrahydro-8-quinolinyl)-1,4-benzenedimethanamine; N-(2-pyridinylmethyl)-N'-(2-guanidinoethyl)-N'-(5,6,7,8-tetrahydro-8-quinolinyl)-1,4-benzenedimethanamine; N-(2-pyridinylmethyl)-N'-[2-[bis-[(2-methoxy)phenylmethyl]amino]ethyl]-N'-(5,6,7,8-tetrahydro-8-quinolinyl)-1,4-benzenedimethanamine; N-(2-pyridinylmethyl)-N'-[2-[(1H-imidazol-4-ylmethyl)amino]ethyl]-N'-(5,6,7,8-tetrahydro-8-quinolinyl)-1,4-benzenedimethanamine; N-(2-pyridinylmethyl)-N'-[2-[(1H-imidazol-2-ylmethyl)amino]ethyl]-N'-(5,6,7,8-tetrahydro-8-quinolinyl)-1,4-benzenedimethanamine; N-(2-pyridinylmethyl)-N'-[2-(phenylureido)ethyl]-N'-(5,6,7,8-tetrahydro-8-quinolinyl)-1,4-benzenedimethanamine; N-(2-pyridinylmethyl)-N'-[[N"-(n-butyl)carboxamido]methyl]-N'-(5,6,7,8-tetrahydro-8-quinolinyl)-1,4-benzenedimethanamine; N-(2-pyridinylmethyl)-N'-(carboxamidomethyl)-N'-(5,6,7,8-tetrahydro-8-quinolinyl)-1,4-benzenedimethanamine; N-(2-pyridinylmethyl)-N'-[(N"-phenyl)carboxamidomethyl]-N'-(5,6,7,8-tetrahydro-8-quinolinyl)-1,4-benzenedimethanamine; N-(2-pyridinylmethyl)-N'-(carboxymethyl)-N'-(5,6,7,8-tetrahydro-8-quinolinyl)-1,4-benzenedimethanamine; N-(2-pyridinylmethyl)-N'-(phenylmethyl)-N'-(5,6,7,8-tetrahydro-8-quinolinyl)-1,4-benzenedimethanamine; N-(2-pyridinylmethyl)-N'-(1H-benzimidazol-2-ylmethyl)-N'-(5,6,7,8-tetrahydro-8-quinolinyl)-1 ,4-benzenedimethanamine; N-(2-pyridinylmethyl)-N'-(5,6-dimethyl-1H-benzimidazol-2-ylmethyl)-N'-(5,6,7,8-tetrahydro-8-quinolinyl)-1,4-benzenedimethanamine (hydrobromide salt); N-(2-pyridinylmethyl)-N'-(5-nitro-1H-benzimidazol-2-ylmethyl)-N'-(5,6,7,8-tetrahydro-8-quinolinyl)-1 ,4-benzenedimethanamine; N-(2-pyridinylmethyl)-N'-[(1H)-5-azabenzimidazol-2-ylmethyl]-N'-(5,6,7,8-tetrahydro-8-quinolinyl)-1,4-benzenedimethanamine; N-(2-pyridinylmethyl)-N-(4-phenyl-1H-imidazol-2-ylmethyl)-N'-(5,6,7,8-tetrahydro-8-quinolinyl)-1,4-benzenedimethanamine; N-(2-pyridinylmethyl)-N'-[2-(2-pyridinyl)ethyl]-N'-(5,6,7,8-tetrahydro-8-quinolinyl)-1,4-benzenedimethanamine; N-(2-pyridinylmethyl)-N'-(2-benzoxazolyl)-N'-(5,6,7,8-tetrahydro-8-quinolinyl)-1,4-benzenedimethanamine; N-(2-pyridinylmethyl)-N'-(trans-2-aminocyclohexyl)-N'-(5,6,7,8-tetrahydro-8-quinolinyl)-1,4-benzenedimethanamine; N-(2-pyridinylmethyl)-N'-(2-phenylethyl)-N'-(5,6,7,8-tetrahydro-8-quinolinyl)-1,4-benzenedimethanamine; N-(2-pyridinylmethyl)-N'-(3-phenylpropyl)-N'-(5,6,7,8-tetrahydro-8-quinolinyl)-1,4-benzenedimethanamine; N-(2-pyridinylmethyl)-N'-(trans-2-aminocyclopentyl)-N'-(5,6,7,8-tetrahydro-8-quinolinyl)-1,4-benzenedimethanamine; N-[[4-[[(2-pyridinylmethyl)amino]methyl]phenyl]methyl]-N-(5,6,7,8-tetrahydro-8-quinolinyl)-glycinamide; N-[[4-[[(2-pyridinylmethyl)amino]methyl]phenyl]methyl]-N-(5,6,7,8-tetrahydro-8-quinolinyl)-(L)-alaninamide; N-[[4-[[(2-pyridinylmethyl)amino]methyl]phenyl]methyl]-N-(5,6,7,8-tetrahydro-8-quinolinyl)-(L)-aspartamide; N-[[4-[[(2-pyridinylmethyl)amino]methyl]phenyl]methyl]-N-(5,6,7,8-tetrahydro-8-quinolinyl)-pyrazinamide; N-[[4-[[(2-pyridinylmethyl)amino]methyl]phenyl]methyl]-N-(5,6,7,8-tetrahydro-8-quinolinyl)-(L)-prolinamide; N-[[4-[[(2-pyridinylmethyl)amino]methyl]phenyl]methyl]-N-(5,6,7,8-tetrahydro-8-quinolinyl)-(L)-lysinamide; N-[[4-[[(2-pyridinylmethyl)amino]methyl]phenyl]methyl]-N-(5,6,7,8-tetrahydro-8-quinolinyl)-benzamide; N-[[4-[[(2-pyridinylmethyl)amino]methyl]phenyl]methyl]-N-(5,6,7,8-tetrahydro-8-quinolinyl)-picolinamide; N'-Benzyl-N-[[4-[[(2-pyridinylmethyl)amino]methyl]phenyl]methyl]-N-(5,6,7,8-tetrahydro-8-quinolinyl)-urea; N'-phenyl-N-[[4-[[(2-pyridinylmethyl)amino]methyl]phenyl]methyl]-N-(5,6,7,8-tetrahydro-8-quinolinyl)-urea; N-(6,7,8,9-tetrahydro-5H-cyclohepta[bacteriapyridin-9-yl)-4-[[(2-pyridinylmethyl)amino]methyl]benzamide; N-(5,6,7,8-tetrahydro-8-quinol inyl)-4-[[(2-pyridinylmethyl)amino]methyl]benzamide; N,N'-bis(2-pyridinylmethyl)-N'-(5,6,7,8-tetrahydro-8-quinolinyl)-1,4-benzenedimethanamine; N,N'-bis(2-pyridinylmethyl)-N'-(6,7,8,9-tetrahydro-5H-cyclohepta[bacteriapyridin-9-yl)-1,4-benzenedimethanamine; N,N'-bis(2-pyridinylmethyl)-N'-(6,7-dihydro-5H-cyclopenta[bacteriapyridin-7-yl)-1,4-benzenedimethanamine; N,N'-bis(2-pyridinylmethyl)-N'-(1,2,3,4-tetrahydro-1-naphthalenyl)-1,4-benzenedimethanamine; N,N'-bis(2-pyridinylmethyl)-N'-[(5,6,7,8-tetrahydro-8-quinolinyl)methyl]-1,4-benzenedimethanamine; N,N'-bis(2-pyridinylmethyl)-N'[(6,7-dihydro-5H-cyclopenta[bacteriapyridin-7-yl)methyl]-1,4-benzenedimethanamine; N-(2-pyridinylmethyl)-N-(2-methoxyethyl)-N'-(5,6,7,8-tetrahydro-8-quinolinyl)-1,4-benzenedimethanamine; N-(2-pyridinylmethyl)-N-[2-(4-methoxyphenyl)ethyl]-N'-(5,6,7,8-tetrahydro-8-quinolinyl)-1,4-benzenedimethanamine; N,N'-bis(2-pyridinylmethyl)-1,4-(5,6,7,8-tetrahydro-8-quinolinyl)benzenedimethanamine; N-[(2,3-dimethoxyphenyl)methyl]-N'-(2-pyridinylmethyl)-N-(5,6,7,8-tetrahydro-8-quinolinyl)-1,4-benzenedimethanamine; N,N'-bis(2-pyridinylmethyl)-N-[1-(N"-phenyl-N"-methylureido)-4-piperidinyl]-1,3-benzenedimethanamine; N,N'-bis(2-pyridinylmethyl)-N-[N"-p-toluenesulfonylphenylalanyl)-4-piperidinyl]-1,3-benzenedimethanamine; N,N'-bis(2-pyridinylmethyl)-N-[1-[3-(2-chlorophenyl)-5-methyl-isoxazol-4-oyl]-4-piperidinyl]-1,3-benzenedimethanamine; N-[(2-hydroxyphenyl)methyl]-N'-(2-pyridinylmethyl)-N-(6,7,8,9-tetrahydro-5H-cyclohepta[bacteriapyridin-9-yl)-1,4-benzenedimethanamine; N-[(4-cyanophenyl)methyl]-N'-(2-pyridinylmethyl)-N-(6,7,8,9-tetrahydro-5H-cyclohepta[bacteriapyridin-9-yl)-1,4-benzenedimethanamine; N-[(4-cyanophenyl)methyl]-N'-(2-pyridinylmethyl)-N-(5,6,7,8-tetrahydro-8-quinolinyl)-1,4-benzenedimethanamine; N-[(4-acetamidophenyl)methyl]-N'-(2-pyridinylmethyl)-N-(5,6,7,8-tetrahydro-8-quinolinyl)-1,4-benzenedimethanamine; N-[(4-phenoxyphenyl)methyl]-N'-(2-pyridinylmethyl)-N-(6,7,8,9-tetrahydro-5H-cyclohepta[bacteriapyridin-9-yl)-1,4-benzenedimethanamine; N-[(1-methyl-2-carboxamido)ethyl]-N,N'-bis(2-pyridinylmethyl)-1,3-benzenedimethanamine; N-[(4-benzyloxyphenyl)methyl]-N'-(2-pyridinylmethyl)-N-(6,7,8,9-tetrahydro-5H-cyclohepta[bacteriapyridin-9-yl)-1,4-benzenedimethanamine; N-[(thiophene-2-yl)methyl]-N'-(2-pyridinylmethyl)-N-(6,7,8,9-tetrahydro-5H-cyclohepta[bacteriapyridin-9-yl)-1,4-benzenedimethanamine; N-[1-(benzyl)-3-pyrrolidinyl]-N,N'-bis(2-pyridinylmethyl)-1,3-benzenedimethanamine; N-[[1-methyl-3-(pyrazol-3-yl)]propyl]-N,N'-bis(2-pyridinylmethyl)-1,3-benzenedimethanamine; N-[1-(phenyl)ethyl]-N,N'-bis(2-pyridinylmethyl)-1,3-benzenedimethanamine; N-[(3,4-methylenedioxyphenyl)methyl]-N'-(2-pyridinylmethyl)-N-(6,7,8,9-tetrahydro-5H-cyclohepta[b]pyridin-9-yl)-1,4-benzenedimethanamine; N-[1-benzyl-3-carboxymethyl-4-piperidinyl]-N,N'-bis(2-pyridinylmethyl)-1,3-benzenedimethanamine; N-[(3,4-methylenedioxyphenyl)methyl]-N'-(2-pyridinylmethyl)-N-(5,6,7,8-tetrahydro-8-quinolinyl)-1,4-benzenedimethanamine; N-(3-pyridinylmethyl)-N'-(2-pyridinylmethyl)-N-(6,7,8,9-tetrahydro-5H-cyclohepta[b]pyridin-9-yl)-1,4-benzenedimethanamine; N-[[1-methyl-2-(2-tolyl)carboxamido]ethyl]-N,N'-bis(2-pyridinylmethyl)-1,3-benzenedimethanamine; N-[(1,5-dimethyl-2-phenyl-3-pyrazolinone-4-yl)methyl]-N'-(2-pyridinylmethyl)-N-(5,6,7,8-tetrahydro-8-quinolinyl)-1,4-benzenedimethanamine; N-[(4-propoxyphenyl)methyl]-N'-(2-pyridinylmethyl)-N-(6,7,8,9-tetrahydro-5H-cyclohepta[b]pyridin-9-yl)-1,4-benzenedimethanamine; N-(1-phenyl-3,5-dimethylpyrazolin-4-ylmethyl)-N'-(2-pyridinylmethyl)-N-(5,6,7,8-tetrahydro-8-quinolinyl)-1,4-benzenedimethanamine; N-[H-imidazol-4-ylmethyl]-N,N'-bis(2-pyridinylmethyl)-1,3-benzenedimethanamine; N-[(3-methoxy-4,5-methylenedioxyphenyl)methyl]-N'-(2-pyridinylmethyl)-N-(6,7,8,9-tetrahydro-5H-cyclohepta[b]pyridin-9-yl)-1,4-benzenedimethanamine; N-[(3-cyanophenyl)methyl]-N'-(2-pyridinylmethyl)-N-(6,7,8,9-tetrahydro-5H-cyclohepta[b]pyridin-9-yl)-1,4-benzenedimethanamine; N-[(3-cyanophenyl)methyl]-N'-(2-pyridinylmethyl)-N-(5,6,7,8-tetrahydro-8-quinolinyl)-1,4-benzenedimethanamine; N-(5-ethylthiophene-2-ylmethyl)-N'-(2-pyridinylmethyl)-N-(6,7,8,9-tetrahydro-5H-cyclohepta[b]pyridin-9-yl)-1,4-benzenedimethanamine; N-(5-ethylthiophene-2-ylmethyl)-N'-(2-pyridinylmethyl)-N-(5,6,7,8-tetrahydro-8-quinolinyl)-1,4-benzenedimethanamine; N-[(2,6-difluorophenyl)methyl]-N'-(2-pyridinylmethyl)-N-(6,7,8,9-tetrahydro-5H-cyclohepta[b]pyridin-9-yl)-1,4-benzenedimethanamine; N-[(2,6-difluorophenyl)methyl]-N'-(2-pyridinylmethyl)-N-(5,6,7,8-tetrahydro-8-quinolinyl)-1,4-benzenedimethanamine; N-[(2-difluoromethoxyphenyl)methyl]-N'-(2-pyridinylmethyl)-N-(6,7,8,9-tetrahydro-5H-cyclohepta[b]pyridin-9-yl)-1,4-benzenedimethanamine; N-(2-difluoromethoxyphenylmethyl)-N'-(2-pyridinylmethyl)-N-(5,6,7,8-tetrahydro-8-quinolinyl)-1,4-benzenedimethanamine; N-(1,4-benzodioxan-6-ylmethyl)-N'-(2-pyridinylmethyl)-N-(6,7,8,9-tetrahydro-5H-cyclohepta[b]pyridin-9-yl)-1,4-benzenedimethanamine; N,N'-bis(2-pyridinylmethyl)-N-[1-(N"-phenyl-N"-methylureido)-4-piperidinyl]-1,4-benzenedimethanamine; N,N'-bis(2-pyridinylmethyl)-N-[N"-p-toluenesulfonylphenylalanyl)-4-piperidinyl]-1,4-benzenedimethanamine; N-[1-(3-pyridinecarboxamido)-4-piperidinyl]-N,N'-bis(2-pyridinylmethyl)-1,4-benzenedimethanamine; N-[1-(cyclopropylcarboxamido)-4-piperidinyl]-N,N'-bis(2-pyridinylmethyl)-1,4-benzenedimethanamine; N-[1-(1-phenylcyclopropylcarboxamido)-4-piperidinyl]-N,N'-bis(2-pyridinylmethyl)-1,4-benzenedimethanamine; N-(1,4-benzodioxan-6-ylmethyl)-N'-(2-pyridinylmethyl)-N-(5,6,7,8-tetrahydro-8-quinolinyl)-1,4-benzenedimethanamine; N-[1-[3-(2-chlorophenyl)-5-methyl-isoxazol-4-carboxamido]-4-piperidinyl]-N,N'-bis(2-pyridinylmethyl)-1,4-benzenedimethanamine; N-[1-(2-thiomethylpyridine-3-carboxamido)-4-piperidinyl]-N,N'-bis(2-pyridinylmethyl)-1,4-benzenedimethanamine; N-[(2,4-difluorophenyl)methyl]-N'-(2-pyridinylmethyl)-N-(5,6,7,8-tetrahydro-8-quinolinyl)-1,4-benzenedimethanamine; N-(1-methylpyrrol-2-ylmethyl)-N'-(2-pyridinylmethyl)-N-(5,6,7,8-tetrahydro-8-quinolinyl)-1,4-benzenedimethanamine; N-[(2-hydroxyphenyl)methyl]-N'-(2-pyridinylmethyl)-N-(5,6,7,8-tetrahydro-8-quinolinyl)-1,4-benzenedimethanamine; N-[(3-methoxy-4,5-methylenedioxyphenyl)methyl]-N'-(2-pyridinylmethyl)-N-(5,6,7,8-tetrahydro-8-quinolinyl)-1,4-benzenedimethanamine; N-(3-pyridinylmethyl)-N'-(2-pyridinylmethyl)-N-(5,6,7,8-tetrahydro-8-quinolinyl)-1,4-benzenedimethanamine; N-[2-(N"-morpholinomethyl)-1-cyclopentyl]-N,N'-bis(2-pyridinylmethyl)-1,4-benzenedimethanamine; N-[(1-methyl-3-piperidinyl)propyl]-N,N'-bis(2-pyridinylmethyl)-1,4-benzenedimethanamine; N-(1-methylbenzimidazol-2-ylmethyl)-N'-(2-pyridinylmethyl)-N-(5,6,7,8-tetrahydro-8-quinolinyl)-1,4-benzenedimethanamine; N-[1-(benzyl)-3-pyrrol idinyl]-N,N'-bis(2-pyridinylmethyl)-1,4-benzenedimethanamine; N-[[(1-phenyl-3-(N"-morpholino)]propyl]-N,N'-bis(2-pyridinylmethyl)-1,4-benzenedimethanamine; N-[1-(iso-propyl)-4-piperidinyl]-N,N'-bis(2-pyridinylmethyl)-1,4-benzenedimethanamine; N-[1-(ethoxycarbonyl)-4-piperidinyl]-N'-(2-pyridinylmethyl)-N-(5,6,7,8-tetrahydro-8-quinolinyl)-1,4-benzenedimethanamine; N-[(1-methyl-3-pyrazolyl)propyl]-N'-(2-pyridinylmethyl)-N-(5,6,7,8-tetrahydro-8-quinolinyl)-1,4-benzenedimethanamine; N-[1-methyl-2-(N",N"-diethylcarboxamido)ethyl]-N,N'-bis(2-pyridinylmethyl)-1,4-benzenedimethanamine; N-[(1-methyl-2-phenylsulfonyl)ethyl]-N'-(2-pyridinylmethyl)-N-(5,6,7,8-tetrahydro-8-quinolinyl)-1,4-benzenedimethanamine; N-[(2-chloro-4,5-methylenedioxyphenyl)methyl]-N'-(2-pyridinylmethyl)-N-(5,6,7,8-tetrahydro-8-quinolinyl)-1,4-benzenedimethanamine; N-[1-methyl-2-[N"-(4-chlorophenyl)carboxamido]ethyl]-N'-(2-pyridinylmethyl)-N-(5,6,7,8-tetrahydro-8-quinolinyl)-1,4-benzenedimethanamine; N-(1-acetoxyindol-3-ylmethyl)-N'-(2-pyridinylmethyl)-N-(6,7,8,9-tetrahydro-5H-cyclohepta[b]pyridin-9-yl)-1,4-benzenedimethanamine; N-[(3-benzyloxy-4-methoxyphenyl)methyl]-N'-(2-pyridinylmethyl)-N-(6,7,8,9-tetrahydro-5H-cyclohepta[b]pyridin-9-yl)-1,4-benzenedimethanamine; N-(3-quinolylmethyl)-N'-(2-pyridinylmethyl)-N-(5,6,7,8-tetrahydro-8-quinolinyl)-1,4-benzenedimethanamine; N-[(8-hydroxy)-2-quinolylmethyl]-N'-(2-pyridinylmethyl)-N-(6,7,8,9-tetrahydro-5H-cyclohepta[b]pyridin-9-yl)-1,4-benzenedimethanamine; N-(2-quinolylmethyl)-N'-(2-pyridinylmethyl)-N-(6,7,8,9-tetrahydro-5H-cyclohepta[b]pyridin-9-yl)-1,4-benzenedimethanamine; N-[(4-acetamidophenyl)methyl]-N'-(2-pyridinylmethyl)-N-(6, 7,8,9-tetrahydro-5H-cyclohepta[b]pyridin-9-yl)-1,4-benzenedimethanamine; N-[1H-imidazol-2-ylmethyl]-N,N'-bis(2-pyridinylmethyl)-1,4-benzenedimethanamine; N-(3-quinolylmethyl)-N'-(2-pyridinylmethyl)-N-(6,7,8,9-tetrahydro-5H-cyclohepta[b]pyridin-9-yl)-1,4-benzenedimethanamine; N-(2-thiazolylmethyl)-N'-(2-pyridinylmethyl)-N-(6,7,8,9-tetrahydro-5H-cyclohepta[b]pyridin-9-yl)-1,4-benzenedimethanamine; N-(4-pyridinylmethyl)-N'-(2-pyridinylmethyl)-N-(6,7,8,9-tetrahydro-5H-cyclohepta[b]pyridin-9-yl)-1,4-benzenedimethanamine; N-[(5-benzyloxy)benzo[b]pyrrol-3-ylmethyl]-N,N'-bis(2-pyridinylmethyl)-1,4-benzenedimethanamine; N-(1-methylpyrazol-2-ylmethyl)-N'-(2-pyridinylmethyl)-N-(6,7,8,9-tetrahydro-5H-cyclohepta[b]pyridin-9-yl)-1,4-benzenedimethanamine; N-[(4-methyl)-1H-imidazol-5-ylmethyl]-N,N'-bis(2-pyridinylmethyl)-1,4-benzenedimethanamine; N-[[(4-dimethylamino)-1-napthalenyl]methyl]-N,N'-bis(2-pyridinylmethyl)-1,4-benzenedimethanamine; N-[1,5-dimethyl-2-phenyl-3-pyrazolinone-4-ylmethyl]-N,N'-bis(2-pyridinylmethyl)-1 ,4-benzenedimethanamine; N-[1-[(1-acetyl-2-(R)-prolinyl]-4-piperidinyl]-N-[2-(2-pyridinyl)ethyl]-N'-(2-pyridinylmethyl)-1,3-benzenedimethanamine; N-[1-[2-acetamidobenzoyl-4-piperidinyl]-4-piperidinyl]-N-[2-(2-pyridinyl)ethyl]-N'-(2-pyridinylmethyl)-1,3-benzenedimethanamine; N-[(2-cyano-2-phenyl)ethyl]-N'-(2-pyridinylmethyl)-N-(6,7,8,9-tetrahydro-5H-cyclohepta[b]pyridin-9-yl)-1,4-benzenedimethanamine; N-[(N"-acetyltryptophanyl)-4-piperidinyl]-N-[2-(2-pyridinyl)ethyl]-N'-(2-pyridinylmethyl)-1,3-benzenedimethanamine; N-[(N"-benzoylvalinyl)-4-piperidinyl]-N-[2-(2-pyridinyl)ethyl]-N'-(2-pyridinylmethyl)-1,3-benzenedimethanamine; N-[(4-dimethylaminophenyl)methyl]-N'-(2-pyridinylmethyl)-N-(6,7,8,9-tetrahydro-5H-cyclohepta[b]pyridin-9-yl)-1,4-benzenedimethanamine; N-(4-pyridinylmethyl)-N'-(2-pyridinylmethyl)-N-(5,6,7,8-tetrahydro-8-quinolinyl)-1,4-benzenedimethanamine; N-(1-methylbenzimadazol-2-ylmethyl)-N'-(2-pyridinylmethyl)-N-(6,7,8,9-tetrahydro-5H-cyclohepta[b]pyridin-9-yl)-1,4-benzenedimethanamine; N-[1-butyl-4-piperidinyl]-N-[2-(2-pyridinyl)ethyl]-N'-(2-pyridinylmethyl)-1,3-benzenedimethanamine; N-[1-benzoyl-4-piperidinyl]-N-[2-(2-pyridinyl)ethyl]-N'-(2-pyridinylmethyl)-1,3-benzenedimethanamine; N-[1-(benzyl)-3-pyrrolidinyl]-N-[2-(2-pyridinyl)ethyl]-N'-(2-pyridinylmethyl)-1,3-benzenedimethanamine; N-[(1-methyl)benzo[b]pyrrol-3-ylmethyl]-N-[2-(2-pyridinyl)ethyl]-N'-(2-pyridinylmethyl)-1,3-benzenedimethanamine; N-[1H-imidazol-4-ylmethyl]-N-[2-(2-pyridinyl)ethyl]-N'-(2-pyridinylmethyl)-1,3-benzenedimethanamine; N-[1-(benzyl)-4-piperidinyl]-N-[2-(2-pyridinyl)ethyl]-N'-(2-pyridinylmethyl)-1,4-benzenedimethanamine; N-[1-methylbenzimidazol-2-ylmethyl]-N-[2-(2-pyridinyl)ethyl]-N'-(2-pyridinylmethyl)-1,4-benzenedimethanamine; N-[(2-phenyl)benzo[b]pyrrol-3-ylmethyl]-N-[2-(2-pyridinyl)ethyl]-N'-(2-pyridinylmethyl)-1,4-benzenedimethanamine; N-[(6-methylpyridin-2-yl)methyl]-N'-(2-pyridinylmethyl)-N-(5,6,7,8-tetrahydro-8-quinolinyl)-1,4-benzenedimethanamine; N-(3-methyl-1H-pyrazol-5-ylmethyl)-N'-(2-pyridinylmethyl)-N-(5,6,7,8-tetrahydro-8-quinolinyl)-1,3-benzenedimethanamine; N-[(2-methoxyphenyl)methyl]-N'-(2-pyridinylmethyl)-N-(5,6,7,8-tetrahydro-8-quinolinyl)-1,3-benzenedimethanamine; N-[(2-ethoxyphenyl)methyl]-N'-(2-pyridinylmethyl)-N-(6,7,8,9-tetrahydro-5H-cyclohepta[b]pyridin-9-yl)-1,3-benzenedimethanamine; N-(benzyloxyethyl)-N'-(2-pyridinylmethyl)-N-(5,6,7,8-tetrahydro-8-quinolinyl)-1,3-benzenedimethanamine; N-[(2-ethoxy-1-naphthalenyl)methyl]-N'-(2-pyridinylmethyl)-N-(5,6,7,8-tetrahydro-8-quinolinyl)-1,3-benzenedimethanamine; N-[(6-methylpyridin-2-yl)methyl]-N'-(2-pyridinylmethyl)-N-(5,6,7,8-tetrahydro-8-quinolinyl)-1,3-benzenedimethanamine; 1-[[4-[[(2-pyridinylmethyl)amino]methyl]phenyl]methyl]guanidine; N-(2-pyridinylmethyl)-N-(8-methyl-8-azabicyclo[3.2.1]octan-3-yl)-1,4-benzenedimethanamine; 1-[[4-[[(2-pyridinylmethyl)amino]methyl]phenyl]methyl]homopiperazine; 1-[[3-[[(2-pyridinylmethyl)amino]methyl]phenyl]methyl]homopiperazine; trans and cis-1-[[4-[[(2-pyridinylmethyl)amino]methyl]phenyl]methyl]-3,5-piperidinediamine; N,N'-[1,4-Phenylenebis(methylene)]bis-4-(2-pyrimidyl)piperazine; 1-[[4-[[(2-pyridinylmethyl)amino]methyl]phenyl]methyl]-1-(2-pyridinyl)methylamine; 2-(2-pyridinyl)-5-[[(2-pyridinylmethyl)amino]methyl]-1,2,3,4-tetrahydroisoquinoline; 1-[[4-[[(2-pyridinylmethyl)amino]methyl]phenyl]methyl]-3,4-diaminopyrrolidine; 1-[[4-[[(2-pyridinylmethyl)amino]methyl]phenyl]methyl]-3,4-diacetylaminopyrrolidine; 8-[[4-[[(2-pyridinylmethyl)amino]methyl]phenyl]methyl]-2,5,8-triaza-3-oxabicyclo [4.3.0]nonane; and 8-[[4-[[(2-pyridinylmethyl)amino]methyl]phenyl]methyl]-2,5,8-triazabicyclo[4.3.0]nonane.

Additional CXCR4 antagonists that may be used to in conjunction with the compositions and methods described herein include those described in WO 2001/085196, WO 1999/050461, WO 2001/094420, and WO 2003/090512, which pertain to compounds that inhibit CXCR4 activity or expression.

### CXCR2 Agonists

### Gro-β, Gro-β T, and variants thereof

Exemplary CXCR2 agonists that may be used in conjunction with the compositions and methods described herein are Gro-β and variants thereof. Gro-β (also referred to as growth-regulated protein β, chemokine (C-X-C motif) ligand 2 (CXCL2), and macrophage inflammatory protein 2-α (MIP2-α)) is a cytokine capable of mobilizing hematopoietic stem and progenitor cells, for example, by stimulating the release of proteases, and particularly MMP9, from peripheral neutrophils. Without being limited by mechanism, MMP9 may induce mobilization of hematopoietic stem and progenitor cells from stem cell niches, such as the bone marrow, to circulating peripheral blood by stimulating the degradation of proteins such as stem cell factor, its corresponding receptor, CD117, and CXCL12, all of which generally maintain hematopoietic stem and progenitor cells immobilized in bone marrow.

In addition to Gro-β*,* exemplary CXCR2 agonists that may be used in conjunction with the compositions and methods described herein are truncated forms of Gro-β*,* such as those that feature a deletion at the N-terminus of Gro-β of from 1 to 8 amino acids (e.g., peptides that feature an N-terminal deletion of 1 amino acids, 2 amino acids, 3 amino acids, 4 amino acids, 5 amino acids, 6 amino acids, 7 amino acids, or 8 amino acids). In some embodiments, CXCR2 agonists that may be used in conjunction with the compositions and methods described herein include Gro-β T, which is characterized by a deletion of the first four amino acids from the N-terminus of Gro-β. Gro-β and Gro-β T are described, for example, in US Patent No. 6,080,398.

In addition, exemplary CXCR2 agonists that may be used in conjunction with the compositions and methods described herein are variants of Gro-β containing an aspartic acid residue in place of the asparagine residue at position 69 of SEQ ID NO: 1. This peptide is referred to herein as Gro-β N69D. Similarly, CXCR2 agonists that may be used with the compositions and methods described herein include variants of Gro-β T containing an aspartic acid residue in place of the asparagine residue at position 65 of SEQ ID NO: 2. This peptide is referred to herein as Gro-β T N65D T. Gro-β N69D and Gro-β T N65D are described, for example, in US Patent No. 6,447,766.

The amino acid sequences of Gro-β, Gro-β T, Gro-β N69D, and Gro-β T N65D are set forth in Table 1, below.

**Table 1. Amino acid sequences of Gro-β and select variants thereof**

| **SEQ ID NO.** | **Description** | **Amino Acid Sequence** |
|---|---|---|
| 1 | Gro-β | APLATELRCQCLQTLQGIHLKNIQSVK VKSPGPHCAQTEVIATLKNGQKACLN PASPMVKKIIEKMLKNGKSN |
| 2 | Gro-β-T | TELRCQCLQTLQGIHLKNIQSVKVKS PGPHCAQTEVIATLKNGQKACLNPAS PMVKKIIEKMLKNGKSN |
| 3 | Gro-β N69D | APLATELRCQCLQTLQGIHLKNIQSVK VKSPGPHCAQTEVIATLKNGQKACLN PASPMVKKIIEKMLKDGKSN |
| 4 | Gro-β-T N65D | TELRCQCLQTLQGIHLKNIQSVKVKS PGPHCAQTEVIATLKNGQKACLNPAS PMVKKIIEKMLKDGKSN |

Additional CXCR2 agonists that may be used in conjunction with the compositions and methods described herein include other variants of Gro-β*,* such as peptides that have one or more amino acid substitutions, insertions, and/or deletions relative to Gro-β. In some embodiments, CXCR2 agonists that may be used in conjunction with the compositions and methods described herein include peptides having at least 85% sequence identity to the amino acid sequence of SEQ ID NO: 1 (e.g., a peptide having at least 85%, 90%, 95%, 96%, 97%, 98%, 99%, 99.5%, or 100% sequence identity to the amino acid sequence of SEQ ID NO: 1). In some embodiments, the amino acid sequence of the CXCR2 agonist differs from that of SEQ ID NO: 1 only by way of one or more conservative amino acid substitutions. In some embodiments, in some embodiments, the amino acid sequence of the CXCR2 agonist differs from that of SEQ ID NO: 1 by no more than 20, no more than 15, no more than 10, no more than 5, or no more than 1 nonconservative amino acid substitutions.

Additional examples of CXCR2 agonists useful in conjunction with the compositions and methods described herein are variants of Gro-β T, such as peptides that have one or more amino acid substitutions, insertions, and/or deletions relative to Gro-β T. In some embodiments, the CXCR2 agonist may be a peptide having at least 85% sequence identity to the amino acid sequence of SEQ ID NO: 2 (e.g., a peptide having at least 85%, 90%, 95%, 96%, 97%, 98%, 99%, 99.5%, or 100% sequence identity to the amino acid sequence of SEQ ID NO: 2). In some embodiments, the amino acid sequence of the CXCR2 agonist differs from that of SEQ ID NO: 2 only by way of one or more conservative amino acid substitutions. In some embodiments, in some embodiments, the amino acid sequence of the CXCR2 agonist differs from that of SEQ ID NO: 2 by no more than 20, no more than 15, no more than 10, no more than 5, or no more than 1 nonconservative amino acid substitutions.

Additional examples of CXCR2 agonists useful in conjunction with the compositions and methods described herein are variants of Gro-β N69D, such as peptides that have one or more amino acid substitutions, insertions, and/or deletions relative to Gro-β N69D. In some embodiments, the CXCR2 agonist may be a peptide having at least 85% sequence identity to the amino acid sequence of SEQ ID NO: 3 (e.g., a peptide having at least 85%, 90%, 95%, 96%, 97%, 98%, 99%, 99.5%, or 100% sequence identity to the amino acid sequence of SEQ ID NO: 3). In some embodiments, the amino acid sequence of the CXCR2 agonist differs from that of SEQ ID NO: 3 only by way of one or more conservative amino acid substitutions. In some embodiments, in some embodiments, the amino acid sequence of the CXCR2 agonist differs from that of SEQ ID NO: 3 by no more than 20, no more than 15, no more than 10, no more than 5, or no more than 1 nonconservative amino acid substitutions.

Additional examples of CXCR2 agonists useful in conjunction with the compositions and methods described herein are variants of Gro-β T N65D, such as peptides that have one or more amino acid substitutions, insertions, and/or deletions relative to Gro-β T N65D. In some embodiments, the CXCR2 agonist may be a peptide having at least 85% sequence identity to the amino acid sequence of SEQ ID NO: 4 (e.g., a peptide having at least 85%, 90%, 95%, 96%, 97%, 98%, 99%, 99.5%, or 100% sequence identity to the amino acid sequence of SEQ ID NO: 4). In some embodiments, the amino acid sequence of the CXCR2 agonist differs from that of SEQ ID NO: 4 only by way of one or more conservative amino acid substitutions. In some embodiments, in some embodiments, the amino acid sequence of the CXCR2 agonist differs from that of SEQ ID NO: 4 by no more than 20, no more than 15, no more than 10, no more than 5, or no more than 1 nonconservative amino acid substitutions.

### Agonistic anti-CXCR2 antibodies and antigen-binding fragments thereof

In some embodiments, the CXCR2 agonist is an antibody or antigen-binding fragment thereof that binds CXCR2 and activates CXCR2 signal transduction. In some embodiments, the CXCR2 agonist may be an antibody or antigen-binding fragment thereof that binds the same epitope on CXCR2 as Gro-β or a variant or truncation thereof, such as Gro-β T, as assessed, for example, by way of a competitive CXCR2 binding assay. In some embodiments, the CXCR2 agonist is an antibody or an antigen-binding fragment thereof that competes with Gro-β or a variant or truncation thereof, such as Gro-β T, for binding to CXCR2.

In some embodiments, the antibody or antigen-binding fragment thereof is selected from the group consisting of a monoclonal antibody or antigen-binding fragment thereof, a polyclonal antibody or antigen-binding fragment thereof, a humanized antibody or antigen-binding fragment thereof, a bispecific antibody or antigen-binding fragment thereof, a dual-variable immunoglobulin domain, a single-chain Fv molecule (scFv), a diabody, a triabody, a nanobody, an antibody-like protein scaffold, a Fv fragment, a Fab fragment, a F(ab')₂ molecule, and a tandem di-scFv. In some embodiments, the antibody has an isotype selected from the group consisting of IgG, IgA, IgM, IgD, and IgE.

### Synthetic CXCR2 Agonists

The peptidic CXCR2 agonists described herein, such as Gro-β*,* Gro-β T, and variants thereof, may be prepared synthetically, for instance, using solid phase peptide synthesis techniques. Systems and processes for performing solid phase peptide synthesis include those that are known in the art and have been described, for instance, in US Patent Nos. 9,169,287; 9,388,212; 9,206,222; 6,028,172; and 5,233,044, among others, which pertain to protocols and techniques for the synthesis of peptides on solid support. Solid phase peptide synthesis is a process in which amino acid residues are added to peptides that have been immobilized on a solid support, such as a polymeric resin (e.g., a hydrophilic resin, such as a polyethylene-glycol-containing resin, or hydrophobic resin, such as a polystyrene-based resin).

Peptides, such as those containing protecting groups at amino, hydroxy, thiol, and carboxy substituents, among others, may be bound to a solid support such that the peptide is effectively immobilized on the solid support. For example, the peptides may be bound to the solid support via their C termini, thereby immobilizing the peptides for subsequent reaction in at a resin-liquid interface.

The process of adding amino acid residues to immobilized peptides can include exposing a deprotection reagent to the immobilized peptides to remove at least a portion of the protection groups from at least a portion of the immobilized peptides. The deprotection reagent exposure step can be configured, for instance, such that side-chain protection groups are preserved, while N-terminal protection groups are removed. For instance, an exemplary amino protecting contains a fluorenylmethyloxycarbonyl (Fmoc) substituent. A deprotection reagent containing a strongly basic substance, such as piperidine (e.g., a piperidine solution in an appropriate organic solvent, such as dimethyl formamide (DMF)) may be exposed to the immobilized peptides such that the Fmoc protecting groups are removed from at least a portion of the immobilized peptides. Other protecting groups suitable for the protection of amino substituents include, for instance, the tert-butyloxycarbonyl (Boc) moiety. A deprotection reagent comprising a strong acid, such as trifluoroacetic acid (TFA) may be exposed to immobilized peptides containing a Boc-protected amino substituent so as to remove the Boc protecting group by an ionization process. In this way, peptides can be protected and deprotected at specific sites, such as at one or more side-chains or at the N- or C-terminus of an immobilized peptide so as to append chemical functionality regioselectively at one or more of these positions. This can be used, for instance, to derivatize a side-chain of an immobilized peptide, or to synthesize a peptide, e.g., from the C-terminus to the N-terminus.

The process of adding amino acid residues to immobilized peptides can include, for instance, exposing protected, activated amino acids to the immobilized peptides such that at least a portion of the activated amino acids are bonded to the immobilized peptides to form newly-bonded amino acid residues. For example, the peptides may be exposed to activated amino acids that react with the deprotected N-termini of the peptides so as to elongate the peptide chain by one amino acid. Amino acids can be activated for reaction with the deprotected peptides by reaction of the amino acid with an agent that enhances the electrophilicity of the backbone carbonyl carbon of the amino acid. For example, phosphonium and uronium salts can, in the presence of a tertiary base (e.g., diisopropylethylamine (DIPEA) and triethylamine (TEA), among others), convert protected amino acids into activated species (for example, BOP, PyBOP, HBTU, and TBTU all generate HOBt esters). Other reagents can be used to help prevent racemization that may be induced in the presence of a base. These reagents include carbodiimides (for example, DCC or WSCDI) with an added auxiliary nucleophile (for example, 1-hydroxy-benzotriazole (HOBt), 1-hydroxy-azabenzotriazole (HOAt), or HOSu) or derivatives thereof. Another reagent that can be utilized to prevent racemization is TBTU. The mixed anhydride method, using isobutyl chloroformate, with or without an added auxiliary nucleophile, can also be used, as well as the azide method, due to the low racemization associated with this reagent. These types of compounds can also increase the rate of carbodiimide-mediated couplings, as well as prevent dehydration of Asn and Gln residues. Typical additional reagents include also bases such as N,N-diisopropylethylamine (DIPEA), triethylamine (TEA) or N-methylmorpholine (NMM). These reagents are described in detail, for instance, in US Patent No. 8,546,350.

During the recombinant expression and folding of Gro-β and Gro-β T in aqueous solution, a particular C-terminal asparagine residue (Asn69 within Gro-β and Asn65 within Gro-β T) is prone to deamidation. This process effectuates the conversion of the asparagine residue to aspartic acid. Without wishing to be bound by any theory, the chemical synthesis of Gro-β and Gro-β T may overcome this problem, for instance, by providing conditions that reduce the exposure of this asparagine residue to nucleophilic solvent. When prepared synthetically (i.e., chemically synthesized), for instance, using, e.g., the solid phase peptide synthesis techniques described above, synthetic Gro-β, Gro-β T, and variants thereof that may be used in conjunction with the compositions and methods described herein may have a purity of, e.g., at least about 95% relative to the deamidated versions of these peptides (i.e., contain less than 5% of the corresponding deamidated peptide). For instance, synthetic Gro-β, Gro-β T, and variants thereof that may be used in conjunction with the compositions and methods described herein may have a purity of about 95%, 95.5%, 96%, 96.5%, 97%, 97.5%, 98%, 98.5%, 99%, 99.5%, 99.6%, 99.7%, 99.8%, 99.9%, 99.99%, or more, relative to the deamidated versions of these peptides(e.g., the Asn69 deamidated version of SEQ ID NO: 1 or the Asn65 deamidated version of SEQ ID NO: 2). For instance, s\Synthetic Gro-β, Gro-β T, and variants thereof may have, for instance, a purity of from about 95% to about 99.99%, such as a purity of from about 95% to about 99.99%, about 96% to about 99.99%, about 97% to about 99.99%, about 98% to about 99.99%, about 99% to about 99.99%, about 99.9% to about 99.99%, about 95% to about 99.5%, about 96% to about 99.5%, about 95% to about 99%, or about 97% to about 99% relative to the deamidated versions of these peptides (e.g., the Asn69 deamidated version of SEQ ID NO: 1 or the Asn65 deamidated version of SEQ ID NO: 2).

### Busulfan Conditioning

High-dose busulfan therapy has several advantages for use in marrow ablation/pretransplant treatment. First, when using chemotherapy alone for conditioning of patients undergoing marrow transplantation, one avoids the dependence on a radiation unit with, usually, limited capacity to deliver the necessary treatment on a fixed schedule. Second, high total-radiation doses are very toxic, especially to the lungs, and may require special protective measures (shielding). Such excessive toxicity is usually not seen with combination chemotherapy. Third, a radiation based regimen can only be delivered to patients who have not been previously irradiated. Many patients with lymphoma, Hodgkin's disease and leukemia have had previous (extensive) radiation for control of locally aggressive disease in sanctuary sites like the central nervous system or to sites of bulky disease such as the mediastinum or the neck. Additional radiation as part of the pretransplantation conditioning regimen may cause irreversible and often fatal toxicity in such cases. However, a majority of previously radiated patients can safely receive a busulfan-based regimen, provided that the previous acute radiation toxicity (usually within the first 2-4 months after therapy) has subsided. Fourth, in selected patients who suffer recurrent leukemia after allogeneic marrow grafting, a second marrow transplant may still offer a chance for long-term disease control or even cure

The methods of the present disclosure may further comprise busulfan conditioning, wherein the busulfan conditioning occurs prior to the administration of the expanded population of hematopoietic stem cells.

The busulfan conditioning may comprise administering busulfan at an amount of less than about 40 mg/kg, less than about 35 mg/kg, less than about 30 mg/kg, less than about 25 mg/kg, less than about 20 mg/kg, less than about 15 mg/kg, less than about 10 mg/kg, less than about 5 mg/kg, less than about 4 mg/kg, less than about 3 mg/kg, less than about 2 mg/kg, less than about 1 mg/kg, less than about 0.5 mg/kg, or less than about 0.1 mg/kg prior to administration of the expanded population of hematopoietic stem cells.

The busulfan conditioning may be a reduced intensity (i.e., lower dosage) than a comparable method wherein the population of hematopoietic stem cells is not expanded, or wherein the population of hematopoietic stem cells is not expanded by contacting a population of hematopoietic stem cells with an aryl hydrocarbon receptor antagonist in an amount sufficient to produce the expanded population of hematopoietic stem cells.

The busulfan conditioning may be reduced at least about 75% in intensity (i.e., lower dosage) relative to a comparable method, reduced at least about 50% in intensity, reduced at leat about 40% in intensity, reduced at least about 30% in intensity, reduced at least about 25% in intensity, reduced at least about 20% in intensity, reduced at least about 15% in intensity, or reduced at least about 10% in intensity (i.e., lower dosage) relative to a comparable method wherein the population of hematopoietic stem cells is not expanded, or wherein the population of hematopoietic stem cells is not expanded by contacting a population of hematopoietic stem cells with an aryl hydrocarbon receptor antagonist in an amount sufficient to produce the expanded population of hematopoietic stem cells.The busulfan conditioning may be administered orally.

A pharmaceutically acceptable formulation for parenteral administration of busulfan may be used for conditioning. The formulation may comprise busulfan dissolved in a water miscible, physiologically acceptable busulfan solvent at a concentration of 1-75 mg/ml. The formulation may further comprise water. The water miscible busulfan solvent may be N',N-dimethylacetamide, an aqueous solution of polyethyleneglycol or a mixture of N'N-dimethylacetamide and an aqueous carrier solution allowing busulfan solubility and stability. The aqueous carrier solution may be a polyethylene glycol solution. The N'N-dimethylacetamide is at a concentration of 5%-99% and the polyethyleneglycol is at a concentration of 5%-50%. The polyethyleneglycol may have a molecular weight between 200 and 2,000 daltons, more preferably between 350 and 450 daltons. The busulfan solvent may be propylene glycol or an aqueous solution of hydroxypropylbetacyclodextrin.

Further pharmaceutically acceptable formulations for parenteral administration of busulfan may be, for example, formulations comprising 1-7.5 mg/ml dissolved busulfan, 35%-45% polyethyleneglycol, 45%-55% water, and 5%-15% N'N-dimethylacetamide. A pharmaceutically acceptable formulation for parenteral administration of busulfan comprising 1-15 mg/ml dissolved busulfan, 35-45% polyethyleneglycol-400, 35-45% water and 15-25% N',N-dimethylacetamide is preferred.

### Stem Cells

In some embodiments, the stem cells of which the population is modified (e.g., expanded) with the compositions and methods described are capable of being expanded upon contacting the aryl hydrocarbon receptor antagonist. In some embodiments, the stem cells are not genetically modified stem cells. In some embodiments, the stem cells are genetically modified stem cells.

In some embodiments, the stem cells are embryonic stem cells or adult stem cells. In some embodiments, the stem cells are totipotentent stem cells, pluripotent stem cells, multipoteltent stem cells, oligopotent stem cells, or unipotent stem cells. In some embodiments, the stem cells are tissue-specific stem cells.

In some embodiments, the stem cells are hematopoietic stem cells, intestinal stem cells, osteoblastic stem cells, mesenchymal stem cells (i.e., lung mesenchymal stem cells, bone marrow-derived mesenchymal stromal cells, or bone marrow stromal cells), neural stem cells (i.e., neuronal dopaminergic stem cells or motor-neuronal stem cells), epithelial stem cells (i.e., lung epithelial stem cells, breast epithelial stem cells, vascular epithelial stem cells, or intestinal epithelial stem cells), cardiac myocyte progenitor stem cells, skin stem cells (i.e., epidermal stem cells or follicular stem cells (hair follicle stem cells)), skeletal muscle stem cells, adipose stem cells, liver stem cells, induced pluripotent stem cells, umbilical cord stem cells, amniotic fluid stem cells, limbal stem cells, dental pulp stem cells, placental stem cells, myoblasts, endothelial progenitor cells, exfoliated teeth derived stem cells, or hair follicle stem cells.

In some embodiments, the stem cells are hematopoietic stem cells.

In some embodiments, the stem cells are primary stem cells. For example, the stem cells are obtained from bone marrow, adipose tissue, or blood. In some embodiments, the stem cells are cultured stem cells.

In some embodiments, the stem cells are CD34+ cells. In some embodiments, the stem cells are CD90+ cells. In some embodiments, the stem cells are CD45RA- cells. In some embodiments, the stem cells are CD34+CD90+ cells. In some embodiments, the stem cells are CD34+CD45RA-cells. In some embodiments, the stem cells are CD90+CD45RA- cells. In some embodiments, the stem cells are CD34+CD90+CD45RA- cells.

In some embodiments, the hematopoietic stem cells are extracted from the bone marrow, mobilized into the peripheral blood and then collected by apheresis, or isolated from umbilical cord blood units.

In some embodiments, the hematopoietic stem cells are CD34+ hematopoietic stem cells. In some embodiments, the hematopoietic stem cells are CD90+ hematopoietic stem cells. In some embodiments, the hematopoietic stem cells are CD45RA- hematopoietic stem cells. In some embodiments, the hematopoietic stem cells are CD34+CD90+ hematopoietic stem cells. In some embodiments, the hematopoietic stem cells are CD34+CD45RA- hematopoietic stem cells. In some embodiments, the hematopoietic stem cells are CD90+CD45RA- hematopoietic stem cells. In some embodiments, the hematopoietic stem cells are CD34+CD90+CD45RA- hematopoietic stem cells.

### Gene-modified Hematopoietic Stem and Progenitor Cells

Hematopoietic stem and progenitor cells for use in conjunction with the compositions and methods described herein include those that have been genetically modified, such as those that have been altered so as to express a therapeutic transgene. Compositions and methods for the genetic modification of hematopoietic stem and progenitor cells are described in the sections that follow.

The compositions and methods described herein provide strategies for disrupting a gene of interest and for promoting the expression of target genes in populations of hematopoietic stem and progenitor cells, as well as for expanding these cells. For instance, a population of hematopoietic stem cells may be expanded according to the methods described herein and may be genetically modified, e.g., so as to exhibit an altered gene expression pattern. Alternatively, a population of cells may be enriched with hematopoietic stem cells, or a population of hematopoietic stem cells may be maintained in a multi-potent state, and the cells may further be modified using established genome editing techniques known in the art. For instance, one may use a genome editing procedure to promote the expression of an exogenous gene or inhibit the expression of an endogenous gene within a hematopoietic stem cell. Populations of hematopoietic stem cells may be expanded, enriched, or maintained in a multi-potent state according to the methods described herein and subsequently genetically modified so as to express a desired target gene, or populations of these cells may be genetically modified first and then expanded, enriched, or maintained in a multi-potent state.

In some embodiments, the populations (e.g., plurality) of hematopoietic stem cells are expanded, enriched, or maintained in a multi-potent state according to the methods described herein by being contacted with an aryl hydrocarbon receptor antagonist as described herein and subsequently genetically modified so as to express a desired target gene and substantially maintain the engraftable properties of the hematopoietic stem cells cells. In some embodiments, the populations (e.g., plurality) of hematopoietic stem cells are expanded, enriched, or maintained in a multi-potent state according to the methods described herein by being contacted with an aryl hydrocarbon receptor antagonist as described herein and subjected to conditions during a period of time sufficient to induce cell cycling, and subsequently genetically modified so as to express a desired target gene and substantially maintain the engraftable properties of the hematopoietic stem cells cells. In one non-limiting embodiment, the conditions sufficient to induce cell cycling may comprise contacting the hematopoietic stem cells with one or more cytokines in amounts sufficient to induce cell cycling. Non-limiting examples of cytokines include SCF, IL6, TPO, FLT3L, and combinations thereof. Other agents or methods may also be used to induce cell cycling.

In some embodiments, the period of time sufficient to induce cell cycling may be at least about 1 day, at least about 2 days, at least about 3 days, at least about 4 days, or at least about 5 days. In some embodiments, the period of time sufficient to induce cell cycling is about 1 to about 5 days, about 1 to about 4 days, about 2 to about 4 days, about 1 to about 3 days, or about 2 to about 3 days. In some embodiments, the period of time sufficient to induce cell cycling may vary depending on the lineage of the cells.

In some embodiments, contacting the hematopoietic stem cells with an aryl hydrocarbon receptor antagonist does not affect cell cycling. Advantageously, actively cycling cells may be more easily genetically modified so as to express a desired target gene than a non-cycling cell. Additionally, in some embodiments, contacting the hematopoietic stem cells with an aryl hydrocarbon receptor antagonist does not prevent stem cells from entering the cell cycle, and allows the stem cells to remain as stem cells (e.g., including dividing so as to multiply in number without substantially differentiating), delaying differentiation and prolonging engraftment potential relative to cells (e.g., hematopoietic stem cells) not contacted with an aryl hydrocarbon receptor antagonist.

In some embodiments, the populations (e.g., plurality) of hematopoietic stem cells are expanded, enriched, or maintained in a multi-potent state according to the methods described herein by being contacted with an aryl hydrocarbon receptor antagonist as described herein during at least a period of time sufficient to induce cell cycling and subsequently genetically modified so as to express a desired target gene resulting in improved genetic modification relative to a comparable method wherein the populations (e.g., plurality) of hematopoietic stem cells are not contacted with an aryl hydrocarbon receptor antagonist as described herein during a period of time sufficient to induce cell cycling prior to being subsequently genetically modified.

In some embodiments, the populations of hematopoietic stem cells are expanded, enriched, or maintained in a multi-potent state according to the methods described herein by being contacted with an aryl hydrocarbon receptor antagonist as described herein during a period of time sufficient to induce cell cycling and subsequently genetically modified so as to express a desired target gene resulting in improved engraftment potential relative to a comparable method wherein the the populations of hematopoietic stem cells are not contacted with an aryl hydrocarbon receptor antagonist as described herein during a period of time sufficient to induce cell cycling prior to being subsequently genetically modified.

In some embodiments, hematopoietic stem cells are expanded, enriched, or maintained in a multi-potent state according to the methods described herein by being contacted with an aryl hydrocarbon receptor antagonist as described herein during a period of time sufficient to induce cell cycling in substantially all of the hematopoietic stem cells.

In some embodiments, the populations (e.g., plurality) of hematopoietic stem cells are expanded subsequently to being genetically modified. For example, the hematopoietic stem cells may be expanded in the presence of an aryl hydrocarbon receptor antagonist subsequently to being genetically modified. Expansion of the genetically modified hematopoietic stem cells may be performed, for example, to increase the number of engraftable genetically modified cells in a hematopoietic stem cell graft.

A wide array of methods has been established for the incorporation of target genes into the genome of a cell (e.g., a mammalian cell, such as a murine or human cell) so as to facilitate the expression of such genes.

### Polynucleotides encoding target genes

One example of a platform that can be used to facilitate the expression of a target gene in a hematopoietic stem cell is by the integration of the polynucleotide encoding a target gene into the nuclear genome of the cell. A variety of techniques have been developed for the introduction of exogenous genes into a eukaryotic genome. One such technique involves the insertion of a target gene into a vector, such as a viral vector. Vectors for use with the compositions and methods described herein can be introduced into a cell by a variety of methods, including transformation, transfection, direct uptake, projectile bombardment, and by encapsulation of the vector in a liposome. Examples of suitable methods of transfecting or transforming cells include calcium phosphate precipitation, electroporation, microinjection, infection, lipofection and direct uptake. Such methods are described in more detail, for example, in Green, et al., Molecular Cloning: A Laboratory Manual, Fourth Edition, Cold Spring Harbor University Press, New York (2014); and Ausubel, et al., Current Protocols in Molecular Biology, John Wiley & Sons, New York (2015).

Exogenous genes can also be introduced into a mammalian cell through the use of a vector containing the gene of interest to cell membrane phospholipids. For example, vectors can be targeted to the phospholipids on the extracellular surface of the cell membrane by linking the vector molecule to a VSV-G protein, a viral protein with affinity for all cell membrane phospholipids. Viral vectors containing the VSV-G protein are described in further detail, e.g., in US 5,512,421; and in US 5,670,354.

Recognition and binding of the polynucleotide encoding a target gene by mammalian RNA polymerase is an important molecular event for gene expression to occur. As such, one may include sequence elements within the polynucleotide that exhibit a high affinity for transcription factors that recruit RNA polymerase and promote the assembly of the transcription complex at the transcription initiation site. Such sequence elements include, e.g., a mammalian promoter, the sequence of which can be recognized and bound by specific transcription initiation factors and ultimately RNA polymerase. Alternatively, promoters derived from viral genomes can be used for the stable expression of target genes in mammalian cells. Examples of functional viral promoters that can be used to promote mammalian expression of these enzymes include adenovirus late promoter, vaccinia virus 7.5K promoter, SV40 promoter, cytomegalovirus promoter, mouse mammary tumor virus (MMTV) promoter, LTR promoter of HIV, promoter of moloney virus, Epstein barr virus (EBV) promoter, Rous sarcoma virus (RSV) promoter, and the cytomegalovirus (CMV) promoter. Additional viral promoters include the SV40 late promoter from simian virus 40, the Baculovirus polyhedron enhancer/promoter element, Herpes Simplex Virus thymidine kinase (HSV tk) promoter, and the 35S promoter from Cauliflower Mosaic Virus. Suitable phage promoters for use with the compositions and methods described herein include, but are not limited to, the E. coli T7 and T3 phage promoters, the S. typhimurium phage SP6 promoter, B. subtilis SP01 phage and B. subtilis phage phi 29 promoters, and N4 phage and K11 phage promoters as described in US 5,547,892.

Upon incorporation of a polynucleotide encoding a target gene has been incorporated into the genome of a cell (e.g., the nuclear genome of a hematopoietic stem cell), the transcription of this polynucleotide can be induced by methods known in the art. For example expression can be induced by exposing the mammalian cell to an external chemical reagent, such as an agent that modulates the binding of a transcription factor and/or RNA polymerase to the mammalian promoter and thus regulate gene expression. The chemical reagent can serve to facilitate the binding of RNA polymerase and/or transcription factors to the mammalian promoter, e.g., by removing a repressor protein that has bound the promoter. Alternatively, the chemical reagent can serve to enhance the affinity of the mammalian promoter for RNA polymerase and/or transcription factors such that the rate of transcription of the gene located downstream of the promoter is increased in the presence of the chemical reagent. Examples of chemical reagents that potentiate polynucleotide transcription by the above mechanisms include tetracycline and doxycycline. These reagents are commercially available (Life Technologies, Carlsbad, CA) and can be administered to a mammalian cell in order to promote gene expression according to established protocols.

Other DNA sequence elements that may be included in polynucleotides for use with the compositions and methods described herein include enhancer sequences. Enhancers represent another class of regulatory elements that induce a conformational change in the polynucleotide comprising the gene of interest such that the DNA adopts a three-dimensional orientation that is favorable for binding of transcription factors and RNA polymerase at the transcription initiation site. Thus, polynucleotides for use with the compositions and methods described herein include those that encode a target gene and additionally include a mammalian enhancer sequence. Many enhancer sequences are now known from mammalian genes, and examples include enhancers from the genes that encode mammalian globin, elastase, albumin, α-fetoprotein, and insulin. Enhancers for use with the compositions and methods described herein also include those that are derived from the genetic material of a virus capable of infecting a eukaryotic cell. Examples include the SV40 enhancer on the late side of the replication origin (bp 100-270), the cytomegalovirus early promoter enhancer, the polyoma enhancer on the late side of the replication origin, and adenovirus enhancers. Additional enhancer sequences that induce activation of eukaryotic gene transcription are disclosed in Yaniv et al. Nature 297:17 (1982). An enhancer may be spliced into a vector containing a polynucleotide encoding a target gene, for example, at a position 5' or 3' to this gene. In a preferred orientation, the enhancer is positioned at the 5' side of the promoter, which in turn is located 5' relative to the polynucleotide encoding the target gene.

In addition to promoting high rates of transcription and translation, stable expression of an exogenous gene in a hematopoietic stem cell can be achieved by integration of the polynucleotide comprising the gene into the nuclear DNA of the cell. A variety of vectors for the delivery and integration of polynucleotides encoding exogenous proteins into the nuclear DNA of a mammalian cell have been developed. Examples of expression vectors are disclosed in, e.g., WO94/11026. Expression vectors for use with the compositions and methods described herein contain a polynucleotide sequence that encodes a target gene, as well as, e.g., additional sequence elements used for the expression of these enzymes and/or the integration of these polynucleotide sequences into the genome of a mammalian cell. Certain vectors that can be used for the expression of target genes include plasmids that contain regulatory sequences, such as promoter and enhancer regions, which direct gene transcription. Other useful vectors for expression of target genes contain polynucleotide sequences that enhance the rate of translation of these genes or improve the stability or nuclear export of the mRNA that results from gene transcription. These sequence elements often encode features within RNA transcripts that enhance the nuclear export, cytosolic half-life, and ribosomal affinity of these molecules, e.g., 5' and 3' untranslated regions, an internal ribosomal entry site (IRES), and polyadenylation signal site in order to direct efficient transcription of the gene carried on the expression vector. Exemplary expression vectors may also contain a polynucleotide encoding a marker for selection of cells that contain such a vector. Non-limiting examples of a suitable marker include genes that encode resistance to antibiotics, such as ampicillin, chloramphenicol, kanamycin, or nourseothricin.

### Vectors for the expression of target genes

Viral genomes provide a rich source of vectors that can be used for the efficient delivery of exogenous genes into a mammalian cell. Viral genomes are particularly useful vectors for gene delivery because the polynucleotides contained within such genomes are typically incorporated into the nuclear genome of a mammalian cell by generalized or specialized transduction. These processes occur as part of the natural viral replication cycle, and often do not require added proteins or reagents in order to induce gene integration. Examples of viral vectors include a retrovirus, adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g. measles and Sendai), positive strand RNA viruses, such as picornavirus and alphavirus, and double stranded DNA viruses including herpes virus (e.g., Herpes Simplex virus types 1 and 2, Epstein-Barr virus, cytomegalovirus), and poxvirus (e.g., vaccinia, modified vaccinia Ankara (MVA), fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D-type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996). Other examples of viral vectors include murine leukemia viruses, murine sarcoma viruses, mouse mammary tumor virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus and lentiviruses. Other examples of vectors are described in, e.g., US 5,801,030.

### Additional transfection methods

Other techniques that can be used to introduce a polynucleotide, such as DNA or RNA (e.g., mRNA, tRNA, siRNA, miRNA, shRNA, chemically modified RNA) into a mammalian cell are well known in the art. For instance, electroporation can be used to permeabilize mammalian cells by the application of an electrostatic potential. Mammalian cells, such as hematopoietic stem cells, subjected to an external electric field in this manner are subsequently predisposed to the uptake of exogenous nucleic acids. Electroporation of mammalian cells is described in detail, e.g., in Chu et al. Nucleic Acids Research 15:1311 (1987). A similar technique, Nucleofection^{™}, utilizes an applied electric field in order to stimulate the update of exogenous polynucleotides into the nucleus of a eukaryotic cell. Nucleofection^{™} and protocols useful for performing this technique are described in detail, e.g., in Distler et al. Experimental Dermatology 14:315 (2005), as well as in US 2010/0317114.

Additional techniques useful for the transfection of hematopoietic stem cells include the squeeze-poration methodology. This technique induces the rapid mechanical deformation of cells in order to stimulate the uptake of exogenous DNA through membranous pores that form in response to the applied stress. This technology is advantageous in that a vector is not required for delivery of nucleic acids into a cell, such as a hematopoietic stem cell. Squeeze-poration is described in detail, e.g., in Sharei et al. Journal of Visualized Experiments 81 :e50980 (2013).

Lipofection represents another technique useful for transfection of hematopoietic stem cells. This method involves the loading of nucleic acids into a liposome, which often presents cationic functional groups, such as quaternary or protonated amines, towards the liposome exterior. This promotes electrostatic interactions between the liposome and a cell due to the anionic nature of the cell membrane, which ultimately leads to uptake of the exogenous nucleic acids, e.g., by direct fusion of the liposome with the cell membrane or by endocytosis of the complex. Lipofection is described in detail, e.g., in US 7,442,386. Similar techniques that exploit ionic interactions with the cell membrane to provoke the uptake of foreign nucleic acids include contacting a cell with a cationic polymer-nucleic acid complex. Cationic molecules that associate with polynucleotides so as to impart a positive charge favorable for interaction with the cell membrane include activated dendrimers (described, e.g., in Dennig, Topics in Current Chemistry 228:227 (2003)) and diethylaminoethyl (DEAE)-dextran, the use of which as a transfection agent is described in detail, e.g., in Gulick et al. Current Protocols in Molecular Biology 40:1:9.2:9.2.1 (1997). Magnetic beads are another tool that can be used to transfect hematopoietic stem cells in a mild and efficient manner, as this methodology utilizes an applied magnetic field in order to direct the uptake of nucleic acids. This technology is described in detail, e.g., in US 2010/0227406.

Another useful tool for inducing the uptake of exogenous nucleic acids by hematopoietic stem cells is laserfection, a technique that involves exposing a cell to electromagnetic radiation of a particular wavelength in order to gently permeabilize the cells and allow polynucleotides to penetrate the cell membrane. This technique is described in detail, e.g., in Rhodes et al. Methods in Cell Biology 82:309 (2007).

Microvesicles represent another potential vehicle that can be used to modify the genome of a hematopoietic stem cell according to the methods described herein. For instance, microvesicles that have been induced by the co-overexpression of the glycoprotein VSV-G with, e.g., a genome-modifying protein, such as a nuclease, can be used to efficiently deliver proteins into a cell that subsequently catalyze the site-specific cleavage of an endogenous polynucleotide sequence so as to prepare the genome of the cell for the covalent incorporation of a polynucleotide of interest, such as a gene or regulatory sequence. The use of such vesicles, also referred to as Gesicles, for the genetic modification of eukaryotic cells is described in detail, e.g., in Quinn et al. Genetic Modification of Target Cells by Direct Delivery of Active Protein [abstract]. In: Methylation changes in early embryonic genes in cancer [abstract], in: Proceedings of the 18th Annual Meeting of the American Society of Gene and Cell Therapy; 2015 May 13, Abstract No. 122.

### Modulation of Gene Expression using Gene Editing Techniques

In addition to viral vectors, a variety of additional tools have been developed that can be used for the incorporation of exogenous genes into hematopoietic stem cells. One such method that can be used for incorporating polynucleotides encoding target genes into hematopoietic stem cells involves the use of transposons. Transposons are polynucleotides that encode transposase enzymes and contain a polynucleotide sequence or gene of interest flanked by 5' and 3' excision sites. Once a transposon has been delivered into a cell, expression of the transposase gene commences and results in active enzymes that cleave the gene of interest from the transposon. This activity is mediated by the site-specific recognition of transposon excision sites by the transposase. In certain cases, these excision sites may be terminal repeats or inverted terminal repeats. Once excised from the transposon, the gene of interest can be integrated into the genome of a mammalian cell by transposase-catalyzed cleavage of similar excision sites that exist within the nuclear genome of the cell. This allows the gene of interest to be inserted into the cleaved nuclear DNA at the complementary excision sites, and subsequent covalent ligation of the phosphodiester bonds that join the gene of interest to the DNA of the mammalian cell genome completes the incorporation process. In certain cases, the transposon may be a retrotransposon, such that the gene encoding the target gene is first transcribed to an RNA product and then reverse-transcribed to DNA before incorporation in the mammalian cell genome. Transposon systems include the piggybac transposon (described in detail in, e.g., WO 2010/085699) and the sleeping beauty transposon (described in detail in, e.g., US2005/0112764).

Another useful tool for the disruption and integration of target genes into the genome of a hematopoietic stem cell is the clustered regularly interspaced short palindromic repeats (CRISPR)/Cas system, a system that originally evolved as an adaptive defense mechanism in bacteria and archaea against viral infection. The CRISPR/Cas system includes palindromic repeat sequences within plasmid DNA and an associated Cas9 nuclease. This ensemble of DNA and protein directs site specific DNA cleavage of a target sequence by first incorporating foreign DNA into CRISPR loci. Polynucleotides containing these foreign sequences and the repeat-spacer elements of the CRISPR locus are in turn transcribed in a host cell to create a guide RNA, which can subsequently anneal to a target sequence and localize the Cas9 nuclease to this site. In this manner, highly site-specific cas9-mediated DNA cleavage can be engendered in a foreign polynucleotide because the interaction that brings cas9 within close proximity of the target DNA molecule is governed by RNA:DNA hybridization. As a result, one can theoretically design a CRISPR/Cas system to cleave any target DNA molecule of interest. This technique has been exploited in order to edit eukaryotic genomes (Hwang et al. Nature Biotechnology 31:227 (2013)) and can be used as an efficient means of site-specifically editing hematopoietic stem cell genomes in order to cleave DNA prior to the incorporation of a gene encoding a target gene. The use of CRISPR/Cas to modulate gene expression has been described in, e.g., US 8,697,359.

The CRISPR/Cas system can be used to create one or more double stranded breaks in a target DNA sequence, which can then be repaired by either the homologous recombination (HR) or non-homologous end joining (NHEJ) DNA repair pathways. The Cas9 enzyme, together with a guide RNA specific to the target DNA (gRNA), can be supplied to a cell to induce one or more double strand breask. The Cas9 enzyme can be supplied as a protein, as a ribonucleoprotein complexed with the guide RNA, or as an RNA or DNA encoding the Cas9 protein that is then used by the cell to synthesize the Cas9 protein. The gRNA may comprise both tracrRNA and crRNA sequences in a chimeric RNA. Alternatively, or in addition, the gRNA may comprise a scaffold region that binds to the Cas9 protein, and a complementary base pairing region, also sometimes called a spacer, that targets the gRNA Cas9 protein complex to a particular DNA sequence. In some cases, the complementary base pairing region can be about 20 nucletodes in length, and is complementary to target DNA sequence immediately adjacent to a protospacer adjacent motif (e.g., a PAM motif). In some cases, the PAM comprises a sequence of NGG, NGA or NAG. The complementary base pairing region of the gRNA hybridizes to the target DNA sequence, and guides the gRNA Cas9 protein complex to the target sequence where the Cas9 endonuclease domains then cut within the target sequence, generating a double strand break that may be 3-4 nucleotides upstream of the PAM. Thus, by altering the complementary base pairing region, almost any DNA sequence can be targeted for the generation of a double stranded break. Methods for selecting an appropriate complementary base pairing region will be known to those skilled in the art. For example, gRNAs can be selected to minimize the number of off-target binding sites of the gRNA in the target DNA sequence. In some cases, modified Cas9 genome editing systems may be used to, for example, increase DNA targeting specificity. An example of a modified Cas9 genome editing system comprises split Cas9 systems such as the Dimeric Cas9-Fok1 genome editing system.

The double strand break or breaks generated by CRISPR/Cas9 genome editing system may be repaired by the non homologous end joining pathway (NHEJ), which ligates the ends of the double strand break together. NHEJ may result in deletions in the DNA around or near the site of the double strand break. Alternatively, the double strand break generated by CRISPR/Cas9 genome editing system may be repaired through a homology directed repair, also called homologous recombination (HR) repair pathway. In the HR pathway, the double strand break is repaired by exchanging sequences between two similar or identical DNA molecules.The HR repair pathway can therefore be used to introduce exogenous DNA sequences into the genome. In using the HR pathway for genome editing, a DNA template is supplied to the cell along with the Cas9 and gRNA. In some cases, the template may contain exogenous sequences to be introduced into the genome via genome editing flanked by homology arms that comprise DNA sequences on either side of the site of the Cas9 induced double strand break. These homology arms may be, for example, between about 50 or 1000 nucleotides, or in other cases up to several kilobases in length or longer. The template may be a linear DNA, or a circular DNA such as a plasmid, or may be supplied using a viral vector or other means of delivery. The template DNA may comprise double stranded or single stranded DNA. All manner of delivering the template DNA, the gRNA and the Cas9 protein to the cell to achieve the desired genome editing are envisaged as being within the scope of the invention.

The CRISPR/Cas9 and HR based genome editing systems of the disclosure provide not only methods of introducing exogenous DNA sequences into a genome or DNA sequence of interest, but also a platform for correcting mutations in genes. An altered or corrected version of a mutated sequence, for example a sequence changing one or more point mutations back to the wild type concensus sequence, inserting a deleted sequence, or deleting an inserted sequence, could be supplied to the cell as a template sequence, and that template sequence used by the cell to fix a CRISPR/Cas9 induced double strand break via the HR pathway. For example, in a patient with one or more disease causing mutations, hematopoietic stem and/or progenitor cells such as the hematopoietic stem and/or progenitor cells of the patient, can be removed from the body. The mutation can then corrected by CRISPR/Cas9 and HR mediated genome editing in the genome of one or more of these hematopoietic stem and/or progenitor cells, the corrected hematopoietic stem and/or progenitor cell(s) expanded with the methods of the disclosure, and then the edited cell population infused back into the patient, thereby supplying a source of the wild type version of the gene and curing the patient of the disease caused by the mutation or mutations in that gene. Mutations that can cause genetic diseases include not only point mutations, but also insertions, deletions and inversions. These mutations can be in protein coding sequence and affect the amino acid sequence of the protein, or they may be in non-coding sequences such as untranslated regions, promoters, cis regulatory elements required for gene expression, sequences required for splicing, or sequences required for DNA structure. All mutations are potentially editable by CRISPR/Cas9 mediated genome editing methods of the disclosure. In some cases, the patient may be conditioned to eliminate or reduce the native hematopoietic stem and/or progenitor cells that carry the mutant version of the gene, thus enriching for the exogenously supplied genome edited hematopoietic stem and/or progenitor cells. Both autologous and allogeneic genome edited hematopoietic stem and/or progenitor cells can be used to treat a genetic disease of a patient of the disclosure.

In addition to the CRISPR/Cas9 system, alternative methods for disruption of a target DNA by site-specifically cleaving genomic DNA prior to the incorporation of a gene of interest in a hematopoietic stem and/or progenitor cell include the use of zinc finger nucleases (ZFNs) and transcription activator-like effector nucleases (TALENs). Unlike the CRISPR/Cas system, these enzymes do not contain a guiding polynucleotide to localize to a specific target sequence. Target specificity is instead controlled by DNA binding domains within these enzymes. The use of ZFNs and TALENs in genome editing applications is described, e.g., in Urnov et al. Nature Reviews Genetics 11:636 (2010); and in Joung et al. Nature Reviews Molecular Cell Biology 14:49 (2013). As with the CRISPR/Cas9 genome editing systems, double strand breaks introduced by TALENS or ZFNs can also repaired via the HR pathway, and this pathway can be used to introduce exogenous DNA sequences or repair mutations in the DNA.

Additional genome editing techniques that can be used to disrupt or incorporate polynucleotides encoding target genes into the genome of a hematopoietic stem cell include the use of ARCUS^{™} meganucleases that can be rationally designed so as to site-specifically cleave genomic DNA. The use of these enzymes for the incorporation of genes encoding target genes into the genome of a mammalian cell is advantageous in view of the defined structure-activity relationships that have been established for such enzymes. Single chain meganucleases can be modified at certain amino acid positions in order to create nucleases that selectively cleave DNA at desired locations, enabling the site-specific incorporation of a target gene into the nuclear DNA of a hematopoietic stem cell. These single-chain nucleases have been described extensively in, e.g., US 8,021,867 and US 8,445,251.

### Methods for Expanding Hematopoietic Stem Cells

A method of producing an expanded population of hematopoietic stem cells ex vivo includes contacting a population of hematopoietic stem cells with any one of the above compounds in an amount sufficient to produce an expanded population of hematopoietic stem cells.

A method of enriching a population of cells with hematopoietic stem cells ex vivo includes contacting a population of hematopoietic stem cells with any one of the above the compounds in an amount sufficient to produce a population of cells enriched with hematopoietic stem cells.

A method of maintaining the hematopoietic stem cell functional potential of a population of hematopoietic stem cells ex vivo for two or more days includes contacting a first population of hematopoietic stem cells with the compound of any one of the above aspects or embodiments, wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the compound.

Said method for expanding hematopoietic stem cells may comprise (a) providing a starting cell population comprising hematopoietic stem cells and (b) culturing said starting cell population ex vivo in the presence of an AHR antagonist agent compound of the above.

The starting cell population comprising hematopoietic stem cells will be selected by the person skilled in the art depending on the envisaged use. Various sources of cells comprising hematopoietic stem cells have been described in the art, including bone marrow, peripheral blood, neonatal umbilical cord blood, placenta or other sources such as liver, particularly fetal liver.

The cell population may first be subjected to enrichment or purification steps, including negative and/or positive selection of cells based on specific cellular markers in order to provide the starting cell population. Methods for isolating said starting cell population based on specific cellular markers may use fluorescent activated cell sorting (FACS) technology also called flow cytometry or solid or insoluble substrate to which is bound antibodies or ligands that interact with specific cell surface markers. For example, cells may be contacted with a solid substrate (e.g., column of beads, flasks, magnetic particles) containing the antibodies and any unbound cells are removed. When a solid substrate comprising magnetic or paramagnetic beads is used, cells bound to the beads can be readily isolated by a magnetic separator.

Said starting cell population may be enriched in a desirable cell marker phenotype (e.g., CD34+, CD133+, CD90+) or based on efflux of dyes such as rhodamine, Hoechst or aldehyde dehydrogenase activity. Specifically, said starting cell population may be enriched in CD34+ cells. Methods for enriching blood cell population in CD34+ cells include kits commercialized by Miltenyi Biotec (CD34+ direct isolation kit, Miltenyi Biotec, Bergisch, Gladbach, Germany) or by Baxter (Isolex 3000).

The hematopoietic stem cells may be CD34+ hematopoietic stem cells. The hematopoietic stem cells may be CD90+ hematopoietic stem cells. The hematopoietic stem cells may be CD45RA-hematopoietic stem cells. The hematopoietic stem cells may be CD34+CD90+ hematopoietic stem cells. The hematopoietic stem cells may be CD34+CD45RA- hematopoietic stem cells. The hematopoietic stem cells may be CD90+CD45RA- hematopoietic stem cells. The hematopoietic stem cells may be CD34+CD90+CD45RA- hematopoietic stem cells.

The hematopoietic stem cells may be mammalian cells, such as human cells. The human cells may be CD34+ cells, such as CD34+ cells are CD34+, CD34+CD38-, CD34+CD38-CD90+, CD34+CD38-CD90+CD45RA-, CD34+CD38-CD90+CD45RA-CD49F+, or CD34+CD90+CD45RA-cells.

The hematopoietic stem cells may be obtained from human cord blood, mobilized human peripheral blood, or human bone marrow. The hematopoietic stem cells may, for example, be freshly isolated from the human or may have been previously cryopreserved.

The amount of cord blood from a single birth is often inadequate to treat an adult or an older child. One advantage of the expansion methods using the compounds of the above, or an agent capable of down-regulating the activity and/or expression of aryl hydrocarbon receptor and/or a downstream effector of aryl hydrocarbon receptor pathway, is that it enables the production of a sufficient amount of hematopoietic stem cells from only one cord blood unit.

Accordingly, the starting cell population may be derived from neonatal umbilical cord blood cells which have been enriched in CD34+ cells. Said starting cell population may be derived from one or two umbilical cord blood units.

The starting cell population may be derived from human mobilized peripheral blood cells which have been enriched in CD34+ cells. Related to that, said starting cell population may be derived from human mobilized peripheral blood cells isolated from only one patient.

Said starting cell population enriched in CD34+ cells may preferably contain at least about 50% CD34+ cells, maybe more than about 90% CD34+ cells, and may comprise between 10⁵ and 10⁹ nucleated cells.

The starting cell population may be used directly for expansion or frozen and stored for use at a later date.

Conditions for culturing the starting cell population for hematopoietic stem cell expansion will vary depending, inter alia, on the starting cell population, the desired final number of cells, and desired final proportion of HSCs.

The culturing conditions may comprise the use of other cytokines and growth factors, generally known in the art for hematopoietic stem cell expansion. Such cytokines and growth factors include without limitation IL-1, IL-3, IL-6, IL-11, G-CSF, GM-CSF, SCF, FIT3-L, thrombopoietin (TPO), erythropoeitin, and analogs thereof. As used herein, "analogs" include any structural variants of the cytokines and growth factors having the biological activity as the naturally occurring forms, including without limitation, variants with enhanced or decreased biological activity when compared to the naturally occurring forms or cytokine receptor agonists such as an agonist antibody against the TPO receptor (for example, VB22B sc(Fv)2 as detailed in patent publication WO 2007/145227, and the like). Cytokine and growth factor combinations are chosen to expand HSC and progenitor cells while limiting the production of terminally differentiated cells. One or more cytokines and growth factors may be selected from the group consisting of SCF, Flt3-L and TPO. At least TPO may be used in a serum-free medium under suitable conditions for HSC expansion. A mixture of IL6, SCF, Flt3-L and TPO may be used in the method for expanding HSCs in combination with the compound of the present disclosure.

The expansion of HSC may be carried out in a basal medium, which may be supplemented with mixtures of cytokines and growth factors. A basal medium typically comprises amino acids, carbon sources, vitamins, serum proteins (e.g. albumin), inorganic salts, divalent cations, buffers and any other element suitable for use in expansion of HSC. Examples of such basal medium appropriate for a method of expanding HSC include, without limitation, StemSpan^{®} SFEM-Serum-Free Expansion Medium (StemCell Technologies, Vancouver, Canada), StemSpan^{®} H3000-Defined Medium (StemCell Technologies, Vancouver, Canada), CellGro^{®} SCGM (CellGenix, Freiburg Germany), StemPro^{®}-34 SFM (Invitrogen).

The compound of the present disclosure is administered during the expansion method of said starting cell population under a concentration appropriate for HSC expansion. Said compound or AHR modulating agent may be administered at a concentration comprised between 1 pM and 100 µM, for example between 10 pM and 10 µM, or between 100 pM and 1 µM.

Where starting cell population essentially consists of CD34+ enriched cells from one or two cord blood units, the cells may be grown under conditions for HSC expansion from about 3 days to about 90 days, for example between 7 and 2 days and/or until the indicated fold expansion and the characteristic cell populations are obtained. The cells may be grown under conditions for HSC expansion not more than 21 days, 14 days or 7 days.

The starting cell population may be cultured during a time sufficient to reach an absolute number of CD34+ cells of at least 10⁵, 10⁶, 10⁷, 10⁸ or 10⁹ cells. Said starting cell population may be cultured during a time sufficient for a 10 to 50000 fold expansion of CD34+ cells, for example between 100 and 10000 fold expansion, for examples between 50 and 1000 fold expansion.

The cell population obtained after the expansion method may be used without further purification or may be subject to further purification or selection steps.

The cell population may then be washed to remove the compound of the present disclosure and/or any other components of the cell culture and resuspended in an appropriate cell suspension medium for short term use or in a long-term storage medium, for example a medium suitable for cryopreservation.

### Cell Population with Expanded Hematopoietic Stem Cells as Obtained by the Expansion Method and Therapeutic Compositions

In another aspect, the disclosure features a composition comprising a population of hematopoietic stem cells, wherein the hematopoietic stem cells or progenitors thereof have been contacted with the compound of any of the above, thereby expanding the hematopoietic stem cells or progenitors thereof.

A cell population with expanded hemapoetic stem cells may be obtainable or obtained by the expansion method described above. Such cell population may be resuspended in a pharmaceutically acceptable medium suitable for administration to a mammalian host, thereby providing a therapeutic composition.

The compound as defined in the present disclosure enables the expansion of HSCs, for example from only one or two cord blood units, to provide a cell population quantitatively and qualitatively appropriate for efficient short and long term engraftment in a human patient in need thereof. In one embodiment, the present disclosure relates to a therapeutic composition comprising a cell population with expanded HSCs derived from not more than one or two cord blood units. In one embodiment, the present disclosure relates to a therapeutic composition containing a total amount of cells of at least about 10⁵, at least about 10⁶, at least about 10⁷, at least about 10⁸ or at least about 10⁹ cells with about 20% to about 100%, for example between about 43% to about 80%, of total cells being CD34+ cells. In certain embodiments, said composition contains between 20-100%, for example between 43-80%, of total cells being CD34+CD90+CD45RA-.

In some embodiments, the hematopoietic stem cells are CD34+ hematopoietic stem cells. In some embodiments, the hematopoietic stem cells are CD90+ hematopoietic stem cells. In some embodiments, the hematopoietic stem cells are CD45RA- hematopoietic stem cells. In some embodiments, the hematopoietic stem cells are CD34+CD90+ hematopoietic stem cells. In some embodiments, the hematopoietic stem cells are CD34+CD45RA- hematopoietic stem cells. In some embodiments, the hematopoietic stem cells are CD90+CD45RA- hematopoietic stem cells. In some embodiments, the hematopoietic stem cells are CD34+CD90+CD45RA- hematopoietic stem cells.

In some embodiments, the hematopoietic stem cells of the therapeutic composition are mammalian cells, such as human cells. In some embodiments, the human cells are CD34+ cells, such as CD34+ cells are CD34+, CD34+CD38-, CD34+CD38-CD90+, CD34+CD38-CD90+CD45RA-, CD34+CD38-CD90+CD45RA-CD49F+, or CD34+CD90+CD45RA- cells.

In some embodiments, the hematopoietic stem cells of the therapeutic composition are obtained from human cord blood, mobilized human peripheral blood, or human bone marrow. The hematopoietic stem cells may, for example, be freshly isolated from the human or may have been previously cryopreserved.

### Other Methods of Treatment

As described herein, hematopoietic stem cell transplant therapy can be administered to a subject in need of treatment so as to populate or repopulate one or more blood cell types, such as a blood cell lineage that is deficient or defective in a patient suffering from a stem cell disorder. Hematopoietic stem and progenitor cells exhibit multi-potency, and can thus differentiate into multiple different blood lineages including, but not limited to, granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells). Hematopoietic stem cells are additionally capable of self-renewal, and can thus give rise to daughter cells that have equivalent potential as the mother cell, and also feature the capacity to be reintroduced into a transplant recipient whereupon they home to the hematopoietic stem cell niche and re-establish productive and sustained hematopoiesis. Thus, hematopoietic stem and progenitor cells represent a useful therapeutic modality for the treatment of a wide array of disorders in which a patient has a deficiency or defect in a cell type of the hematopoietic lineage. The deficiency or defect may be caused, for example, by depletion of a population of endogenous cells of the hematopoietic system due to administration of a chemotherapeutic agent (e.g., in the case of a patient suffering from a cancer, such as a hematologic cancer described herein). The deficiency or defect may be caused, for example, by depletion of a population of endogenous hematopoietic cells due to the activity of self-reactive immune cells, such as T lymphocytes or B lymphocytes that cross-react with self antigens (e.g., in the case of a patient suffering from an autoimmune disorder, such as an autoimmune disorder described herein). Additionally or alternatively, the deficiency or defect in cellular activity may be caused by aberrant expression of an enzyme (e.g., in the case of a patient suffering from various metabolic disorders, such as a metabolic disorder described herein).

Thus, hematopoietic stem cells can be administered to a patient defective or deficient in one or more cell types of the hematopoietic lineage in order to re-constitute the defective or deficient population of cells in vivo, thereby treating the pathology associated with the defect or depletion in the endogenous blood cell population. Hematopoietic stem and progenitor cells can be used to treat, e.g., a non-malignant hemoglobinopathy (e.g., a hemoglobinopathy selected from the group consisting of sickle cell anemia, thalassemia, Fanconi anemia, aplastic anemia, and Wiskott-Aldrich syndrome). In these cases, for example, a CXCR4 antagonist and/or a CXCR2 agonist may be administered to a donor, such as a donor identified as likely to exhibit release of a population of hematopoietic stem and progenitor cells from a stem cell niche, such as the bone marrow, into circulating peripheral blood in response to such treatment. The hematopoietic stem and progenitor cells thus mobilized may then be withdrawn from the donor and administered to a patient, where the cells may home to a hematopoietic stem cell niche and re-constitute a population of cells that are damaged or deficient in the patient.

Hematopoietic stem or progenitor cells mobilized to the peripheral blood of a subject may be withdrawn (e.g., harvested or collected) from the subject by any suitable technique. For example, the hematopoietic stem or progenitor cells may be withdrawn by a blood draw. Hematopoietic stem or progenitor cells mobilized to a subject's peripheral blood as contemplated herein may be harvested (i.e., collected) using apheresis. Apheresis may be used to enrich a donor's blood with mobilized hematopoietic stem or progenitor cells.

Additionally or alternatively, hematopoietic stem and progenitor cells can be used to treat an immunodeficiency, such as a congenital immunodeficiency. Additionally or alternatively, the compositions and methods described herein can be used to treat an acquired immunodeficiency (e.g., an acquired immunodeficiency selected from the group consisting of HIV and AIDS). In these cases, for example, a population of hematopoietic stem cells may be expanded ex vivo by culturing the cells in the presence of an aryl hydrocarbon receptor antagonist described herein. In these cases, for example, a CXCR4 antagonist and/or a CXCR2 agonist may be administered to a donor, such as a donor identified as likely to exhibit release of a population of hematopoietic stem and progenitor cells from a stem cell niche, such as the bone marrow, into circulating peripheral blood in response to such treatment. The hematopoietic stem and progenitor cells thus mobilized may then be withdrawn from the donor and administered to a patient, where the cells may home to a hematopoietic stem cell niche and re-constitute a population of immune cells (e.g., T lymphocytes, B lymphocytes, NK cells, or other immune cells) that are damaged or deficient in the patient.

Hematopoietic stem and progenitor cells can also be used to treat a metabolic disorder (e.g., a metabolic disorder selected from the group consisting of glycogen storage diseases, mucopolysaccharidoses, Gaucher's Disease, Hurler syndrome or Hurler's Disease, sphingolipidoses, Sly Syndrome, alpha-Mannosidosis, X-ALD, Aspartylglucosaminuria, Wolman Disease, late infantile metachromatic leukodystrophy, Niemann Pick Type C disease, Niemann Pick Type B disease, Juvenile Tay Sachs, Infantile Tay Sachs, Juvenile Sandhoff, Infantile Sandhoff, GM1 gangliosidosis, MPSIV (Morquio), Presymptomatic or milder forms of globoid cell leukodystrophy, infantile Krabbe when newborn and asymptomatic, early diagnosis fucosidosis, Fabry, MPSIS, MPSIH/S, MPSII, MPSVI in conjunction with ERT or where alloantibodies attenuate efficacy of ERT, Pompe where alloantibodies attenuate efficacy of ERT, Mucolipidosis II, and metachromatic leukodystrophy). In these cases, for example, a CXCR4 antagonist and/or a CXCR2 agonist may be administered to a donor, such as a donor identified as likely to exhibit release of a population of hematopoietic stem and progenitor cells from a stem cell niche, such as the bone marrow, into circulating peripheral blood in response to such treatment. The hematopoietic stem and progenitor cells thus mobilized may then be withdrawn from the donor and administered to a patient, where the cells may home to a hematopoietic stem cell niche and re-constitute a population of hematopoietic cells that are damaged or deficient in the patient. In these cases, for example, a population of hematopoietic stem cells may be expanded ex vivo by culturing the cells in the presence of an aryl hydrocarbon receptor antagonist described herein. The hematopoietic stem cells thus expanded may then be administered to a patient, where the cells may home to a hematopoietic stem cell niche and re-constitute a population of hematopoietic cells that are damaged or deficient in the patient.

Additionally or alternatively, hematopoietic stem or progenitor cells can be used to treat a malignancy or proliferative disorder, such as a hematologic cancer or myeloproliferative disease. In the case of cancer treatment, for example, a CXCR4 antagonist and/or a CXCR2 agonist may be administered to a donor, such as a donor identified as likely to exhibit release of a population of hematopoietic stem and progenitor cells from a stem cell niche, such as the bone marrow, into circulating peripheral blood in response to such treatment. The hematopoietic stem and progenitor cells thus mobilized may then be withdrawn from the donor and administered to a patient, where the cells may home to a hematopoietic stem cell niche and re-constitute a population of cells that are damaged or deficient in the patient, such as a population of hematopoietic cells that is damaged or deficient due to the administration of one or more chemotherapeutic agents to the patient. In some embodiments, hematopoietic stem or progenitor cells may be infused into a patient in order to repopulate a population of cells depleted during cancer cell eradication, such as during systemic chemotherapy. Exemplary hematological cancers that can be treated by way of administration of hematopoietic stem and progenitor cells in accordance with the compositions and methods described herein are acute myeloid leukemia, acute lymphoid leukemia, chronic myeloid leukemia, chronic lymphoid leukemia, multiple myeloma, diffuse large B-cell lymphoma, and non-Hodgkin's lymphoma, as well as other cancerous conditions, including neuroblastoma.

Additionally or alternatively, hematopoietic stem or progenitor cells can be used to treat a malignancy or proliferative disorder, such as a hematologic cancer or myeloproliferative disease. In the case of cancer treatment, for example, a population of hematopoietic stem cells may be expanded ex vivo by culturing the cells in the presence of an aryl hydrocarbon receptor antagonist described herein. The hematopoietic stem cells thus expanded may then be administered to a patient, where the cells may home to a hematopoietic stem cell niche and re-constitute a population of cells that are damaged or deficient in the patient, such as a population of hematopoietic cells that is damaged or deficient due to the administration of one or more chemotherapeutic agents to the patient. In some embodiments, hematopoietic stem or progenitor cells may be infused into a patient in order to repopulate a population of cells depleted during cancer cell eradication, such as during systemic chemotherapy. Exemplary hematological cancers that can be treated by way of administration of hematopoietic stem and progenitor cells in accordance with the compositions and methods described herein are acute myeloid leukemia, acute lymphoid leukemia, chronic myeloid leukemia, chronic lymphoid leukemia, multiple myeloma, diffuse large B-cell lymphoma, and non-Hodgkin's lymphoma, as well as other cancerous conditions, including neuroblastoma.

Additional diseases that can be treated by the administration of hematopoietic stem and progenitor cells to a patient include, without limitation, adenosine deaminase deficiency and severe combined immunodeficiency, hyper immunoglobulin M syndrome, Chediak-Higashi disease, hereditary lymphohistiocytosis, osteopetrosis, osteogenesis imperfecta, storage diseases, thalassemia major, systemic sclerosis, systemic lupus erythematosus, multiple sclerosis, and juvenile rheumatoid arthritis.

In addition, administration of hematopoietic stem and progenitor cells can be used to treat autoimmune disorders. Upon infusion into a patient, transplanted hematopoietic stem and progenitor cells may home to a stem cell niche, such as the bone marrow, and establish productive hematopoiesis. This, in turn, can re-constitute a population of cells depleted during autoimmune cell eradication, which may occur due to the activity of self-reactive lymphocytes (e.g., self-reactive T lymphocytes and/or self-reactive B lymphocytes). Autoimmune diseases that can be treated by way of administering hematopoietic stem and progenitor cells to a patient include, without limitation, psoriasis, psoriatic arthritis, Type 1 diabetes mellitus (Type 1 diabetes), rheumatoid arthritis (RA), human systemic lupus (SLE), multiple sclerosis (MS), inflammatory bowel disease (IBD), lymphocytic colitis, acute disseminated encephalomyelitis (ADEM), Addison's disease, alopecia universalis, ankylosing spondylitisis, antiphospholipid antibody syndrome (APS), aplastic anemia, autoimmune hemolytic anemia, autoimmune hepatitis, autoimmune inner ear disease (AIED), autoimmune lymphoproliferative syndrome (ALPS), autoimmune oophoritis, Balo disease, Behcet's disease, bullous pemphigoid, cardiomyopathy, Chagas' disease, chronic fatigue immune dysfunction syndrome (CFIDS), chronic inflammatory demyelinating polyneuropathy, Crohn's disease, cicatrical pemphigoid, coeliac sprue-dermatitis herpetiformis, cold agglutinin disease, CREST syndrome, Degos disease, discoid lupus, dysautonomia, endometriosis, essential mixed cryoglobulinemia, fibromyalgiafibromyositis, Goodpasture's syndrome, Grave's disease, Guillain-Barre syndrome (GBS), Hashimoto's thyroiditis, Hidradenitis suppurativa, idiopathic and/or acute thrombocytopenic purpura, idiopathic pulmonary fibrosis, IgA neuropathy, interstitial cystitis, juvenile arthritis, Kawasaki's disease, lichen planus, Lyme disease, Meniere disease, mixed connective tissue disease (MCTD), myasthenia gravis, neuromyotonia, opsoclonus myoclonus syndrome (OMS), optic neuritis, Ord's thyroiditis, pemphigus vulgaris, pernicious anemia, polychondritis, polymyositis and dermatomyositis, primary biliary cirrhosis, polyarteritis nodosa, polyglandular syndromes, polymyalgia rheumatica, primary agammaglobulinemia, Raynaud phenomenon, Reiter's syndrome, rheumatic fever, sarcoidosis, scleroderma, Sjögren's syndrome, stiff person syndrome, Takayasu's arteritis, temporal arteritis (also known as "giant cell arteritis"), ulcerative colitis, collagenous colitis, uveitis, vasculitis, vitiligo, vulvodynia ("vulvar vestibulitis"), and Wegener' s granulomatosis.

Hematopoietic stem cell transplant therapy may additionally be used to treat neurological disorders, such as Parkinson's disease, Alzheimer's disease, multiple sclerosis, Amyotrophic lateral sclerosis, Huntington's disease, mild cognitive impairment, amyloidosis, AIDS-related dementia, encephalitis, stroke, head trauma, epilepsy, mood disorders, and dementia. As described herein, upon transplantation into a patient, hematopoietic stem cells may migrate to the central nervous system and differentiate into, for example, microglial cells, thereby re-constituting a population of cells that may be damaged or deficient in a patient suffering from a neurological disorder. In these cases, for example, a population of hematopoietic stem cells may be administered to a patient suffering from a neurological disorder, where the cells may home to the central nervous system, such as the brain of the patient, and re-constitute a population of hematopoietic cells (e.g., microglial cells) that are damaged or deficient in the patient.

As described herein, upon transplantation into a patient, hematopoietic stem cells may migrate to the central nervous system and differentiate into, for example, microglial cells, thereby re-constituting a population of cells that may be damaged or deficient in a patient suffering from a neurological disorder. In these cases, for example, a population of hematopoietic stem cells may be expanded ex vivo by culturing the cells in the presence of an aryl hydrocarbon receptor antagonist described herein. The hematopoietic stem cells thus expanded may then be administered to a patient suffering from a neurological disorder, where the cells may home to the central nervous system, such as the brain of the patient, and re-constitute a population of hematopoietic cells (e.g., microglial cells) that are damaged or deficient in the patient.

As described herein, hematopoietic stem cell transplant therapy can be administered to a subject in need of treatment so as to populate or repopulate one or more blood cell types, such as a blood cell lineage that is deficient or defective in a patient suffering from a stem cell disorder. Hematopoietic stem and progenitor cells exhibit multi-potency, and can thus differentiate into multiple different blood lineages including, in one embodiment, microglia.

The methods disclosed herein for treating disorders in a subject in need thereof comprise the administration of an expanded population of hematopoietic stem cells to a subject in need thereof. The number of expanded hematopoietic stem cells administered to the subject may be equal to or greater than the amount of hematopoietic stem cells needed to achieve a therapeutic benefit. The number of expanded hematopoietic stem cells administered to the subject may be greater than the amount of hematopoietic stem cells needed to achieve a therapeutic benefit. The therapeutic benefit achieved may be proportional to the number of expanded hematopoietic stem cells that are administered.

A dose of the expanded hematopoietic stem cell composition of the disclosure is deemed to have achieved a therapeutic benefit if it alleviates a sign or a symptom of the disease. The sign or symptom of the disease may comprise one or more biomarkers associated with the disease, or one or more clinical symptoms of the disease.

For example, administration of the expanded hematopoietic stem cell composition may result in the reduction of a biomarker that is elevated in individuals suffering from the disease, or elevate the level of a biomarker that is reduced in individuals suffering from the disease.

For example, administering the expanded hematopoietic stem cell composition of the disclosure may elevate the level of an enzyme that is reduced in an individual suffering from a metabolic disorder. This change in biomarker level may be partial, or the level of the biomarker may return to levels normally seen in healthy individuals.

### Selection of donors and patients

The patient may be the donor. In such cases, withdrawn hematopoietic stem or progenitor cells may be re-infused into the patient, such that the cells may subsequently home hematopoietic tissue and establish productive hematopoiesis, thereby populating or repopulating a line of cells that is defective or deficient in the patient (e.g., a population of megakaryocytes, thrombocytes, platelets, erythrocytes, mast cells, myeoblasts, basophils, neutrophils, eosinophils, microglia, granulocytes, monocytes, osteoclasts, antigen-presenting cells, macrophages, dendritic cells, natural killer cells, T-lymphocytes, and B-lymphocytes). In this scenario, the transplanted hematopoietic stem or progenitor cells are least likely to undergo graft rejection, as the infused cells are derived from the patient and express the same HLA class I and class II antigens as expressed by the patient.

Alternatively, the patient and the donor may be distinct. The patient and the donor may be related, and may, for example, be HLA-matched. As described herein, HLA-matched donor-recipient pairs have a decreased risk of graft rejection, as endogenous T cells and NK cells within the transplant recipient are less likely to recognize the incoming hematopoietic stem or progenitor cell graft as foreign, and are thus less likely to mount an immune response against the transplant. Exemplary HLA-matched donor-recipient pairs are donors and recipients that are genetically related, such as familial donor-recipient pairs (e.g., sibling donor-recipient pairs).

The patient and the donor may be HLA-mismatched, which occurs when at least one HLA antigen, in particular with respect to HLA-A, HLA-B and HLA-DR, is mismatched between the donor and recipient. To reduce the likelihood of graft rejection, for example, one haplotype may be matched between the donor and recipient, and the other may be mismatched.

### Administration and Dosing of Hematopoietic Stem or Progenitor Cells

Hematopoietic stem and progenitor cells described herein may be administered to a subject, such as a mammalian subject (e.g., a human subject) suffering from a disease, condition, or disorder described herein, by one or more routes of administration. For instance, hematopoietic stem cells described herein may be administered to a subject by intravenous infusion. Hematopoietic stem cells may be administered at any suitable dosage. Non-limiting examples of dosages include about 1 × 10⁵ CD34+ cells/kg of recipient to about 1 × 10⁷ CD34+ cells/kg (e.g., from about 2 × 10⁵ CD34+ cells/kg to about 9 × 10⁶ CD34+ cells/kg, from about 3 × 10⁵ CD34+ cells/kg to about 8 × 10⁶ CD34+ cells/kg, from about 4 × 10⁵ CD34+ cells/kg to about 7 × 10⁶ CD34+ cells/kg, from about 5 × 10⁵ CD34+ cells/kg to about 6 × 10⁶ CD34+ cells/kg, from about 5 × 10⁵ CD34+ cells/kg to about 1 × 10⁷ CD34+ cells/kg, from about 6 × 10⁵ CD34+ cells/kg to about 1 × 10⁷ CD34+ cells/kg, from about 7 × 10⁵ CD34+ cells/kg to about 1 × 10⁷ CD34+ cells/kg, from about 8 × 10⁵ CD34+ cells/kg to about 1 × 10⁷ CD34+ cells/kg, from about 9 × 10⁵ CD34+ cells/kg to about 1 × 10⁷ CD34+ cells/kg, or from about 1 × 10⁶ CD34+ cells/kg to about 1 × 10⁷ CD34+ cells/kg, among others).

Hematopoietic stem or progenitor cells and pharmaceutical compositions described herein may be administered to a subject in one or more doses. When multiple doses are administered, subsequent doses may be provided one or more days, weeks, months, or years following the initial dose.

The disclosure having been described, the following example are offered by way of illustration and not limitation.

### Examples

The following examples are put forth so as to provide those of ordinary skill in the art with a description of how the compositions and methods described herein may be used, made, and evaluated, and are intended to be purely exemplary of the invention and are not intended to limit the scope of what the inventors regard as their invention.

Aryl hydrocarbon receptor antagonists represented by formula (**III**) can be prepared by the methods described in U.S. Patent Nos. 8,927,281 and 9,580,426.

### Example 1. Capacity of Compounds (7) and (18) to expand hematopoietic stem cells

To determine the ability of compounds (7) and (18) to inhibit the activity of the aryl hydrocarbon receptor and to induce the proliferation of hematopoietic stem cells, a series of HSC expansion experiments were conducted. In the first experiment, compounds (**7**) and (**18**) were assessed for their capacity to attenuate aryl hydrocarbon receptor signaling. To this end, HepG2 hepatocytes were transiently transfected with a luciferase reporter construct under the control of a promoter responsive to aryl hydrocarbon receptor signal transduction. The cells were plated at a density of 25,000 cells per well in a microtiter plate. The HepG2 cells were immediately treated with compound (**7**) or (**18**) in the absence (FIG. 1) or presence (FIG. 2) of the aryl hydrocarbon receptor agonist, VAF347 (80 nM). Luciferase activity was subsequently analyzed six hours after plating.

As shown in FIGS. 1 and 2, compounds (**7**) and (**18**) were capable of suppressing aryl hydrocarbon receptor activity even in the presence of the activator VAF347.

To assess the ability of compounds (**7**) and (**18**) to induce the proliferation of hematopoietic stem cells, a population of mononuclear peripheral blood cells enriched in CD34+ cells were plated at a density of 2,350 cells per well (50 µL) in a microtiter plate in the presence of each compound. The percentage of CD34+ hematopoietic stem cells was assessed seven days following the initial plating. The results of this experiment are reported in FIG. 3. As shown therein, compounds (**7**) and (**18**) were capable of potentiating hematopoietic stem cell growth in a dose-dependent manner. The compounds of formula (**IV**) and (**V**) described herein can thus be used to expand hematopoietic stem cells ex vivo in order to obtain sufficient quantities of such cells for in vivo applications.

Surprisingly, compounds (**7**) and (**18**) were capable of promoting hematopoietic stem cell expansion with a potency greater than that reported for StemRegenin1 (SR1, i.e., the compound of Formula (1), which is described, for example, in U.S. Patent No. 8,927,281. This difference in biological activity is expected to have a significant clinical benefit, as a reduced quantity of aryl hydrocarbon receptor antagonists according to formulas (**IV**) and (**V**) described herein relative to SR1 may be used to prepare an amplified population of hematopoietic stem cells suitable for transplantation to a patient in need thereof (for instance, as described in Example 2, below).

### Example 2. Administration of hematopoietic stem cells to a human patient in need thereof

Using the methods disclosed herein, a population of hematopoietic stem cells that have been expanded ex vivo using the aryl hydrocarbon receptor antagonists of formula (**IV**) or (**V**) can be administered to a human patient in need of hematopoietic stem cell transplant therapy. Prior to the transplantation, a population of hematopoietic stem cells may be cultured in the presence of the aryl hydrocarbon receptor antagonist for one or more days (e.g., for one, two, three, four, five, six, seven, eight, nine, ten, or more days, replenishing culture medium as needed). The hematopoietic stem cell population may be expanded to 1 × 10⁶ to 1 × 10¹² hematopoietic stem cells prior to infusion into the patient in need of transplant therapy.

Following the conclusion of the expansion process, the patient may receive an infusion (e.g., an intravenous infusion) of the expanded, exogenous hematopoietic stem cells, such as from a practitioner that performed the ex vivo expansion or from a different physician. The patient may then be administered an infusion of autologous, syngeneic, or allogeneic hematopoietic stem cells, for instance, at a dosage of from 1 × 10³ to 1 × 10⁹ hematopoietic stem cells/kg. The engraftment of the hematopoietic stem cell transplant may be monitored, for example, by detecting an increase in concentration of hematopoietic stem cells or cells of the hematopoietic lineage (such as megakaryocytes, thrombocytes, platelets, erythrocytes, mast cells, myoblasts, basophils, neutrophils, eosinophils, microglia, granulocytes, monocytes, osteoclasts, antigen-presenting cells, macrophages, dendritic cells, natural killer cells, T-lymphocytes, and B-lymphocytes) in a blood sample isolated from the patient following administration of the transplant. This analysis may be conducted, for example, from 1 hour to 6 months, or more, following hematopoietic stem cell transplant therapy (e.g., 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 2 days, 3 days, 4 days, 5 days, 6 days, 7 days, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, or more). A finding that the concentration of hematopoietic stem cells or cells of the hematopoietic lineage has increased (e.g., by 1%, 2%, 3%, 4%, 5%, 6%, 7%, 8%, 9%, 10%, 20%, 30%, 40%, 50%, 60%, 70%, 80%, 90%, 100%, 200%, 500%, or more) following the transplant therapy relative to the concentration of the corresponding cell type prior to transplant therapy provides one indication that the transplantation therapy is successful.

### Example 3. Engraftment of microglial cells in the central nervous system following hematopoietic stem cell transplant

To investigate the ability of hematopoietic stem cells to differentiate into microglial cells and subsequently engraft in central nervous system tissue, such as the brain of a hematopoietic stem cell transplant recipient, a series of experiments were conducted in which human hematopoietic stem cells were first expanded ex vivo in the presence of an aryl hydrocarbon receptor antagonist (compound (**18**) or compound (**26**)) and were subsequently transplanted into NSG mice, in accordance with the scheme shown in FIG. 4. The frequency of human CD45+ cells in the peripheral blood of the mice was then determined, as well as the profile of microglial cells in the brain tissue using flow cytometry and immunohistochemistry techniques.

As shown in FIGS. 5A and 5B, upon transplantation of hematopoietic stem cells expanded ex vivo in the presence of compound (**18**), NSG mice exhibited an increased frequency of hCD45+ cells in peripheral blood, as well as an increased engraftment of hCD45+CD11b+ microglial cells in the brain. Similar results were obtained upon transplantation of hematopoietic stem cells expanded ex vivo in the presence of compound (**26**), as shown in FIGS. 6A and 6B.

Collectively, these data demonstrate the ability of hematopoietic stem cells expanded in the presence of aryl hydrocarbon receptor antagonists described herein to promote the engraftment of microglial cells in central nervous system tissue of a hematopoietic stem cells transplant recipient. These findings provide further evidence that hematopoietic stem cell transplantation can be used to treat a wide array of neurological disorders, including Parkinson's disease, Alzheimer's disease, multiple sclerosis, Amyotrophic lateral sclerosis, Huntington's disease, mild cognitive impairment, amyloidosis, AIDS-related dementia, encephalitis, stroke, head trauma, epilepsy, mood disorders, and dementia, among others.

### Example 4. Expansion of hematopoietic stem or progenitor cells by treatment with an aryl hydrocarbon receptor antagonist

Achieving a high dosage of hematopoietic stem cells is important for successful therapy. Ex vivo expansion of hematopoietic stem cells represents a method by which elevated quantities of cells may be obtained for therapeutic applications. A clinical trial in which patients received cord blood (CB)-derived hematopoietic stem cells that had been expanded ex vivo by culturing the cells in the presence of an AHR antagonist demonstrated an improvement in time to engraftment, as shown in FIG. 7. This example demonstrates the ability of AHR antagonists to expand hematopoietic stem cells ex vivo, and to promote the engraftment of such cells in vivo.

*Methods.* A series of aryl hydrocarbon receptor antagonists, including SR1, along with histone deacetylase (HDAC) inhibitors, and UM171 were evaluated in the presence of cytokines to expand primary human CD34+ cells *ex vivo.* Cell number and immunophenotype were assessed by flow cytometry, and HSC function was evaluated by cell and molecular assays *in vitro.* The expanded cells were transplanted into sub-lethally irradiated NSG mice to evaluate engraftment potential *in vivo.* Engraftment rates were evaluated by flow cytometry of the peripheral blood and bone marrow.

Structure of UM171.

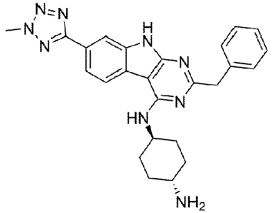

*Results.* Based on the results of these experiments, cultures expanded with an AHR antagonist showed the largest improvement in NSG engraftment levels compared to unmanipulated cells. Culture of CD34+ cells with SR1 or another AHR antagonist, A, led to a 6-fold increase in CD34+ number and a significant increase in engraftment in NSG mice relative to vehicle-cultured CB derived CD34+ cells. The aryl hydrocarbon receptor antagonist displayed complete AHR antagonism in the dioxin response element luciferase reporter assay and was a more potent antagonist compared to SR1 (a 12-fold increase in potency). The expanded culture contained 3.4-fold more CD34+CD90+ cells than the vehicle-treated cells. Upon transplant, mice receiving the expanded cells showed greater than 2-fold increase in engraftment compared to those receiving vehicle-treated cells.

*Conclusions.* These studies demonstrate that AHR antagonism is an effective strategy to expand functional HSCs and that small molecules inhibiting AHR can expand HSC from mPB and BM.

### Example 5. Treatment of a hematologic disorder by administration of a hematopoietic stem or progenitor cell graft

Using the compositions and methods described herein, a stem cell disorder, such as a hematologic pathology described herein, can be treated by administering to a patient a hematopoietic stem or progenitor cell graft. For example, a population of hematopoietic stem or progenitor cells can be isolated from a donor. Following the isolation process, a patient may then receive an infusion (e.g., an intravenous infusion) of the mobilized and isolated hematopoietic stem or progenitor cells. The patient may be the donor, or may be a patient that is HLA-matched with respect to the donor, thereby reducing the likelihood of graft rejection. The patient may be one that is suffering, for instance, from a cancer, such as a hematologic cancer described herein. Additionally or alternatively, the patient may be one that is suffering from an autoimmune disease or metabolic disorder described herein.

The engraftment of the hematopoietic stem cell transplant can be monitored, for example, by withdrawing a blood sample from the patient and determining the increase in concentration of hematopoietic stem cells or cells of the hematopoietic lineage (such as megakaryocytes, thrombocytes, platelets, erythrocytes, mast cells, myeoblasts, basophils, neutrophils, eosinophils, microglia, granulocytes, monocytes, osteoclasts, antigen-presenting cells, macrophages, dendritic cells, natural killer cells, T-lymphocytes, and B-lymphocytes) following administration of the transplant. This analysis may be conducted, for example, from 1 hour to 6 months, or more, following hematopoietic stem cell transplant therapy (e.g., 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 2 days, 3 days, 4 days, 5 days, 6 days, 7 days, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, or more). A finding that the concentration of hematopoietic stem cells or cells of the hematopoietic lineage has increased (e.g., by 1%, 2%, 3%, 4%, 5%, 6%, 7%, 8%, 9%, 10%, 20%, 30%, 40%, 50%, 60%, 70%, 80%, 90%, 100%, 200%, 500%, or more) following the transplant therapy relative to the concentration of the corresponding cell type prior to transplant therapy provides one indication that the hematopoietic stem or progenitor cell transplant therapy is efficacious in treating the stem cell disorder.

### Example 6. Engraftment of microglial cells in the brains of NSG mice following hematopoietic stem cell transplantation

Approximately 1,000 allogeneic hematopoietic cell transplantations (HSCTs) have been performed over the last three decades for treatment of different inherited metabolic disorders to prevent symptom onset, suppress disease progression, and improve patient outcomes. The goal of HSCT in these diseases is to provide cells that produce functional enzymes otherwise deficient in patients with inherited metabolic disorders. Mechanistically, this is accomplished through repopulation of the myeloid compartment, including brain microglia, by donor derived cells. Microglia catabolize storage material in tissues; replacement of defective microglia by normal cells reestablishes an important scavenging function defective in patients with inherited metabolic disorders. Further, these normal cells secrete lysosomal enzymes, which can be taken up by neighboring cells, and thereby cross correct the metabolic disorder. Although HSCT effectively halts disease progression, central nervous system stabilization takes 6-12 months post-HSCT, perhaps reflecting the slow kinetics of microglia replacement by donor-derived cells.

We compared the ability of unmanipulated cord blood or cord blood expanded *ex vivo* using an aryl hydrocarbon receptor (AHR) antagonist (MGTA-456) to engraft the brain microglia compartment. The design of the experiments described in this Example is shown in FIG. 8. The AHR antagonist used in the experiments described in this Example is Compound 2, represented by Formula (2). These examples demonstrate the utility of AHR antagonism as an effective strategy to expand cord blood-derived CD34+ cells that reduces graft failure, accelerates neutrophil recovery and provides stable long-term engraftment (Wagner, J. E. et al Cell Stem Cell, 2016, 18, pp.144-155.).

In this study, mice transplanted with MGTA-456 showed 2.8-fold higher human CD45 engraftment in the peripheral blood at week 13 compared to mice transplanted with non-expanded fresh cord blood or vehicle treated CD34+ cells (FIGS. 9A and 9B). As shown in FIG. 10, we observed an approximately 10-fold increase in human CD45+CD11b+ myeloid cells in the brains of transplanted NSG mice with MGTA-456 (n=15, p<0.0001). To confirm microglia engraftment in the brain, we also assessed the presence of Ku80+lba1+ microglia in brain sections by morphological assessment and immunohistochemistry following transplantation, the results of which are shown, e.g., in FIG. 11.

These data demonstrate that *ex vivo* expanded human cord blood CD34+ cells, (MGTA-456) significantly improves engraftment of human microglia in the brain of NSG mice. These findings demonstrate that hematopoietic stem cells expanded ex vivo with an aryl hydrocarbon receptor antagonist, e.g., MGTA-456 expanded with Compound 2, are an effective method to accelerate recovery in patients with neurologic and inherited metabolic disorders.

### Example 7. Only CD90+ Cells Contribute to Microglial Engraftment.

It has been highly debated as to which cell types are responsible for short-term and long-term engraftment after transplant. The hematopoietic differentiation pathway (FIG. 15) consists of an HSC with self-renewal capabilities, which give rise to multipotent progenitor cells (MPP), lineage-committed progenitors, and mature cell types. These cell types can be distinguished by cell surface markers, including, for example, CD34, CD90, and CD45RA. As HSCs differentiate, they lose CD90 expression and gain expression of CD45RA.

In order to determine which of these cell types contribute to engraftment, stem and progenitor cells were transplanted, and their ability to engraft was followed. It has previously been shown that CD90+ cells contributed to either short-term or long-term engraftment in non-human primates (data not shown), and that this population contributed to robust multilineage hematopoiesis (Radtke et al., 2017, Science Translation Medicine, 9(131) eaan11445).

Similar experiments were run with human HSCs in NSG immunodeficient mice, and it was determined that only CD90+ cells contributed to engraftment. Human cord blood cells were sorted based on CD34 and CD90 expression using FACS, and unsorted, CD90+, CD90- and recombined sorted cells transplanted into NSG mice (FIG. 12). When the percent engraftment was assayed by flow cytometry using antibodies against hCD45 and mCD45 at weeks 4, 8 and 12 (FIG. 13B, FIG. 16) following transplantation, only those populations of transplanted cells that contained CD90+ cells showed elevated levels of engraftment (FIG. 13A, FIG. 16). The same was true for cells that were cultured prior to engraftment, and cells that were uncultured (FIG. 13A, FIG. 13C, FIG. 16). The numbers of engrafted microglia in the brain were also quantified (FIG. 14) in mice that were transplanted with unsorted, CD90+, CD90- and recombined CD90+ and CD90- cells. When the numbers of hCD45+ CD11b+ cells, or the numbers of hCD45+ CD11b+ lba1 + cells in the brains of engrafted mice were counted, only the unsorted, CD90+ or CD90+ CD90- recombined transplant cell populations gave rise to an elevated level of microglia engraftment (FIG. 14A). Similarly, in cells that were cultured for 10 days, only the unsorted, CD90+ or recombined cell populations to an elevated level of microglia engraftment (FIG. 14B).

Thus, the number of CD90+ cells in the transplanted cell population contribute to the number of engrafted cells following transplantation. Thus, monitoring CD90+ expressing cells after culture with expansion agents may provide an indication of an agent's ability to promote expansion of HSCs.

### Example 8. Expansion of HSCs with Small Molecules.

The expansion of HSCs with several types of agents is known, including HDAC inhibitors such as trichostatin A (TSA) (Araki et al. Exp. Hematology, (2006) 34(2): 140-149), valproic acid (VPA) (Chaurasia et al., J. Clinical Investigation (2014) 124(6): 2378-2395) and UM171, small molecule with an unknown mechanism (Fares et al. Science (2014) 6203: 1509-12). In all cases, culturing CD34+ cord blood cells, frequently in the presence of cytokines or using cytokines to prime the culture, led to an increase in CD90+ cells.

Boitano et. al. found that SR1 (compound 1) enhances the number of phenotypic HSCs (Boitano et al. Science (2010) 329(5997): 1345-1348) in cultured primary human CD34+ cells. SR1 is the only published expansion agent to work clinically to date. Historical controls have shown that the average time to neutrophil recovery is 25 days (Wagner et al. Cell Stem Ce112016, 18, 144-155). When patients are transplanted with SR1-expanded cord blood cells i the time to neutrophil recovery is decreased by 2 weeks (to 10.5 days), demonstrating that expansion with SR1 increases the number of stem cells.

Expansion of mobilized peripheral blood cells was performed for 7 days in the presence of SR1, UM171, or various HDAC inhibitors using a 10-point dose response to identify optimal compounds and doses for expansion of HSCs (FIG. 17). On day 7, about half of the cells cultured in DMSO vehicle differentiated into CD34- cells (FIG. 17, top left panel). In contrast, SR1 prevented the differentiation of CD34+ cells. UM171 and HDAC inhibitors also had a similar effect. When CD90+ expression in these cells was assayed using FACS, it was observed that while SR1 increased the number of CD90+ cells, and UM171 had a similar effect, the HDAC inhibitors trichostatin A (TSA) and valproic acid (VPA) showed the greatest increase in the number of CD90+ cells after 7 days of expansion.

### Example 9. Analysis of Cord Blood Expansion

The effect of SR1, UM171 and Entinostat, LMK235, Romidepsin, Scriptaid, TSA and VPA at optimal concentrations on CD90+ cell number following expansion was confirmed (FIG. 18). All compounds lead to an increased number of CD90+ expressing cells at their optimal doses (FIG. 18). The largest increases in CD90+ cell numbers were seen with Romidepsin, Scriptaid and TSA. However, the difference between SR1 and vehicle control was statistically significant (p<0.001).

### Example 10. SR1 Shows the Largest Increase in Engraftable HSCs.

In order to determine if the increase in CD90+ expression would lead to expansion of long-term HSCs, the percent engraftment of cells expanded using SR1, UM171, Entinostat, LMK235, Romidepsin, Scriptaid, TSA or VPA at varying concentrations was assessed in NSG mice. CD34+ cord blood cells, either fresh or cultured for 10 days with or without SR1, UM171, Entinostat, LMK235, Romidepsin, Scriptaid, TSA or VPA were transplanted into NSG mice. 12 weeks after transplantation, the frequency of human CD45+ (hCD45+) cells was assessed. Unexpectedly, given the low number of CD90+ cells seen in FIG. 17 when cells were cultured with SR1, CD34+ cells cultured with SR1 led to the largest increase in engraftable HSCs. It was therefore hypothesized that the CD90+ cells observed after expansion UM171, Entinostat, LMK235, Romidepsin, Scriptaid, TSA or VPA were not true HSCs. Compounds such as TSA, VPA and UM171 are known to upregulate CD90 in CD90-cells. Thus, one potential explanation for the disconnect between the CD90+ phenotype observed in culture by FACS and the % engraftment observed in mice *in vivo* is that culturing cells with agents other than SR1 such as UM171, Entinostat, LMK235, Romidepsin, Scriptaid, TSA or VPA does not cause CD90+ cells to proliferate in culture. Rather, culturing cells with these agents instead causes CD90- cells to turn on the expression of CD90 during the culturing and expansion process.

This hypothesis was confirmed by looking at the ability of CD90- cells to turn on the expression of CD90 in culture, and to form colonies, as outlined in FIG. 20. First, unsorted cells were sorted using CD34 and CD90 into two populations, a CD34+ CD90- population and a CD34+ CD90+ population. CD90- cells were cultured for 8 days with DMSO, SR1, UM171 or valproic acid (VPA). These cultured cells were the phenotyped for CD34 and CD90 expression using FACS. While DMSO and SR1 cultured cells remained uniformly CD90-, a sub-population of CD90- cells cultured with UM171 or VPA gained CD90 expression after being cultured (FIG. 21, top row, see boxed portions of the FACS plots). This confirmed that culturing with UM171 and VPA induced expression of CD90 in otherwise CD90- cells. The number of CD90- cells that had gained CD90+ expression correlated with the fold expansion seen with SR, UM171 or VPA (FIG. 21, bottom row). Second, cells cultured with various compounds were assessed for their ability to form colonies on methylcellulose. If the CD90+ cells resulting from culturing with UM171, VPA or other compounds were true HSCs, these cells should be able to proliferate and form a colony when plated on methylcellulose. When CD90- sorted cells were cultured with vehicle, SR1, UM171, Scriptaid, (100nM), Scriptaid (300 nM), TSA (30 nM) or TSA (100 nM), none of these cultured cells were able to form colonies better than the vehicle alone. This indicates that none of the CD90+ cells derived from culturing CD90- cells with UM171 or VPA had gained the colony forming potential associated with a true CD90+ HSC. In contrast, when CD90+ sorted cells were cultured with vehicle, SR1, UM171, Scriptaid, (100nM), Scriptaid (300 nM), TSA (30 nM) or TSA (100 nM), all cultured CD90+ cells were able to form colonies, at least at approximately the same level as vehicle alone, with the exception of TSA (100 nM). However, only SR1 cultured CD90+ cells performed significantly better than vehicle alone in their ability to form colonies. This indicates that only SR1 has the ability to induce expansion of CD90+ HSCs in culture (resulting in more colonies).

Several compounds (UM171, HDAC inhibitors) have been described as promoting the expansion of CD90+ cells in culture. The data presented here indicates that this may not strictly be the case. UM171 and HDAC inhibitors such as VPA, instead of, or in addition to, promoting the expansion of CD90+ HSCs, act to activate CD90 expression in cells that are not true HSCs (e.g. MPPs, see FIG. 23). This confounds any analysis of HSC expansion in vitro that uses CD90 as marker. Moreover, the data presented here indicate that CD90- cells in which CD90 has been upregulated do not behave like HSCs: in vitro, these are unable to form colonies at the same level as HSCs, and in vivo, they do not show the advantage in terms of engraftment expected of a CD09+ HSC. Of the compounds tested, only SR appears to robustly promote the expansion of CD90+ HSCs without this confounding effect. Given the importance of using CD90+ HSC transplantation in order to get transplanted cell types such as microglia, the HR antagonist-mediated expansion of HSCs has the potential for a significant impact on patient outcomes.

### Example 11. Cord Blood Cells Expanded in the Presence of an Aryl Hydrocarbon Receptor Antagonist Enhance Human Microglia Engraftment in the Brains of NSG Mice and Enables a Reduced Intensity Conditioning Regimen

Allogenic bone marrow transplant (BMT) can prevent or ameliorate neurological symptoms arising from certain inherited metabolic disorders (IMDs). Donor-derived microglial cells limit progression of neuorological disease following transplant. Cord blood (CB) is the preferred source for IMD patients lacking an HLA-matched, non-carrier related donor due to its wide availability and ability to tolerate less HLA match but is associated with delayed recovery and low engraftment due to small numbers of hematopoietic stem and/or progenitor cells (HSPCs). MGTA-456, produced by expanding a single cord blood unit with an aryl hydrocarbon receptor antagonist, contains 100-fold higher number of CD34+CD90+ cells, a cell population enriched for hematopoietic stem cells. As microglia are thought to be derived from HSPCs, the high number of HSPCs in MGTA-456 may lead to enhanced microglial engraftment.

Relative to naive CB CD34+ cells, MGTA-456 led to an 8-fold increase in hematopoietic engraftment and a 10-fold increase in microglial engraftment (p < 0.0001, n = 15) in sublethallyirradiated mice, with most donor cells localized to the non-perivascular region. As high dose busulfan results in enhanced microglial engraftment, the engraftment with MGTA-456 after conditioning with busulfan at a low (20 mg/kg) or high (40 mg/kg) dose was assessed. MGTA-456 led to a 60-fold increase in microglial engraftment relative to mice transplanted with unexpanded or vehicle-expanded CB CD34+ cells (p < 0.001, n = 8). Notably, transplant of MGTA-456 into mice conditioned with lowdose busulfan led to a 21-fold increase in microglial engraftment relative to high-dose busulfanconditioned animals transplanted with unmanipulated CB CD34+ cells (p < 0.01, n = 8). The brains were evaluated from 1 through 16 weeks post-transplant to evaluate speed of microglial engraftment. A 28-fold increase in microglial engraftment was observed as early as 2 weeks post-transplant with MGTA-456 (p < 0.0001, n = 8). The number of engrafting hematopoietic cells in peripheral blood correlated with the number of engrafting microglia in the brain (p < 0.0001). Further, as demonstrated, the subpopulations of MGTA-456 were evaluated to determine the source of microglial engraftment indicating that only CD34+CD90+ and not CD34+CD90- or CD34- cells, led to brain engraftment.

This data is summarized in FIG. 25A and FIG. 25B. MGTA-456 increased hematopoietic and microglial engraftment in NSG mice compared to uncultured controls. Hematopoietic and microglial engraftment with MGTA-456 in mice receiving one dose of busulfan was superior to mice receiving two doses of busulfan and uncultured cells. The study demonstrates that microglial engraftment is more robust in recipients of MGTA-456 due to the high number of NSG-engrafting CD34+CD90+ cells contained in MGTA-456 and this may enable use of a reduced-intensity conditioning regimen. Mechanistically, the number of microglia cells in brains correlate with peripheral blood engraftment and are derived from CD34+CD90+ cells. Collectively this data indicates the potential of MGTA-456 as a cell therapy for transplant in IMD patients. These data suggest that MGTA-456 enhances level of engraftment relative to the standard of care and potentially enables a reduced intensity conditioning regimen.

Importantly, as can be seen in FIG. 26A and FIG. 26B, MGTA-456 led to fast hematopoietic engraftment, as early as 1 week post transplant, and brain engraftment, as early as 2 weeks post transplant, relative to the standard of care. Collectively, this data demonstrates that MGTA-456 leads to quick and sustainable engraftment. Replacement of defective microglial with functional, or donor-derived microglia may be clinically-beneficial to halt disease progression in patients replacing defective microglia quickly and sustainably.

### Materials and Methods

### Cord Blood Expansion and Transplantation

Approximately 60,000 cord blood CD34+ cells were seeded in T25 flasks at a final volume of 12 mL in HSC growth media (SFEM supplemented with Pen/Strep, 50 ng/mL FLT3L, TPO, SCF, and IL-6). Flasks were incubated for 10 days at 37°C/5% CO₂. Cells were cultured in the presence of 500 nM of AHR antagonist, where indicated. Cells were transferred to a larger flask when needed to maintain cells at a density less than 1×10⁶ cells/mL throughout the culture period.

At the time of thaw, an equal number of cells to the starting cell cultures were injected into NSG mice, sublethally irradiated (200 cGy) 24 hours prior to injection. After 10 days of culture, the entire progeny of the cultures was injected into NSG mice. Peripheral blood was harvested by retroorbital bleeding at approximately weeks 4 and 8 or by cardiac puncture at week 12 or 16 or as otherwise indicated in FIG. 26 and chimerism was assessed by flow cytometry using antibodies against hCD45, mCD45, hCD33, hCD19, hCD3 and a viability dye.

### Brain Harvesting and Processing

At 3 months, brains were harvested. 1 hemisphere was fixed in formalin, embedded, and used for immunohistochemistry. The other hemisphere was crushed in Dounce buffer (15 mM HEPES/0.5% glucose in phenol red-free HBSS) and filtered through a 40 µM filter to create a single cell suspension and resuspended in 900 µL 0.5% BSA/PBS. Myelin was depleted from brain samples, per manufacturer's instructions, by incubating with 100 µL myelin removal beads (Miltenyi Biotec), incubating for 15 minutes at 4°C, washing with PBS, and resuspending in 1 mL MACS Buffer prior to deletion on an AutoMACs Pro.

### Flow Cytometric Detection of Microglia

Myelin-depleted samples were resuspended in 100 µL PBS and stained with antibodies against hCD45, mCD45, CD11b, CD19, CD3, and 7-AAD viability dye. Cells were washed once in PBS and resuspended in 300 µL final volume. The entire sample was acquired by flow cytometry (BD Celesta) to quantitate the number of microglia per brain hemisphere.

### Immunohistochemical Detection of Microglia

Embedded brains were sectioned at approximately 5 microns and stained with Ku80 (brown) and Iba-1 (red) primary antibodies). Mouse brains were analyzed from each transplanted mouse and five levels were analyzed each. Glass slides were scanned at 20X using an Aperio AT2 whole slide scanner. Image analysis was performed on the digital slide images using Visiopharm software.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the invention that come within known or customary practice within the art to which the invention pertains and may be applied to the essential features hereinbefore set forth, and follows in the scope of the claims.

Other embodiments are within the claims.

## Claims

1. A composition for use in treating an inherited metabolic disorder in a subject, said composition comprising an expanded population of hematopoietic stem cells, wherein the expanded hematopoietic stem cells result in microglia engraftment in the brain of the subject; and optionally, the inherited metabolic disorder has a neurological component.

2. The composition for use of claim 1, wherein the expanded population of hematopoietic stem cells are produced *ex vivo,* wherein producing the expanded population of hematopoietic stem cells comprises contacting a population of hematopoietic stem cells with an aryl hydrocarbon receptor antagonist in an amount sufficient to produce the expanded population of hematopoietic stem cells.

3. The composition for use of claim 1 or 2, wherein the population of hematopoietic stem cells comprises CD34+ cells; and optionally, the population of hematopoietic stem cells comprises a population enriched for CD90+ cells.

4. The composition for use of anyone of claims 1-3, wherein the cells are human cells; and optionally, the human cells are derived from umbilical cord blood cells.

5. The composition for use of any one of claims 1-4, wherein the number of expanded hematopoietic stem cells is 10³ cells/kg, 10⁴ cells/kg, 10⁵ cells/kg, 10⁶ cells/kg, 10⁷ cells/kg, 10⁸ cells/kg, or any number in between, inclusive of the end points.

6. The composition for use of any one of claims 1-5, wherein the inherited metabolic disorder is Hurler syndrome (Hurler's Disease), a mucopolysaccharide disorder (Maroteaux Lamy syndrome), a lysosomal storage disorder, a peroxisomal disorder (X-linked adrenoleukodystrophy), a glycogen storage disease, a mucopolysaccharidose disorder, Gaucher's Disease, a sphingolipidose disorder, Mucolipidosis II, or metachromatic leukodystrophy.

7. The composition for use of any one of claims 1-6, further comprising busulfan conditioning; and optionally, the busulfan conditioning comprises administering busulfan at an amount of less than about 40 mg/kg, less than about 35 mg/kg, less than about 30 mg/kg, less than about 25 mg/kg, less than about 20 mg/kg, less than about 15 mg/kg, less than about 10 mg/kg, less than about 5 mg/kg, less than about 4 mg/kg, less than about 3 mg/kg, less than about 2 mg/kg, less than about 1 mg/kg, less than about 0.5 mg/kg, or less than about 0.1 mg/kg prior to the administration of the expanded population of hematopoietic stem cells.

8. The composition for use of any one of claims 1-7, wherein prior to expansion, the hematopoietic stem or progenitor cells are mobilized and isolated from a donor; and optionally,
(i) the donor is a human, or
(ii) the hematopoietic stem or progenitor cells are mobilized by contacting the hematopoietic stem or progenitor cells with a mobilizing amount of a CXCR4 antagonist and/or a CXCR2 agonist.

9. The composition for use of claim 8, wherein the CXCR4 antagonist is plerixafor or a pharmaceutically acceptable salt thereof, or the CXCR2 agonist is Gro-β, Gro-β T, or a variant thereof.

10. The composition for use of any one of claims 1-9, wherein the expanded hematopoietic stem cells result in microglia engraftment in the brain of the subject in less than about 10 weeks after administering, less than about 8 weeks after administering, less than about 6 weeks after administering, less than about 4 weeks after administering, less than about 3 weeks after administering, less than about 2 weeks after administering, or less than about 1 week after administering; and optionally, the expanded hematopoietic stem cells result in microglia engraftment in the brain of the subject that is maintained for greater than 1 week after administering, greater than 2 weeks after administering, greater than 4 weeks after administering, greater than 8 weeks after administering, greater than 16 weeks after administering, greater than 32 weeks after administering, or greater than 40 weeks after administering.

11. The composition for use of any one of claims 2-10, wherein the aryl hydrocarbon receptor antagonist is SR-1 represented by formula (1), or Compound 2:

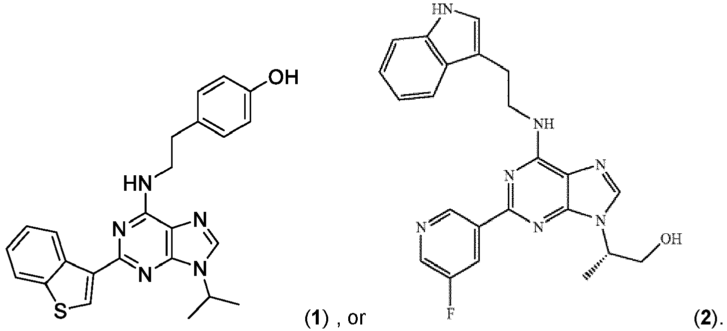

12. The composition for use of any one of claims 2-10, wherein the aryl hydrocarbon receptor antagonist is a compound represented by formula (IV) 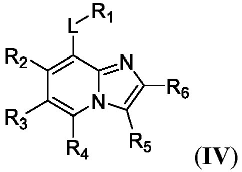
wherein L is selected from the group consisting of -NR₇ₐ(CR₈ₐR_{8b})ₙ-, -O(CR₈ₐR_{8b})ₙ-, - C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, -NR₇ₐC(O)(CR₈ₐR_{8b})ₙ-, -NR₇ₐC(S)(CR₈ₐR_{8b})ₙ-, -OC(O)(CR₈ₐR_{8b})ₙ-, -OC(S)(CR₈ₐR_{8b})ₙ-, -C(O)NR₇ₐ(CR₈ₐR_{8b})ₙ-, - C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-, - NR₇ₐS(O)₂(CR₈ₐR_{8b})ₙ-, -NR₇ₐC(O)NR_{7b}(CR₈ₐR_{8b})ₙ-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, - NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, - C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, - OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
R₂ is selected from the group consisting of hydrogen and optionally substituted C1-4 alkyl;
R₃ is selected from the group consisting of optionally substituted aryl, optionally substituted heteroaryl, optionally substituted cycloalkyl, and optionally substituted heterocycloalkyl;
R₄ is selected from the group consisting of hydrogen and optionally substituted C1-4 alkyl;
R₅ is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

13. The composition for use of any one of claims 2-10, wherein the aryl hydrocarbon receptor antagonist is compound (**3**), (**4**), (**5**), (**6**), (**7**), (**8**), (**9**), (**10**), (**11**), (**12**), (**13**), (**25**), (**27**), or (**28**) 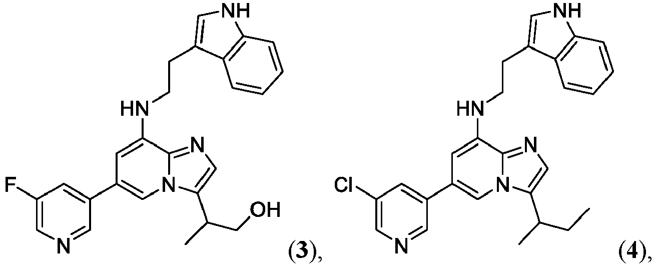 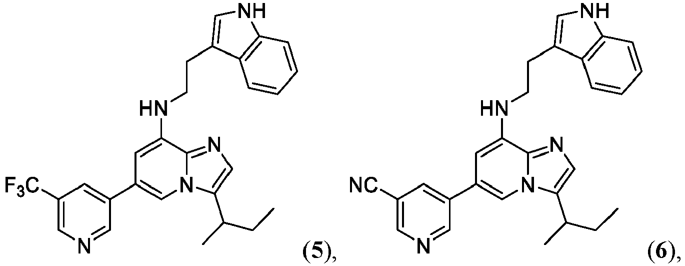 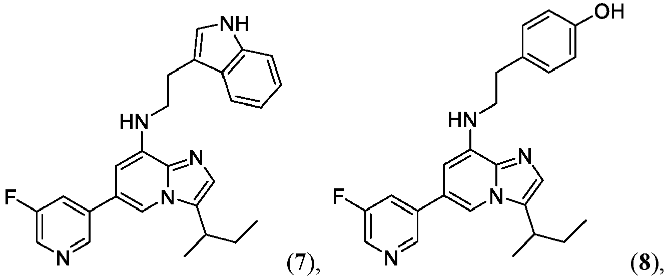 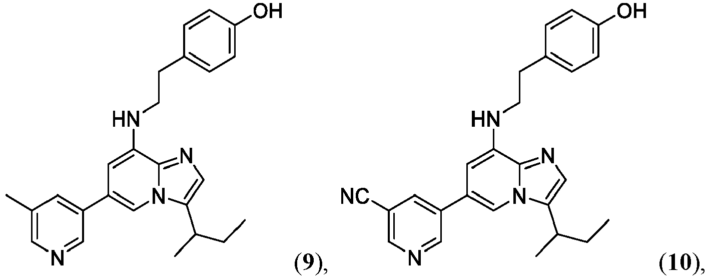 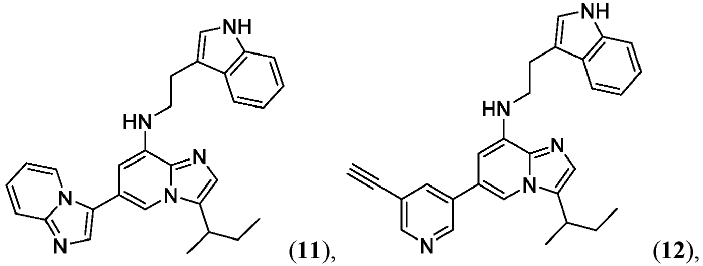 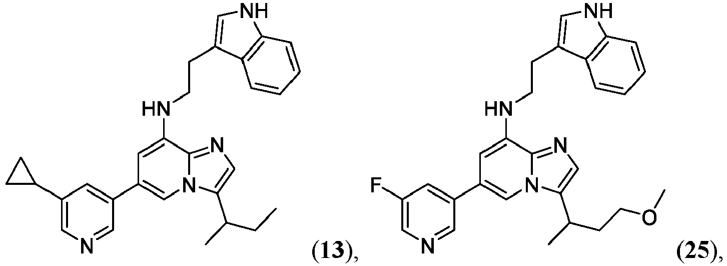 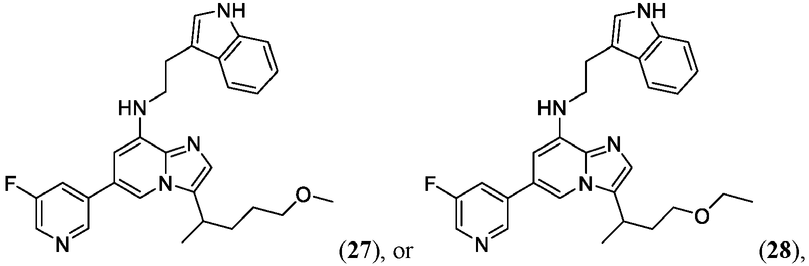 or a salt thereof.

14. The composition for use of any one of claims 2-10, wherein the aryl hydrocarbon receptor antagonist is a compound represented by formula (**V**) 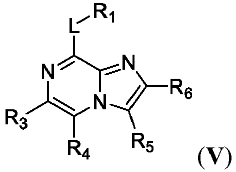
wherein L is selected from the group consisting of -NR₇ₐ(CR₈ₐR_{8b})ₙ-, -O(CR₈ₐR_{8b})ₙ-, - C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, -NR₇ₐC(O)(CR₈ₐR_{8b})ₙ-, -NR₇ₐC(S)(CR₈ₐR_{8b})ₙ-, -OC(O)(CR₈ₐR_{8b})ₙ-, -OC(S)(CR₈ₐR_{8b})ₙ-, -C(O)NR₇ₐ(CR₈ₐR_{8b})ₙ-, - C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-, - NR₇ₐS(O)₂(CR₈ₐR_{8b})ₙ-, -NR₇ₐC(O)NR_{7b}(CR₈ₐR_{8b})ₙ-, and -NR₇ₐC(O)O(CR₈ₐR_{8b})ₙ-, wherein R₇ₐ, R_{7b}, R₈ₐ, and R_{8b} are each independently selected from the group consisting of hydrogen and optionally substituted C1-4 alkyl, and each n is independently an integer from 2 to 6;
R₁ is selected from the group consisting of -S(O)₂NR₉ₐR_{9b}, -NR₉ₐC(O)R_{9b}, - NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, - C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, - OC(S)CR₉ₐR_{9b}R_{9c}, optionally substituted aryl, optionally substituted heteroaryl, optionally substituted cycloalkyl, and optionally substituted heterocycloalkyl, wherein R₉ₐ, R_{9b}, and R_{9c} are each independently selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
R₃ is selected from the group consisting of optionally substituted aryl, optionally substituted heteroaryl, optionally substituted cycloalkyl, and optionally substituted heterocycloalkyl;
R₄ is selected from the group consisting of hydrogen and optionally substituted C1-4 alkyl;
R₅ is selected from the group consisting of optionally substituted aryl, optionally substituted heteroaryl, optionally substituted alkyl, optionally substituted heteroalkyl, optionally substituted cycloalkyl, and optionally substituted heterocycloalkyl; and
R₆ is selected from the group consisting of hydrogen, optionally substituted aryl, optionally substituted heteroaryl, optionally substituted alkyl, optionally substituted heteroalkyl, optionally substituted cycloalkyl, and optionally substituted heterocycloalkyl;
or a salt thereof.

15. The composition for use of any one of claims 2-10, wherein the aryl hydrocarbon receptor antagonist is compound (**14**), (**15**), (**16**), (**17**), (**18**), (**19**), (**20**), (**21**), (**22**), (**23**), (**24**), (**26**), (**29**), or (**30**) 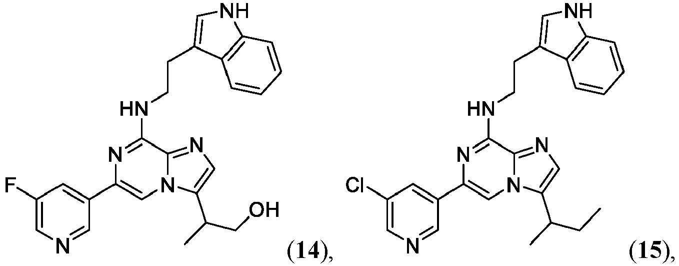 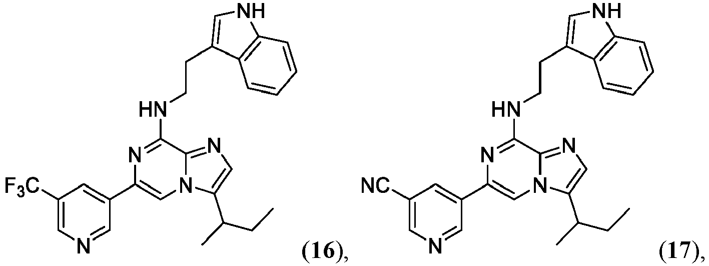 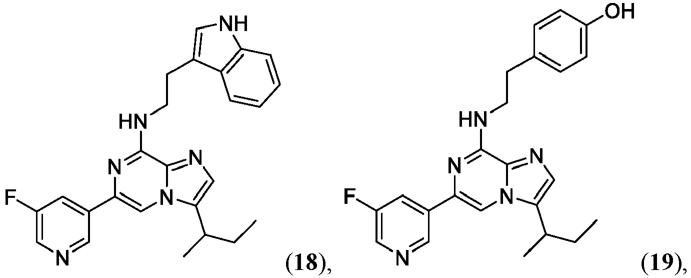 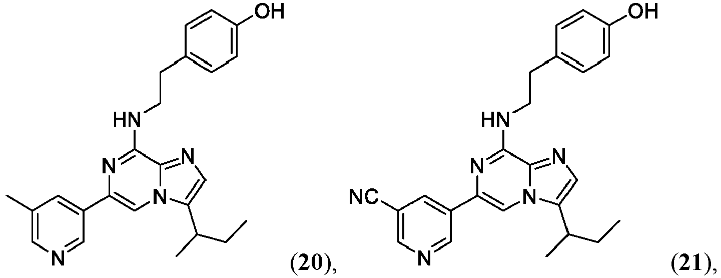 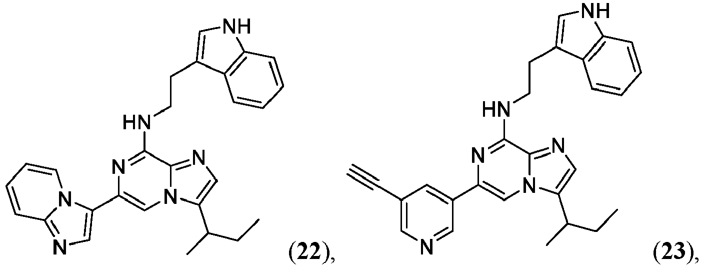 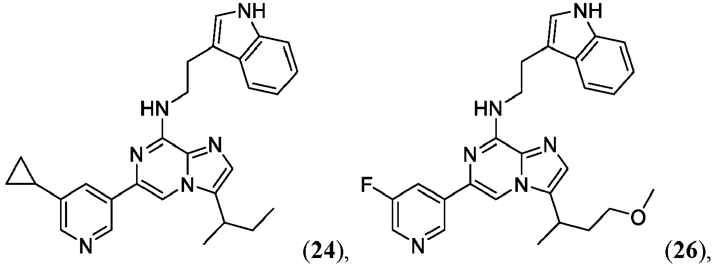 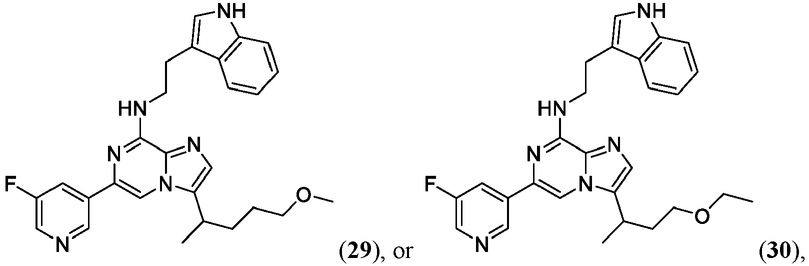 or a salt thereof.

## Patentansprüche

1. Zusammensetzung zur Verwendung beim Behandeln einer vererbten metabolischen Störung bei einem Subjekt, die Zusammensetzung umfassend eine vermehrte Population hämatopoetischer Stammzellen, wobei die vermehrten hämatopoetischen Stammzellen in einem Angehen von Mikroglia im Gehirn des Subjekts resultieren; und optional die vererbte metabolische Störung eine neurologische Komponente aufweist.

2. Zusammensetzung zur Verwendung nach Anspruch 1, wobei die vermehrte Population hämatopoetischer Stammzellen *ex vivo* produziert wird, wobei die Herstellung der vermehrten Population hämatopoetischer Stammzellen ein Inkontaktbringen einer Population hämatopoetischer Stammzellen mit einem Arylkohlenwasserstoffrezeptor-Antagonisten in einer Menge umfasst, die ausreichend ist, um die vermehrte Population hämatopoetischer Stammzellen zu produzieren.

3. Zusammensetzung zur Verwendung nach Anspruch 1 oder 2, wobei die Population hämatopoetischer Stammzellen CD34+-Zellen umfasst; und optional die Population hämatopoetischer Stammzellen eine mit CD90+-Zellen angereicherte Population umfasst.

4. Zusammensetzung zur Verwendung nach einem der Ansprüche 1-3, wobei die Zellen menschliche Zellen sind; und optional die menschlichen Zellen von Nabelschnurblutzellen abgeleitet sind.

5. Zusammensetzung zur Verwendung nach einem der Ansprüche 1-4, wobei die Anzahl vermehrter hämatopoetischer Stammzellen 10³ Zellen/kg, 10⁴ Zellen/kg, 10⁵ Zellen/kg, 10⁶ Zellen/kg, 10⁷ Zellen/kg, 10⁸ Zellen/kg oder eine beliebige Zahl dazwischen, einschließlich der Endpunkte, ist.

6. Zusammensetzung zur Verwendung nach einem der Ansprüche 1-5, wobei die vererbte metabolische Störung Hurler-Syndrom (Hurler-Krankheit), eine Mukopolysaccharid-Störung (Maroteaux-Lamy-Syndrom), eine lysosomale Speicherkrankheit, eine peroxisomale Störung (X-chromosomale Adrenoleukodystrophie), eine Glykogenspeicherkrankheit, eine Mukopolysaccharidose-Störung, Gaucher-Krankheit, eine Sphingolipidose-Störung, Mukolipidose II oder metachromatische Leukodystrophie ist.

7. Zusammensetzung zur Verwendung nach einem der Ansprüche 1-6, ferner umfassend Busulfan-Konditionierung; und optional wobei die Busulfan-Konditionierung ein Verabreichen von Busulfan in einer Menge von weniger als etwa 40 mg/kg, weniger als etwa 35 mg/kg, weniger als etwa 30 mg/kg, weniger als etwa 25 mg/kg, weniger als etwa 20 mg/kg, weniger als etwa 15 mg/kg, weniger als etwa 10 mg/kg, weniger als etwa 5 mg/kg, weniger als etwa 4 mg/kg, weniger als etwa 3 mg/kg, weniger als etwa 2 mg/kg, weniger als etwa 1 mg/kg, weniger als etwa 0,5 mg/kg oder weniger als etwa 0,1 mg/kg vor der Verabreichung der vermehrten Population hämatopoetischer Stammzellen umfasst.

8. Zusammensetzung zur Verwendung nach einem der Ansprüche 1-7, wobei die hämatopoetischen Stamm- oder Vorläuferzellen vor einer Expansion mobilisiert und von einem Donor isoliert werden; und optional
(i) der Donor ein Mensch ist, oder
(ii) die hämatopoetischen Stamm- oder Vorläuferzellen mobilisiert werden, indem die hämatopoetischen Stamm- oder Vorläuferzellen mit einer mobilisierenden Menge eines CXCR4-Antagonisten und/oder eines CXCR2-Agonisten in Kontakt gebracht werden.

9. Zusammensetzung zur Verwendung nach Anspruch 8, wobei der CXCR4-Antagonist Plerixafor oder ein pharmazeutisch annehmbares Salz davon ist, oder der CXCR2-Agonist Gro-β, Gro-β T oder eine Variante davon ist.

10. Zusammensetzung zur Verwendung nach einem der Ansprüche 1-9, wobei die vermehrten hämatopoetischen Stammzellen in einem Angehen von Mikroglia im Gehirn des Subjekts in weniger als etwa 10 Wochen nach Verabreichung, weniger als etwa 8 Wochen nach Verabreichung, weniger als etwa 6 Wochen nach Verabreichung, weniger als etwa 4 Wochen nach Verabreichung, weniger als etwa 3 Wochen nach Verabreichung, weniger als etwa 2 Wochen nach Verabreichung oder weniger als etwa 1 Woche nach Verabreichung resultieren; und optional die vermehrten hämatopoetischen Stammzellen in einem Angehen von Mikroglia im Gehirn des Subjekts resultiert, das mehr als 1 Woche nach Verabreichung, mehr als 2 Wochen nach Verabreichung, mehr als 4 Wochen nach Verabreichung, mehr als 8 Wochen nach Verabreichung, mehr als 16 Wochen nach Verabreichung, mehr als 32 Wochen nach Verabreichung oder mehr als 40 Wochen nach Verabreichung anhält.

11. Zusammensetzung zur Verwendung nach einem der Ansprüche 2-10, wobei der ArylKohlenwasserstoffrezeptor-Antagonist SR-1, dargestellt durch Formel (1), oder Verbindung 2 ist:

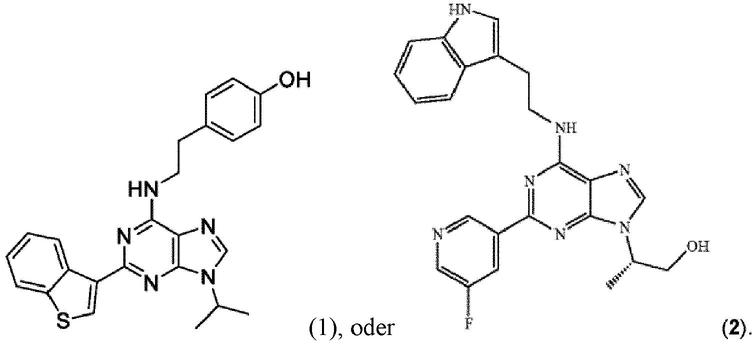

12. Zusammensetzung zur Verwendung nach einem der Ansprüche 2-10, wobei der ArylKohlenwasserstoffrezeptor-Antagonist eine Verbindung ist, die durch Formel (**IV**) dargestellt ist 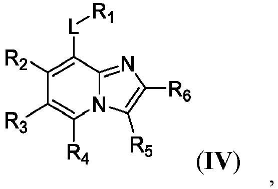
wobei L ausgewählt ist aus der Gruppe bestehend aus -NR₇ₐ(CR₈ₐR_{8b})ₙ-, -O(CR₈ₐR_{8b})ₙ-, - C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, -NR₇ₐC(O)(CR₈ₐR_{8b})ₙ-, - NR₇ₐC(S)(CR₈ₐR_{8b})ₙ-, -OC(O)(CR₈ₐR_{8b})ₙ-, -OC(S)(CR₈ₐR_{8b})ₙ-, -C(O)NR₇ₐ(CR₈ₐR_{8b})ₙ-, - C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-, - NR₇ₐS(O)₂(CR₈ₐR_{8b})ₙ-, -NR₇ₐC(O)NR_{7b}(CR₈ₐR_{8b})ₙ- und -NR₇ₐC(O)O(CR₈ₐR_{8b})ₙ-, wobei R₇ₐ, R_{7b}, R₈ₐ und R_{8b} jeweils unabhängig ausgewählt sind aus der Gruppe bestehend aus Wasserstoff und optional substituiertem C1-4-Alkyl und jedes n unabhängig eine ganze Zahl von 2 bis 6 ist;
R₁ ausgewählt ist aus der Gruppe bestehend aus -S(O)₂NR₉ₐR_{9b}, -NR₉ₐC(O)R_{9b}, -NR₉ₐC(S)R_{9b}.-NR₉ₐC(O)NR_{9b}R₉C, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b}, - C(S)NR₉ₐR_{9b}, -NR₉ₐS(O)₂R_{9b}, -NR₉ₐC(O)OR_{9b}, -OC(O)CR₉ₐR_{9b}R₉c, - OC(S)CR₉ₐR_{9b}R_{9c}, optional substituiertem Aryl, optional substituiertem Heteroaryl, optional substituiertem Cycloalkyl und optional substituiertem Heterocycloalkyl, wobei R₉ₐ, R_{9b} und R_{9c} jeweils unabhängig ausgewählt sind aus der Gruppe bestehend aus Wasserstoff, optional substituiertem Aryl, optional substituiertem Heteroaryl, optional substituiertem Alkyl, optional substituiertem Heteroalkyl, optional substituiertem Cycloalkyl und optional substituiertem Heterocycloalkyl;
R₂ ausgewählt ist aus der Gruppe bestehend aus Wasserstoff und optional substituiertem C1-4-Alkyl;
R₃ ausgewählt ist aus der Gruppe bestehend aus optional substituiertem Aryl, optional substituiertem Heteroaryl, optional substituiertem Cycloalkyl und optional substituiertem Heterocycloalkyl,
R₄ ausgewählt ist aus der Gruppe bestehend aus Wasserstoff und optional substituiertem C1-4-Alkyl;
R₅ ausgewählt ist aus der Gruppe bestehend aus optional substituiertem Aryl, optional substituiertem Heteroaryl, optional substituiertem Alkyl, optional substituiertem Heteroalkyl, optional substituiertem Cycloalkyl und optional substituiertem Heterocycloalkyl; und
R₆ ausgewählt ist aus der Gruppe bestehend aus Wasserstoff, optional substituiertem Aryl, optional substituiertem Heteroaryl, optional substituiertem Alkyl, optional substituiertem Heteroalkyl, optional substituiertem Cycloalkyl und optional substituiertem Heterocycloalkyl;
oder ein Salz davon ist.

13. Zusammensetzung zur Verwendung nach einem der Ansprüche 2-10, wobei der ArylKohlenwasserstoffrezeptor-Antagonist Verbindung (**3**), (**4**), (**5**), (**6**), (**7**), (**8**), (**9**), (**10**), (**11**), (**12**), (**13**), (**25**), (**27**) oder (**28**) 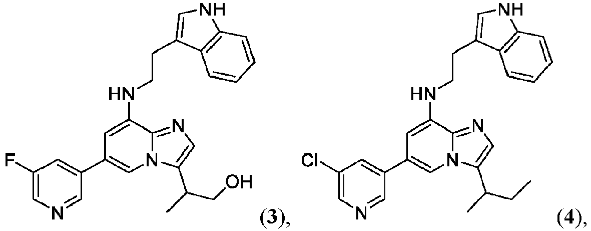 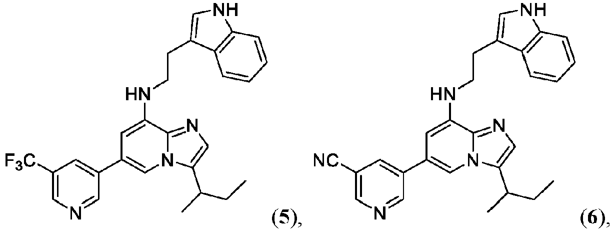 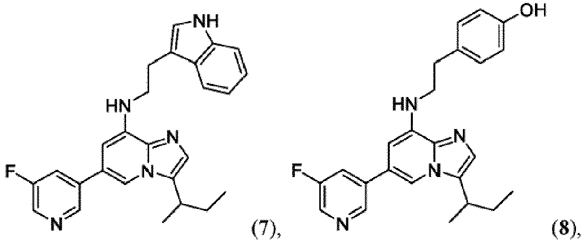 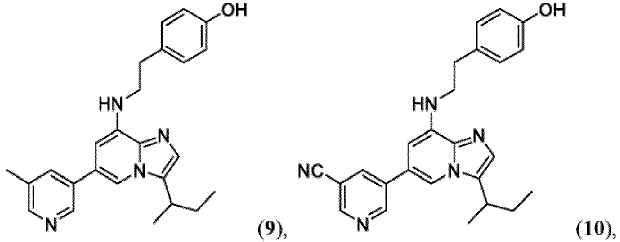 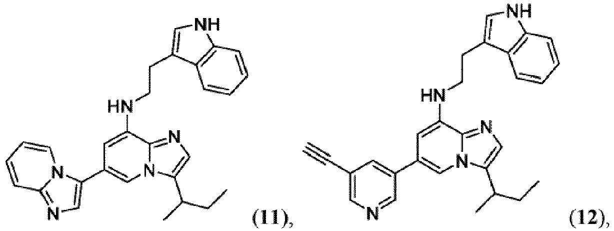 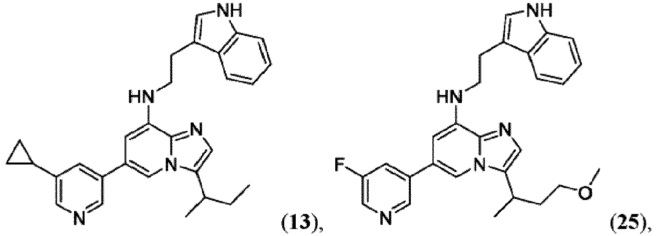 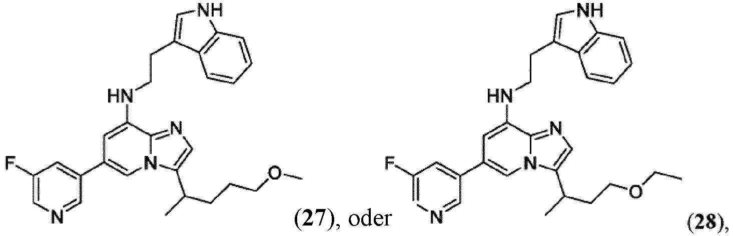 oder ein Salz davon ist.

14. Zusammensetzung zur Verwendung nach einem der Ansprüche 2-10, wobei der Arylkohlenwasserstoffrezeptor-Antagonist eine Verbindung ist, die durch Formel (**V**) dargestellt ist 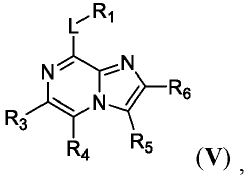
wobei L ausgewählt ist aus der Gruppe bestehend aus -NR₇ₐ(CR₈ₐR_{8b})ₙ-, -O(CR₈ₐR_{8b})ₙ-, - C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, -NR₇ₐC(O)(CR₈ₐR_{8b})ₙ-, - NR₇ₐC(S)(CR₈ₐR_{8b})ₙ-, -OC(O)(CR₈ₐR_{8b})ₙ-, -OC(S)(CR₈ₐR_{8b})ₙ-, -C(O)NR₇ₐ(CR₈ₐR_{8b})ₙ-, - C(S)NR₇ₐ(CR₈ₐR_{8b})ₙ-, -C(O)O(CR₈ₐR_{8b})ₙ-, -C(S)O(CR₈ₐR_{8b})ₙ-, -S(O)₂NR₇ₐ(CR₈ₐR_{8b})ₙ-, - NR₇ₐS(O)₂(CR₈ₐR_{8b})ₙ-, -NR₇ₐC(O)NR_{7b}(CR₈ₐR_{8b})ₙ- und -NR₇ₐC(O)O(CR₈ₐR_{8b})ₙ-, wobei R₇ₐ, R_{7b}, R₈ₐ und R_{8b} jeweils unabhängig ausgewählt sind aus der Gruppe bestehend aus Wasserstoff und optional substituiertem C1-4-Alkyl und jedes n unabhängig eine ganze Zahl von 2 bis 6 ist;
R₁ ausgewählt ist aus der Gruppe bestehend aus -S(O)₂NR₉ₐR_{9b}, -NR₉ₐC(O)R_{9b}, -NR₉ₐC(S)R_{9b}.-NR₉ₐC(O)NR_{9b}R₉c, -C(O)R₉ₐ, -C(S)R₉ₐ, -S(O)₀₋₂R₉ₐ, -C(O)OR₉ₐ, -C(S)OR₉ₐ, -C(O)NR₉ₐR_{9b}, - C(S)NR₉ₐR_{9b}, -NR₉ₐS(O)₂R_{9b}, -NR₉ₐC(O)OR_{9b}, -OC(O)CR₉ₐR_{9b}R₉c, - OC(S)CR₉ₐR_{9b}R_{9c}, optional substituiertem Aryl, optional substituiertem Heteroaryl, optional substituiertem Cycloalkyl und optional substituiertem Heterocycloalkyl, wobei R₉ₐ, R_{9b} und R_{9c} jeweils unabhängig ausgewählt sind aus der Gruppe bestehend aus Wasserstoff, optional substituiertem Aryl, optional substituiertem Heteroaryl, optional substituiertem Alkyl, optional substituiertem Heteroalkyl, optional substituiertem Cycloalkyl und optional substituiertem Heterocycloalkyl;
R₃ ausgewählt ist aus der Gruppe bestehend aus optional substituiertem Aryl, optional substituiertem Heteroaryl, optional substituiertem Cycloalkyl und optional substituiertem Heterocycloalkyl,
R₄ ausgewählt ist aus der Gruppe bestehend aus Wasserstoff und optional substituiertem C1-4-Alkyl;
R₅ ausgewählt ist aus der Gruppe bestehend aus optional substituiertem Aryl, optional substituiertem Heteroaryl, optional substituiertem Alkyl, optional substituiertem Heteroalkyl, optional substituiertem Cycloalkyl und optional substituiertem Heterocycloalkyl; und
R₆ ausgewählt ist aus der Gruppe bestehend aus Wasserstoff, optional substituiertem Aryl, optional substituiertem Heteroaryl, optional substituiertem Alkyl, optional substituiertem Heteroalkyl, optional substituiertem Cycloalkyl und optional substituiertem Heterocycloalkyl;
oder ein Salz davon ist.

15. Zusammensetzung zur Verwendung nach einem der Ansprüche 2-10, wobei der ArylKohlenwasserstoffrezeptor-Antagonist Verbindung (**14**), (**15**), (**16**), (**17**), (**18**), (**19**), (**20**), (**21**), (**22**), (**23**), (**24**), (**26**), (**29**) oder (**30**) 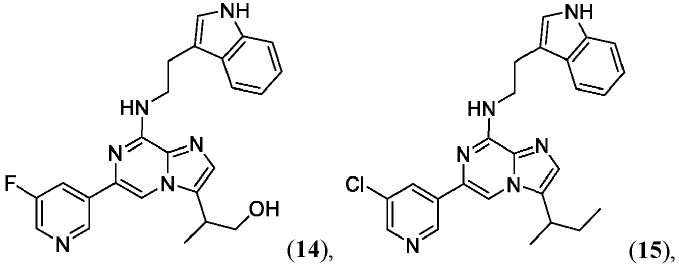 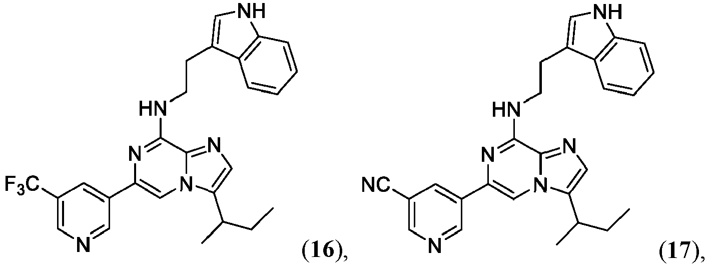 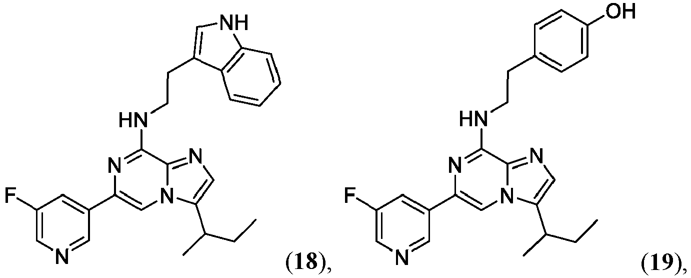 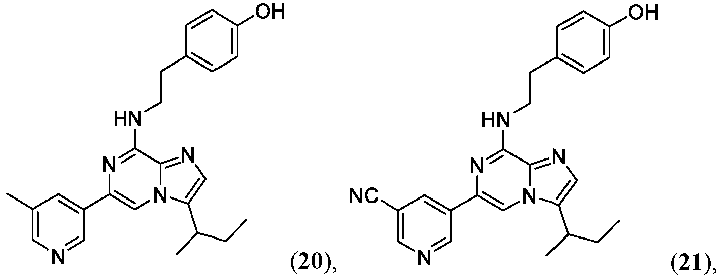 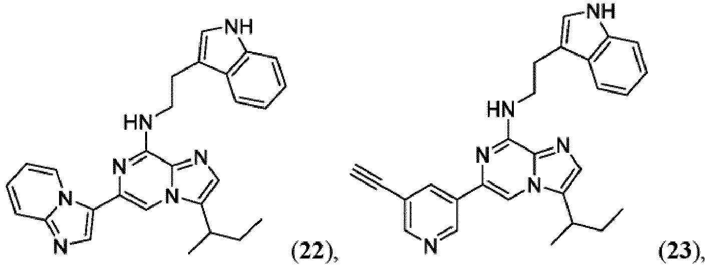 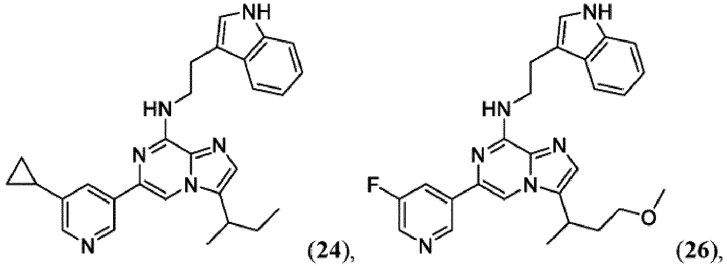 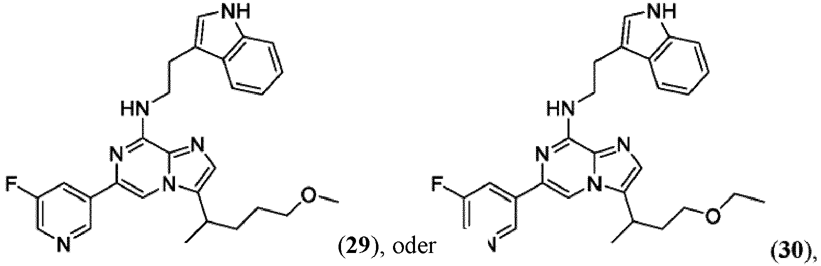 oder ein Salz davon ist.

## Revendications

1. Composition destinée à être utilisée dans le traitement d'un désordre métabolique héréditaire chez un sujet, ladite composition comprenant une population multipliée de cellules souches hématopoïétiques, lesdites cellules souches hématopoïétiques multipliées conduisant à un greffe de microglie dans le cerveau du sujet ; et éventuellement, ledit désordre métabolique héréditaire comportant une composante neurologique.

2. Composition destinée à être utilisée selon la revendication 1, ladite population multipliée de cellules souches hématopoïétiques étant produite *ex vivo,* ladite production de la population multipliée de cellules souches hématopoïétiques comprenant la mise en contact d'une population de cellules souches hématopoïétiques avec un antagoniste de récepteur d'hydrocarbure aryle en quantité suffisante pour produire la population multipliée de cellules souches hématopoïétiques.

3. Composition destinée à être utilisée selon l'une des revendications 1 ou 2, ladite population de cellules souches hématopoïétiques comprenant des cellules CD34+ ; et éventuellement, ladite population de cellules souches hématopoïétiques comprenant une population enrichie en cellules CD90+.

4. Composition destinée à être utilisée selon l'une quelconque des revendications 1 à 3, lesdites cellules étant des cellules humaines ; et éventuellement, lesdites cellules humaines provenant de cellules sanguines de cordon ombilical.

5. Composition destinée à être utilisée selon l'une quelconque des revendications 1 à 4, le nombre de cellules souches hématopoïétiques multipliées étant de 10³ cellules/kg, 10⁴ cellules/kg, 10⁵ cellules/kg, 10⁶ cellules/kg, 10⁷ cellules/kg, 10⁸ cellules/kg, ou tout nombre intermédiaire, limites incluses.

6. Composition destinée à être utilisée selon l'une quelconque des revendications 1 à 5, ledit désordre métabolique héréditaire étant le syndrome de Hurler (maladie de Hurler), un trouble des mucopolysaccharides (syndrome de Maroteaux Lamy), un trouble de surcharge lysocomale, un trouble péroxysomal (adrénoleucodystrophie liée à l'X), une maladie de surcharge en glycogène, un trouble de mucopolysaccharidose, la maladie de Gaucher, un trouble de sphingolipidose, la mucolipidose II ou une leucodystrophie métachromique.

7. Composition destinée à être utilisée selon l'une quelconque des revendications 1 à 6, comprenant en outre un conditionnement par busulfan ; et, éventuellement, le conditionnement par busulfan comprenant l'administration de busulfan à une quantité inférieure à environ 40 mg/kg, inférieure à environ 35 mg/kg, inférieure à environ 30 mg/kg, inférieure à environ 25 mg/kg, inférieure à environ 20 mg/kg, inférieure à environ 15 mg/kg, inférieure à environ 10 mg/kg, inférieure à environ 5 mg/kg, inférieure à environ 4 mg/kg, inférieure à environ 3 mg/kg, inférieure à environ 2 mg/kg, inférieure à environ 1 mg/kg, inférieure à environ 0,5 mg/kg, ou inférieure à environ 0,1 mg/kg avant l'administration de la population multipliée de cellules souches hématopoïétiques.

8. Composition destinée à être utilisée selon l'une quelconque des revendications 1 à 7, avant la multiplication, lesdites cellules souches ou progénitrices hématopoïétiques étant mobilisées et isolées d'un donneur ; et éventuellement,
(i) ledit donneur étant un humain, ou
(ii) lesdites cellules souches ou progénitrices hématopoïétiques étant mobilisées par la mise en contact des cellules souches ou progénitrices hématopoïétiques avec une quantité mobilisante d'un antagoniste de CXCR4 et/ou un antagoniste de CXCR2.

9. Composition destinée à être utilisée selon la revendication 8, ledit antagoniste de CXCR4 étant le plérixafor ou un sel pharmaceutiquement acceptable de celui-ci, ou ledit antagoniste de CXCR2 étant Gro-β, Gro-β T ou un variant de ceux-ci.

10. Composition destinée à être utilisée selon l'une quelconque des revendications 1 à 9, lesdites cellules souches hématopoïétiques multipliées conduisant à un greffe de microglie dans le cerveau du sujet en moins d'environ 10 semaines après l'administration, en moins d'environ 8 semaines après l'administration, en moins d'environ 6 semaines après l'administration, en moins d'environ 4 semaines après l'administration, en moins d'environ 3 semaines après l'administration, en moins d'environ 2 semaines après l'administration, ou en moins d'environ 1 semaine après l'administration ; et éventuellement, lesdites cellules souches hématopoïétiques multipliées conduisant à un greffe de microglie dans le cerveau du sujet qui est maintenue pendant plus de 1 semaine après l'administration, plus de 2 semaines après l'administration, plus de 4 semaines après l'administration, plus de 8 semaines après l'administration, plus de 16 semaines après l'administration, plus de 32 semaines après l'administration, ou plus de 40 semaines après l'administration.

11. Composition destinée à être utilisée selon l'une quelconque des revendications 2 à 10, ledit antagoniste de récepteur d'hydrocarbure aryle étant SR-1 représenté par la formule (1), ou le composé 2 :

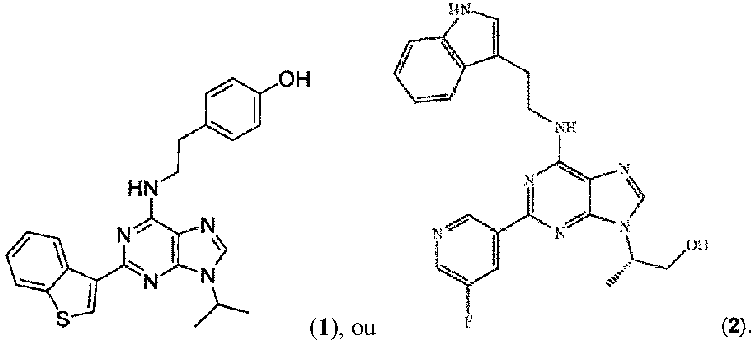

12. Composition destinée à être utilisée selon l'une quelconque des revendications 2 à 10, ledit antagoniste de récepteur d'hydrocarbure aryle étant un composé représenté par la formule (IV) 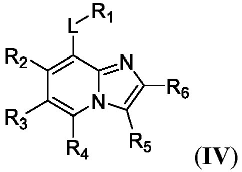
dans laquelle L est choisi dans le groupe constitué par les groupes -NR₇ₐ(CR₈ₐR_{8b})ₙ-, - O(CR₈ₐR_{8b})ₙ-, -C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, - NR₇ₐC(O)(CR₈ₐR_{8b})ₙ-, -NR₇ₐC(S)(CR₈ₐR_{8b})ₙ-, -OC(O)(CR₈ₐR_{8b})ₙ-, -OC(S)(CR₈ₐR_{8b})ₙ-, - C(O)NR₇ₐ(CR₈ₐR_{8b})ₙ-, -C(S)NR₇ₐ(CR₈ₐR_{8b})ₙ-, -C(O)O(CR₈ₐR_{8b})ₙ-, -C(S)O(CR₈ₐR_{8b})ₙ-, - S(O)₂NR₇ₐ(CR₈ₐR_{8b})ₙ-, -NR₇ₐS(O)₂(CR₈ₐR_{8b})ₙ-, -NR₇ₐC(O)NR_{7b}(CR₈ₐR_{8b})ₙ- et-NR₇ₐC(O)O(CR₈ₐR_{8b})ₙ-, dans lesquels R₇ₐ, R_{7b}, R₈ₐ et R_{8b} sont chacun indépendamment choisis dans le groupe constitué par un atome d'hydrogène et un groupe C1-4 alkyle éventuellement substitué, et chaque n représente indépendamment un entier de 2 à 6 ;
R₁ est choisi dans le groupe constitué par les groupes -S(O)₂NR₉ₐR_{9b}, -NR₉ₐC(O)R_{9b}, - NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, - C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, - OC(S)CR₉ₐR_{9b}R_{9c}, aryle éventuellement substitué, hétéroaryle éventuellement substitué, cycloalkyle éventuellement substitué, et hétérocycloalkyle éventuellement substitué, dans lesquels R₉ₐ, R_{9b} et R_{9c} sont chacun indépendamment choisis dans le groupe constitué par un atome d'hydrogène, les groupes aryle éventuellement substitué, hétéroaryle éventuellement substitué, alkyle éventuellement substitué, hétéroalkyle éventuellement substitué, cycloalkyle éventuellement substitué, et hétérocycloalkyle éventuellement substitué ;
R₂ est choisi dans le groupe constitué par un atome d'hydrogène et un groupe C1-4 alkyle éventuellement substitué ;
R₃ est choisi dans le groupe constitué par les groupes aryle éventuellement substitué, hétéroaryle éventuellement substitué, cycloalkyle éventuellement substitué, et hétérocycloalkyle éventuellement substitué ;
R₄ est choisi dans le groupe constitué par un atome d'hydrogène et un groupe C1-4 alkyle éventuellement substitué ;
R₅ est choisi dans le groupe constitué par les groupes aryle éventuellement substitué, hétéroaryle éventuellement substitué, alkyle éventuellement substitué, hétéroalkyle éventuellement substitué, cycloalkyle éventuellement substitué, et hétérocycloalkyle éventuellement substitué ; et
R₆ est choisi dans le groupe constitué par un atome d'hydrogène, les groupes aryle éventuellement substitué, hétéroaryle éventuellement substitué, alkyle éventuellement substitué, hétéroalkyle éventuellement substitué, cycloalkyle éventuellement substitué, et hétérocycloalkyle éventuellement substitué ;
ou un sel de celui-ci.

13. Composition destinée à être utilisée selon l'une quelconque des revendications 2 à 10, ledit antagoniste de récepteur d'hydrocarbure aryle étant le composé (**3**), (**4**), (**5**), (**6**), (**7**), (**8**), (**9**), (**10**), (**11**), (**12**), (**13**), (**25**), (**27**), ou (**28**) 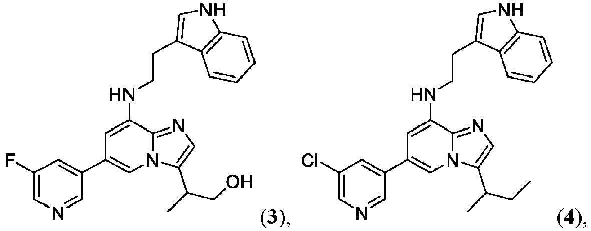 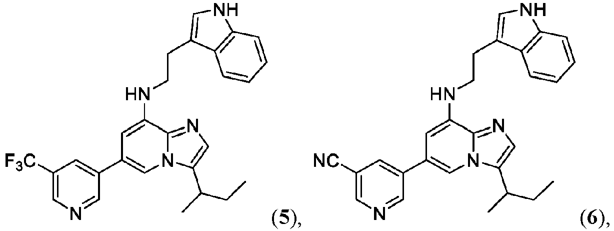 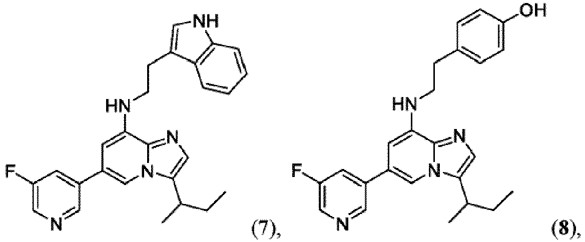 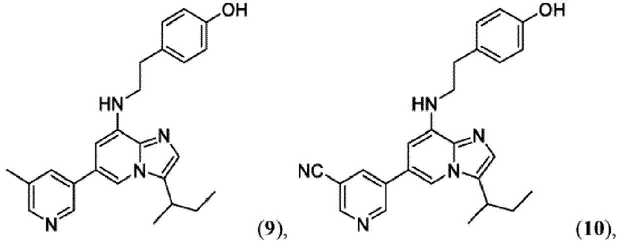 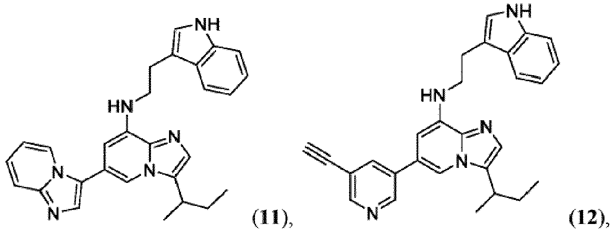 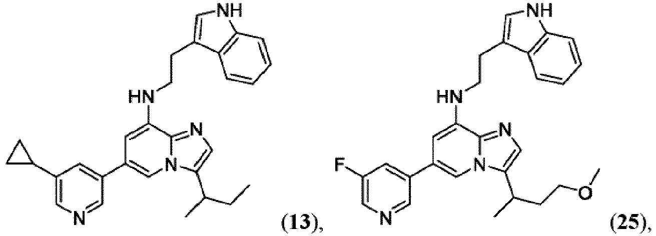 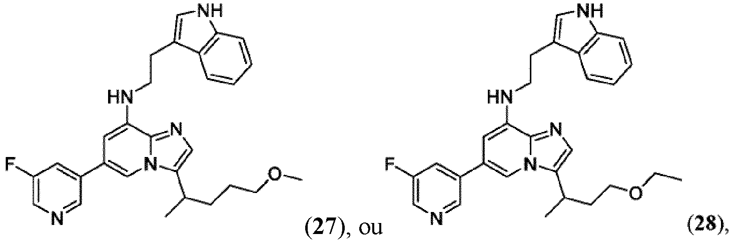 ou un sel pharmaceutiquement acceptable de celui-ci.

14. Composition destinée à être utilisée selon l'une quelconque des revendications 2 à 10, ledit antagoniste de récepteur d'hydrocarbure aryle étant un composé représenté par la formule (**V**) 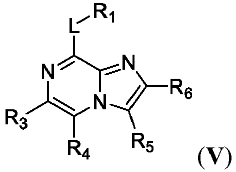
dans laquelle L est choisi dans le groupe constitué par les groupes -NR₇ₐ(CR₈ₐR_{8b})ₙ-, - O(CR₈ₐR_{8b})ₙ-, -C(O)(CR₈ₐR_{8b})ₙ-, -C(S)(CR₈ₐR_{8b})ₙ-, -S(O)₀₋₂(CR₈ₐR_{8b})ₙ-, -(CR₈ₐR_{8b})ₙ-, - NR₇ₐC(O)(CR₈ₐR_{8b})ₙ-, -NR₇ₐC(S)(CR₈ₐR_{8b})ₙ-, -OC(O)(CR₈ₐR_{8b})ₙ-, -OC(S)(CR₈ₐR_{8b})ₙ-, - C(O)NR₇ₐ(CR₈ₐR_{8b})ₙ-, -C(S)NR₇ₐ(CR₈ₐR_{8b})ₙ-, -C(O)O(CR₈ₐR_{8b})ₙ-, -C(S)O(CR₈ₐR_{8b})ₙ-, - S(O)₂NR₇ₐ(CR₈ₐR_{8b})ₙ-, -NR₇ₐS(O)₂(CR₈ₐR_{8b})ₙ-, -NR₇ₐC(O)NR_{7b}(CR₈ₐR_{8b})ₙ- et-NR₇ₐC(O)O(CR₈ₐR_{8b})ₙ-, dans lesquels R₇ₐ, R_{7b}, R₈ₐ, et R_{8b} sont chacun indépendamment choisis dans le groupe constitué par un atome d'hydrogène et un groupe C1-4 alkyle éventuellement substitué, et chaque n représente indépendamment un entier de 2 à 6 ;
R₁ est choisi dans le groupe constitué par les groupes -S(O)₂NR₉ₐR_{9b}, -NR₉ₐC(O)R_{9b}, - NR₉ₐC(S)R_{9b}, -NR₉ₐC(O)NR_{9b}R_{9c}, -C(O)R₉ₐ, -C(S)R₉ₐ, -S(O)₀₋₂R₉ₐ, -C(O)OR₉ₐ, -C(S)OR₉ₐ, - C(O)NR₉ₐR_{9b}, -C(S)NR₉ₐR_{9b}, -NR₉ₐS(O)₂R_{9b}, -NR₉ₐC(O)OR_{9b}, -OC(O)CR₉ₐR_{9b}R_{9c}, - OC(S)CR₉ₐR_{9b}R_{9c}, aryle éventuellement substitué, hétéroaryle éventuellement substitué, cycloalkyle éventuellement substitué, et hétérocycloalkyle éventuellement substitué, dans lesquels R₉ₐ, R_{9b} et R_{9c} sont chacun indépendamment choisis dans le groupe constitué par un atome d'hydrogène, les groupes aryle éventuellement substitué, hétéroaryle éventuellement substitué, alkyle éventuellement substitué, hétéroalkyle éventuellement substitué, cycloalkyle éventuellement substitué, et hétérocycloalkyle éventuellement substitué ;
R₃ est choisi dans le groupe constitué par les groupes aryle éventuellement substitué, hétéroaryle éventuellement substitué, cycloalkyle éventuellement substitué, et hétérocycloalkyle éventuellement substitué ;
R₄ est choisi dans le groupe constitué par un atome d'hydrogène et un groupe C1-4 alkyle éventuellement substitué ;
R₅ est choisi dans le groupe constitué par les groupes aryle éventuellement substitué, hétéroaryle éventuellement substitué, alkyle éventuellement substitué, hétéroalkyle éventuellement substitué, cycloalkyle éventuellement substitué, et hétérocycloalkyle éventuellement substitué ; et
R₆ est choisi dans le groupe constitué par un atome d'hydrogène, les groupes aryle éventuellement substitué, hétéroaryle éventuellement substitué, alkyle éventuellement substitué, hétéroalkyle éventuellement substitué, cycloalkyle éventuellement substitué, et hétérocycloalkyle éventuellement substitué ;
ou un sel de celui-ci.

15. Composition destinée à être utilisée selon l'une quelconque des revendications 2 à 10, ledit antagoniste de récepteur d'hydrocarbure aryle étant le composé (**14**), (**15**), (**16**), (**17**), (**18**), (**19**), (**20**), (**21**), (**22**), (**23**), (**24**), (**26**), (**29**) ou (**30**) 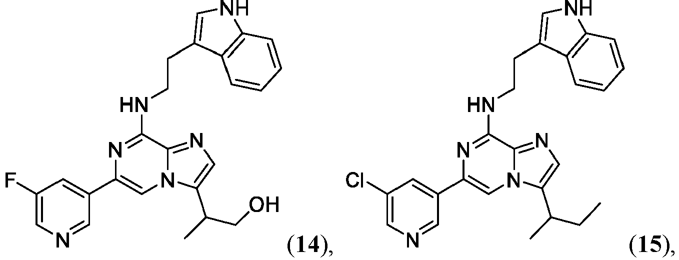 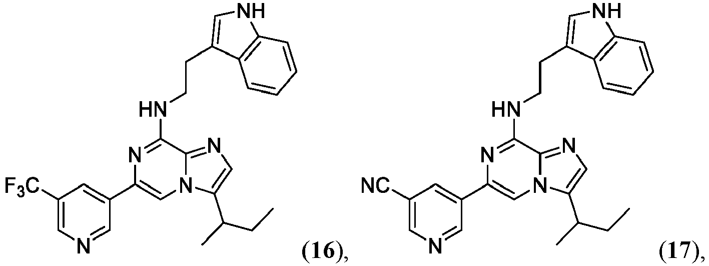 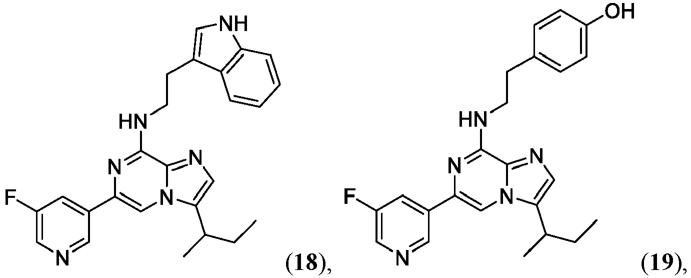 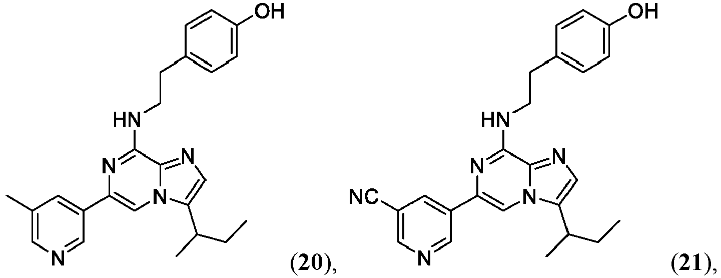 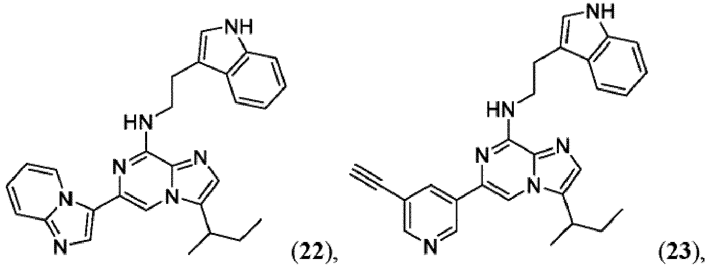 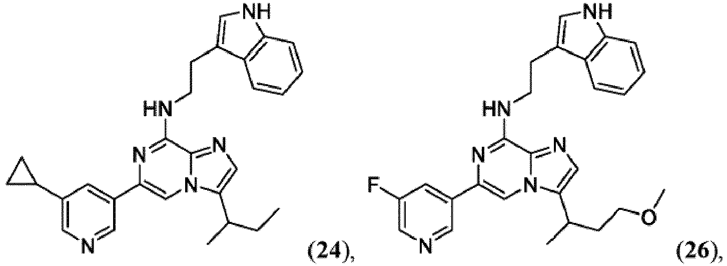 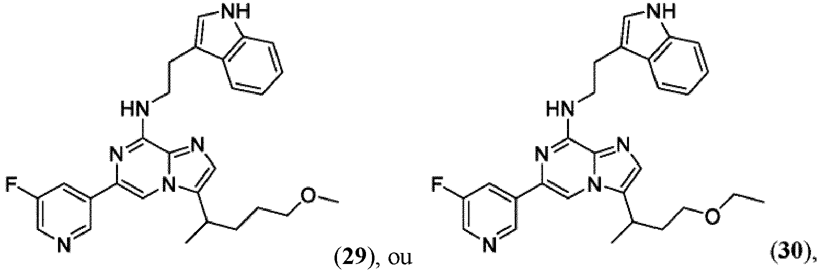 ou un sel de celui-ci.
